# EUROPEAN PATENT APPLICATION

(11) **EP 4 623 930 A2**
(43) Date of publication of application: **01.10.2025**
(21) Application number: 25184078.1
(22) Date of filing: 02.12.2021
(51) Int. Cl.: A61K 39/00

(54) **METHODS FOR IDENTIFYING LILRB-BLOCKING ANTIBODIES**

(30) Priority: 03.12.2020 US 202063121057 P; 30.08.2021 US 202163238717 P; 03.09.2021 US 202163240684 P
(62) Divisional of application: 21901469.3
(71) Applicant: THE BOARD OF REGENTS OF THE UNIVERSITY OF TEXAS SYSTEM, Austin, TX 78701 (US)
(72) Inventor: ZHANG, Chengcheng, Southlake 76092 (US); WU, Guojin, Dallas 75390 (US); KIM, Jaehyu, Dallas 75390 (US); CHEN, Heyu, Dallas 75390 (US); DENG, Mi, Dallas 75390 (US); AN, Zhiqiang, Houston 77021 (US); ZHANG, Ningyan, Houston 77021 (US); HUANG, Ryan, Dallas 75390 (US)
(74) Representative: Potter Clarkson

(57) **Abstract**

Provided herein are methods and compositions for the identification of modulators of LILRB3 activation. Also provided herein are methods of treating cancer comprising the administration of an inhibitor LILRB3 activation. Also provided are methods of treating autoimmune disease or inhibiting the onset of transplant rejection or treating an inflammatory disorder comprising administering an agonist of LILRB3 activation to a subject.

## Description

### PRIORITY CLAIM

This application claims benefit of priority to U.S Provisional Application Serial Nos. 63/121,057 (filed December 3, 2020), 63/238,717 (filed August 30, 2021) and 63/240,684 (filed September 3, 3021), the entire contents of each application being hereby incorporated by reference.

### BACKGROUND

### 1. Field

The present disclosure relates generally to the field of molecular biology. More particularly, it concerns methods and compositions for identifying LILRB3 antibodies and the use of such antibodies in cancer therapy and diagnosis.

### 2. Description of Related Art

Immune checkpoint blockade therapies effectively treat some types of cancers. However, for most cancer patients immune evasion and resistance lead to a failure to respond to these therapies or relapse after treatment (Robert *et al.,* 2015a; 2015b; Postow *et al.,* 2015). For leukemia patients, low mutational burdens and low levels of IFN-γ result in a weaker response to immune checkpoint blockade (Curran *et al.,* 2017). In particular, CTLA4 and PD-1/PD-L1 targeting monotherapies have been ineffective for treating patients with acute myeloid leukemia (AML) (Curran *et al.,* 2017). Several new immunotherapeutics have recently been approved. These include liposome-encapsulated chemodrugs, anti-CD33-drug conjugates, and inhibitors of BCL-2, IDH1, IDH2, Flt3, and hedgehog. Some of these therapeutics have significant toxicities. Further, these therapeutics are efficacious only in subpopulations of AML patients and often result in relapse (Click *et al.,* 2018). It is critical that the molecular mechanisms of AML development and immunosuppression are identified in order to guide development of more effective treatments.

The LILRBs with intracellular immunoreceptor tyrosine-based inhibitory motifs (ITIMs) can recruit tyrosine phosphatases SHP1, SHP2, and/or the inositol-phosphatase SHIP (Trowsdale *et al.,* 2015; Daeron *et al.,* 2008; Takai *et al.,* 2011; Katz, 2016; Kang *et al.,* 2016; Hirayasu & Arase, 2015; Deng *et al.,* 2021; van der Touw *et al.,* 2017). Because of the negative roles of phosphatases in immune activation, LILRBs are considered to be immune checkpoint factors (Carosella *et al.,* 2015). Numerous groups have contributed to the current understanding of the functions of LILRBs (Trowsdale *et al.,* 2015; Daeron *et al.,* 2008; Takai *et al.,* 2011; Katz, 2016; Kang *et al.,* 2016; Hirayasu & Arase, 2015; Deng *et al.,* 2021; van der Touw *et al.,* 2017). The inventors have studied how signaling mediated by LILRBs influences cancer development. The showed that LILRB2 is a receptor for the hormone Angptl2 and that several LILRBs and a related ITIM-receptor LAIR1 support AML development (John *et al.,* 2018; Zheng *et al.,* 2012; Kang *et al.,* 2015; Deng *et al.,* 2018; Gui *et al.,* 2019; Anami *et al.,* 2020; Li *et al.,* 2019; Churchill *et al.,* 2020; Bergstrom *et al.,* 2021). Recently, the inventors and others have demonstrated that blocking signaling mediated by LILRB1, LILRB2, or LILRB4 in human myeloid or natural killer cells promotes their pro-inflammatory activity and enhances anti-tumor responses (Deng *et al.,* 2018; Gui *et al.,* 2019; Li *et al.,* 2019; Barkal *et al.,* 2018; Chen *et al.,* 2018).

LILRB3 is a member of LILRB family that is restrictively expressed on myeloid cells, including monocytes, neutrophils, eosinophils, and basophils (as well as on *in vitro* differentiated mast cells and osteoclasts) (Deng *et al.,* 2021; Tedia *et al.,* 2003). LILRB3 contains four cytoplasmic ITIM motifs that may contribute to negative regulation of immune response (Coxon *et al.,* 2017). Ligation of LILRB3 in human myeloid cells led to inhibition of immune activation (Sloane *et al.,* 2004; Yeboah *et al.,* 2020). LILRB3 may be an inhibitor of allergic inflammation and autoimmunity (Renauer *et al.,* 2015). However, the ligand for LILRB3 has not been identified (Jones *et al.,* 2016), and the downstream signaling of LILRB3 is unclear. It is noteworthy that LILRBs, including LILRB3, are primate specific. The expression pattern and ligand of PirB, the mouse relative of LILRB3, differ from those of LILRB3 (Kang *et al.,* 2016). PirB is more broadly expressed than LILRB3 (Kang *et al.,* 2016). LILRB3 is also expressed on some myeloid leukemia, B lymphoid leukemia, and myeloma cells (Deng *et al.,* 2021; Pfistershammer *et al.,* 2009). It is reportedly co-expressed with stem cell marker CD34 and with myeloma marker CD138 (Pfistershammer *et al.,* 2009).

### SUMMARY

Embodiments of the present disclosure provide a method of identifying a modulator of LILRB3 activation comprising (a) contacting a reporter cell with galectin-4 and a candidate substance, wherein said reporter cell expresses a chimeric receptor having an external domain of LILRB3; and (b) detecting a level of receptor activation in the reporter cell, wherein a change in the level of receptor activation as compared to a reference level indicates that the candidate substance is a modulator of LILRB3 activation. The cell may be a mouse T-cell hybridoma cell. The receptor may comprise an intracellular domain of paired immunoglobulin-like receptor β (PILRβ). The receptor may be expressed in the cell through a viral expression vector, such as a retroviral expression vector.

The reporter cell may express a reporter gene that encodes a detectable label and is operably linked to a promoter regulated by activation of the receptor. The promoter may be a nuclear factor of activated T cells (NFAT) promoter, an inducible promoter, a tissue specific promoter or a constitutive promoter. The detectable label may be a colorometric label, fluorescent label, bioluminescent label, or chemiluminescent label, and in particular may be GFP, YFP, RFP, or D-luciferin. Detecting may comprise flow cytometry analysis or quantification of luminescence.

The candidate substance may be an antibody, such as a monoclonal antibody, a chimeric antibody, a CDR-grafted antibody, a humanized antibody, a Fab, a Fab', a F(ab')2, a Fv, or a scFv. The reference level may be obtained in the reporter cell when it is contacted with galectin-4. An increase in the level of receptor activation as compared to the reference level indicates that the modulator is an agonist. A decrease in the level of receptor activation as compared to the reference level indicates that the modulator is an antagonist. The candidate substance may be linked to a substrate, or the candidate substance may belinked to a cell expressing FcR.

Also provided is composition comprising a candidate LILRB3 modulator; galectin-4; and a reporter cell that expresses a chimeric receptor having an extracellular domain of LILRB3, wherein the reporter cell has a phenotype indicating receptor activation.

In another embodiment, there is provided an isolated monoclonal antibody or an antigen-binding fragment thereof comprising a heavy chain (HC) variable region (VH) and a light chain (LC) variable region (VL) comprising clone-paired CDR sequences as set forth in Tables 3 and 4; and variants thereof wherein one or more of the HC-CDRs and/or LC-CDRs has one, two, or three amino acid substitutions, additions, deletions, or combinations thereof. The isolated monoclonal antibody or an antigen binding fragment may be a murine, a rodent, a rabbit, a chimeric, humanized, or human antibody. The isolated antigen-binding fragment may be a recombinant ScFv (single chain fragment variable) antibody, Fab fragment, F(ab')2 fragment, or Fv fragment. The isolated monoclonal antibody may be a human antibody.

The isolated monoclonal antibody or an antigen-binding fragment thereof may have VH and VL chains having amino acid sequences at least 90% or 95% identical to clone-paired sequences of Appendices II and IV, respectively. The isolated monoclonal antibody or an antigen-binding fragment thereof may have VH and VL chains encoded by nucleic acid sequences at least 80% or 90% identical to clone-paired sequences of Appendices I and III, respectively. The isolated monoclonal antibody or an antigen-binding fragment thereof may have VH and VL chains having amino acid sequences identical to clone-paired sequences of Appendices II and IV, respectively. The isolated monoclonal antibody or an antigen binding fragment thereof may have VH and VL chains encoded by nucleic acid sequences identical to clone-paired sequences of Appendices I and III, respectively.

The isolated monoclonal antibody may be a humanized antibody. The isolated monoclonal antibody may be a chimeric antibody. The isolated monoclonal antibody or an antigen binding fragment thereof may induce the activation of LILRB3. The isolated monoclonal antibody or an antigen binding fragment thereof may suppress the activation of LILRB3.

In still other embodiments, there are provided (a) an isolated monoclonal antibody or an antigen binding fragment thereof, which competes for the same epitope with an isolated monoclonal antibody or an antigen-binding fragment defined herein; (b) a pharmaceutical composition comprising an isolated monoclonal antibody or an antigen-binding fragment thereof as defined herein; (c) an isolated nucleic acid that encodes an isolated monoclonal antibody as defined herein, or a vector comprising such isolated nucleic acid, or a host cell comprising such a vector; or (d) a hybridoma or engineered cell encoding and/or producing an isolated monoclonal antibody or an antigen-binding fragment thereof as defined herein. The host cell may be a mammalian cell, such as a CHO cell. Also provided a process for producing an antibody, comprising culturing such host cell under conditions suitable for expressing the antibody, and recovering the antibody.

In a further embodiment, there is provided a chimeric antigen receptor (CAR) protein comprising sequences of an antibody or antigen-binding fragment thereof as defined herein, as well as an isolated nucleic acid that encoding said CAR, a vector comprising and/or an engineered cell comprising this isolated nucleic acid, such as a T cell, NK cell, or macrophage.

In still a further embodiment, there is provided a method of treating or ameliorating the effect of a cancer in a subject, the method comprising administering to the subject a therapeutically effective amount of an antibody or an antigen-binding fragment thereof as defined herein or an engineered cell as defined herein.

The method may reduce or eradicate the tumor burden in the subject. The method may reduce the number of tumor cells, reduce the tumor size, reduce or prevent tumor metastasis, or eradicate the tumor in the subject. The cancer may be a solid cancer, such as adrenal cancer, bile duct carcinoma, bone cancer, brain cancer, breast cancer, cervical cancer, choriocarcinoma, colon cancer, colorectal cancer, esophageal cancer, eye cancer, gastric cancer, glioblastoma, head and neck cancer, kidney cancer, liver cancer, lung cancer, mesothelioma, melanoma, merkel cell cancer, nasopharyngeal carcinoma, neuroblastoma, oral cancer, ovarian cancer, pancreatic cancer, penile cancer, pinealoma, prostate cancer, renal cell cancer, retinoblastoma, sarcoma, skin cancer, testicular cancer, thymic carcinoma, thyroid cancer, uterine cancer, and vaginal cancer. The method may target monocytes, macrophages, dendritic cells, neutrophils and other myeloid cells, myeloid-derived suppressor cells, tumor-associated macrophages, and other immunosuppressive myeloid cells.

The cancer may be a hematologic malignancy, such as acute lymphocytic leukemia (ALL), acute myeloid leukemia (AML), B-cell leukemia, chronic lymphoblastic leukemia (CLL), blastic plasmacytoid dendritic cell neoplasm (BPDCN), chronic myelomonocytic leukemia (CMML), chronic myelocytic leukemia (CML), pre-B acute lymphocytic leukemia (Pre-B ALL), diffuse large B-cell lymphoma (DLBCL), extranodal NK/T-cell lymphoma, hairy cell leukemia, heavy chain disease, HHV8-associated primary effusion lymphoma, plasmablastic lymphoma, primary CNS lymphoma, primary mediastinal large B-cell lymphoma, T-cell/histiocyte-rich B-cell lymphoma, Hodgkin's lymphoma, non-Hodgkin's lymphoma, Waldenstrom's macroglobulinemia, multiple myeloma (MM), myelodysplastic syndromes (MDS), myeloproliferative neoplasms, and polycythemia vera.

The antibody or an antigen-binding fragment thereof may be administered intravenously, intra-arterially, intra-tumorally, or subcutaneously. The method may further comprise administering to the subject one or more drugs selected from the group consisting of a topoisomerase inhibitor, an anthracycline topoisomerase inhibitor, an anthracycline, a daunorubicin, a nucleoside metabolic inhibitor, a cytarabine, a hypomethylating agent, a low dose cytarabine (LDAC), a combination of daunorubicin and cytarabine, a daunorubicin and cytarabine liposome for injection, Vyxeos^{®}, an azacytidine, Vidaza^{®}, a decitabine, an all-trans-retinoic acid (ATRA), an arsenic, an arsenic trioxide, a histamine dihydrochloride, Ceplene^{®}, an interleukin-2, an aldesleukin, Proleukin^{®}, a gemtuzumab ozogamicin, Mylotarg^{®}, an FLT-3 inhibitor, a midostaurin, Rydapt^{®}, a clofarabine, a farnesyl transferase inhibitor, a decitabine, an IDH1 inhibitor, an ivosidenib, Tibsovo^{®}, an IDH2 inhibitor, an enasidenib, Idhifa^{®}, a smoothened (SMO) inhibitor, a glasdegib, an arginase inhibitor, an IDO inhibitor, an epacadostat, a BCL-2 inhibitor, a venetoclax, Venclexta^{®}, a platinum complex derivative, oxaliplatin, a kinase inhibitor, a tyrosine kinase inhibitor, a PI3 kinase inhibitor, a BTK inhibitor, an ibrutinib, IMBRUVICA^{®}, an acalabrutinib, CALQUENCE^{®}, a zanubrutinib, a PD-1 antibody, a PD-L1 antibody, a CTLA-4 antibody, a LAG3 antibody, an ICOS antibody, a TIGIT antibody, a TIM3 antibody, a CD40 antibody, a 4-1BB antibody, a CD47 antibody, a SIRP1α antibody or fusions protein, a CD70 antibody, and CLL1 antibody, a CD123 antibody, an antagonist of E-selectin, an antibody binding to a tumor antigen, an antibody binding to a T-cell surface marker, an antibody binding to a myeloid cell or NK cell surface marker, an alkylating agent, a nitrosourea agent, an antimetabolite, an antitumor antibiotic, an alkaloid derived from a plant, a hormone therapy medicine, a hormone antagonist, an aromatase inhibitor, and a P-glycoprotein inhibitor.

The isolated monoclonal antibody or an antigen binding fragment thereof may further comprise an antitumor drug linked thereto. The antitumor drug may be linked to said antibody through a photolabile linker or through an enzymatically cleaved linker. The antitumor drug may be a toxin, a radioisotope, a cytokine, or an enzyme.

Also provided is method of detecting a cancer cell or cancer stem cell in a sample or subject comprising (a) contacting a subject or a sample from the subject with an antibody or an antigen-binding fragment thereof as defined herein; and (b) detecting binding of said antibody to a cancer cell or cancer stem cell in said subject or sample. The sample may be a body fluid or biopsy. The sample may be blood, bone marrow, sputum, tears, saliva, mucous, serum, urine or feces. Detection may comprise immunohistochemistry, flow cytometry, an immunoassay (including ELISA, RIA etc.) or Western blot. The method may further comprise performing steps (a) and (b) a second time and determining a change in detection levels as compared to the first time. The isolated monoclonal antibody or an antigen binding fragment thereof may further comprise a label, such as a peptide tag, an enzyme, a magnetic particle, a chromophore, a fluorescent molecule, a chemo-luminescent molecule, or a dye. The isolated monoclonal antibody or an antigen binding fragment thereof may be conjugated to a liposome or nanoparticle.

An additional embodiment is a method of treating or ameliorating the effect of an autoimmune disease in a subject, the method comprising administering to the subject a therapeutically effective amount of an antibody or an antigen-binding fragment thereof as defined herein or an engineered cell as defined herein. The method may target monocytes, macrophages, dendritic cells, and neutrophils and other myeloid cells. The antibody or an antigen-binding fragment thereof may be administered intravenously, intra-arterially, intra-tumorally, or subcutaneously. The method may further comprise administering to the subject one or more drugs selected from the group consisting of a steroid or an NSAID. The autoimmune disease may be Guillain-Barre syndrome, Chronic inflammatory demyelinating polyneuropathy, ankylosing spondylitis, psoriatic arthritis, enteropathic arthritis, reactive arthritis, undifferentiated spondyloarthropathy, juvenile spondyloarthropathy, Behcet's disease, enthesitis, ulcerative colitis, Crohn's disease, irritable bowel syndrome, inflammatory bowel disease, fibromyalgia, chronic fatigue syndrome, pain conditions associated with systemic inflammatory disease, systemic lupus erythematosus, Sjogren's syndrome, rheumatoid arthritis, juvenile rheumatoid arthritis, juvenile onset diabetes mellitus (also known as Type I diabetes mellitus), Wegener's granulomatosis, polymyositis, dermatomyositis, inclusion body myositis, multiple endocrine failure, Schmidt's syndrome, autoimmune uveitis, Addison's disease, Grave's Disease, Hashimoto's thyroiditis, autoimmune thyroid disease, pernicious anemia, gastric atrophy, chronic hepatitis, lupoid hepatitis, atherosclerosis, multiple sclerosis, amyotrophic lateral sclerosis, hypoparathyroidism, Dressler's syndrome, myasthenia gravis, Eaton-Lambert syndrome, autoimmune thrombocytopenia, idiopathic thrombocytopenic purpura, hemolytic anemia, pemphigus vulgaris, pemphigus, dermatitis herpetiformis, alopecia, scleroderma, progressive systemic sclerosis, CREST syndrome (calcinosis, Raynaud's phenomenon, esophageal dysmotility, sclerodactyly, and telangtasia), adult onset diabetes mellitus (also known as Type II diabetes mellitus), mixed connective tissue disease, polyarteritis nodosa, systemic necrotizing vasculitis, glomerulonephritis, atopic dermatitis, atopic rhinitis, Goodpasture's syndrome, Chagas' disease, sarcoidosis, rheumatic fever, asthma, anti-phospholipidsyndrome, erythema multiforme, Cushing's syndrome, autoimmune chronic active hepatitis, allergic disease, allergic encephalomyelitis, transfusion reaction, leprosy, malaria, leshmaniasis, trypanosomiasis, Takayasu's arteritis, polymyalgia rheumatica, temporal arteritis, shistosomiasis, giant cell arteritis, eczema, lymphomatoid granulomatosis, Kawasaki's disease, endophthalmitis, psoriasis, erythroblastosis fetalis, eosinophilic faciitis, Shulman's syndrome, Felty's syndrome, Fuch's cyclitis, IgA nephropathy, Henoch-Schonlein purpura, graft versus host disease, transplantation rejection, tularemia, periodic fever syndromes, pyogenic arthritis, Familial Mediterranean Fever, TNF-receptor associated periodic syndrome (TRAPS), Muckle-Wells syndrome, or hyper-IgD syndrome.

Also provides is a non-human cell comprising a coding region for human LILRB3, such as a mouse cell. The LILBR3 coding region may be under the control of a promoter, such as a heterologous promoter, including an inducible promoter or a constitutive promoter. Expression of LILRB3 may be negatively regulated by transcription or translation inhibitor element, such as a stop codon flanked by Lox sites. The cell make be located in a transgenic non-human animal, such as a transgenic mouse.

Other objects, features and advantages of the present disclosure will become apparent from the following detailed description. It should be understood, however, that the detailed description and the specific examples, while indicating preferred embodiments of the disclosure, are given by way of illustration only, since various changes and modifications within the spirit and scope of the disclosure will become apparent to those skilled in the art from this detailed description.

### BRIEF DESCRIPTION OF THE DRAWINGS

The following drawings form part of the present specification and are included to further demonstrate certain aspects of the present disclosure. The disclosure may be better understood by reference to one or more of these drawings in combination with the detailed description of specific embodiments presented herein.

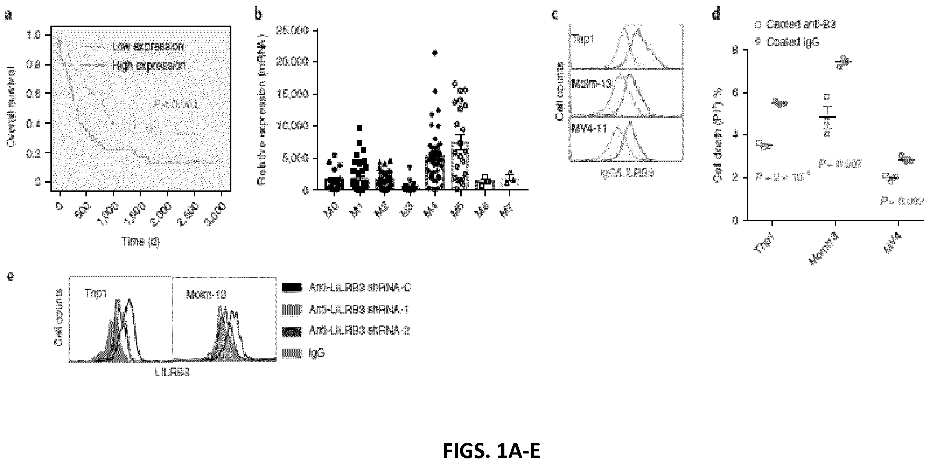

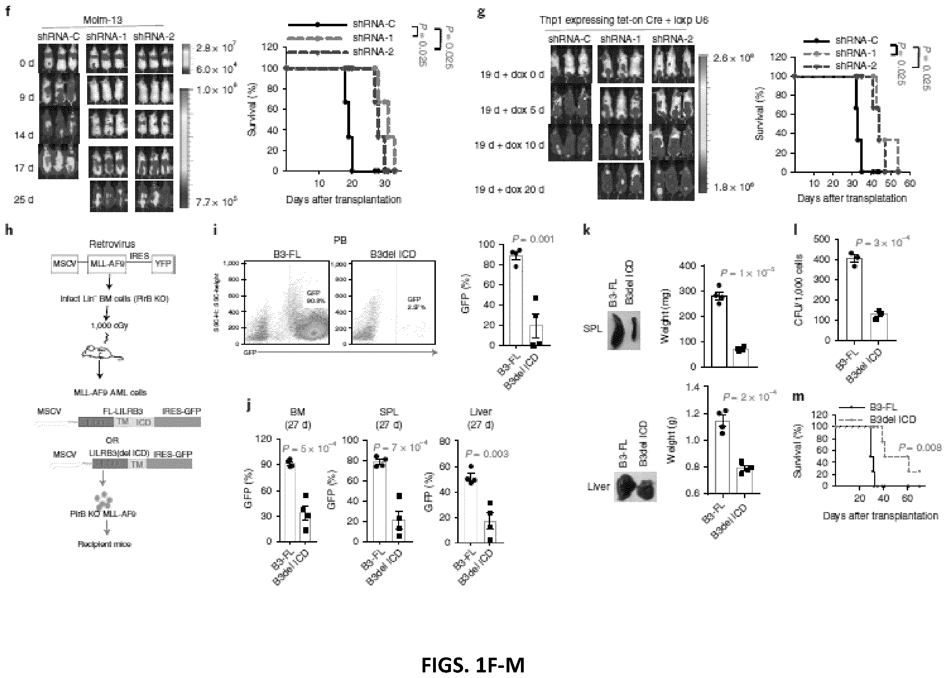

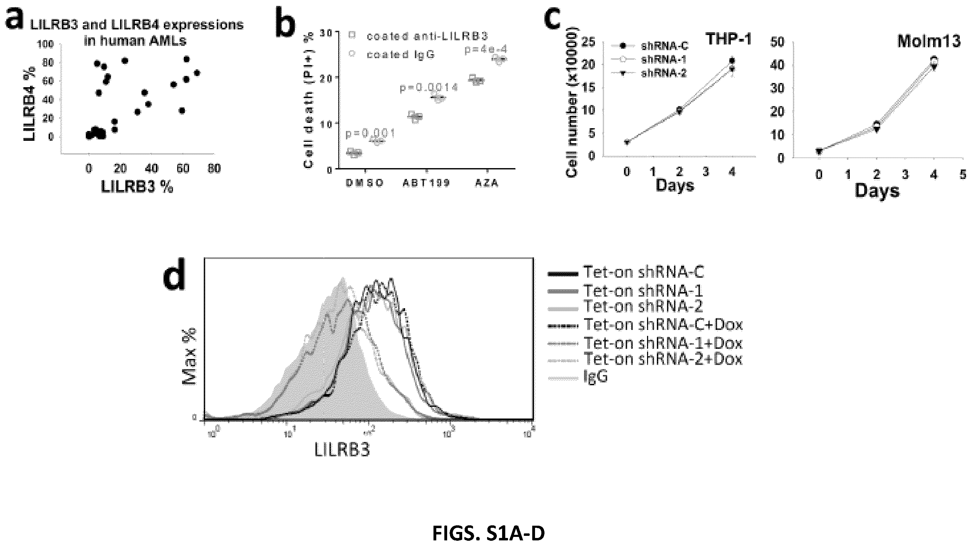

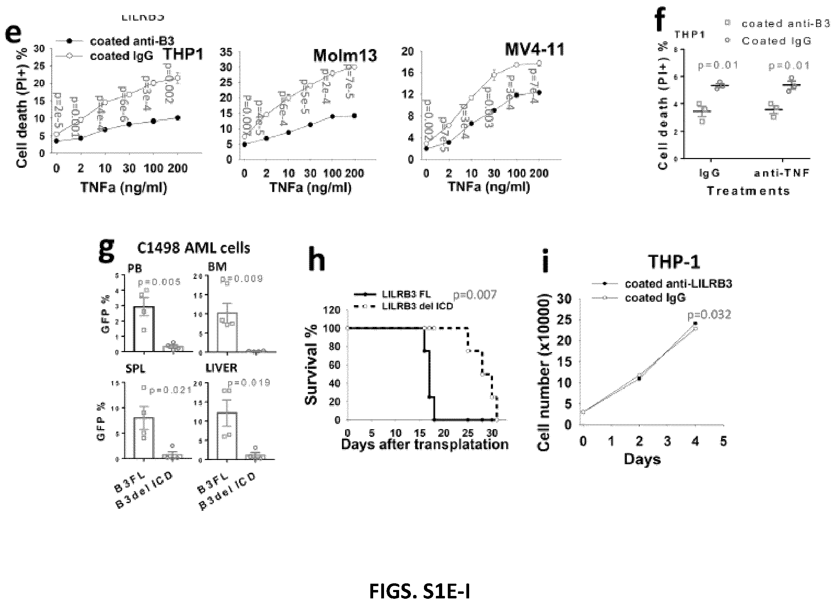

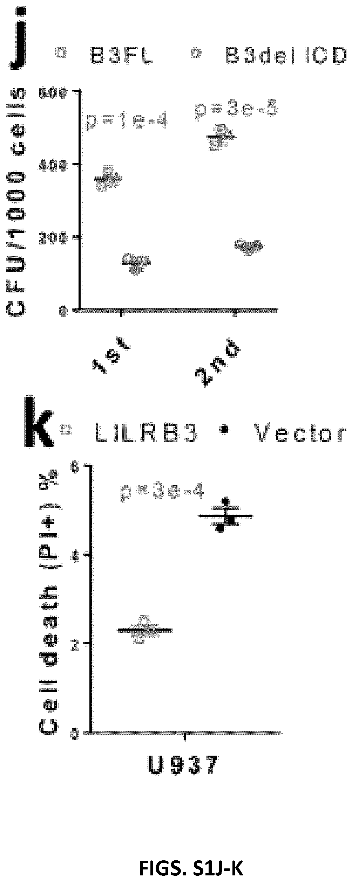

**FIGS. 1A-M****.** LILRB3 promotes monocytic AML progression. (FIG. 1A) Overall survival of AML patients grouped based on *LILRB3* expression; data from the TCGA database were analyzed. (FIG. 1B) Relative *LILRB3* expression in different AML subtypes (mRNA expression normalized to GAPDH); data from the TCGA database. (FIG. 1C) LILRB3 expression on AML cell lines. (FIG. 1D) Percentages of dead cells in AML cultures treated with anti-LILRB3 antibody or IgG (n=3 independent cell cultures). (FIG. 1E) LILRB3 expression on AML cells expressing control shRNA (shRNA-C) or *LILRB3*-specific shRNAs (shRNA-1 or -2). (FIG. 1F) Left: Whole-body images for luciferase of NSG mice engrafted with Molm13 cells expressing luciferase and indicated shRNAs over time. Right: Survival of mice (n=3 independent mice). (FIG. 1G) Left: Images of NGS mice engrafted with THP-1 AML and treated with dox at 19 days (once, gavage 2 mg/mouse) post-engraftment to induce shRNA expression. Right: Survival of mice (n=3 independent mice). (FIG. 1H) Schematic of retroviral vector used to create a mouse model to study the function of LILRB3. (FIG. 1I) Representative FACS analyses and plot of percentages of GFP⁺ AML cells expressing B3-FL and B3del ICD in peripheral blood of engrafted mice (normal peripheral blood sample as a negative control for gating GFP positive population). (FIG. 1J) Percentages of GFP⁺ AML cells expressing B3-FL and B3del ICD in bone marrow (BM), spleen (SPL), and liver of mice at 27 days after engraftment (n=4 independent mice). (FIG. 1K) Representative images and average weights of spleen and liver of mice engrafted with GFP⁺ AML cells expressing B3-FL and B3del ICD at 27 days (n=4 independent mice). (FIG. 1L) Clone forming assay (CFU) of GFP⁺ AML cells expressing B3-FL or B3del ICD isolated from bone marrow (n=3 independent cell cultures). (FIG. 1M) Survival of the mice engrafted with AML cells expressing B3-FL and B3del ICD (n=4 independent mice). The data are presented as mean ± s.e.m, and p values were calculated by two-tailed t-test except for a, f, g and m by log-rank test.

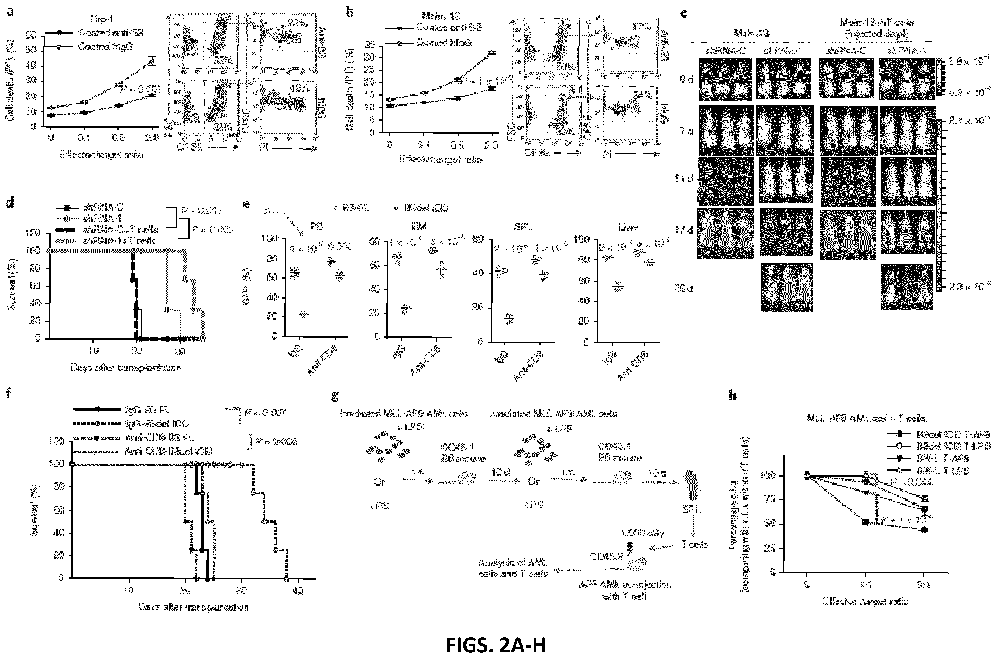

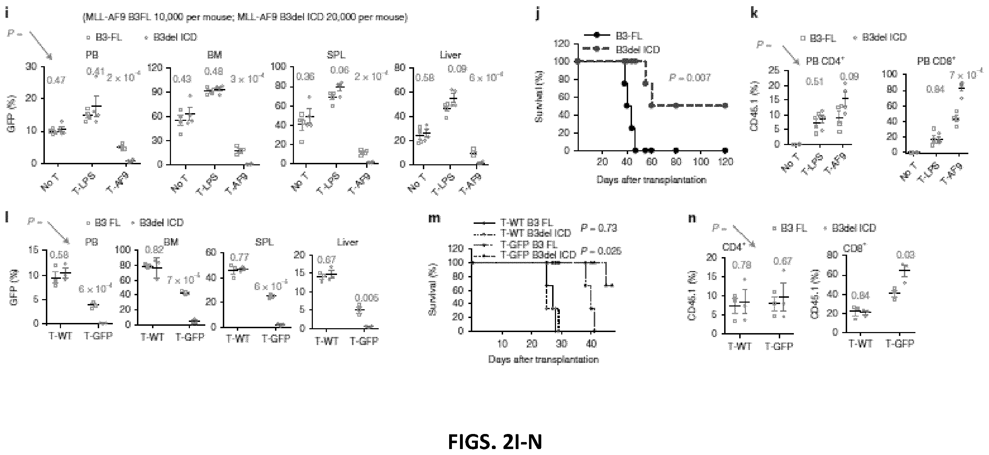

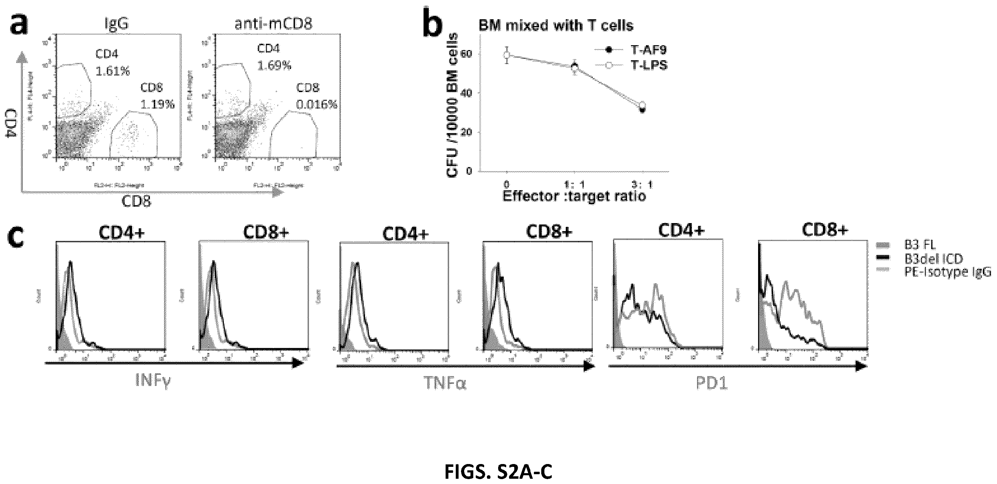

**FIGS. 2A-N****.** LILRB3 increases the sensitivity of monocytic AML cells to cytotoxic T cells. (FIGS. 2A-B) cell death of CFSE-stained (FIG. 2A) THP-1 or (FIG. 2B) Molm13 cells when co-cultured with activated T cells at different ratios. The FACS images to the right are with effector to target (E:T) ratio of 2 (n=3 independent cell cultures). (FIGS. 2C-D) NSG xongrafts with Molm13-luciferase cells introduced with shRNA-C or LILRB3-specific shRNA-1 with or without human T cell transplantation. Images of mice (FIG. 2C). Overall survival (FIG. 2D). (FIG. 2E) The percentage of GFP⁺ AML cells expressing B3-FL or B3del ICD in peripheral blood (PB), bone marrow (BM), spleen (SPL), and liver in C57BL/6 mice treated with IgG or anti-mCD8 (n=4 independent mice ). (FIG. 2F) Survival of mice in FIG. 2E. (FIG. 2G) Flow chart for immunizing CD45.1 B6 mice with MLL-AF9 mouse AML cells. (FIG. 2H) CFUs of MLL-AF9 AML cells when co-cultured with AML-specific T-AF9 or non-specific T-LPS T cells for 12 hours. (n=3 independent cell cultures). (FIG. 2I) The percentage of GFP⁺ AML cells expressing B3-FL or B3del ICD in peripheral blood (PB), bone marrow (BM), spleen (SPL), and liver in mice not treated with T cells (noT) or treated with T-LPS or T-AF9 (n=4 independent mice). (FIG. 2J) Overall survival of mice engrafted with AML cells expressing B3-FL or B3del ICD and injected with CD45.1 T cells (n=4 independent mice). (FIG. 2K) Percentages of CD45.1⁺/CD4⁺ and CD45.1⁺/CD8⁺ T cells in total CD4⁺ and CD8⁺ T cell populations, respectively, in peripheral blood of mice in FIG. 18I (n=4 independent mice). (FIG. 2L) The percentage of GFP⁺ AML cells expressing B3-FL or B3del ICD in peripheral blood (PB), bone marrow (BM), spleen (SPL), and liver in mice treated with wild-type T cells (T-WT, CD45.1) or GFP specific T cells (T-GFP, CD45.1) (n=3 independent mice). (FIG. 2M) Survival of mice engrafted with indicated AML cells and T cells as above (n=3 mice). (FIG. 2N) Percentages of CD45.1+/CD4+ and CD45.1+/CD8+ T cells in total CD4+ and CD8+ T cell populations, respectively, in peripheral blood of mice in l. The data are presented as mean ± s.e.m, and p values were calculated by two-tailed t-test except for FIG. 2D, FIG. 2F, FIG. 2J and FIG. 2M by log-rank test.

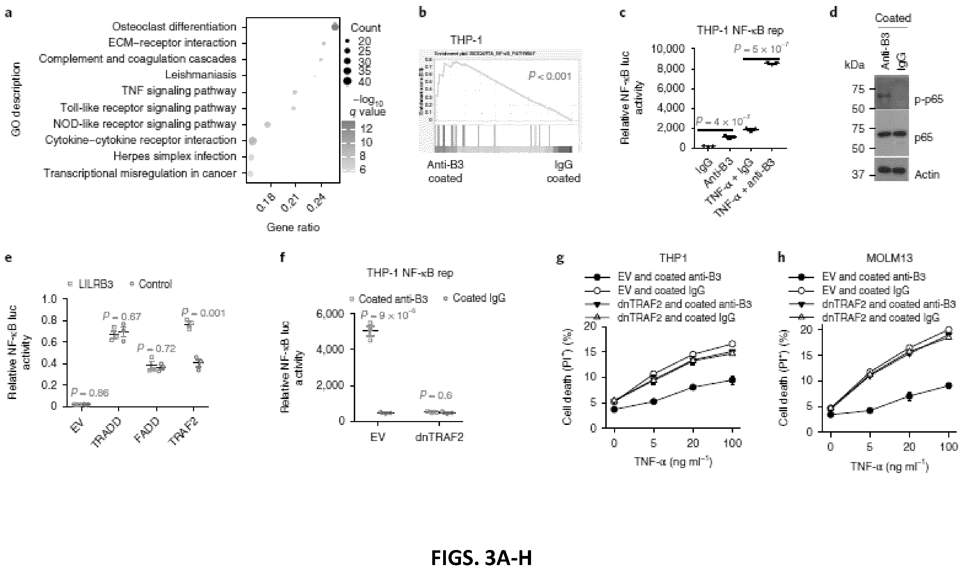

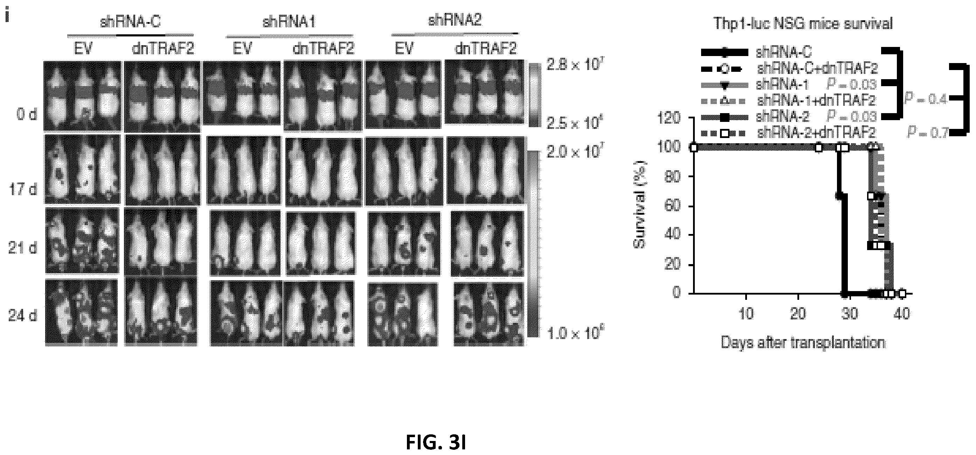

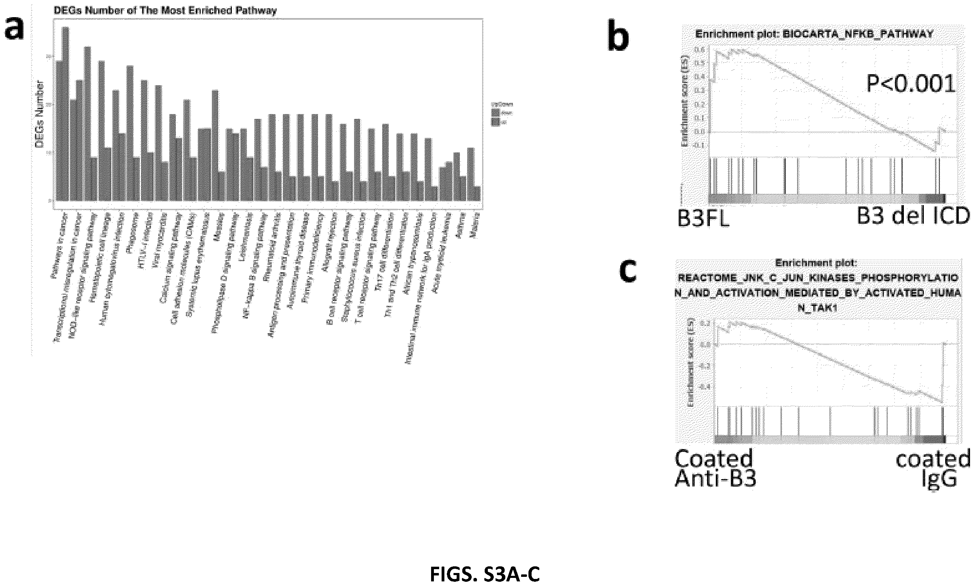

**FIGS. 3A-I****.** LILRB3 enhances NF-κB signaling and monocytic AML survival via TRAF2. (FIG. 3A) Gene ontology enrichment analysis of RNA-seq data from THP-1 cells cultured in plates coated with anti-LILRB3 antibody or IgG. (FIG. 3B) GSEA of the correlation between NF-κB signaling and LILRB3 signaling (p values were calculated by Kolmogorov Smirnov (K-S) test in GSEA analysis). (FIG. 3C) Luciferase signal from THP-1-Lucia^{™} cells that stably express an NF-κB-inducible luciferase reporter after culture in plates coated with anti-LILRB3 antibody or IgG in the presence of TNF-α (10 ng/ml) or not (n=3 independent cell cultures). (FIG. 3D) Phosphorylated p65 (p-p65) and p65 levels in THP-1 cell cultured in plates coated anti-LILRB3 antibody or IgG. (FIG. 3E) Luciferase signal in 293T cells transfected with plasmid for expression of NF-κB promoter-driven firefly *luciferase* and control *Renilla luciferase* and empty vector (EV) or TRADD, FADD, or TRAF2 expression plasmids with a vector for expression of LILRB3 or a control vector (n=3 independent cell cultures). (FIG. 3F) Luciferase signal from THP-1-Lucia^{™} cells transfected with empty vector (EV) or vector for expression of dominant-negative TRAF2 (dnTRAF2) cultured in plates coated anti-LILRB3 antibody or IgG (n=3 independent cell cultures). (FIGS. 3G-H) Percentages of dead cells in cultures of THP-1 cells (FIG. 3G) or Molm13 cells (FIG. 3H) expressing dnTRAF2 or empty vector and cultured in plates coated with anti-LILRB3 antibody or IgG (n=3 independent cell cultures). (FIG. 3I) Left: Images of NSG mice transplanted with THP-1 cells expressing luciferase and shRNA-C or LILRB3-specific shRNAs in the presence of dnTRAF2 or not. Right: Overall survival of the NSG mice (n=3 independent mice, p values were calculated by log-rank test). The data are presented as mean ± s.e.m, and p values were calculated by two-tailed t-test except for FIG. 3B and FIG. 3I.

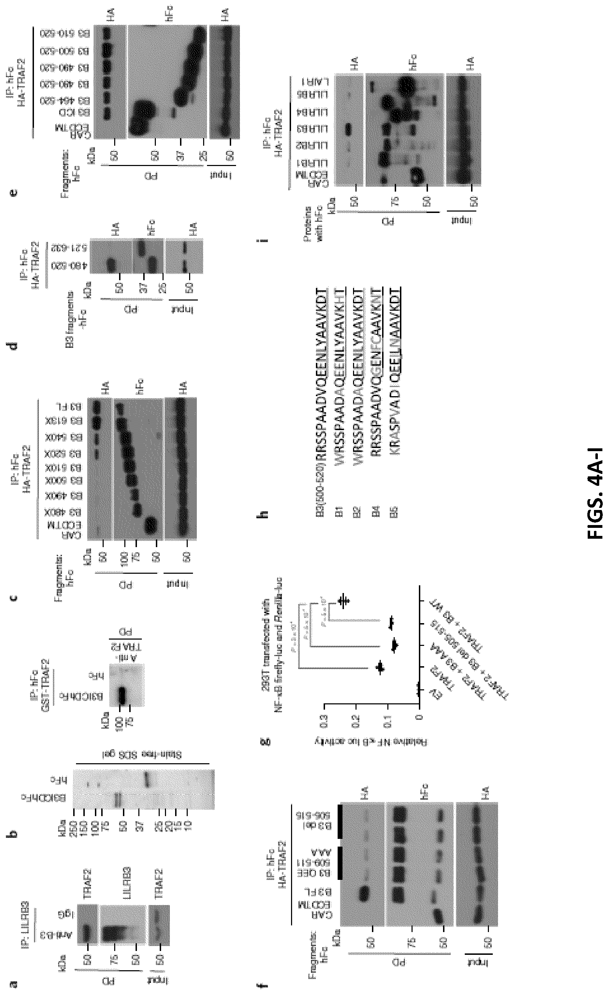

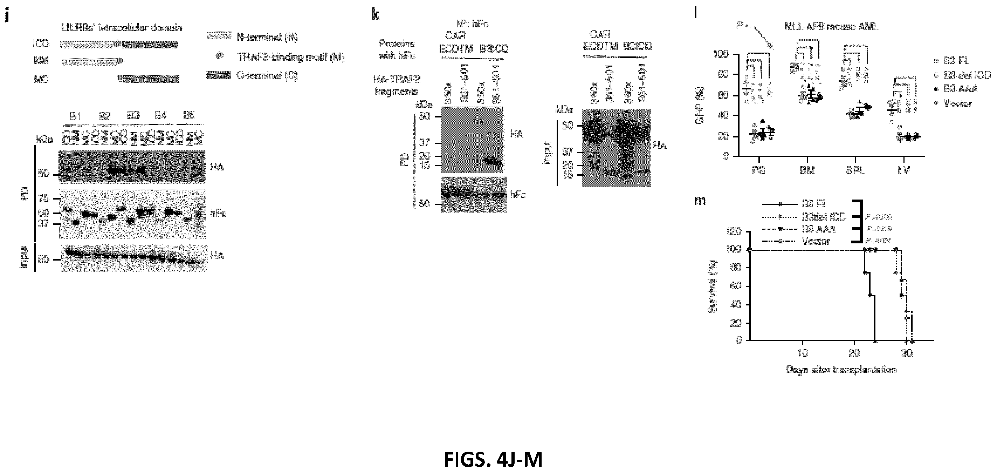

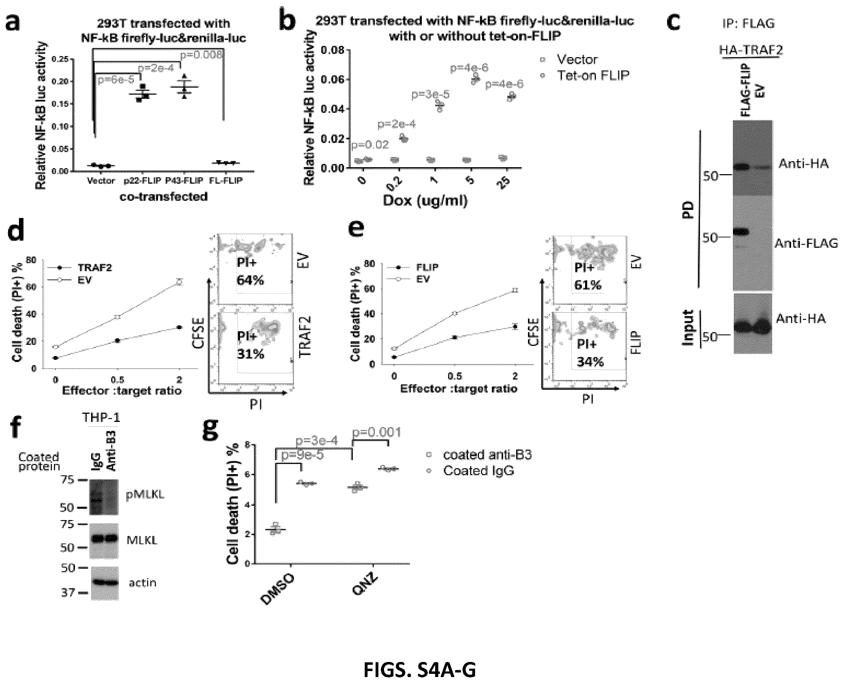

**FIGS. 4A-M****.** LILRB3 interacted with TRAF2. (FIG. 4A) Interaction of TRAF2 and LILRB3 in M5 AML patient sample detected by immunoprecipitation with human anti-LILRB3 antibody. (FIG. 4B) LILRB3 interacts with TRAF2 *in vitro.* Left: SDS-PAGE of purified LILRB3 intracellular domain fused to hFc at C-terminus (B3ICDhFc) or hFc alone, exogenously expressed in 293T cells. Right; purified GST-TRAF2 (0.5 M) interacts with purified B3ICDhFc or hFc. (FIGS. 4C-E) Co-immunoprecipitation analysis of exogenous HA-TRAF2 with LILRB3 fragments in 293T cells. The C-terminal of LILRB3 fragments was fused to human Fc and the extracellular domain and transmembrane domain of CAR, an unrelated protein served as control. (FIG. 4F) Co-immunoprecipitation analysis of exogenous HA-TRAF2 and LILRB3 mutants in 293T cells. (FIG. 4G) Relative NF-κB activities in 293T cells that express TRAF2 and indicated LILRB3 mutants (n=3 independent cell cultures). (FIG. 4H) Conservation of LILRB3 sequence critical for binding to TRAF2 in other LILRBs. (SEQ ID NOS: 444-448) (FIG. 4I) Co-immunoprecipitation analysis of exogenous HA-TRAF2 with LILRBs fused with human in 293T cells. (FIG. 4J) Interactions of TRAF2 with intracellular segments of different LILRBs. (FIG. 4K) Co-immunoprecipitation analysis of exogenous HA-TRAF2 fragments and the LILRB3 intracellular domain (B3ICD) fused to human Fc in 293T cells. (FIG. 4L) The percentage of GFP⁺ MLL-AF9 AML cells (with PirB knockout) expressing B3-FL, B3del ICD, B3 AAA mutant (n=4 independent mice) or empty vector (n=3 independent mice) in peripheral blood (PB), bone marrow (BM), spleen (SPL), and liver. (FIG. 4M) Survival of mice engrafted with indicated AML cells as above (n=4 independent mice). The data are presented as mean ± s.e.m, and p values were calculated by two-tailed t-test except for m by log-rank test.

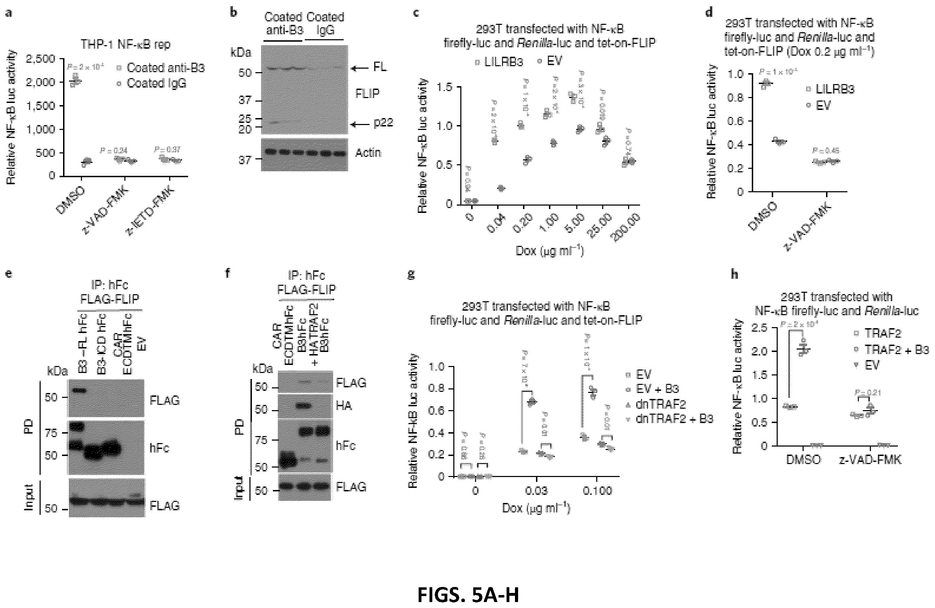

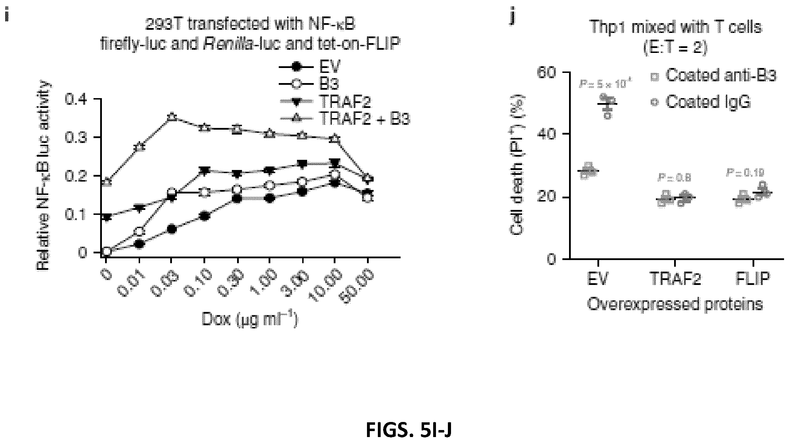

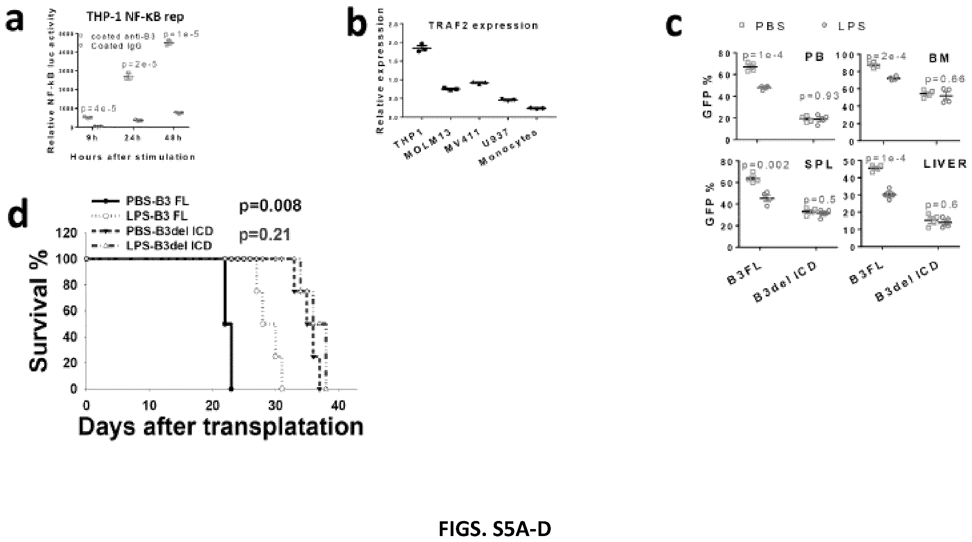

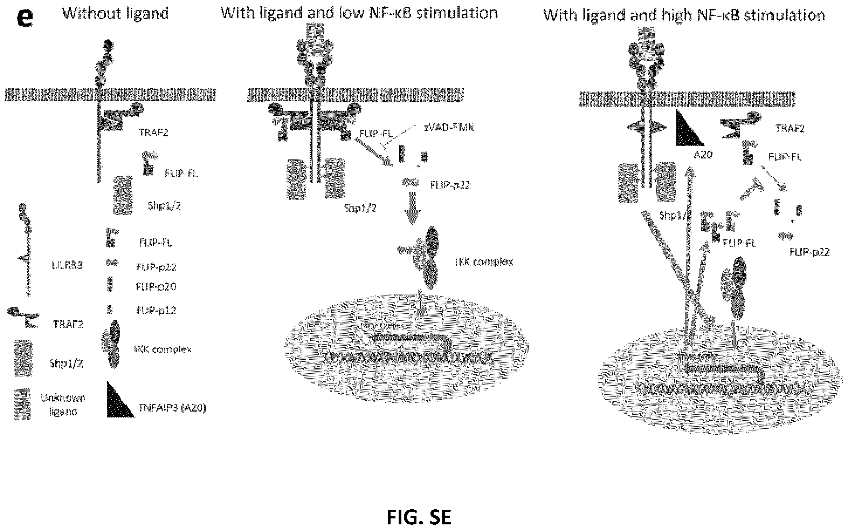

**FIGS. 5A-J****.** LILRB3 enhancement of NF-κB signaling depends on cFLIP. (FIG. 5A) NF-κB reporter gene activities in THP-1-Lucia^{™} cells activated with anti-LILRB3 antibody or IgG in the presence of DMSO, caspase inhibitor z-VAD-FMK, or caspase 8 inhibitor z-IETD-FMK (n=3 independent cell cultures). (FIG. 5B) Western blot analysis for cFLIP in THP-1 cells activated by anti-LILRB3 antibody or IgG. (FIG. 5C) Relative NF-κB reporter gene activities in 293T cells co-transfected with tet-on cFLIP plus empty vector (EV) or vector for expression of LILRB3 at different concentration of dox (n=3 independent experiments). (FIG. 5D) Relative NF-κB reporter gene activities in 293T cells co-transfected with tet-on cFLIP plus empty vector or vector for expression of LILRB3 in the presence of DMSO or z-VAD-FMK (with 0.2 ug/ml of dox) (n=3 independent experiments ). (FIG. 5E) Co-immunoprecipitation assay of exogenously expressed FLAG-cFLIP and hFc-tagged B3-FL or B3del ICD in 293T cells. (FIG. 5F) Co-immunoprecipitation assay of exogenously expressed FLAG-cFLIP and LILRB3-hFc in the presence of HA-TRAF2 or empty vector in 293T cells. (FIG. 5G) Relative NF-κB reporter gene activities in 293T cells co-transfected with tet-on cFLIP in the presence of LILRB3 and dnTRAF2 or empty vector at different concentrations of dox (n=3 wells). (FIG. 5H) Relative NF-κB reporter gene activities in 293T cells co-transfected with TRAF2 or LILRB3 in the presence of DMSO or z-VAD-FMK (n=3 independent experiments). (FIG. 5I) Relative NF-κB reporter gene activities in 293T cells co-transfected with tet-on cFLIP in the presence of LILRB3 and TRAF2 or empty vector at different concentrations of dox (n=3 independent experiments). (FIG. 5J) CFSE-stained THP-1 cells with forced expression of TRAF2, FLIP or empty vector (EV) were co-cultured with activated T cells in plates coated with anti-LILRB3 or IgG for 12 hours before cell death analysis. The plots are of percent dead cells in CSFE positive cells (n=3 independent experiments). The data are presented as mean ± s.e.m, and p values were calculated by two-tailed t-test.

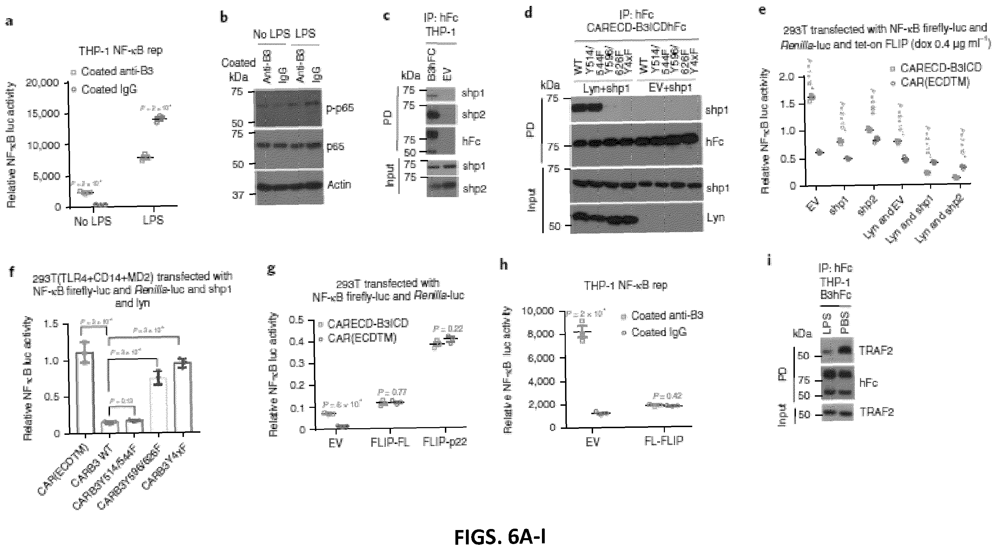

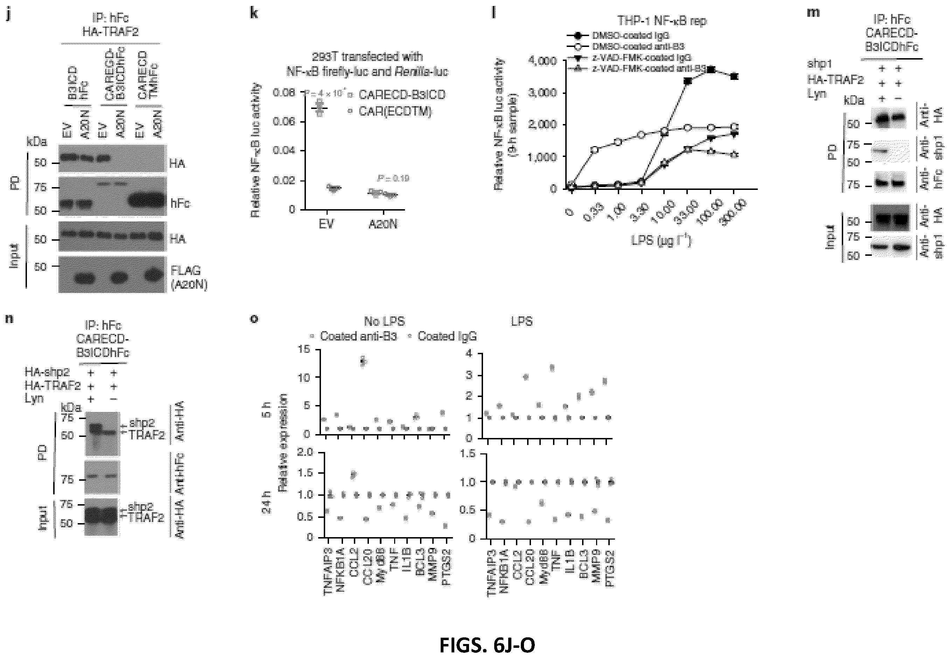

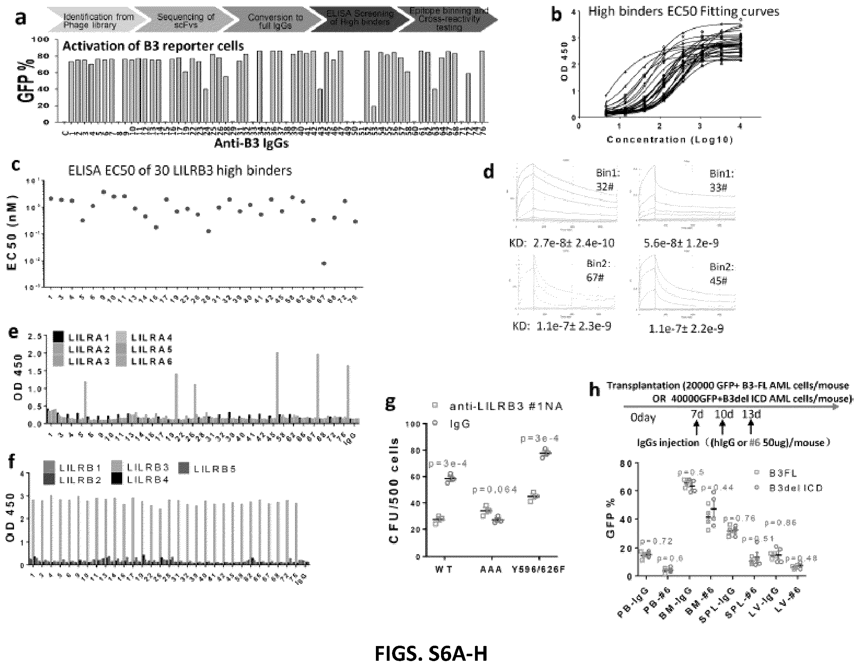

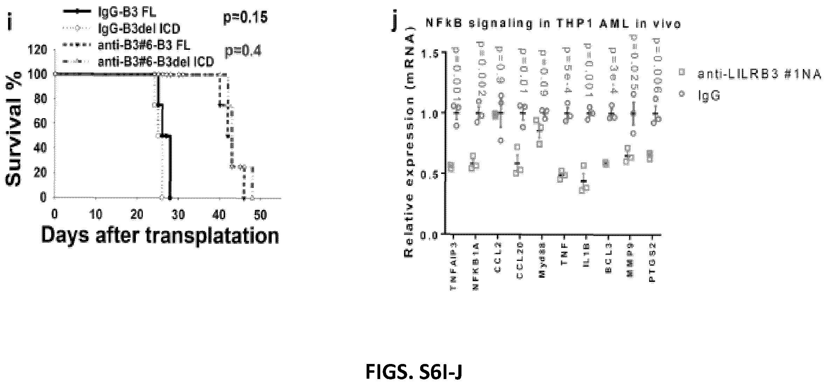

**FIGS. 6A-O****.** LILRB3 negatively regulates NF-κB signaling with strong LPS stimulation. (FIGS. 6A-B) In the presence or absence of 200 µg/ L LPS, Signal from THP-1-Lucia^{™} cells (a) (n=3 independent cell cultures) and Levels of phosphorylated p65 (p-p65) and p65 in THP-1 cell (b) when treated with anti-LILRB3 antibody or IgG for 12 hours. (FIG. 6C) Co-immunoprecipitation of endogenous SHP-1 and SHP-2 in THP-1 cells that stably express LILRB3-hFc (B3hFc) or empty vector (EV). (FIG. 6D) Co-immunoprecipitation of exogenous SHP-1 and CARECD-B3ICDhFc with or without ITIM mutations in 293T cells in the presence of exogenously expressed Lyn or not (EV). Y4xF indicates protein with four ITIM mutations. (FIGS. 6E-G) Relative NF-κB signaling activities in 293T cells co-transfected with vectors tet-on cFLIP and CARECD-B3ICD or CARECDTM in the presence of empty vector or vectors for expressing of SHP-1 or SHP-2 with or without Lyn expression (FIG. 6E); expressing TLR4, CD14, MD2, SHP-1, and Lyn plus CARECDTM or CARECD-B3ICD with different ITIM mutations in the presence of 200 µg/L LPS (FIG. 6F);or co-transfected with CARECD-B3ICD or CARECDTM in the presence of empty vector, cFLIP-FL, or p22 (FIG. 6G). (n=3 wells) (FIG. 6H) Signal from THP-1-Lucia^{™} cells infected with empty vector or FL-cFLIP and activated with anti-LILRB3 antibody or IgG. (n=3 independent cell cultures) (FIG. 6I) Co-immunoprecipitation of endogenous TRAF2with LILRB3-hFc in THP-1 cells with LPS or PBS. (FIG. 6J) Interactions of exogenous HA-TRAF2 with B3ICDhFc, CARECDTMhFc, or CARECD-B3ICDhFc in presence or absence of the FLAG-tagged N terminus of A20 (A20N). (FIG. 6K) Relative NF-κB signaling activities in 293T cells co-transfected with CARECD-B3ICD or CARECDTM in the presence of empty vector or A20N. (n=3 independent experiments) (FIG. 6L) Signal of THP-1-Lucia^{™} cells activated with anti-LILRB3 antibody or IgG in the presence of DMSO or z-VAD-FMK at different concentration of LPS. (n=3 wells). (FIGS. 22M-N) Co-immunoprecipitation of SHP-1 (FIG. 6M or SHP-2 (FIG. 6N) with TRAF2 and CARECD-B3ICDhFc with or without Lyn. (FIG. 6O) qPCR of NF-κB target gene expression in normal monocytes (CD14⁺ cells) culture in the presence of anti-LILRB3 antibody or IgG with or without 200 µg/L LPS. (n=3 independent experiments). The data are presented as mean ± s.e.m, and P values were calculated by two-tailed t-test.

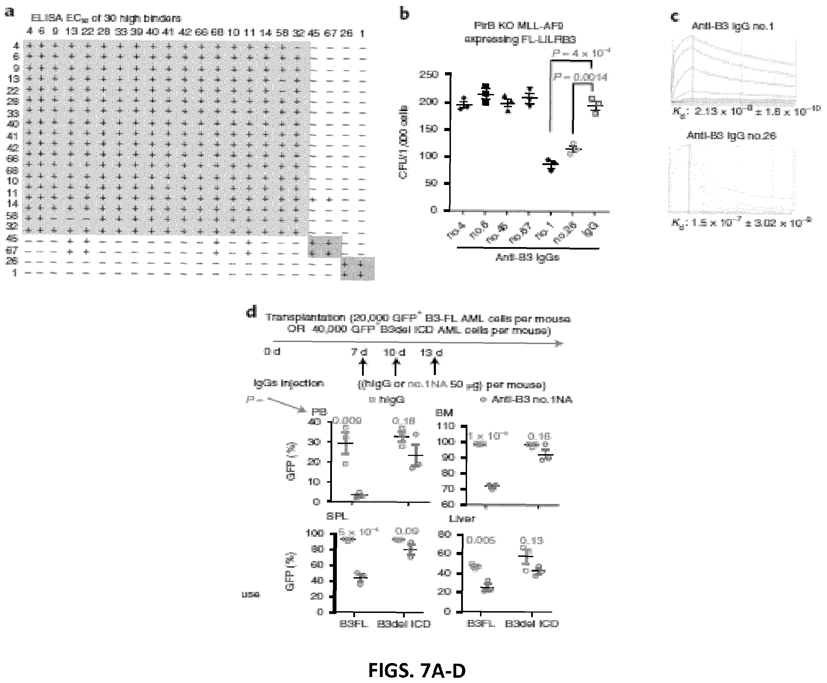

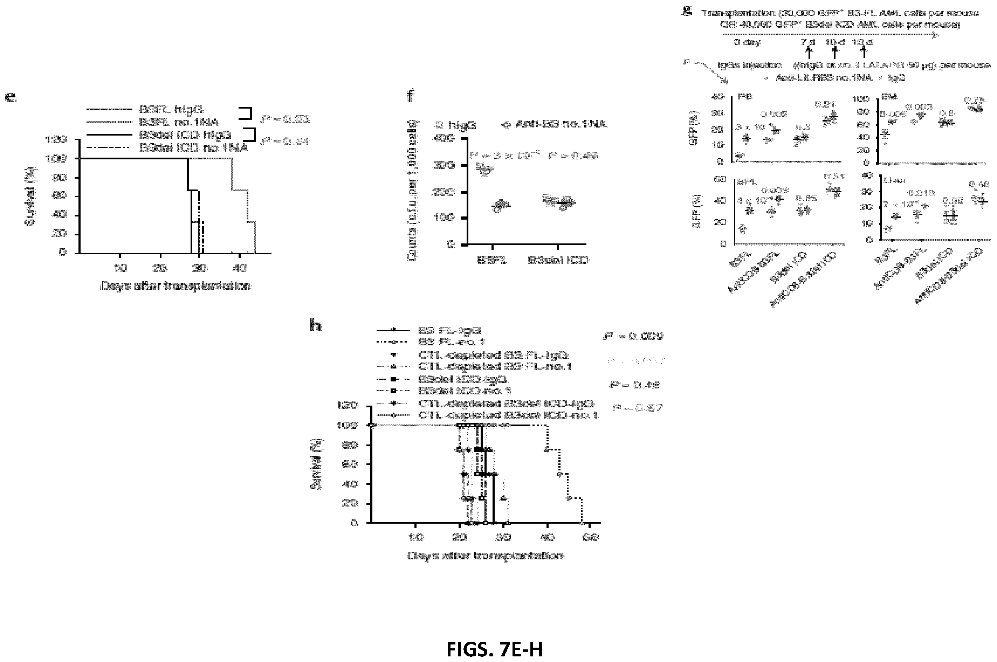

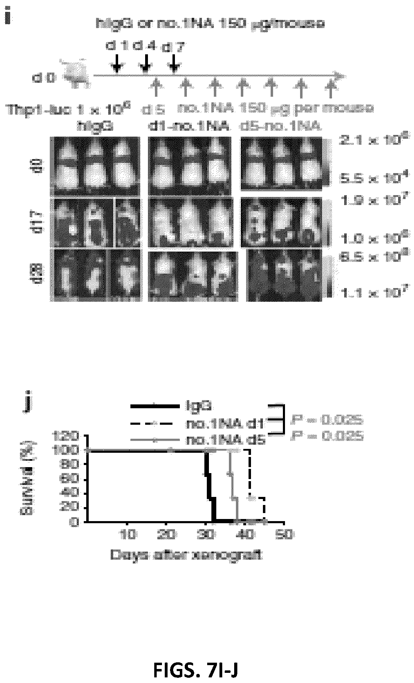

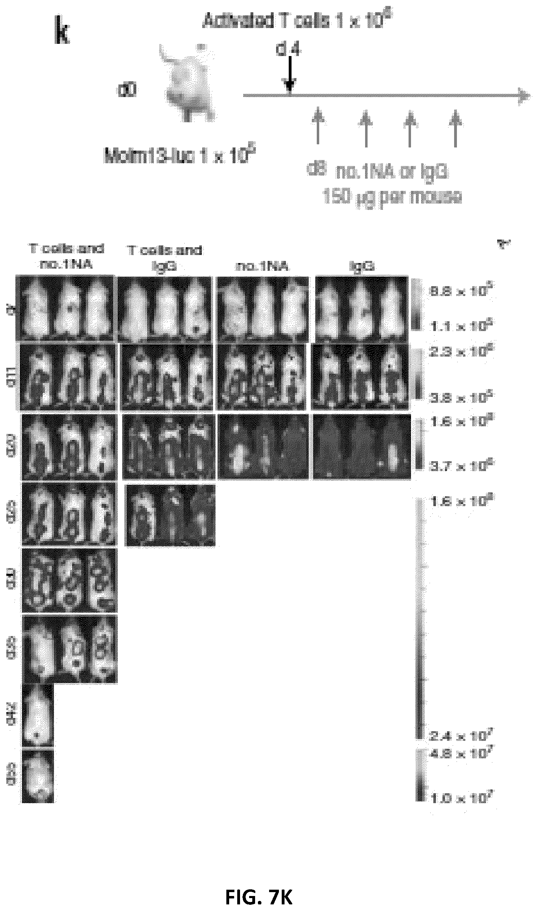

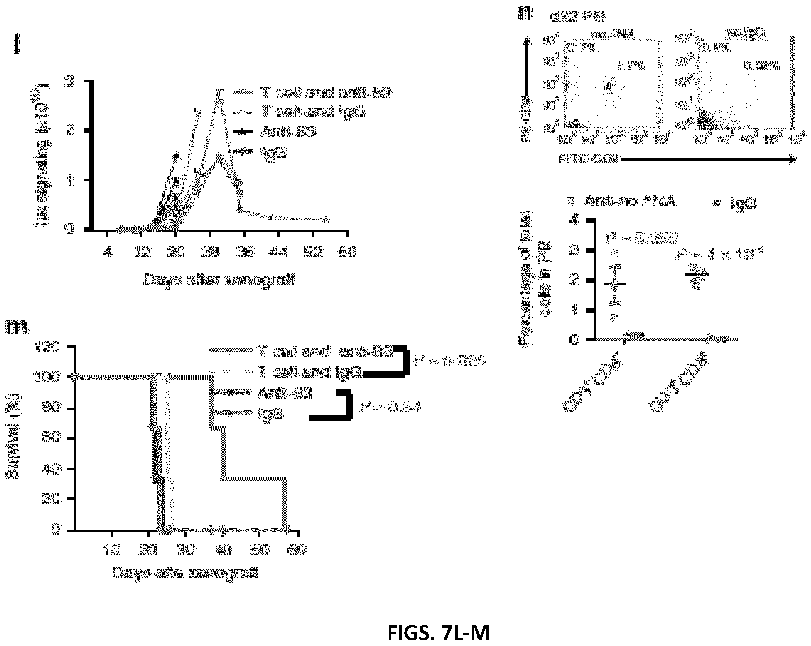

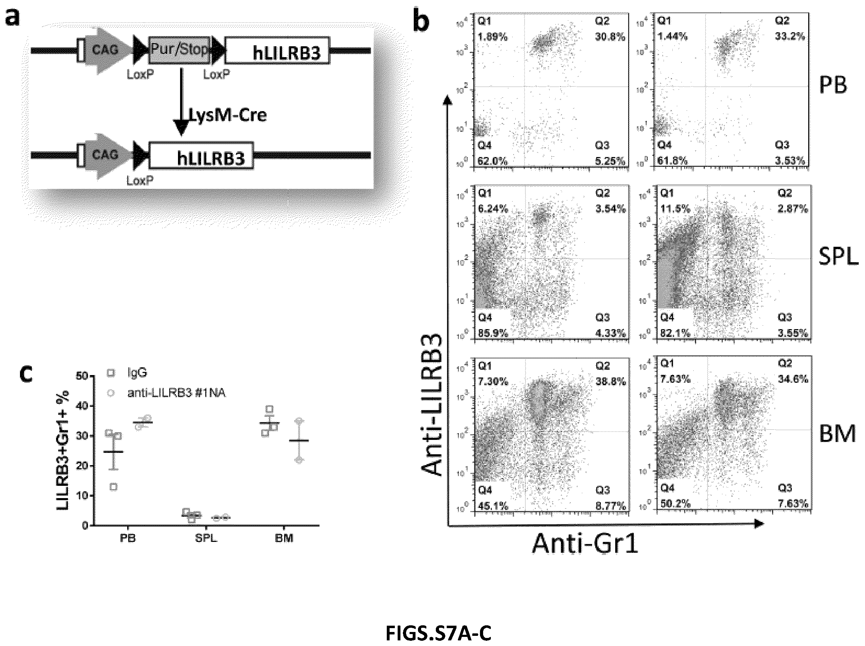

**FIGS. 7A-N****.** Blocking anti-LILRB3 antibody prevents monocytic AML development. (FIG. 7A) Epitope binning of 30 high-affinity IgGs. (FIG. 7B) CFU assay of mouse MLL-AF9 AML cells in the presence of above IgGs (n=3 independent experiments). (FIG. 7C) Binding affinities of the two IgGs from bin #3. (FIG. 7D) Upper: Schematic of treatment. Lower: Percentages of GFP⁺ MLL-AF9 mouse AML cells in peripheral blood (PB), bone marrow (BM), spleen (SPL), and liver (LV) of mice transplanted with AML cells expressing B3-FL or B3delICD and injected with IgG or anti-LILRB3 #1 N297A (n=3 independent mice). (FIG. 7E) Overall survival of mice transplanted with indicated AML cells as above. (FIG. 7F) CFU assay of MLL-AF9 mouse AML cells expressing B3-FL or B3delICD in the presence of IgG or anti-LILRB3 #1 N297A (n=3 independent experiments) (FIG. 7G) Upper: Schematic of treatment. Recipient mice were injected with mouse IgG or anti-mCD8 to deplete CD8 T cells. Lower: Percentages of GFP⁺ MLL-AF9 mouse AML cells in peripheral blood (PB), bone marrow (BM), spleen (SPL), and liver (LV) of mice transplanted with AML cells expressing B3-FL or B3delICD and injected with IgG or anti-LILRB3 #1 LALAPG (n=4 mice). (FIG. 7H) Survival of mice as in panel h (n=4 independent mice). (FIG. 7I) Upper: Schematic of treatment. Lower: Whole-body images of NSG mice transplanted with luciferase-expressing THP-1 cells and treated with IgG or anti-LILRB3 #1 N297A (#1NA). (FIG. 7J) Overall survival of the mice shown in panel h (n=3 independent mice). (FIG. 7K) Upper: Schematic of treatment. Lower: Whole-body images of NSG mice transplanted with luciferase-expressing Molm13 cells, injected with activated T cells, and treated with IgG or #1NA. (FIG. 7L) Luciferase signaling as a function of time in mice treated as described in panel k. (FIG. 7M) Overall survival of mice treated as described in panel k (n=3 independent mice). (FIG. 7N) Analyses of T cells in peripheral blood of mice treated as described in panel l at 22 days after Molm13 AML cell transplantation. Upper: Flow cytometry analyses. Lower: Plot of T cell percentages (n=3 independent mice). The data are presented as mean ± s.e.m, and p values were calculated by two-tailed t-test except for FIG. 7E, FIG. 7H, FIG. 7J and FIG. 7M by log-rank test.

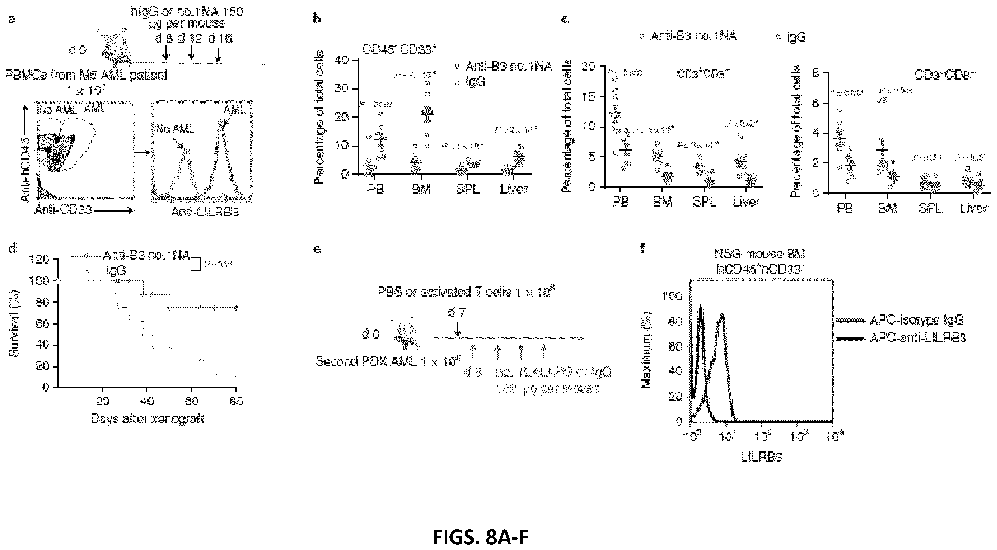

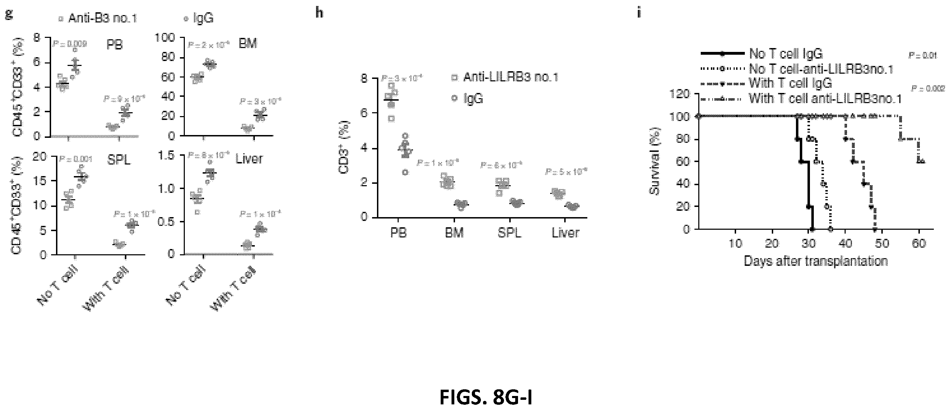

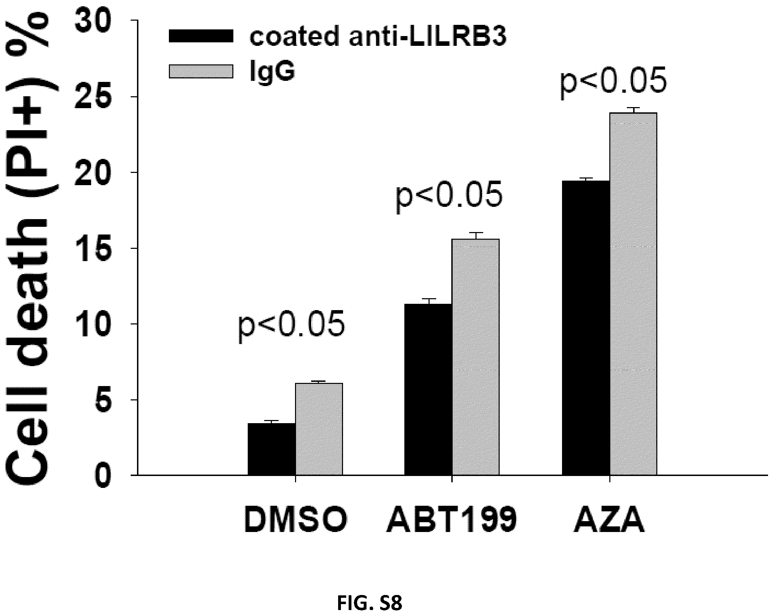

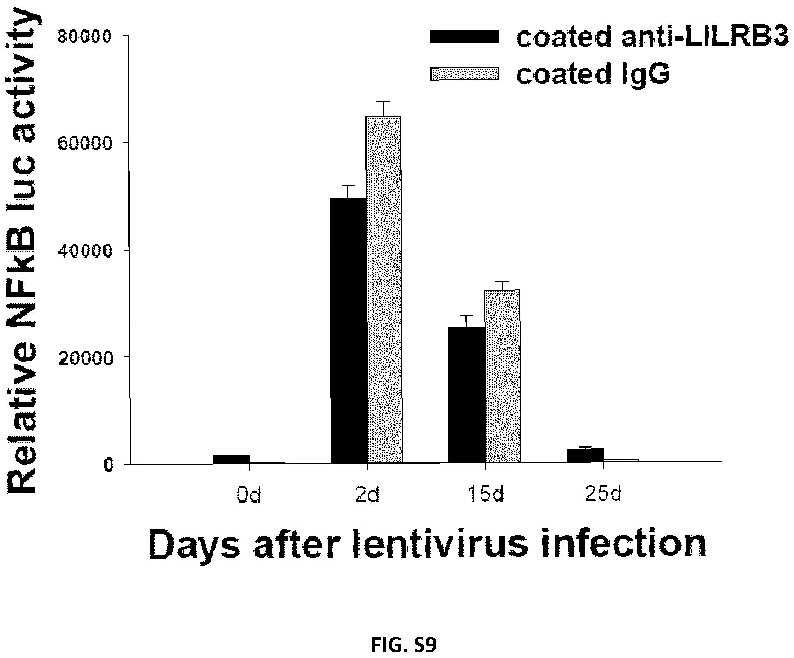

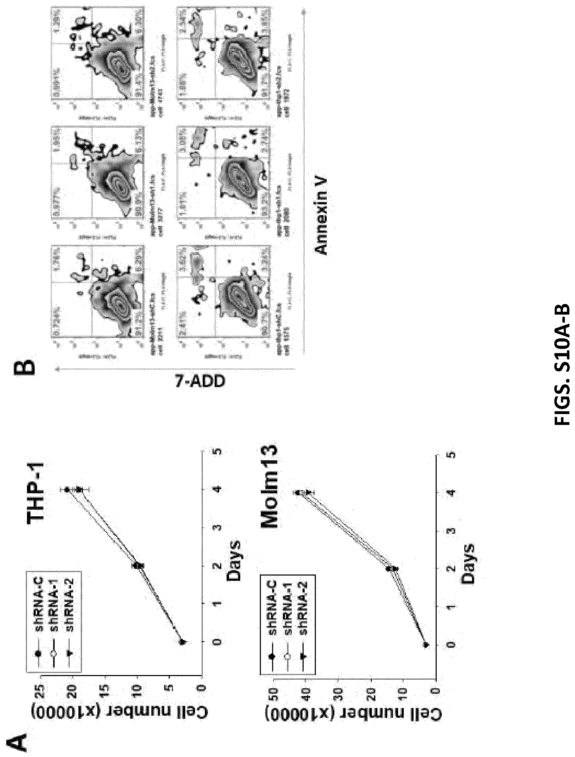

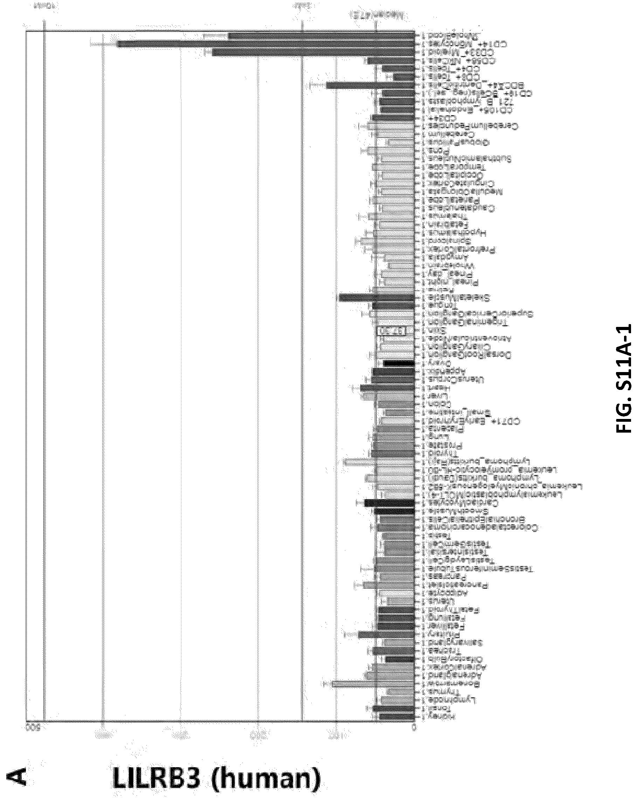

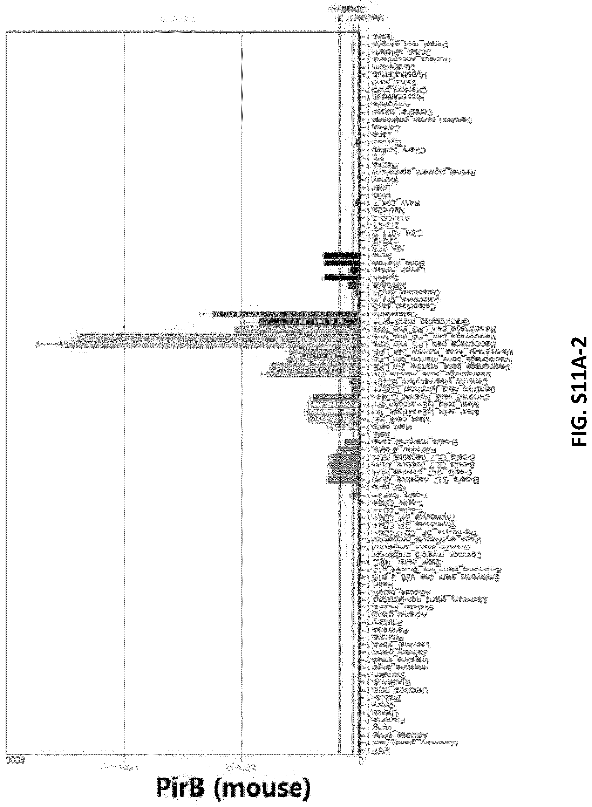

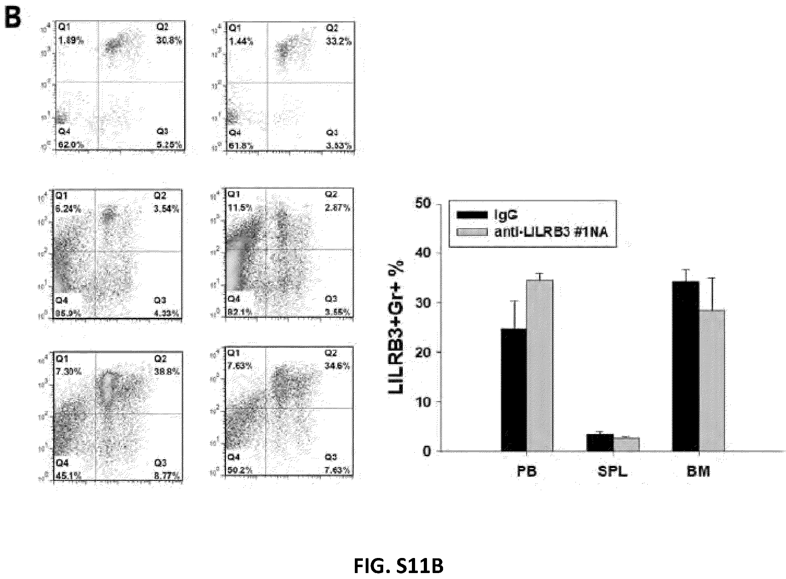

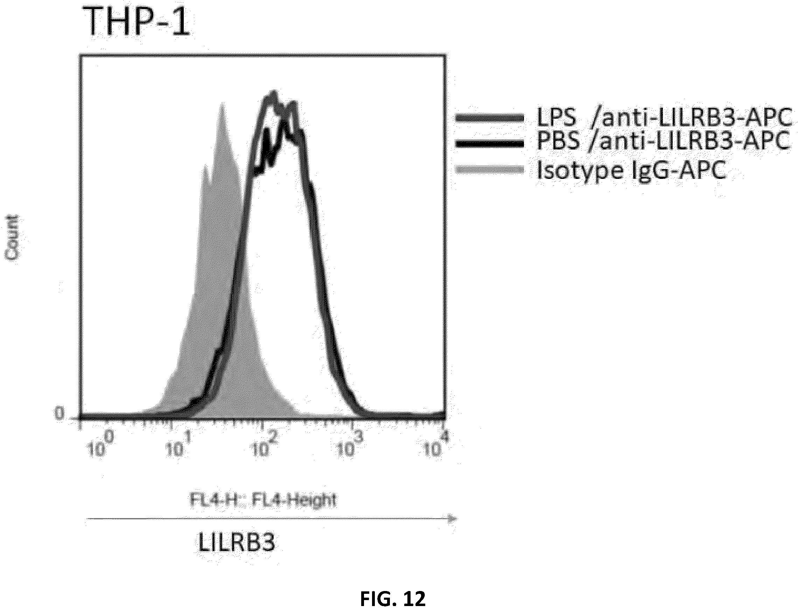

**FIGS. 8A-I****.** Anti-LILRB3 blocking antibody prevents development of patient derived AML. (FIG. 8A) Upper: Schematic of treatment. NSG mice were transplanted with monocytic AML patient peripheral blood sample (depleted red blood cells) and given IgG or #1NA. Lower: FACS analyses of the mouse bone marrow cells (bone marrow samples stained with isotype IgGs were used as negative control) (FIG. 8B) The percentages of human AML cells (CD45⁺/CD33⁺) in NSG mice after injection of IgG or #1NA treated as indicated in panel a (n=8 independent mice). (FIG. 8C) The percentages of human T cells in NSG mice treated as indicated in panel a (n=8 independent mice). (FIG. 8D) Overall survival of the NSG mice treated as indicated in panel a (n=8 independent mice). (FIG. 8E) Schematic of treatment. NSG mice were transplanted with monocytic AML cells (derived from BM of NSG mice engrafted with monocytic AML patient peripheral blood samples) with treatment of IgG or anti-LILRB3 #1 LALAPG. (FIG. 8F) Flow cytometry analyses of LILRB3 expression on human AML cells in the mouse bone marrow. (FIG. 8G) The percentages of human AML cells (CD45⁺/CD33⁺) in NSG mice after treatment of IgG or #1NA as indicated in panel e. (n=5 independent mice). (FIG. 8H) The percentages of human T cells in NSG mice treated as indicated in panel e (n=5 independent mice). (FIG. 8I), Survival of NSG mice treated as indicated in panel e (n=5 independent mice). The data are presented as mean ± s.e.m, and p values were calculated by two-tailed t-test except for d and i by log-rank test.
**FIGS. S1A-K.** LILRB3 enhances AML cell survival and promotes monocytic AML progression. (FIG. S1a) Analysis of LILRB3 and LILRB4 expression in patient AML samples (n=35) as determined by flow cytometry. (FIG. S1b) The cell death of THP-1 cells cultured with coated anti-LILRB3 or IgG in presence of DMSO, ABT199 (1 µM) or AZA (10 µM). (n=3 wells). (FIG. S1c) Knockdown of LILRB3 in AML cell lines does not affect cell growth in culture (n=3 wells). (FIG. S1d) THP-1 cells expressing Tet-on Cre and loxp U6 driven shRNAs were treated with Dox (1 µg/ml) for one day, and surface LILRB3 expression was analyzed by flow cytometry one week later. (FIG. S1e) Percentages of dead cells in AML cultures treated with anti-LILRB3 antibody or IgG in the presence of different concentrations of TNFα (n=3 wells). (FIG. S1f) Percentages of dead cells in THP-1 cells treated with anti-LILRB3 antibody or IgG in the presence of anti-TNFα (5 µg/ml) or control IgG (n=3 wells). (FIG. S1g) Percentages of GFP⁺ AML cells in peripheral blood (PB), bone marrow (BM), spleen (SPL), and liver of mice transplanted with C1498 AML cells expressing B3-FL or B3del ICD (n=4 mice). (FIG. S1h) Survival curve of the mice treated as in FIG. S1e. (FIG. S6a) THP-1 cell growth in plates coated anti-LILRB3 or IgG (n=3 wells). (FIG. S1j) Serial colony-forming unit (CFU) replating with MLL-AF9 mouse AML cells (n=3 wells). (FIG. S1k) Percentages of dead cells in U937 cells overexpressing LILRB3 or a control vector (n=3 wells). The data are presented as mean ± s.e.m, and p values were calculated by two-tailed t-test except for h by log-rank test.
**FIGS. S2A-C.** LILRB3 increases the survival of monocytic AML cells against cytotoxic T cells. (FIG. S7a) Percentages of CD4 and CD8 T cells in spleens of mice injected with mouse IgG or anti-mCD8 (10 mg/kg). (FIG. S2b) CFU assays (MethoCult^{™} GF M3434 ) of regular BM cells mixed with mouse T cells specific to MLL-AF9 AML cells (T-AF9) or non-specific T cells (T-LPS) (n=3 wells). (FIG. S2C) Expression of INFγ, TNFα and PD-1 on CD4 and CD8 T cells from spleens of mice engrafted with MLL-AF9 AML cells expressing LILRB3 FL or LILRB3 with intracellular domain truncation.
**FIGS. S3A-C.** LILRB3 enhances NF-κB signaling but not JNK signaling. (FIG. S3a) KEGG analysis of the top 20 processes affected by LILRB3 in mouse MLL-AF9 AML cells with whole-genome RNA-seq analysis. RNA was isolated from mouse MLL-AF9 AML cells expressing B3-FL or B3del ICD. "Down" and "Up" indicate genes expressed at lower or higher levels in AML cells that express B3del ICD versus those that express B3-FL. (FIG. S3b) GSEA of the correlation between NF-κB signaling and LILRB3 in mouse MLL-AF9 AML cells (p values were calculated by Kolmogorov Smirnov (K-S) test in GSEA analysis). (FIG. S3c) LILRB3 does not enhance the JNK signaling. GSEA of gene expression in THP-1 cells cultured in plates coated with anti-LILRB3 antibody or IgG.
**FIGS. S4A-G.** TRAF2 and cFLIP interact, stimulate NF-κB signaling, and increase resistance of AML cells to the killing of cytotoxic T cells. (FIG. S4a) Relative NF-xB activities in 293T cells co-transfected with NF-κB reporter plus empty vector, p22-FLIP, p43-CFLIP, or full-length cFLIP (n=3 wells). (FIG. S4b) Relative NF-κB activities in 293T cells co-transfected with NF-κB reporter plus empty vector or tet-on cFLIP in the presence of dox (n=3 wells). (FIG. S4c) Co-immunoprecipitation assay of exogenous expressed FLAG-cFLIP and HA-TRAF2 in 293T cells. (FIGS. S4d-e) Overexpression of TRAF2 and cFLIP increase the resistance of monocytic AML cells to cytotoxic T cells. CFSE-stained THP-1 cells that overexpress TRAF2 or empty vector (EV) (FIG. S4d) or cFLIP or empty vector (FIG. S4e) were co-cultured with activated T cells at the different ratios for 12 hours and cell death was quantified. Left: Plots of percentage of dead cells versus E:T ration. Right: FACS analyses with E:T ratio of 2 (n=3 wells). (FIG. S4f) West blotting of pMLKL (pS358) and MLKL in THP-1 cells treated with coated IgG or anti-LILRB3 for 12 hours. (FIG. S4g) Percentages of dead cells in THP-1 cells treated with anti-LILRB3 antibody or IgG in the presence of DMSO or NF-κB inhibitor QNZ (10 µM) (n=3 wells). The data are presented as mean ± s.e.m, and p values were calculated by two-tailed t-test.
**FIGS. S5A-E.** LILRB3 balances NF-κB signaling with TRAF2 and SHP1/2. (FIG. S5a) Relative luciferase activity from THP-1-Lucia^{™} cells at different times after activation with anti-LILRB3 antibody or IgG (n=3 individual samples). (FIG. S5b) TRAF2 mRNA levels in AML cell lines and normal monocytes (n=3 wells) (FIG. S5c) The percentage of GFP⁺ MLL-AF9 AML cells (with PirB knockout) expressing B3-FL or B3del ICD in peripheral blood (PB), bone marrow (BM), spleen (SPL), and liver in mice treated with PBS or LPS (n=4 mice). (FIG. S5d) Survival of mice engrafted with AML cells as treated in panel d (n=4 mice). The data are presented as mean ± s.e.m, and p values were calculated by two-tailed t-test except for FIG. 1S0e by log-rank test. (FIG. S5e) Mechanistic scheme of LILRB3 signaling. Without ligand-induced crosslinking of LILRB3, TRAF2 remains associated with LILRB3 but does not stimulate downstream signaling. When NF-κB signaling is at a low level, upon ligand-induced crosslinking of LILRB3, TRAF2 recruits cFLIP, and cFLIP is cleaved to p22-FLIP by caspase 8 (whose activity can be inhibited by zVAD-FMK). p22-FLIP binds to the IKK complex and stimulates NF-κB signaling. Meanwhile, after ligand binding to LILRB3, the ITIMs of LILRB3 are phosphorylated, which recruits SHP-1 and SHP-2. When there is a high level of NF-κB signaling stimulated by other cues (*e.g.,* LPS), higher expression of cFLIP and A20 (TNFAIP3) is induced. Increased cFLIP inhibits caspase 8 activity, and A20 disrupts the interaction between TRAF2 and LILRB3. Thus the inhibitory effect of LILRB3 on NF-κB signaling mediated by SHPs becomes dominant.
**FIGS. S6A-J.** Development of anti-LILRB3 blocking antibodies for suppressing AML development. (FIG. S6a) Upper: Flow chart of strategy for development of fully humanized antibodies against LILRB3. Lower: The identified antibodies were tested in the LILRB3 chimeric receptor reporter cell assay. (FIG. S6b) ELISA results for LILRB3 binders. (FIG. S6c) EC50 values of the anti-LILRB3 antibodies based on ELISA. (FIG. S6d) Affinities of antibodies #32, #33, #67, and #45 to LILRB3 as determined by Octet. (FIG. S6e) Cross-reactivity of the anti-LILRB3 antibodies with LILRAs evaluated with LILRA binding analyses. (FIG. S6f) Cross-reactivity of the anti-LILRB3 antibodies with other LILRBs evaluated with LILRB binding analyses. (FIG. S6g) Interaction with TRAF2 contributes to the effect of LILRB3 on AML development. CFU assay of MLL-AF9 AML cells expressing wild-type LILRB3 or mutant LILRB3 with mutations disrupting TRAF2 binding (AAA, QEE509-511AAA) or disrupting SHP-1/2 interactions (Y596/626F) in the presence of control or anti-LILRB3 antibodies (n=3 wells). (FIG. S6h) Evaluation of the effect of Fc-mediated effector functions of anti-LILRB3. Upper: Schematic of treatment. Lower: Percentages of GFP+ MLL-AF9 mouse AML cells in PB, BM, SPL and LV of mice transplanted with AML cells expressing B3-FL or B3delICD and injected with IgG or anti-LILRB3 #6 (n=4 mice). (FIG. S6i) Survival of mice as treated in panel FIG. S6h. (FIG. S6j) NF-κB signaling target gene expression (measured by qPCR) in THP-1 cells from BM of xenografted NSG mice treated with anti-LILRB3 #1NA or IgG (n=3 individual samples). The data are presented as mean ± s.e.m, and p values were calculated by two-tailed t-test except for FIG. S6i by log-rank test.
**FIGS. S7A-C.** Anti-LILRB3 #1N297A antibody did not affect normal hematopoiesis and leukocytosis. (FIG. S7a) Schematic of generation of myeloid-specific LILRB3 transgenic mice. (FIGS. S7b-c) LILRB3 is expressed on myeloid cells in peripheral blood (**PB**), spleen (**SPL**) and bone marrow (**BM**) of LysM-Cre driven LILRB3 transgenic mice, which were treated by anti-LILRB3 #1 antibody (n=2 mice) or IgG (n=3 mice). Shown are representative flow cytometry plots (FIG. S7b) and result summary (FIG. S7c).
**FIG. S8.** THP-1 cells were cultured in 96-well plate treated with coated anti-LILRB3 or IgG, and the cells were then treated with DMSO, ABT199 (1 µM) or AZA (10 µM). The cell death was detected as PI positive 24 hours later.
**FIG. S9.** THP-1 NF-κB luciferase reporter cells were infected with lentivirus and treated with coated anti-LILRB3 or IgG. The luciferase activity of NF-κB reporter cells was measured at indicated time points.
**FIGS. S10A-B.** Knockdown of LILRB3 in AML cell lines does not affect cell growth (FIG. S10A) and survival (FIG. S10B) in culture.
**FIGS. S11A1/2-B**. LILRB3 and PirB expression patterns and LILRB3-transgenic mice. (FIG. S11A1/2) The expression of LILRB3 in human tissues and PirB in mouse tissues. (FIG. S11B). LILRB3 is expressed on myeloid cells of LysM-Cre driven LILRB3 transgenic mice, which were treated by anti-LILRB3 #1 antibody or IgG.
**FIG. S12.** THP-1 cells were treated with LPS or PBS and surface LILRB3 expression was detected by flow cytometry

### DESCRIPTION OF ILLUSTRATIVE EMBODIMENTS

Targeted therapy may induce rapid tumor regression, whereas immunotherapy may achieve long-lasting anti-tumor effects. Thus, it would be ideal to identify molecular targets that enable the combination of the strengths of targeted therapy and immunotherapy. The function of leukocyte immunoglobulin-like receptor family (LILRBs) that are expressed on both immune cells and leukemia cells remain to be understood.

The present disclosure relates to LILRB3, which is expressed on some myeloid leukemia, B lymphoid leukemia, and myeloma cells (Pfistershammer *et al.,* 2009). It is reportedly co-expressed with stem cell marker CD34 and with myeloma marker CD138 (Pfistershammer *et al.,* 2009). In this study, the inventors found LILRB3 expression on monocytic AML cells enhanced the survival of these leukemia cells in the presence or absence of cytotoxic T lymphocytes (CTLs) by recruiting TRAF2 and cFLIP to stimulate NF-κB activity. They also showed that blockade of LILRB3 signaling with antagonizing antibodies increased leukemia cell death and the cytotoxic effects of CTLs. Accordingly, LILRB3 is a potential target for treatment of AML and possibly other cancers. The present disclosure also provides methods of identifying LILRB3 antagonists (*e.g*., anti-LILRB3 antibodies). Thus, the methods and compositions of the present disclosure provide methods of identifying galectin-induced LILRB3 activation and their use thereof in the treatment of cancer, specifically AML.

The following description of the disclosure is merely intended to illustrate various embodiments of the disclosure. As such, the specific modifications discussed are not to be construed as limitations on the scope of the disclosure. It will be apparent to one skilled in the art that various equivalents, changes, and modifications may be made without departing from the scope of the disclosure, and it is understood that such equivalent embodiments are to be included herein. All references cited herein, including publications, patents and patent applications are incorporated herein by reference in their entirety.

### I. Definitions

It is to be understood that both the foregoing general description and the following detailed description are exemplary and explanatory only and are not restrictive of the invention as claimed. In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise. Also, the use of the term "portion" can include part of a moiety or the entire moiety.

As used herein, the singular forms "a", "an" and "the" include plural references unless the context clearly dictates otherwise.

The term "about" as used herein when referring to a measurable value such as an amount, a temporal duration, and the like, is meant to encompass variations of up to ±10% from the specified value. Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the disclosed subject matter. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements.

The term "antibody" refers to an intact immunoglobulin of any isotype, or a fragment thereof that can compete with the intact antibody for specific binding to the target antigen, and includes, for instance, chimeric, humanized, fully human, and bispecific antibodies. An "antibody" is a species of an antigen binding protein. An intact antibody will generally comprise at least two full-length heavy chains and two full-length light chains, but in some instances can include fewer chains such as antibodies naturally occurring in camelids which can comprise only heavy chains. Antibodies can be derived solely from a single source, or can be "chimeric," that is, different portions of the antibody can be derived from two different antibodies as described further below. The antigen binding proteins, antibodies, or binding fragments can be produced in hybridomas, by recombinant DNA techniques, or by enzymatic or chemical cleavage of intact antibodies. Unless otherwise indicated, the term "antibody" includes, in addition to antibodies comprising two full-length heavy chains and two full-length light chains, derivatives, variants, fragments, and muteins thereof, examples of which are described below. Furthermore, unless explicitly excluded, antibodies include monoclonal antibodies, bispecific antibodies, minibodies, domain antibodies, synthetic antibodies (sometimes referred to herein as "antibody mimetics"), chimeric antibodies, humanized antibodies, human antibodies, antibody fusions (sometimes referred to herein as "antibody conjugates"), and fragments thereof, respectively. In some embodiments, the term also encompasses peptibodies.

Naturally occurring antibody structural units typically comprise a tetramer. Each such tetramer typically is composed of two identical pairs of polypeptide chains, each pair having one full-length "light" (in certain embodiments, about 25 kDa) and one full-length "heavy" chain (in certain embodiments, about 50-70 kDa). The amino-terminal portion of each chain typically includes a variable region of about 100 to 110 or more amino acids that typically is responsible for antigen recognition. The carboxy-terminal portion of each chain typically defines a constant region that can be responsible for effector function. Human light chains are typically classified as kappa and lambda light chains. Heavy chains are typically classified as mu, delta, gamma, alpha, or epsilon, and define the antibody's isotype as IgM, IgD, IgG, IgA, and IgE, respectively. IgG has several subclasses, including, but not limited to, IgG1, IgG2, IgG3, and IgG4. IgM has subclasses including, but not limited to, IgM1 and IgM2. IgA is similarly subdivided into subclasses including, but not limited to, IgA1 and IgA2. Within full-length light and heavy chains, typically, the variable and constant regions are joined by a "J" region of about 12 or more amino acids, with the heavy chain also including a "D" region of about 10 more amino acids. See, *e.g*., Fundamental Immunology, Ch. 7 (Paul, W., ed., 2nd ed. Raven Press, N.Y. (1989)) (incorporated by reference in its entirety for all purposes). The variable regions of each light/heavy chain pair typically form the antigen binding site.

The term "variable region" or "variable domain" refers to a portion of the light and/or heavy chains of an antibody, typically including approximately the amino-terminal 120 to 130 amino acids in the heavy chain and about 100 to 110 amino terminal amino acids in the light chain. In certain embodiments, variable regions of different antibodies differ extensively in amino acid sequence even among antibodies of the same species. The variable region of an antibody typically determines specificity of a particular antibody for its target.

The variable regions typically exhibit the same general structure of relatively conserved framework regions (FR) joined by three hyper variable regions, also called complementarity determining regions or CDRs. The CDRs from the two chains of each pair typically are aligned by the framework regions, which can enable binding to a specific epitope. From N-terminal to C-terminal, both light and heavy chain variable regions typically comprise the domains FR1, CDR1, FR2, CDR2, FR3, CDR3 and FR4. The assignment of amino acids to each domain is typically in accordance with the definitions of Kabat Sequences of Proteins of Immunological Interest (National Institutes of Health, Bethesda, Md. (1987 and 1991)), Chothia & Lesk, J. Mol. Biol., 196:901-917 (1987) or Chothia et al., Nature, 342:878-883 (1989).

In certain embodiments, an antibody heavy chain binds to an antigen in the absence of an antibody light chain. In certain embodiments, an antibody light chain binds to an antigen in the absence of an antibody heavy chain. In certain embodiments, an antibody binding region binds to an antigen in the absence of an antibody light chain. In certain embodiments, an antibody binding region binds to an antigen in the absence of an antibody heavy chain. In certain embodiments, an individual variable region specifically binds to an antigen in the absence of other variable regions.

In certain embodiments, definitive delineation of a CDR and identification of residues comprising the binding site of an antibody is accomplished by solving the structure of the antibody and/or solving the structure of the antibody-ligand complex. In certain embodiments, that can be accomplished by any of a variety of techniques known to those skilled in the art, such as X-ray crystallography. In certain embodiments, various methods of analysis can be employed to identify or approximate the CDR regions. Examples of such methods include, but are not limited to, the Kabat definition, the Chothia definition, the AbM definition and the contact definition.

The Kabat definition is a standard for numbering the residues in an antibody and is typically used to identify CDR regions. See, *e.g*., Johnson & Wu, Nucleic Acids Res., 28: 214-8 (2000). The Chothia definition is similar to the Kabat definition, but the Chothia definition takes into account positions of certain structural loop regions. See, *e.g.,* Chothia et al., J. Mol. Biol., 196: 901-17 (1986); Chothia et al., Nature, 342: 877-83 (1989). The AbM definition uses an integrated suite of computer programs produced by Oxford Molecular Group that model antibody structure. See, *e.g.,* Martin et al., Proc Natl Acad Sci (USA), 86:9268-9272 (1989); "AbM^{™}, A Computer Program for Modeling Variable Regions of Antibodies," Oxford, UK; Oxford Molecular, Ltd. The AbM definition models the tertiary structure of an antibody from primary sequence using a combination of knowledge databases and ab initio methods, such as those described by Samudrala et al., "Ab Initio Protein Structure Prediction Using a Combined Hierarchical Approach," in PROTEINS, Structure, Function and Genetics Suppl., 3:194-198 (1999). The contact definition is based on an analysis of the available complex crystal structures. See, *e.g.,* MacCallum et al., J. Mol. Biol., 5:732-45 (1996).

By convention, the CDR regions in the heavy chain are typically referred to as H1, H2, and H3 and are numbered sequentially in the direction from the amino terminus to the carboxy terminus. The CDR regions in the light chain are typically referred to as L1, L2, and L3 and are numbered sequentially in the direction from the amino terminus to the carboxy terminus.

The term "light chain" includes a full-length light chain and fragments thereof having sufficient variable region sequence to confer binding specificity. A full-length light chain includes a variable region domain, VL, and a constant region domain, CL. The variable region domain of the light chain is at the amino-terminus of the polypeptide. Light chains include kappa chains and lambda chains.

The term "heavy chain" includes a full-length heavy chain and fragments thereof having sufficient variable region sequence to confer binding specificity. A full-length heavy chain includes a variable region domain, VH, and three constant region domains, CH1, CH2, and CH3. The VH domain is at the amino-terminus of the polypeptide, and the CH domains are at the carboxyl-terminus, with the CH3 being closest to the carboxy-terminus of the polypeptide. Heavy chains can be of any isotype, including IgG (including IgG1, IgG2, IgG3 and IgG4 subtypes), IgA (including IgA1 and IgA2 subtypes), IgM and IgE.

A bispecific or bifunctional antibody typically is an artificial hybrid antibody having two different heavy/light chain pairs and two different binding sites. Bispecific antibodies can be produced by a variety of methods including, but not limited to, fusion of hybridomas or linking of Fab' fragments. See, *e.g.,* Songsivilai et al., Clin. Exp. Immunol., 79: 315-321 (1990); Kostelny et al., J. Immunol., 148:1547-1553 (1992).

The term "antigen" refers to a substance capable of inducing adaptive immune responses. Specifically, an antigen is a substance which serves as a target for the receptors of an adaptive immune response. Typically, an antigen is a molecule that binds to antigen-specific receptors but cannot induce an immune response in the body by itsself. Antigens are usually proteins and polysaccharides, less frequently also lipids. Suitable antigens include without limitation parts of bacteria (coats, capsules, cell walls, flagella, fimbrai, and toxins), viruses, and other microorganisms. Antigens also include tumor antigens, *e.g*., antigens generated by mutations in tumors. As used herein, antigens also include immunogens and haptens.

An "antigen binding protein" ("ABP") as used herein means any protein that binds a specified target antigen. In the instant application, the specified target antigen is the LILRB protein or fragment thereof. "Antigen binding protein" includes but is not limited to antibodies and antigen-binding fragment thereof. Peptibodies are another example of antigen binding proteins.

The term "antigen-binding fragment" as used herein refers to a portion of a protein which is capable of binding specifically to an antigen. In certain embodiment, the antigen-binding fragment is derived from an antibody comprising one or more CDRs, or any other antibody fragment that binds to an antigen but does not comprise an intact native antibody structure. In certain embodiments, the antigen-binding fragment is not derived from an antibody but rather is derived from a receptor. Examples of antigen-binding fragment include, without limitation, a diabody, a Fab, a Fab', a F(ab')₂, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), an scFv dimer (bivalent diabody), a multispecific antibody, a single domain antibody (sdAb), a camelid antibody or a nanobody, a domain antibody, and a bivalent domain antibody. In certain embodiments, an antigen-binding fragment is capable of binding to the same antigen to which the parent antibody binds. In certain embodiments, an antigen-binding fragment may comprise one or more CDRs from a particular human antibody grafted to a framework region from one or more different human antibodies. In certain embodiments, the antigen-binding fragment is derived from a receptor and contains one or more mutations. In certain embodiments, the antigen-binding fragment does not bind to the natural ligand of the receptor from which the antigen-binding fragment is derived.

A "Fab fragment" comprises one light chain and the CH1 and variable regions of one heavy chain. The heavy chain of a Fab molecule cannot form a disulfide bond with another heavy chain molecule.

A "Fab' fragment" comprises one light chain and a portion of one heavy chain that contains the VH domain and the CH1 domain and also the region between the CH1 and CH2 domains, such that an interchain disulfide bond can be formed between the two heavy chains of two Fab' fragments to form an F(ab')₂ molecule.

A "F(ab')₂ fragment" contains two light chains and two heavy chains containing a portion of the constant region between the CH1 and CH2 domains, such that an interchain disulfide bond is formed between the two heavy chains. A F(ab')₂ fragment thus is composed of two Fab' fragments that are held together by a disulfide bond between the two heavy chains.

An "Fc" region comprises two heavy chain fragments comprising the CH1 and CH2 domains of an antibody. The two heavy chain fragments are held together by two or more disulfide bonds and by hydrophobic interactions of the CH3 domains.

The "Fv region" comprises the variable regions from both the heavy and light chains but lacks the constant regions.

"Single-chain antibodies" are Fv molecules in which the heavy and light chain variable regions have been connected by a flexible linker to form a single polypeptide chain, which forms an antigen binding region. Single chain antibodies are discussed in detail in International Patent Application Publication No. WO 88/01649 and U.S. Pat. No. 4,946,778 and No. 5,260,203, the disclosures of which are incorporated by reference.

A "domain antibody" is an immunologically functional immunoglobulin fragment containing only the variable region of a heavy chain or the variable region of a light chain. In some instances, two or more VH regions are covalently joined with a peptide linker to create a bivalent domain antibody. The two VH regions of a bivalent domain antibody can target the same or different antigens.

A "bivalent antigen binding protein" or "bivalent antibody" comprises two antigen binding sites. In some instances, the two binding sites have the same antigen specificities. Bivalent antigen binding proteins and bivalent antibodies can be bispecific, see, infra. A bivalent antibody other than a "multispecific" or "multifunctional" antibody, in certain embodiments, typically is understood to have each of its binding sites identical.

A "multispecific antigen binding protein" or "multispecific antibody" is one that targets more than one antigen or epitope.

A "bispecific," "dual-specific" or "bifunctional" antigen binding protein or antibody is a hybrid antigen binding protein or antibody, respectively, having two different antigen binding sites. Bispecific antigen binding proteins and antibodies are a species of multispecific antigen binding protein antibody and can be produced by a variety of methods including, but not limited to, fusion of hybridomas or linking of Fab' fragments. See, *e.g.,* Songsivilai and Lachmann, 1990, Clin. Exp. Immunol. 79:315-321; Kostelny et al., 1992, J. Immunol. 148:1547-1553. The two binding sites of a bispecific antigen binding protein or antibody will bind to two different epitopes, which can reside on the same or different protein targets.

"Binding affinity" generally refers to the strength of the sum total of noncovalent interactions between a single binding site of a molecule (*e.g.,* an antibody) and its binding partner (*e.g.,* an antigen). Unless indicated otherwise, as used herein, "binding affinity" refers to intrinsic binding affinity that reflects a 1:1 interaction between members of a binding pair (*e.g*., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative and exemplary embodiments for measuring binding affinity are described in the following.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope. For example, the LILRB3 specific antibodies of the present invention are specific to LILRB3. In some embodiments, the antibody that binds to LILRB3 has a dissociation constant (Kd) of ≦100 nM, ≦10 nM, ≦1 nM, ≦0.1 nM, ≦0.01 nM, or ≦0.001 nM (*e.g.,* 10⁻⁸M or less, *e.g.,* from 10⁻⁸M to 10⁻¹³M, *e.g.,* from 10⁻⁹M to 10⁻¹³ M). The dissociation constant Kd used herein refers to the ratio of the dissociation rate to the association rate (k_{off}/kₒₙ), which may be determined by using any conventional method known in the art, including but are not limited to surface plasmon resonance method, microscale thermophoresis method, HPLC-MS method and flow cytometry (such as FACS) method. In certain embodiments, the Kd value can be appropriately determined by using flow cytometry.

The term "compete" when used in the context of antigen binding proteins (*e.g*., antibody or antigen-binding fragment thereof) that compete for the same epitope means competition between antigen binding proteins as determined by an assay in which the antigen binding protein (*e.g*., antibody or antigen-binding fragment thereof) being tested prevents or inhibits (*e.g.,* reduces) specific binding of a reference antigen binding protein (*e.g.,* a ligand, or a reference antibody) to a common antigen (*e.g*., LILRB or a fragment thereof). Numerous types of competitive binding assays can be used to determine if one antigen binding protein competes with another, for example: solid phase direct or indirect radioimmunoassay (RIA), solid phase direct or indirect enzyme immunoassay (EIA), sandwich competition assay (see, *e.g.,* Stahli et al., 1983, Methods in Enzymology 9:242-253); solid phase direct biotin-avidin EIA (see, *e.g.,* Kirkland et al., 1986, J. Immunol. 137:3614-3619) solid phase direct labeled assay, solid phase direct labeled sandwich assay (see, *e.g*., Harlow and Lane, 1988, Antibodies, A Laboratory Manual, Cold Spring Harbor Press); solid phase direct label RIA using 1-125 label (see, *e.g.,* Morel et al., 1988, Molec. Immunol. 25:7-15); solid phase direct biotin-avidin EIA (see, *e.g.,* Cheung, et al., 1990, Virology 176:546-552); and direct labeled RIA (Moldenhauer et al., 1990, Scand. J. Immunol. 32:77-82). Typically, such an assay involves the use of purified antigen bound to a solid surface or cells bearing either of these, an unlabelled test antigen binding protein and a labeled reference antigen binding protein. Competitive inhibition is measured by determining the amount of label bound to the solid surface or cells in the presence of the test antigen binding protein. Usually the test antigen binding protein is present in excess. Antigen binding proteins identified by competition assay (competing antigen binding proteins) include antigen binding proteins binding to the same epitope as the reference antigen binding proteins and antigen binding proteins binding to an adjacent epitope sufficiently proximal to the epitope bound by the reference antigen binding protein for steric hindrance to occur. Additional details regarding methods for determining competitive binding are provided in the examples herein. Usually, when a competing antigen binding protein is present in excess, it will inhibit (*e.g*., reduce) specific binding of a reference antigen binding protein to a common antigen by at least 40-45%, 45-50%, 50-55%, 55-60%, 60-65%, 65-70%, 70-75% or 75% or more. In some instances, binding is inhibited by at least 80-85%, 85-90%, 90-95%, 95-97%, or 97% or more.

The term "epitope" as used herein refers to the specific group of atoms or amino acids on an antigen to which an antibody binds. The epitope can be either linear epitope or a conformational epitope. A linear epitope is formed by a continuous sequence of amino acids from the antigen and interacts with an antibody based on their primary structure. A conformational epitope, on the other hand, is composed of discontinuous sections of the antigen's amino acid sequence and interacts with the antibody based on the 3D structure of the antigen. In general, an epitope is approximately five or six amino acid in length. Two antibodies may bind the same epitope within an antigen if they exhibit competitive binding for the antigen.

A "cell", as used herein, can be prokaryotic or eukaryotic. A prokaryotic cell includes, for example, bacteria. A eukaryotic cell includes, for example, a fungus, a plant cell, and an animal cell. The types of an animal cell (*e.g.,* a mammalian cell or a human cell) includes, for example, a cell from circulatory/immune system or organ, *e.g.,* a B cell, a T cell (cytotoxic T cell, natural killer T cell, regulatory T cell, T helper cell), a natural killer cell, a granulocyte (*e.g*., basophil granulocyte, an eosinophil granulocyte, a neutrophil granulocyte and a hypersegmented neutrophil), a monocyte or macrophage, a red blood cell (*e.g.,* reticulocyte), a mast cell, a thrombocyte or megakaryocyte, and a dendritic cell; a cell from an endocrine system or organ, *e.g.,* a thyroid cell (*e.g.,* thyroid epithelial cell, parafollicular cell), a parathyroid cell (*e.g.,* parathyroid chief cell, oxyphil cell), an adrenal cell (*e.g.,* chromaffin cell), and a pineal cell (*e.g.,* pinealocyte); a cell from a nervous system or organ, *e.g.,* a glioblast (*e.g*., astrocyte and oligodendrocyte), a microglia, a magnocellular neurosecretory cell, a stellate cell, a boettcher cell, and a pituitary cell (*e.g.,* gonadotrope, corticotrope, thyrotrope, somatotrope, and lactotroph); a cell from a respiratory system or organ, *e.g.,* a pneumocyte (a type I pneumocyte and a type II pneumocyte), a clara cell, a goblet cell, and an alveolar macrophage; a cell from circular system or organ (*e.g*., myocardiocyte and pericyte); a cell from digestive system or organ, *e.g.,* a gastric chief cell, a parietal cell, a goblet cell, a paneth cell, a G cell, a D cell, an ECL cell, an I cell, a K cell, an S cell, an enteroendocrine cell, an enterochromaffin cell, an APUD cell, and a liver cell (*e.g.,* a hepatocyte and Kupffer cell); a cell from integumentary system or organ, *e.g.,* a bone cell (*e.g.,* an osteoblast, an osteocyte, and an osteoclast), a teeth cell (*e.g.,* a cementoblast, and an ameloblast), a cartilage cell (*e.g.*, a chondroblast and a chondrocyte), a skin/hair cell (*e.g.,* a trichocyte, a keratinocyte, and a melanocyte (Nevus cell), a muscle cell (*e.g.,* myocyte), an adipocyte, a fibroblast, and a tendon cell; a cell from urinary system or organ (*e.g.,* a podocyte, a juxtaglomerular cell, an intraglomerular mesangial cell, an extraglomerular mesangial cell, a kidney proximal tubule brush border cell, and a macula densa cell); and a cell from reproductive system or organ (*e.g.,* a spermatozoon, a Sertoli cell, a leydig cell, an ovum, an oocyte). A cell can be normal, healthy cell; or a diseased or unhealthy cell (*e.g.,* a cancer cell). A cell further includes a mammalian zygote or a stem cell which include an embryonic stem cell, a fetal stem cell, an induced pluripotent stem cell, and an adult stem cell. A stem cell is a cell that is capable of undergoing cycles of cell division while maintaining an undifferentiated state and differentiating into specialized cell types. A stem cell can be an omnipotent stem cell, a pluripotent stem cell, a multipotent stem cell, an oligopotent stem cell and a unipotent stem cell, any of which may be induced from a somatic cell. A stem cell may also include a cancer stem cell. A mammalian cell can be a rodent cell, *e.g.,* a mouse, rat, hamster cell. A mammalian cell can be a lagomorpha cell, *e.g.,* a rabbit cell. A mammalian cell can also be a primate cell, *e.g.,* a human cell.

The term "chimeric antigen receptor" or "CAR" as used herein refers to an artificially constructed hybrid protein or polypeptide containing an antigen binding domain of an antibody (*e.g.,* a single chain variable fragment (scFv)) linked to a domain or signaling, *e.g.,* T-cell signaling or T-cell activation domains, that activates an immune cell, *e.g.,* a T cell or a NK cell (see, *e.g.,* Kershaw *et al.,* supra, Eshhar et al., Proc. Natl. Acad. Sci. USA, 90(2): 720-724 (1993), and Sadelain et al., Curr. Opin. Immunol. 21(2): 215-223 (2009)). CARs are capable of redirecting the immune cell specificity and reactivity toward a selected target in a non-MHC-restricted manner, taking advantage of the antigen-binding properties of monoclonal antibodies. The non-MHC-restricted antigen recognition confers immune cells expressing CARs on the ability to recognize an antigen independent of antigen processing, thus bypassing a major mechanism of tumor escape. In addition, when expressed in T-cells, CARs advantageously do not dimerize with endogenous T-cell receptor (TCR) alpha and beta chains.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

The term "host cell" means a cell that has been transformed, or is capable of being transformed, with a nucleic acid sequence and thereby expresses a gene of interest. The term includes the progeny of the parent cell, whether or not the progeny is identical in morphology or in genetic make-up to the original parent cell, so long as the gene of interest is present.

The term "identity" refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) are preferably addressed by a particular mathematical model or computer program (*i.e*., an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAM J. Applied Math. 48:1073.

In calculating percent identity, the sequences being compared are typically aligned in a way that gives the largest match between the sequences. One example of a computer program that can be used to determine percent identity is the GCG program package, which includes GAP (Devereux et al., 1984, Nucl. Acid Res. 12:387; Genetics Computer Group, University of Wisconsin, Madison, Wis.). The computer algorithm GAP is used to align the two polypeptides or polynucleotides for which the percent sequence identity is to be determined. The sequences are aligned for optimal matching of their respective amino acid or nucleotide (the "matched span", as determined by the algorithm). A gap opening penalty (which is calculated as 3× the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm. In certain embodiments, a standard comparison matrix (see, Dayhoff et al., 1978, Atlas of Protein Sequence and Structure 5:345-352 for the PAM 250 comparison matrix; Henikoff et al., 1992, Proc. Natl. Acad. Sci. U.S.A. 89:10915-10919 for the BLOSUM 62 comparison matrix) is also used by the algorithm.

Examples of parameters that can be employed in determining percent identity for polypeptides or nucleotide sequences using the GAP program can be found in Needleman et al., 1970, J. Mol. Biol. 48:443-453.

Certain alignment schemes for aligning two amino acid sequences may result in matching of only a short region of the two sequences, and this small aligned region may have very high sequence identity even though there is no significant relationship between the two full-length sequences. Accordingly, the selected alignment method (GAP program) can be adjusted if so desired to result in an alignment that spans at least 50 or other number of contiguous amino acids of the target polypeptide.

The term "link" as used herein refers to the association via intramolecular interaction, *e.g*., covalent bonds, metallic bonds, and/or ionic bonding, or inter-molecular interaction, *e.g*., hydrogen bond or noncovalent bonds.

Leukocyte immunoglobulin-like receptor subfamily B member 2 (LILRB3) is a protein that in humans is encoded by the *LILRB3* gene. This gene is a member of the leukocyte immunoglobulin-like receptor (LIR) family, which is found in a gene cluster at chromosomal region 19q13.4. The encoded protein belongs to the subfamily B class of LIR receptors which contain two or four extracellular immunoglobulin domains, a transmembrane domain, and two to four cytoplasmic immunoreceptor tyrosine-based inhibitory motifs (ITIMs).

The term "operably linked" refers to an arrangement of elements wherein the components so described are configured so as to perform their usual function. Thus, a given signal peptide that is operably linked to a polypeptide directs the secretion of the polypeptide from a cell. In the case of a promoter, a promoter that is operably linked to a coding sequence will direct the expression of the coding sequence. The promoter or other control elements need not be contiguous with the coding sequence, so long as they function to direct the expression thereof. For example, intervening untranslated yet transcribed sequences can be present between the promoter sequence and the coding sequence and the promoter sequence can still be considered "operably linked" to the coding sequence.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

The term "polynucleotide" or "nucleic acid" includes both single-stranded and double-stranded nucleotide polymers. The nucleotides comprising the polynucleotide can be ribonucleotides or deoxyribonucleotides or a modified form of either type of nucleotide. Said modifications include base modifications such as bromouridine and inosine derivatives, ribose modifications such as 2',3'-dideoxyribose, and internucleotide linkage modifications such as phosphorothioate, phosphorodithioate, phosphoroselenoate, phosphorodiselenoate, phosphoroanilothioate, phoshoraniladate and phosphoroamidate.

The terms "polypeptide" or "protein" means a macromolecule having the amino acid sequence of a native protein, that is, a protein produced by a naturally-occurring and non-recombinant cell; or it is produced by a genetically-engineered or recombinant cell, and comprise molecules having the amino acid sequence of the native protein, or molecules having deletions from, additions to, and/or substitutions of one or more amino acids of the native sequence. The term also includes amino acid polymers in which one or more amino acids are chemical analogs of a corresponding naturally-occurring amino acid and polymers. The terms "polypeptide" and "protein" specifically encompass LILRB antigen binding proteins, antibodies, or sequences that have deletions from, additions to, and/or substitutions of one or more amino acid of antigen-binding protein. The term "polypeptide fragment" refers to a polypeptide that has an amino-terminal deletion, a carboxyl-terminal deletion, and/or an internal deletion as compared with the full-length native protein. Such fragments can also contain modified amino acids as compared with the native protein. In certain embodiments, fragments are about five to 500 amino acids long. For example, fragments can be at least 5, 6, 8, 10, 14, 20, 50, 70, 100, 110, 150, 200, 250, 300, 350, 400, or 450 amino acids long. Useful polypeptide fragments include immunologically functional fragments of antibodies, including binding domains. In the case of a LILRB-binding antibody, useful fragments include but are not limited to a CDR region, a variable domain of a heavy and/or light chain, a portion of an antibody chain or just its variable region including two CDRs, and the like.

The pharmaceutically acceptable carriers useful in this invention are conventional. Remington's Pharmaceutical Sciences, by E. W. Martin, Mack Publishing Co., Easton, PA, 15th Edition (1975), describes compositions and formulations suitable for pharmaceutical delivery of the fusion proteins herein disclosed. In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (*e.g*., powder, pill, tablet, or capsule forms) , conventional non-toxic solid carriers can include, for example, pharmaceutical grades of mannitol, lactose, starch or magnesium stearate. In addition to biologically- neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

As used herein, the term "subject" refers to a human or any non-human animal (*e.g*., mouse, rat, rabbit, dog, cat, cattle, swine, sheep, horse or primate). A human includes pre- and post-natal forms. In many embodiments, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

The term "therapeutically effective amount" or "effective dosage" as used herein refers to the dosage or concentration of a drug effective to treat a disease or condition. For example, with regard to the use of the monoclonal antibodies or antigen-binding fragments thereof disclosed herein to treat cancer, a therapeutically effective amount is the dosage or concentration of the monoclonal antibody or antigen-binding fragment thereof capable of reducing the tumor volume, eradicating all or part of a tumor, inhibiting or slowing tumor growth or cancer cell infiltration into other organs, inhibiting growth or proliferation of cells mediating a cancerous condition, inhibiting or slowing tumor cell metastasis, ameliorating any symptom or marker associated with a tumor or cancerous condition, preventing or delaying the development of a tumor or cancerous condition, or some combination thereof.

"Treating" or "treatment" of a condition as used herein includes preventing or alleviating a condition, slowing the onset or rate of development of a condition, reducing the risk of developing a condition, preventing or delaying the development of symptoms associated with a condition, reducing or ending symptoms associated with a condition, generating a complete or partial regression of a condition, curing a condition, or some combination thereof.

As used herein, a "vector" refers to a nucleic acid molecule as introduced into a host cell, thereby producing a transformed host cell. A vector may include nucleic acid sequences that permit it to replicate in the host cell, such as an origin of replication. A vector may also include one or more therapeutic genes and/or selectable marker genes and other genetic elements known in the art. A vector can transduce, transform or infect a cell, thereby causing the cell to express nucleic acids and/or proteins other than those native to the cell. A vector optionally includes materials to aid in achieving entry of the nucleic acid into the cell, such as a viral particle, liposome, protein coating or the like.

### II. LILRB3 and Related Diseases

The leukocyte immunoglobulin-like receptors (LILR) are a family of receptors possessing extracellular immunoglobulin domains. They are also known as CD85, ILTs and LIR, and can exert immunomodulatory effects on a wide range of immune cells. The human genes encoding these receptors are found in a gene cluster at chromosomal region 19q13.4. They include, LILRA1, LILRA2, LILRA3, LILRA4, LILRA5 or LILRB7, LILRA6 or LILRB6, LILRB1, LILRB2, LILRB3, LILRB4, and LILRB5. A subset of LILRs recognize MHC class I molecules (also known as HLA class I in humans). Of these, the inhibitory receptors LILRB1 and LILRB2 show a broad specificity for classical and non-classical MHC alleles with preferential binding to β2m-associated complexes. In contrast, the activating receptors LILRA1 and LILRA3 prefer b2m-independent free heavy chains of MHC class I, and in particular HLA-C alleles. For LILRs and following descriptions of LILRB1-5 and LAIR1, see review ²².

Leukocyte immunoglobulin-like receptor subfamily B member 3 is a protein that in humans is encoded by the *LILRB3* gene. This gene found in a gene cluster at chromosomal region 19q13.4. LILRB3 is restrictively expressed in myeloid cells, including monocytes, neutrophils, eosinophils, and basophils (as well as mast cells and some osteoclasts based on evidence from in vitro differentiation experiments). LILRB3 contains four cytoplasmic ITIM motifs and contributes to negative regulation of immune response. The ligand for LILRB3 is unknown, and relatively little is known about the function of LILRB3. Co-ligation of LILRB3 with activating receptors in human basophils led to inhibition of cell activation. LILRB3 was also suggested to be an inhibitor of allergic inflammation and autoimmunity. It is noteworthy that LILRB family receptors including LILRB3 are primate specific. The expression pattern and ligand of the mouse ortholog PirB for LILRB3 are different.

The inventors have demonstrated that LILRB3 expressing acute myeloid leukemia (AML) cells have increased survival and suppressed the activity of cytotoxic T lymphocytes (CTLs). Mechanistically, they found that the intracellular domain of LILRB3 is constitutively associated with the adaptor protein TRAF2, which is also high in AML cells. LILRB3 activation leads to stimulation of NF-kB signaling and enhanced AML cell survival. Activated LILRB3 can further recruit the signaling protein cFLIP via TRAF2. As full-length cFLIP can be cleaved by caspase 8 to p22-FLIP to stimulate NF-kB, activation of LILRB3 causes full-length cFLIP to be cleaved and induces NF-kB signaling. They also showed that inhibition of caspase activity prevented LILRB3 to stimulate NF-kB signaling. Upregulation of NF-kB signaling stimulated by high level of LPS disrupted the interaction of LILRB3 and TRAF2 by A20, a protein rapidly induced by high level of NF-kB. Consequently, the SHP-1/2 mediated inhibitory function of LILRB3 becomes predominant. Thus, LILRB3 can behave as a stimulator or an inhibitor of NF-kB, depending on signaling context.

In one aspect, antagonists of LILRB3 can be used to treat hyperproliferative diseases. While hyperproliferative diseases can be associated with any disease which causes a cell to begin to reproduce uncontrollably, the prototypical example is cancer. Examples of cancer can be generally categorized into solid tumors and hematologic malignancies. Solid tumors include but are not limited to, adrenal cancer, bile duct carcinoma, bone cancer, brain cancer (*e.g*., astrocytoma, brain stem glioma, craniopharyngioma, ependymoma, hemangioblastoma, medulloblastoma, meningioma, oligodendroglioma, spinal axis tumor), breast cancer (including acoustic neuroma, basal breast carcinoma, ductal carcinoma and lobular breast carcinoma), cervical cancer, choriocarcinoma, colon cancer, colorectal cancer, esophageal cancer, eye cancer, gastric cancer, glioblastoma, head and neck cancer, kidney cancer (including Wilms tumor), liver cancer (including hepatocellular carcinoma (HCC)), lung cancer (including bronchogenic carcinoma, non-small cell lung cancer (squamous/non-squamous), bronchioloalveolar cell lung cancer, papillary adenocarcinomas), mesothelioma, melanoma, merkel cell cancer, nasopharyngeal carcinoma, neuroblastoma, oral cancer, ovarian cancer, pancreatic cancer, penile cancer, pinealoma, prostate cancer, renal cell cancer, retinoblastoma, sarcoma (including chondrosarcoma, Ewing's sarcoma, fibrosarcoma, leiomyosarcoma, liposarcoma, myxosarcoma, osteogenic sarcoma, rhabdomyosarcoma, synovial sarcoma), skin cancer (including basal cell carcinoma, sebaceous gland carcinoma, squamous cell carcinoma), testicular cancer (including seminoma), thymic carcinoma, thyroid cancer (*e.g*., medullary thyroid carcinoma, papillary thyroid carcinoma), uterine cancer, and vaginal cancer.

Hematologic malignancies include but are not limited to blastic plasmacytoid dendritic cell neoplasm (BPDCN), heavy chain disease, leukemias (including but not limited to acute lymphocytic leukemia (ALL), acute myeloid leukemia (AML) (including but not limited to acute promyelocytic leukemia (APL) or M3 AML, acute myelomonocytic leukemia or M4 AML, acute monocytic leukemia or M5 AML), B-cell leukemia, chronic lymphoblastic leukemia (CLL), chronic myelomonocytic leukemia (CMML), chronic myelocytic leukemia (CML), pre-B acute lymphocytic leukemia (Pre-B ALL), diffuse large B-cell lymphoma (DLBCL), extranodal NK/T-cell lymphoma, hairy cell leukemia, HHV8-associated primary effusion lymphoma, plasmablastic lymphoma, primary CNS lymphoma, primary mediastinal large B-cell lymphoma, T-cell/histiocyte-rich B-cell lymphoma), lymphomas (including but not limited to Hodgkin's lymphoma, non-Hodgkin's lymphoma, Waldenstrom's macroglobulinemia), multiple myeloma (MM), myelodysplastic syndromes (MDS), myeloproliferative neoplasms, and polycythemia vera.

Immunotherapy holds great promise to achieve long-lasting anti-tumor effects. Immune checkpoint PD-1 and CTLA-4 blockade therapies have been successful in treating some types of cancers but not others. These immunotherapies target inhibitory molecules on T cells to reactivate dysfunctional T cells within the tumor microenvironment (TME). Other populations of immune cells, including monocytic cells, are present in the TME in even larger numbers than T cells. In fact, monocyte-derived macrophages are the most abundant immune cell population in tumor tissues. While these innate cells possess the capacity to kill tumor cells and to prime or reactivate T cells, they become dysfunctional in TME and turn into MDSCs and tumor-associated macrophages (TAMs) that support tumor development and suppress immune surveillance and attack. MDSCs, including monocytic MDSCs (M-MDSCs) and polymorphonuclear MDSCs (PMN-MDSCs), represent a heterogeneous population of immature myeloid cells that fail to terminally differentiate. TAMs are a mixed macrophage population in TME. They are anti-inflammatory and correlated with a poor prognosis. Despite their phenotypic plasticity, MDSCs and TAMs are defined by their immunosuppressive function. Removing, reprogramming, or blocking trafficking of these immune-suppressive monocytic cells is becoming an attractive anti-cancer therapeutic strategy.

Therapeutic activating or agonizing LILRB3 has the potential to treat autoimmune or inflammatory diseases. Autoimmune or inflammatory diseases include, but are not limited to, Acquired Immunodeficiency Syndrome (AIDS, which is a viral disease with an autoimmune component), alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigold, cold agglutinin disease, crest syndrome, Crohn's disease, Degos' disease, dermatomyositis-juvenile, discoid lupus, essential mixed cryoglobulinemia, fibromyalgia-fibromyositis, Graves' disease, Guillain-Barre syndrome, Hashimoto's thyroiditis, idiopathic pulmonary fibrosis, idiopathic thrombocytopenia purpura (ITP), IgA nephropathy, insulin-dependent diabetes mellitus, juvenile chronic arthritis (Still's disease), juvenile rheumatoid arthritis, Meniere's disease, mixed connective tissue disease, multiple sclerosis, myasthenia gravis, pemacious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma, systemic scleroderma, progressive systemic sclerosis (PSS), systemic sclerosis (SS), Sjogren's syndrome, stiff-man syndrome, systemic lupus erythematosus (SLE), Takayasu arteritis, temporal arteritis/giant cell arteritis, inflammatory bowel disease (IBD), ulcerative colitis, Cohn's disease, intestinal mucosal inflammation, wasting disease associated with colitis, uveitis, vitiligo and Wegener's granulomatosis, Alzheimer's disease, asthma, atopic allergy, allergy, atherosclerosis, bronchial asthma, eczema, glomerulonephritis, graft vs. host disease, hemolytic anemias, osteoarthritis, sepsis, stroke, transplantation of tissue and organs, vasculitis, diabetic retinopathy, ventilator induced lung injury, viral infections, autoimmune diabetes and the like. Inflammatory disorders include, for example, chronic and acute inflammatory disorders.

### III. Monoclonal Antibodies and Production Thereof

The monoclonal antibodies described herein can be prepared using standard methods, followed by screening, characterization and functional assessment. Variable regions can be sequenced and then subcloned into a human expression vector to produce the chimeric antibody genes, which are then expressed and purified. These chimeric antibodies can be tested for antigen binding, signaling blocking, and in xenograft experiments. The monoclonal antibodies described herein can also be prepared using phage display method, in which a large library of phage displayed human scFv is panned against the target protein. The human scFv selected to specifically binding to the target protein can be sequenced and then subcloned into a human expression vector to produce the desired human antibody.

### A. General Methods

It will be understood that monoclonal antibodies binding to LILRB3 will have several applications. These include the production of diagnostic kits for use in detecting and diagnosing cancer, as well as for cancer therapies. In these contexts, one may link such antibodies to diagnostic or therapeutic agents, use them as capture agents or competitors in competitive assays, or use them individually without additional agents being attached thereto. The antibodies may be mutated or modified, as discussed further below. Methods for preparing and characterizing antibodies are well known in the art (see, *e.g*., Antibodies: A Laboratory Manual, Cold Spring Harbor Laboratory, 1988; U.S. Patent 4,196,265).

The classical methods for generating monoclonal antibodies (MAbs) generally begin along the same lines as those for preparing polyclonal antibodies. The first step for both these methods is immunization of an appropriate host. As is well known in the art, a given composition for immunization may vary in its immunogenicity. It is often necessary therefore to boost the host immune system, as may be achieved by coupling a peptide or polypeptide immunogen to a carrier. Exemplary and preferred carriers are keyhole limpet hemocyanin (KLH) and bovine serum albumin (BSA). Other albumins such as ovalbumin, mouse serum albumin or rabbit serum albumin can also be used as carriers. Means for conjugating a polypeptide to a carrier protein are well known in the art and include glutaraldehyde, m-maleimidobencoyl-N-hydroxysuccinimide ester, carbodiimyde and bis-biazotized benzidine. As also is well known in the art, the immunogenicity of a particular immunogen composition can be enhanced by the use of non-specific stimulators of the immune response, known as adjuvants. Exemplary and preferred adjuvants include complete Freund's adjuvant (a non-specific stimulator of the immune response containing killed *Mycobacterium tuberculosis),* incomplete Freund's adjuvants and aluminum hydroxide adjuvant.

The amount of immunogen composition used in the production of polyclonal antibodies varies upon the nature of the immunogen as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization. A second, booster injection, also may be given. The process of boosting and titering is repeated until a suitable titer is achieved. When a desired level of immunogenicity is obtained, the immunized animal can be bled and the serum isolated and stored, and/or the animal can be used to generate MAbs.

Following immunization, somatic cells with the potential for producing antibodies, specifically B lymphocytes (B cells), are selected for use in the MAb generating protocol. These cells may be obtained from biopsied spleens or lymph nodes, or from circulating blood. The antibody-producing B lymphocytes from the immunized animal are then fused with cells of an immortal myeloma cell, generally one of the same species as the animal that was immunized or human or human/mouse chimeric cells. Myeloma cell lines suited for use in hybridoma-producing fusion procedures preferably are non-antibody-producing, have high fusion efficiency, and enzyme deficiencies that render then incapable of growing in certain selective media which support the growth of only the desired fused cells (hybridomas). Any one of a number of myeloma cells may be used, as are known to those of skill in the art (Goding, pp. 65-66, 1986; Campbell, pp. 75-83, 1984).

Methods for generating hybrids of antibody-producing spleen or lymph node cells and myeloma cells usually comprise mixing somatic cells with myeloma cells in a 2:1 proportion, though the proportion may vary from about 20:1 to about 1:1, respectively, in the presence of an agent or agents (chemical or electrical) that promote the fusion of cell membranes. Fusion methods using Sendai virus have been described by Kohler and Milstein (1975; 1976), and those using polyethylene glycol (PEG), such as 37% (v/v) PEG, by Gefter *et al.* (1977). The use of electrically induced fusion methods also is appropriate (Goding, pp. 71-74, 1986). Fusion procedures usually produce viable hybrids at low frequencies, about 1 x 10⁻⁶ to 1 x 10⁻⁸. However, this does not pose a problem, as the viable, fused hybrids are differentiated from the parental, infused cells (particularly the infused myeloma cells that would normally continue to divide indefinitely) by culturing in a selective medium. The selective medium is generally one that contains an agent that blocks the *de novo* synthesis of nucleotides in the tissue culture media. Exemplary and preferred agents are aminopterin, methotrexate, and azaserine. Aminopterin and methotrexate block *de novo* synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine. Ouabain is added if the B cell source is an Epstein Barr virus (EBV) transformed human B cell line, in order to eliminate EBV transformed lines that have not fused to the myeloma.

The preferred selection medium is HAT or HAT with ouabain. Only cells capable of operating nucleotide salvage pathways are able to survive in HAT medium. The myeloma cells are defective in key enzymes of the salvage pathway, *e.g*., hypoxanthine phosphoribosyl transferase (HPRT), and they cannot survive. The B cells can operate this pathway, but they have a limited life span in culture and generally die within about two weeks. Therefore, the only cells that can survive in the selective media are those hybrids formed from myeloma and B cells. When the source of B cells used for fusion is a line of EBV-transformed B cells, as here, ouabain is also used for drug selection of hybrids as EBV-transformed B cells are susceptible to drug killing, whereas the myeloma partner used is chosen to be ouabain resistant.

Culturing provides a population of hybridomas from which specific hybridomas are selected. Typically, selection of hybridomas is performed by culturing the cells by single-clone dilution in microtiter plates, followed by testing the individual clonal supernatants (after about two to three weeks) for the desired reactivity. The assay should be sensitive, simple and rapid, such as radioimmunoassays, enzyme immunoassays, cytotoxicity assays, plaque assays dot immunobinding assays, and the like. The selected hybridomas are then serially diluted or single-cell sorted by flow cytometric sorting and cloned into individual antibody-producing cell lines, which clones can then be propagated indefinitely to provide mAbs. The cell lines may be exploited for MAb production in two basic ways. A sample of the hybridoma can be injected (often into the peritoneal cavity) into an animal (*e.g.,* a mouse). Optionally, the animals are primed with a hydrocarbon, especially oils such as pristane (tetramethylpentadecane) prior to injection. When human hybridomas are used in this way, it is optimal to inject immunocompromised mice, such as SCID mice, to prevent tumor rejection. The injected animal develops tumors secreting the specific monoclonal antibody produced by the fused cell hybrid. The body fluids of the animal, such as serum or ascites fluid, can then be tapped to provide MAbs in high concentration. The individual cell lines could also be cultured *in vitro,* where the MAbs are naturally secreted into the culture medium from which they can be readily obtained in high concentrations. Alternatively, human hybridoma cells lines can be used *in vitro* to produce immunoglobulins in cell supernatant. The cell lines can be adapted for growth in serum-free medium to optimize the ability to recover human monoclonal immunoglobulins of high purity.

MAbs produced by either means may be further purified, if desired, using filtration, centrifugation and various chromatographic methods such as FPLC or affinity chromatography. Fragments of the monoclonal antibodies of the disclosure can be obtained from the purified monoclonal antibodies by methods which include digestion with enzymes, such as pepsin or papain, and/or by cleavage of disulfide bonds by chemical reduction. Alternatively, monoclonal antibody fragments encompassed by the present disclosure can be synthesized using an automated peptide synthesizer.

It also is contemplated that a molecular cloning approach may be used to generate monoclonals. For this, RNA can be isolated from the hybridoma line and the antibody genes obtained by RT-PCR and cloned into an immunoglobulin expression vector. Alternatively, combinatorial immunoglobulin phagemid libraries are prepared from RNA isolated from the cell lines and phagemids expressing appropriate antibodies are selected by panning using viral antigens. The advantages of this approach over conventional hybridoma techniques are that approximately 10⁴ times as many antibodies can be produced and screened in a single round, and that new specificities are generated by H and L chain combination which further increases the chance of finding appropriate antibodies.

Recently, additional methods for generating mAb, such as scFv phage display, have been developed (see CM Hammers and JR Stanley, Antibody phage display: technique and applications, J Invest Dermatol (2014) 134: e17). Generally, a panel of human mAbs that bind to a target protein, *e.g*., human LILRB3, are generated by panning a large diversity of human scFv phage displayed antibody library.

To generate the human scFv phage displayed antibody library, RNA is extracted from the chosen cell source, *e.g*., peripheral blood mononuclear cells. The RNA is then reversed-transcribed into cDNA, which is used for PCR of the VH and VL chains of the encoded antibodies. Defined sets of primers specific for the different VH and VL chain region gene families allow the amplification of all transcribed rearranged variable regions within a given immunoglobulin repertoire, reflecting all antibody specificities in a particular individual.

The VH and VL PCR products that represent the antibody repertoire are ligated into a phage display vector that is engineered to express the VH and VL as an scFv fused to the pIII minor capsid protein of a filamentous bacteriophage of *E. coli* that was originally derived from the M13 bacteriophage. This generates a library of phages, each of which expresses on its surface a scFv and harbors the vector with the respective nucleotide sequence within.

The library is then screened for phage binding to a target antigen through its expressed surface scFv by a technique called bio-panning. In short, the target protein is coated on solid phase for incubation with phage libraries. After washing and elution, antigen enriched phages are recovered and used for next rounds of phage panning. After at least three rounds of phage panning, single bacterial colonies are picked for phage ELISA and other functional/genetic analysis.

The positive hits are sequenced for the scFv region and are converted to full human IgG heavy and light chain constructs, which are used to generate the mAb of interest using the methods disclosed supra. For example, the IgG expressing plasmids are cotransfected into Expi293 cells using transfection reagent PEI. After 7 days of expression, supernatants are harvested, and antibodies are purified by affinity chromatography using protein A resin.

Other U.S. patents, each incorporated herein by reference, that teach the production of antibodies useful in the present disclosure include U.S. Patent 5,565,332, which describes the production of chimeric antibodies using a combinatorial approach; U.S. Patent 4,816,567 which describes recombinant immunoglobulin preparations; and U.S. Patent 4,867,973 which describes antibody-therapeutic agent conjugates.

### B. Antibodies of the Present Disclosure

### 1. Antibodies to LILRB3

Antibodies or antigen-binding fragments thereof according to the present disclosure may be defined, in the first instance, by their binding specificity, which in this case is for LILRB3. Those of skill in the art, by assessing the binding specificity/affinity of a given antibody using techniques well known to those of skill in the art, can determine whether such antibodies fall within the scope of the instant claims.

In one aspect, there are provided antibodies and antigen-binding fragments specifically bind to LILRB3. In some embodiments, when bound to LILRB3, such antibodies modulate the activation of LILRB3. In certain embodiments, the antibody or antigen-binding fragment, when bound to LILRB3, activates LILRB3. In certain embodiments, the antibody or antigen-binding fragment, when bound to LILRB3, suppresses activation of LILRB3. In certain embodiments, the antibody or antigen-binding fragment, when bound to LILRB3, can specifically interfere with, block or reduce the interaction between LILRB3 and its binding partners. In certain embodiments, the antibody or antigen-binding fragment provided herein is capable of inhibiting the immunosuppressive activity of MDSCs and other solid tumor-infiltrating myeloid cells, such as tumor-associated macrophages (TAMs) and tolerogenic dendritic cells (DCs). In certain embodiments, the antibodies or antigen-binding fragments provided herein specifically or selectively bind to human LILRB3.

In some embodiments, the antibodies or antigen-binding fragments bind specifically to human LILRB3 and/or substantially inhibits binding of human LILRB3 to galectin-4 by at least about 20%-40%, 40-60%, 60-80%, 80-85%, or more (for example, by an assay as disclosed in the Example). In some embodiments, the antibody or antigen-binding fragment has a Kd of less (binding more tightly) than 10⁻⁶, 10⁻⁷, 10⁻⁸, 10⁻⁹, 10⁻¹⁰, 10⁻¹¹, 10⁻¹², 10⁻¹³ M. In some embodiments, the antibody or antigen-binding fragment has an IC50 for blocking the binding of galectin-4 to LILRB3 of less than 10 uM, 10 uM to 1 uM, 1000 nM to 100 nM, 100 nM to 10 nM, 10 nM to 1 nM, 1000 pM to 500 pM, 500 pM to 200 pM, less than 200 pM, 200 pM to 150 pM, 200 pM to 100 pM, 100 pM to 10 pM, 10 pM to 1 pM.

In some embodiments, the antibodies or antigen-binding fragments provided herein having the clone-paired CDRs illustrated in Tables 3 and 4.

In certain embodiments, the antibodies may be defined by their variable sequence, which include additional "framework" regions. The antibody is characterized by clone-paired heavy chain and light chain amino acid sequences from Appendices I and III. Furthermore, the antibodies sequences may vary from these sequences, particularly in regions outside the CDRs. For example, the amino acids may vary from those set out above by a given percentage, *e.g.,* 80%, 85%, 90%, 91%, 92%, 93%, 94%, 95%, 96%, 97%, 98% or 99% homology, or the amino acids may vary from those set out above by permitting conservative substitutions (discussed below). Each of the foregoing apply to the amino acid sequences of Appendices I and III. In another embodiment, the antibody derivatives of the present disclosure comprise VL and VH domains having up to 0, 1, 2, 3, 4, 5, 6, 7, 8, 9, 10 or more conservative or non-conservative amino acid substitutions, while still exhibiting the desired binding and functional properties.

While the antibodies of the present disclosure were generated as IgG's, it may be useful to modify the constant regions to alter their function. The constant regions of the antibodies typically mediate the binding of the antibody to host tissues or factors, including various cells of the immune system (*e.g*., effector cells) and the first component (Clq) of the classical complement system. Thus, the term "antibody" includes intact immunoglobulins of types IgA, IgG, IgE, IgD, IgM (as well as subtypes thereof), wherein the light chains of the immunoglobulin may be of types kappa or lambda. Within light and heavy chains, the variable and constant regions are joined by a 35 "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989).

The present disclosure further comprises nucleic acids which hybridize to nucleic acids encoding the antibodies disclosed herein. In general, the nucleic acids hybridize under moderate or high stringency conditions to nucleic acids that encode antibodies disclosed herein and also encode antibodies that maintain the ability to specifically bind to an LILRB3. A first nucleic acid molecule is "hybridizable" to a second nucleic acid molecule when a single stranded form of the first nucleic acid molecule can anneal to the second nucleic acid molecule under the appropriate conditions of temperature and solution ionic strength (see Sambrook et al., MOLECULAR CLONING: A LABORATORY MANUAL, 3rd ed., Cold Spring Harbor Press, Cold Spring Harbor, N.Y. 2001). The conditions of temperature and ionic strength determine the "stringency" of the hybridization. Typical moderate stringency hybridization conditions are 40% formamide, with 5X or 6X SSC and 0.1% SDS at 42°C. High stringency hybridization conditions are 50% formamide, 5X or 6X SSC (0.15M NaC1 and 0.015M Na-citrate) at 42°C or, optionally, at a higher temperature (*e.g*., 57°C, 59°C, 60°C, 62°C, 63°C, 65°C or 68°C). Hybridization requires that the two nucleic acids contain complementary sequences, although, depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the higher the stringency under which the nucleic acids may hybridize. For hybrids of greater than 100 nucleotides in length, equations for calculating the melting temperature have been derived (see Sambrook *et al., supra*). For hybridization with shorter nucleic acids, *e.g.,* oligonucleotides, the position of mismatches becomes more important, and the length of the oligonucleotide determines its specificity (see Sambrook *et al., supra*).

### 2. Exemplary Epitopes and Competing Antigen Binding Proteins

In another aspect, the present disclosure provides epitopes to which anti-LILRB3 antibodies bind. In some embodiments, epitopes that are bound by the antibodies described herein are useful. In certain embodiments, an epitope provided herein can be utilized to isolate antibodies or antigen binding proteins that bind to LILRB3. In certain embodiments, an epitope provided herein can be utilized to generate antibodies or antigen binding proteins which bind to LILRB3. In certain embodiments, an epitope or a sequence comprising an epitope provided herein can be utilized as an immunogen to generate antibodies or antigen binding proteins that bind to LILRB3. In certain embodiments, an epitope described herein or a sequence comprising an epitope described herein can be utilized to interfere with biological activity of LILRB3.

In some embodiments, antibodies or antigen-binding fragments thereof that bind to any of the epitopes are particularly useful. In some embodiments, an epitope provided herein, when bound by an antibody, modulates the biological activity of LILRB3. In some embodiments, an epitope provided herein, when bound by an antibody, activates LILRB3. In some embodiments, an epitope provided herein, when bound by an antibody, suppress the activation of LILRB3. In some embodiments, an epitope provided herein, when bound by an antibody, block the interaction between LILRB3 and its binding partners.

In some embodiments, the domain(s)/region(s) containing residues that are in contact with or are buried by an antibody can be identified by mutating specific residues in LILRB3 and determining whether the antibody can bind the mutated LILRB3 protein. By making a number of individual mutations, residues that play a direct role in binding or that are in sufficiently close proximity to the antibody such that a mutation can affect binding between the antibody and antigen can be identified. From knowledge of these amino acids, the domain(s) or region(s) of the antigen that contain residues in contact with the antigen binding protein or covered by the antibody can be elucidated. Such a domain can include the binding epitope of an antigen binding protein.

In another aspect, the present disclosure provides antigen-binding proteins that compete with one of the exemplified antibodies or antigen-binding fragment binding to the epitope described herein for specific binding to LILRB3. Such antigen binding proteins can also bind to the same epitope as one of the herein exemplified antibodies or the antigen-binding fragment, or an overlapping epitope. Antigen-binding proteins that compete with or bind to the same epitope as the exemplified antibodies are expected to show similar functional properties. The exemplified antibodies include those described above, including those with the heavy and light chain variable regions and CDRs included in Tables 3 and 4, heavy and light chains as shown in Appendices I and III, and heavy and light chain coding regions as shown in Appendices II and IV.

### C. Engineering of Antibody Sequences

In various embodiments, one may choose to engineer sequences of the identified antibodies for a variety of reasons, such as improved expression, improved cross-reactivity or diminished off-target binding. The following is a general discussion of relevant techniques for antibody engineering.

Hybridomas may be cultured, then cells lysed, and total RNA extracted. Random hexamers may be used with RT to generate cDNA copies of RNA, and then PCR performed using a multiplex mixture of PCR primers expected to amplify all human variable gene sequences. PCR product can be cloned into pGEM-T Easy vector, then sequenced by automated DNA sequencing using standard vector primers. Assay of binding and neutralization may be performed using antibodies collected from hybridoma supernatants and purified by FPLC, using Protein G columns. Recombinant full-length IgG antibodies may be generated by subcloning heavy and light chain Fv DNAs from the cloning vector into an IgG plasmid vector, transfected into 293 Freestyle cells or CHO cells, and antibodies collected a purified from the 293 or CHO cell supernatant.

The rapid availability of antibody produced in the same host cell and cell culture process as the final cGMP manufacturing process has the potential to reduce the duration of process development programs. Lonza has developed a generic method using pooled transfectants grown in CDACF medium, for the rapid production of small quantities (up to 50 g) of antibodies in CHO cells. Although slightly slower than a true transient system, the advantages include a higher product concentration and use of the same host and process as the production cell line. Example of growth and productivity of GS-CHO pools, expressing a model antibody, in a disposable bioreactor: in a disposable bag bioreactor culture (5 L working volume) operated in fed-batch mode, a harvest antibody concentration of 2 g/L was achieved within 9 weeks of transfection.

Antibody molecules will comprise fragments (such as F(ab'), F(ab')₂) that are produced, for example, by the proteolytic cleavage of the mAbs, or single-chain immunoglobulins producible, for example, via recombinant means. Such antibody derivatives are monovalent. In one embodiment, such fragments can be combined with one another, or with other antibody fragments or receptor ligands to form "chimeric" binding molecules. Significantly, such chimeric molecules may contain substituents capable of binding to different epitopes of the same molecule.

### 1. Antigen Binding Modifications

In related embodiments, the antibody is a derivative of the disclosed antibodies, *e.g*., an antibody comprising the CDR sequences identical to those in the disclosed antibodies (*e.g*., a chimeric, or CDR-grafted antibody). Alternatively, one may wish to make modifications, such as introducing conservative changes into an antibody molecule. In making such changes, the hydropathic index of amino acids may be considered. The importance of the hydropathic amino acid index in conferring interactive biologic function on a protein is generally understood in the art (Kyte and Doolittle, 1982). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like.

It also is understood in the art that the substitution of like amino acids can be made effectively on the basis of hydrophilicity. U.S. Patent 4,554,101, incorporated herein by reference, states that the greatest local average hydrophilicity of a protein, as governed by the hydrophilicity of its adjacent amino acids, correlates with a biological property of the protein. As detailed in U.S. Patent 4,554,101, the following hydrophilicity values have been assigned to amino acid residues: basic amino acids: arginine (+3.0), lysine (+3.0), and histidine (-0.5); acidic amino acids: aspartate (+3.0 ± 1), glutamate (+3.0 ± 1), asparagine (+0.2), and glutamine (+0.2); hydrophilic, nonionic amino acids: serine (+0.3), asparagine (+0.2), glutamine (+0.2), and threonine (-0.4), sulfur containing amino acids: cysteine (-1.0) and methionine (-1.3); hydrophobic, nonaromatic amino acids: valine (-1.5), leucine (-1.8), isoleucine (-1.8), proline (-0.5 ± 1), alanine (-0.5), and glycine (0); hydrophobic, aromatic amino acids: tryptophan (-3.4), phenylalanine (-2.5), and tyrosine (-2.3).

It is understood that an amino acid can be substituted for another having a similar hydrophilicity and produce a biologically or immunologically modified protein. In such changes, the substitution of amino acids whose hydrophilicity values are within ± 2 is preferred, those that are within ± 1 are particularly preferred, and those within ± 0.5 are even more particularly preferred.

As outlined above, amino acid substitutions generally are based on the relative similarity of the amino acid side-chain substituents, for example, their hydrophobicity, hydrophilicity, charge, size, and the like. Exemplary substitutions that take into consideration the various foregoing characteristics are well known to those of skill in the art and include: arginine and lysine; glutamate and aspartate; serine and threonine; glutamine and asparagine; and valine, leucine and isoleucine.

The present disclosure also contemplates isotype modification. By modifying the Fc region to have a different isotype, different functionalities can be achieved. For example, changing to IgG₁ can increase antibody dependent cell cytotoxicity, switching to class A can improve tissue distribution, and switching to class M can improve valency.

Modified antibodies may be made by any technique known to those of skill in the art, including expression through standard molecular biological techniques, or the chemical synthesis of polypeptides. Methods for recombinant expression are addressed elsewhere in this document.

### 2. Fc Region Modifications

The antibodies disclosed herein can also be engineered to include modifications within the Fc region, typically to alter one or more functional properties of the antibody, such as serum half-life, complement fixation, Fc receptor binding, and/or effector function (*e.g*., antigen-dependent cellular cytotoxicity). Furthermore, the antibodies disclosed herein can be chemically modified (*e.g.,* one or more chemical moieties can be attached to the antibody) or be modified to alter its glycosylation, again to alter one or more functional properties of the antibody. Each of these embodiments is described in further detail below. The numbering of residues in the Fc region is that of the EU index of Kabat. The antibodies disclosed herein also include antibodies with modified (or blocked) Fc regions to provide altered effector functions. See, *e.g.,* U.S. Patent 5,624,821; WO2003/086310; WO2005/120571; WO2006/0057702. Such modification can be used to enhance or suppress various reactions of the immune system, with possible beneficial effects in diagnosis and therapy. Alterations of the Fc region include amino acid changes (substitutions, deletions and insertions), glycosylation or deglycosylation, and adding multiple Fc. Changes to the Fc can also alter the half-life of antibodies in therapeutic antibodies, enabling less frequent dosing and thus increased convenience and decreased use of material. This mutation has been reported to abolish the heterogeneity of inter-heavy chain disulfide bridges in the hinge region.

In one embodiment, the hinge region of CH1 is modified such that the number of cysteine residues in the hinge region is increased or decreased. This approach is described further in U.S. Patent 5,677,425. The number of cysteine residues in the hinge region of CH1 is altered, for example, to facilitate assembly of the light and heavy chains or to increase or decrease the stability of the antibody. In another embodiment, the antibody is modified to increase its biological half-life. Various approaches are possible. For example, one or more of the following mutations can be introduced: T252L, T254S, T256F, as described in U.S. Patent 6,277,375. Alternatively, to increase the biological half-life, the antibody can be altered within the CH1 or CL region to contain a salvage receptor binding epitope taken from two loops of a CH2 domain of an Fc region of an IgG, as described in U.S. Patents 5,869,046 and 6,121,022. In yet other embodiments, the Fc region is altered by replacing at least one amino acid residue with a different amino acid residue to alter the effector function(s) of the antibodies. For example, one or more amino acids selected from amino acid residues 234, 235, 236, 237, 297, 318, 320 and 322 can be replaced with a different amino acid residue such that the antibody has an altered affinity for an effector ligand but retains the antigen binding ability of the parent antibody. The effector ligand to which affinity is altered can be, for example, an Fc receptor or the C1 component of complement. This approach is described in further detail in U.S. Patents 5,624,821 and 5,648,260.

In another example, one or more amino acid residues within amino acid positions 231 and 239 are altered to thereby alter the ability of the antibody to fix complement. This approach is described further in PCT Publication WO 94/29351. In yet another example, the Fc region is modified to increase or decrease the ability of the antibodies to mediate antibody dependent cellular cytotoxicity (ADCC) and/or to increase or decrease the affinity of the antibodies for an Fcγ receptor by modifying one or more amino acids at the following positions: 238, 239, 243, 248, 249, 252, 254, 255, 256, 258, 264, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439. This approach is described further in PCT Publication WO 00/42072. Moreover, the binding sites on human IgG1 for FcγR1, FcγRII, FcγRIII and FcRn have been mapped and variants with improved binding have been described. Specific mutations at positions 256, 290, 298, 333, 334 and 339 were shown to improve binding to FcγRIII. Additionally, the following combination mutants were shown to improve FcγRIII binding: T256A/S298A, S298A/E333A, S298A/K224A and S298A/E333A/K334A.

In one embodiment, the Fc region is modified to decrease the ability of the antibodies to mediate effector function and/or to increase anti-inflammatory properties by modifying residues 243 and 264. In one embodiment, the Fc region of the antibody is modified by changing the residues at positions 243 and 264 to alanine. In one embodiment, the Fc region is modified to decrease the ability of the antibody to mediate effector function and/or to increase anti-inflammatory properties by modifying residues 243, 264, 267 and 328.

In one embodiment, the Fc region is modified to abolish the ability of the antibodies to mediate effector function by modifying residues 234, 235 and 329 to alanine or glycine (L234A-L235A-P329G).

In still another embodiment, the antibody comprises a particular glycosylation pattern. For example, an aglycosylated antibody can be made (*i.e.,* the antibody lacks glycosylation). The glycosylation pattern of an antibody may be altered to, for example, increase the affinity or avidity of the antibody for an antigen. Such modifications can be accomplished by, for example, altering one or more of the glycosylation sites within the antibody sequence. For example, one or more amino acid substitutions can be made that result removal of one or more of the variable region framework glycosylation sites to thereby eliminate glycosylation at that site. Such aglycosylation may increase the affinity or avidity of the antibody for antigen. *See, e.g.,* U.S. Patents 5,714,350 and 6,350,861.

An antibody may also be made in which the glycosylation pattern includes hypofucosylated or afucosylated glycans, such as a hypofucosylated antibodies or afucosylated antibodies have reduced amounts of fucosyl residues on the glycan. The antibodies may also include glycans having an increased amount of bisecting GlcNac structures. Such altered glycosylation patterns have been demonstrated to increase the ADCC ability of antibodies. Such modifications can be accomplished by, for example, expressing the antibodies in a host cell in which the glycosylation pathway was been genetically engineered to produce glycoproteins with particular glycosylation patterns. These cells have been described in the art and can be used as host cells in which to express recombinant antibodies of the invention to thereby produce an antibody with altered glycosylation. For example, the cell lines Ms704, Ms705, and Ms709 lack the fucosyltransferase gene, FUT8 (α (1,6)-fucosyltransferase), such that antibodies expressed in the Ms704, Ms705, and Ms709 cell lines lack fucose on their carbohydrates. The Ms704, Ms705, and Ms709 FUT8-/- cell lines were created by the targeted disruption of the FUT8 gene in CHO/DG44 cells using two replacement vectors (*see* U.S. Patent Publication No. 20040110704. As another example, EP 1 176 195 describes a cell line with a functionally disrupted FUT8 gene, which encodes a fucosyl transferase, such that antibodies expressed in such a cell line exhibit hypofucosylation by reducing or eliminating the α-1,6 bond-related enzyme. EP 1 176 195 also describes cell lines which have a low enzyme activity for adding fucose to the N-acetylglucosamine that binds to the Fc region of the antibody or does not have the enzyme activity, for example the rat myeloma cell line YB2/0 (ATCC CRL 1662). PCT Publication WO 03/035835 describes a variant CHO cell line, Lec13 cells, with reduced ability to attach fucose to Asn(297)-linked carbohydrates, also resulting in hypofucosylation of antibodies expressed in that host cell. Antibodies with a modified glycosylation profile can also be produced in chicken eggs, as described in PCT Publication WO 06/089231. Alternatively, antibodies with a modified glycosylation profile can be produced in plant cells, such as *Lemna* (US Patent 7,632,983). Methods for production of antibodies in a plant system are disclosed in the U.S. Patents 6,998,267 and 7,388,081. PCT Publication WO 99/54342 describes cell lines engineered to express glycoprotein-modifying glycosyl transferases (*e.g*., β(1,4)-N-acetylglucosaminyltransferase III (GnTIII)) such that antibodies expressed in the engineered cell lines exhibit increased bisecting GlcNac structures which results in increased ADCC activity of the antibodies.

Alternatively, the fucose residues of the antibodies can be cleaved off using a fucosidase enzyme; *e.g.,* the fucosidase α-L-fucosidase removes fucosyl residues from antibodies. Antibodies disclosed herein further include those produced in lower eukaryote host cells, in particular fungal host cells such as yeast and filamentous fungi have been genetically engineered to produce glycoproteins that have mammalian- or human-like glycosylation patterns. A particular advantage of these genetically modified host cells over currently used mammalian cell lines is the ability to control the glycosylation profile of glycoproteins that are produced in the cells such that compositions of glycoproteins can be produced wherein a particular N-glycan structure predominates (see, *e.g.,* U.S. Patents 7,029,872 and 7,449,308). These genetically modified host cells have been used to produce antibodies that have predominantly particular N-glycan structures.

In addition, since fungi such as yeast or filamentous fungi lack the ability to produce fucosylated glycoproteins, antibodies produced in such cells will lack fucose unless the cells are further modified to include the enzymatic pathway for producing fucosylated glycoproteins (See for example, PCT Publication WO2008112092). In particular embodiments, the antibodies disclosed herein further include those produced in lower eukaryotic host cells and which comprise fucosylated and nonfucosylated hybrid and complex N-glycans, including bisected and multiantennary species, including but not limited to *N-*glycans such as GlcNAc(1-4)Man3GlcNAc2; Gal(1-4)GlcNAc(1-4)Man3GlcNAc2; NANA(1-4)Gal(1-4)GlcNAc(1-4)Man3GlcNAc2. In particular embodiments, the antibody compositions provided herein may comprise antibodies having at least one hybrid N-glycan selected from the group consisting of GlcNAcMan5GlcNAc2; GalGlcNAcMan5GlcNAc2; and NANAGalGlcNAcMan5GlcNAc2. In particular aspects, the hybrid N-glycan is the predominant N-glycan species in the composition. In further aspects, the hybrid N-glycan is a particular N-glycan species that comprises about 30%, 40%, 50%, 60%, 70%, 80%, 90%, 95%, 97%, 98%, 99%, or 100% of the hybrid N-glycans in the composition.

In particular embodiments, the antibody compositions provided herein comprise antibodies having at least one complex N-glycan selected from the group consisting of GlcNAcMan3GlcNAc2; GalGlcNAcMan3GlcNAc2; NANAGalGlcNAcMan3GlcNAc2; GlcNAc2Man3GlcNAc2; GalGlcNAc2Man3GlcNAc2; Gal2GlcNAc2Man3GlcNAc2; NANAGal2GlcNAc2Man3GlcNAc2; and NANA2Gal2GlcNAc2Man3 GlcNAc2. In particular aspects, the complex N-glycan is the predominant N-glycan species in the composition. In further aspects, the complex N-glycan is a particular N-glycan species that comprises about 30%, 40%, 50%, 60%, 70%, 80%, 90%, 95%, 97%, 98%, 99%, or 100% of the complex N-glycans in the composition. In particular embodiments, the N-glycan is fusosylated. In general, the fucose is in an α1,3-linkage with the GlcNAc at the reducing end of the N-glycan, an α1,6-linkage with the GlcNAc at the reducing end of the N-glycan, an α1,2-linkage with the Gal at the non-reducing end of the N-glycan, an α1,3-linkage with the GlcNac at the non-reducing end of the N-glycan, or an α1,4-linkage with a GlcNAc at the non-reducing end of the N-glycan.

Therefore, in particular aspects of the above the glycoprotein compositions, the glycoform is in an α1,3-linkage or α1,6-linkage fucose to produce a glycoform selected from the group consisting of Man5GlcNAc2(Fuc), GlcNAcMan5GlcNAc2(Fuc), Man3GlcNAc2(Fuc), GlcNAcMan3GlcNAc2(Fuc), GlcNAc2Man3GlcNAc2(Fuc), GalGlcNAc2Man3GlcNAc2(Fuc), Gal2GlcNAc2Man3 GlcNAc2(Fuc), NANAGal2GlcNAc2Man3GlcNAc2(Fuc), and NANA2Gal2GlcNAc2Man3GlcNAc2(Fuc); in an α1,3-linkage or α1,4-linkage fucose to produce a glycoform selected from the group consisting of GlcNAc(Fuc)Man5GlcNAc2, GlcNAc(Fuc)Man3GlcNAc2, GlcNAc2(Fuc1-2)Man3GlcNAc2, GalGlcNAc2(Fuc1-2)Man3GlcNAc2, Gal2GlcNAc2(Fuc1-2)Man3GlcNAc2, NANAGal2GlcNAc2(Fuc1-2)Man3GlcNAc2, and NANA2Gal2GlcNAc2(Fuc1-2)Man3GlcNAc2; or in an α1,2-linkage fucose to produce a glycoform selected from the group consisting of Gal(Fuc)GlcNAc2Man3GlcNAc2, Gal2(Fuc1-2)GlcNAc2Man3GlcNAc2, NANAGal2(Fucl-2)GlcNAc2Man3GlcNAc2, and NANA2Gal2(Fuc1-2)GlcNAc2Man3GlcNAc2.

In further aspects, the antibodies comprise high mannose *N*-glycans, including but not limited to, Man8GlcNAc2, Man7GlcNAc2, Man6GlcNAc2, Man5GlcNAc2, Man4GlcNAc2, or *N*-glycans that consist of the Man3GlcNAc2 *N*-glycan structure. In further aspects of the above, the complex *N*-glycans further include fucosylated and non-fucosylated bisected and multiantennary species. As used herein, the terms "*N*-glycan" and "glycoform" are used interchangeably and refer to an N-linked oligosaccharide, for example, one that is attached by an asparagine-*N*-acetylglucosamine linkage to an asparagine residue of a polypeptide. *N*-linked glycoproteins contain an *N*-acetylglucosamine residue linked to the amide nitrogen of an asparagine residue in the protein.

### D. Single Chain Antibodies

A Single Chain Variable Fragment (scFv) is a fusion of the variable regions of the heavy and light chains of immunoglobulins, linked together with a short (usually serine, glycine) linker. This chimeric molecule retains the specificity of the original immunoglobulin, despite removal of the constant regions and the introduction of a linker peptide. This modification usually leaves the specificity unaltered. These molecules were created historically to facilitate phage display where it is highly convenient to express the antigen binding domain as a single peptide. Alternatively, scFv can be created directly from subcloned heavy and light chains derived from a hybridoma. Single chain variable fragments lack the constant Fc region found in complete antibody molecules, and thus, the common binding sites (*e.g*., protein A/G) used to purify antibodies. These fragments can often be purified/immobilized using Protein L since Protein L interacts with the variable region of kappa light chains.

Flexible linkers generally are comprised of helix- and turn-promoting amino acid residues such as alanine, serine and glycine. However, other residues can function as well. Tang *et al.* (1996) used phage display as a means of rapidly selecting tailored linkers for single-chain antibodies (scFvs) from protein linker libraries. A random linker library was constructed in which the genes for the heavy and light chain variable domains were linked by a segment encoding an 18-amino acid polypeptide of variable composition. The scFv repertoire (approx. 5 × 10⁶ different members) was displayed on filamentous phage and subjected to affinity selection with hapten. The population of selected variants exhibited significant increases in binding activity but retained considerable sequence diversity. Screening 1054 individual variants subsequently yielded a catalytically active scFv that was produced efficiently in soluble form. Sequence analysis revealed a conserved proline in the linker two residues after the V_{H} C terminus and an abundance of arginines and prolines at other positions as the only common features of the selected tethers.

The recombinant antibodies of the present disclosure may also involve sequences or moieties that permit dimerization or multimerization of the receptors. Such sequences include those derived from IgA, which permit formation of multimers in conjunction with the J-chain. Another multimerization domain is the Gal4 dimerization domain. In other embodiments, the chains may be modified with agents such as biotin/avidin, which permit the combination of two antibodies.

In a separate embodiment, a single-chain antibody can be created by joining receptor light and heavy chains using a non-peptide linker or chemical unit. Generally, the light and heavy chains will be produced in distinct cells, purified, and subsequently linked together in an appropriate fashion (*i.e.,* the N-terminus of the heavy chain being attached to the C-terminus of the light chain via an appropriate chemical bridge).

Cross-linking reagents are used to form molecular bridges that tie functional groups of two different molecules, *e.g.,* a stabilizing and coagulating agent. However, it is contemplated that dimers or multimers of the same analog or heteromeric complexes comprised of different analogs can be created. To link two different compounds in a step-wise manner, hetero-bifunctional cross-linkers can be used that eliminate unwanted homopolymer formation.

An exemplary hetero-bifunctional cross-linker contains two reactive groups: one reacting with primary amine group (*e.g*., N-hydroxy succinimide) and the other reacting with a thiol group (*e.g.,* pyridyl disulfide, maleimides, halogens, *etc.*)*.* Through the primary amine reactive group, the cross-linker may react with the lysine residue(s) of one protein (*e.g.,* the selected antibody or fragment) and through the thiol reactive group, the cross-linker, already tied up to the first protein, reacts with the cysteine residue (free sulfhydryl group) of the other protein (*e.g.,* the selective agent).

It is preferred that a cross-linker having reasonable stability in blood will be employed. Numerous types of disulfide-bond containing linkers are known that can be successfully employed to conjugate targeting and therapeutic/preventative agents. Linkers that contain a disulfide bond that is sterically hindered may prove to give greater stability *in vivo,* preventing release of the targeting peptide prior to reaching the site of action. These linkers are thus one group of linking agents.

Another cross-linking reagent is SMPT, which is a bifunctional cross-linker containing a disulfide bond that is "sterically hindered" by an adjacent benzene ring and methyl groups. It is believed that steric hindrance of the disulfide bond serves a function of protecting the bond from attack by thiolate anions such as glutathione which can be present in tissues and blood, and thereby help in preventing decoupling of the conjugate prior to the delivery of the attached agent to the target site.

The SMPT cross-linking reagent, as with many other known cross-linking reagents, lends the ability to cross-link functional groups such as the SH of cysteine or primary amines (*e.g*., the epsilon amino group of lysine). Another possible type of cross-linker includes the hetero-bifunctional photoreactive phenylazides containing a cleavable disulfide bond such as sulfosuccinimidyl-2-(p-azido salicylamido) ethyl-1,3'-dithiopropionate. The N-hydroxy-succinimidyl group reacts with primary amino groups and the phenylazide (upon photolysis) reacts non-selectively with any amino acid residue.

In addition to hindered cross-linkers, non-hindered linkers also can be employed in accordance herewith. Other useful cross-linkers, not considered to contain or generate a protected disulfide, include SATA, SPDP and 2-iminothiolane (Wawrzynczak & Thorpe, 1987). The use of such cross-linkers is well understood in the art. Another embodiment involves the use of flexible linkers.

U.S. Patent 4,680,338 describes bifunctional linkers useful for producing conjugates of ligands with amine-containing polymers and/or proteins, especially for forming antibody conjugates with chelators, drugs, enzymes, detectable labels and the like. U.S. Patents 5,141,648 and 5,563,250 disclose cleavable conjugates containing a labile bond that is cleavable under a variety of mild conditions. This linker is particularly useful in that the agent of interest may be bonded directly to the linker, with cleavage resulting in release of the active agent. Particular uses include adding a free amino or free sulfhydryl group to a protein, such as an antibody, or a drug.

U.S. Patent 5,856,456 provides peptide linkers for use in connecting polypeptide constituents to make fusion proteins, *e.g*., single chain antibodies. The linker is up to about 50 amino acids in length, contains at least one occurrence of a charged amino acid (preferably arginine or lysine) followed by a proline, and is characterized by greater stability and reduced aggregation. U.S. Patent 5,880,270 discloses aminooxy-containing linkers useful in a variety of immunodiagnostic and separative techniques.

### E. Purification

In certain embodiments, the antibodies of the present disclosure may be purified. The term "purified," as used herein, is intended to refer to a composition, isolatable from other components, wherein the protein is purified to any degree relative to its naturally-obtainable state. A purified protein therefore also refers to a protein, free from the environment in which it may naturally occur. Where the term "substantially purified" is used, this designation will refer to a composition in which the protein or peptide forms the major component of the composition, such as constituting about 50%, about 60%, about 70%, about 80%, about 90%, about 95% or more of the proteins in the composition.

Protein purification techniques are well known to those of skill in the art. These techniques involve, at one level, the crude fractionation of the cellular milieu to polypeptide and non-polypeptide fractions. Having separated the polypeptide from other proteins, the polypeptide of interest may be further purified using chromatographic and electrophoretic techniques to achieve partial or complete purification (or purification to homogeneity). Analytical methods particularly suited to the preparation of a pure peptide are ion-exchange chromatography, exclusion chromatography; polyacrylamide gel electrophoresis; isoelectric focusing. Other methods for protein purification include, precipitation with ammonium sulfate, PEG, antibodies and the like or by heat denaturation, followed by centrifugation; gel filtration, reverse phase, hydroxylapatite and affinity chromatography; and combinations of such and other techniques.

In purifying an antibody of the present disclosure, it may be desirable to express the polypeptide in a prokaryotic or eukaryotic expression system and extract the protein using denaturing conditions. The polypeptide may be purified from other cellular components using an affinity column, which binds to a tagged portion of the polypeptide. As is generally known in the art, it is believed that the order of conducting the various purification steps may be changed, or that certain steps may be omitted, and still result in a suitable method for the preparation of a substantially purified protein or peptide.

Commonly, complete antibodies are fractionated utilizing agents (*i.e.,* protein A) that bind the Fc portion of the antibody. Alternatively, antigens may be used to simultaneously purify and select appropriate antibodies. Such methods often utilize the selection agent bound to a support, such as a column, filter or bead. The antibodies is bound to a support, contaminants removed (*e.g*., washed away), and the antibodies released by applying conditions (salt, heat, *etc*.)*.*

Various methods for quantifying the degree of purification of the protein or peptide will be known to those of skill in the art in light of the present disclosure. These include, for example, determining the specific activity of an active fraction, or assessing the amount of polypeptides within a fraction by SDS/PAGE analysis. Another method for assessing the purity of a fraction is to calculate the specific activity of the fraction, to compare it to the specific activity of the initial extract, and to thus calculate the degree of purity. The actual units used to represent the amount of activity will, of course, be dependent upon the particular assay technique chosen to follow the purification and whether or not the expressed protein or peptide exhibits a detectable activity.

Various It is known that the migration of a polypeptide can vary, sometimes significantly, with different conditions of SDS/PAGE (Capaldi *et al.,* 1977). It will therefore be appreciated that under differing electrophoresis conditions, the apparent molecular weights of purified or partially purified expression products may vary.

### IV. Treatment of Cancer

### A. Formulation and Administration

The present disclosure provides pharmaceutical compositions comprising anti-LILRB antibodies and antigens for generating the same. Such compositions comprise a prophylactically or therapeutically effective amount of an antibody or a fragment thereof, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a particular carrier when the pharmaceutical composition is administered intravenously. Saline solutions and aqueous dextrose and glycerol solutions can also be employed as liquid carriers, particularly for injectable solutions. Other suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like. Oral formulations can include standard carriers such as pharmaceutical grades of mannitol, lactose, starch, magnesium stearate, sodium saccharine, cellulose, magnesium carbonate, *etc.* Examples of suitable pharmaceutical agents are described in "Remington's Pharmaceutical Sciences." Such compositions will contain a prophylactically or therapeutically effective amount of the antibody or fragment thereof, preferably in purified form, together with a suitable amount of carrier so as to provide the form for proper administration to the patient. The formulation should suit the mode of administration, which can be oral, intravenous, intraarterial, intrabuccal, intranasal, nebulized, bronchial inhalation, or delivered by mechanical ventilation.

Antibodies of the present disclosure, as described herein, can be formulated for parenteral administration, *e.g.,* formulated for injection *via* the intradermal, intravenous, intra-arterial, intramuscular, subcutaneous, intra-tumoral or even intraperitoneal routes. The antibodies could alternatively be administered by a topical route directly to the mucosa, for example by nasal drops, inhalation, or by nebulizer. Pharmaceutically acceptable salts include the acid salts and those which are formed with inorganic acids such as, for example, hydrochloric or phosphoric acids, or such organic acids as acetic, oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

Passive transfer of antibodies, known as artificially acquired passive immunity, generally will involve the use of intravenous injections. The forms of antibody can be human or animal blood plasma or serum, as pooled human immunoglobulin for intravenous (IVIG) or intramuscular (IG) use, as high-titer human IVIG or IG from immunized or from donors recovering from disease, and as monoclonal antibodies (MAb). Such immunity generally lasts for only a short period of time, and there is also a potential risk for hypersensitivity reactions, and serum sickness, especially from gamma globulin of non-human origin. However, passive immunity provides immediate protection. The antibodies will be formulated in a carrier suitable for injection, *i.e.,* sterile and syringeable.

Generally, the ingredients of compositions of the disclosure are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compositions of the disclosure can be formulated as neutral or salt forms. Pharmaceutically acceptable salts include those formed with anions such as those derived from hydrochloric, phosphoric, acetic, oxalic, tartaric acids, *etc.,* and those formed with cations such as those derived from sodium, potassium, ammonium, calcium, ferric hydroxides, isopropylamine, triethylamine, 2-ethylamino ethanol, histidine, procaine, *etc.*

### B. Cell Therapies

In another aspect, the present disclosure provides immune cells which express a chimeric antigen receptor (CAR). In some embodiment, The CAR comprises an antigen-binding fragment provided herein. In an embodiment, the CAR protein includes from the N-terminus to the C-terminus: a leader peptide, an anti-LILRB3 heavy chain variable domain, a linker domain, an anti-LILRB3 light chain variable domain, a human IgG1-CH2-CH3 domain, a spacer region, a CD28 transmembrane domain, a 4-1BB intracellular co-stimulatory signaling and a CD3 ζ intracellular T cell signaling domain.

Also provided are methods for immunotherapy comprising administering an effective amount of the immune cells of the present disclosure. In one embodiment, a medical disease or disorder is treated by transfer of an immune cell population that elicits an immune response. In certain embodiments of the present disclosure, cancer or infection is treated by transfer of an immune cell population that elicits an immune response. Provided herein are methods for treating or delaying progression of cancer in an individual comprising administering to the individual an effective amount of an antigen-specific cell therapy.

The immune cells may be T cells (*e.g*., regulatory T cells, CD4+ T cells, CD8+ T cells, or gamma-delta T cells), NK cells, invariant NK cells, NKT cells, or macrophages. Also provided herein are methods of producing and engineering the immune cells as well as methods of using and administering the cells for adoptive cell therapy, in which case the cells may be autologous or allogeneic. Thus, the immune cells may be used as immunotherapy, such as to target cancer cells.

The immune cells may be isolated from subjects, particularly human subjects. The immune cells can be obtained from healthy human subjects, healthy volunteers, or healthy donors. The immune cells can be obtained from a subject of interest, such as a subject suspected of having a particular disease or condition, a subject suspected of having a predisposition to a particular disease or condition, or a subject who is undergoing therapy for a particular disease or condition. Immune cells can be collected from any location in which they reside in the subject including, but not limited to, blood, cord blood, spleen, thymus, lymph nodes, and bone marrow. The isolated immune cells may be used directly, or they can be stored for a period of time, such as by freezing.

The immune cells may be enriched/purified from any tissue where they reside including, but not limited to, blood (including blood collected by blood banks or cord blood banks), spleen, bone marrow, tissues removed and/or exposed during surgical procedures, and tissues obtained via biopsy procedures. Tissues/organs from which the immune cells are enriched, isolated, and/or purified may be isolated from both living and non-living subjects, wherein the non-living subjects are organ donors. In particular embodiments, the immune cells are isolated from blood, such as peripheral blood or cord blood. In some aspects, immune cells isolated from cord blood have enhanced immunomodulation capacity, such as measured by CD4- or CD8-positive T cell suppression. In specific aspects, the immune cells are isolated from pooled blood, particularly pooled cord blood, for enhanced immunomodulation capacity. The pooled blood may be from 2 or more sources, such as 3, 4, 5, 6, 7, 8, 9, 10 or more sources (*e.g*., donor subjects).

The population of immune cells can be obtained from a subject in need of therapy or suffering from a disease associated with reduced immune cell activity. Thus, the cells will be autologous to the subject in need of therapy. Alternatively, the population of immune cells can be obtained from a donor, preferably a histocompatibility matched donor. The immune cell population can be harvested from the peripheral blood, cord blood, bone marrow, spleen, or any other organ/tissue in which immune cells reside in said subject or donor. The immune cells can be isolated from a pool of subjects and/or donors, such as from pooled cord blood.

When the population of immune cells is obtained from a donor distinct from the subject, the donor is preferably allogeneic, provided that the cells obtained are subject-compatible in that they can be introduced into the subject. Allogeneic donor cells may or may not be human-leukocyte-antigen (HLA)-compatible. To be rendered subject-compatible, allogeneic cells can be treated to reduce immunogenicity.

The immune cells can be genetically engineered to express antigen receptors such as engineered TCRs and/or chimeric antigen receptors (CARs). For example, the host cells (*e.g*., autologous or allogeneic T-cells) are modified to express a T cell receptor (TCR) having antigenic specificity for a cancer antigen. In particular embodiments, NK cells are engineered to express a TCR. The NK cells may be further engineered to express a CAR. Multiple CARs and/or TCRs, such as to different antigens, may be added to a single cell type, such as T cells or NK cells.

Suitable methods of modification are known in the art. See, for instance, Sambrook *et al., supra;* and Ausubel et al., CURRENT PROTOCOLS IN MOLECULAR BIOLOGY, Greene Publishing Associates and John Wiley & Sons, NY, 1994. For example, the cells may be transduced to express a T cell receptor (TCR) having antigenic specificity for a cancer antigen using transduction techniques described in Heemskerk *et al.* (2008) and Johnson *et al.* (2009).

In some embodiments, the cells comprise one or more nucleic acids introduced via genetic engineering that encode one or more antigen receptors, and genetically engineered products of such nucleic acids. In some embodiments, the nucleic acids are heterologous, *i.e.*, normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature (*e.g.,* chimeric).

### C. Combination Therapies

It may also be desirable to provide combination treatments using antibodies of the present disclosure in conjunction with additional anti-cancer therapies. These therapies would be provided in a combined amount effective to achieve a reduction in one or more disease parameter. This process may involve contacting the cells/subjects with the both agents/therapies at the same time, *e.g*., using a single composition or pharmacological formulation that includes both agents, or by contacting the cell/subject with two distinct compositions or formulations, at the same time, wherein one composition includes the antibody and the other includes the other agent.

Alternatively, the antibody may precede or follow the other treatment by intervals ranging from minutes to weeks. One would generally ensure that a significant period of time did not expire between the time of each delivery, such that the therapies would still be able to exert an advantageously combined effect on the cell/subject. In such instances, it is contemplated that one would contact the cell with both modalities within about 12-24 hours of each other, within about 6-12 hours of each other, or with a delay time of only about 12 hours. In some situations, it may be desirable to extend the time period for treatment significantly; however, where several 10 days (2, 3, 4, 5, 6 or 7) to several weeks (1, 2, 3, 4, 5, 6, 7 or 8) lapse between the respective administrations.

It also is conceivable that more than one administration of either the anti-LILRB3 antibody or the other therapy will be desired. Various combinations may be employed, where the antibody is "A," and the other therapy is "B," as exemplified below:
A/B/A B/A/B B/B/A A/A/B B/A/A A/B/B B/B/B/A B/B/A/B
A/A/B/B A/B/A/B A/B/B/A B/B/A/A B/A/B/A B/A/A/B B/B/B/A
A/A/A/B B/A/A/A A/B/A/A A/A/B/A A/B/B/B B/A/B/B B/B/A/B

Other combinations are contemplated. To kill cells, inhibit cell growth, inhibit metastasis, inhibit angiogenesis or otherwise reverse or reduce the malignant phenotype of tumor cells, using the methods and compositions of the present invention, one may contact a target cell or site with an antibody and at least one other therapy. These therapies would be provided in a combined amount effective to kill or inhibit proliferation of cancer cells. This process may involve contacting the cells/site/subject with the agents/therapies at the same time.

Particular agents contemplated for combination therapy with antibodies of the present disclosure include chemotherapy and hematopoietic stem cell transplantation. Chemotherapy may include cytarabine (ara-C) and an anthracycline (most often daunorubicin), high-dose cytarabine alone, all-trans-retinoic acid (ATRA) in addition to induction chemotherapy, usually an anthracycline, histamine dihydrochloride (Ceplene) and interleukin 2 (Proleukin) after the completion of consolidation therapy, gemtuzumab ozogamicin (Mylotarg) for patients aged more than 60 years with relapsed AML who are not candidates for high-dose chemotherapy, clofarabine, as well as targeted therapies, such as kinase inhibitors, farnesyl transferase inhibitors, decitabine, and inhibitors of MDR1 (multidrug-resistance protein), or arsenic trioxide or relapsed acute promyelocytic leukemia (APL).

In certain embodiments, the agents for combination therapy are one or more drugs selected from the group consisting of a topoisomerase inhibitor, an anthracycline topoisomerase inhibitor, an anthracycline, a daunorubicin, a nucleoside metabolic inhibitor, a cytarabine, a hypomethylating agent, a low dose cytarabine (LDAC), a combination of daunorubicin and cytarabine, a daunorubicin and cytarabine liposome for injection, Vyxeos^{®}, an azacytidine, Vidaza^{®}, a decitabine, an all-trans-retinoic acid (ATRA), an arsenic, an arsenic trioxide, a histamine dihydrochloride, Ceplene^{®}, an interleukin-2, an aldesleukin, Proleukin^{®}, a gemtuzumab ozogamicin, Mylotarg^{®}, an FLT-3 inhibitor, a midostaurin, Rydapt^{®}, a clofarabine, a farnesyl transferase inhibitor, a decitabine, an IDH1 inhibitor, an ivosidenib, Tibsovo^{®}, an IDH2 inhibitor, an enasidenib, Idhifa^{®}, a smoothened (SMO) inhibitor, a glasdegib, an arginase inhibitor, an IDO inhibitor, an epacadostat, a BCL-2 inihbitor, a venetoclax, Venclexta^{®}, a platinum complex derivative, oxaliplatin, a kinase inhibitor, a tyrosine kinase inhibitor, a PI3 kinase inhibitor, a BTK inhibitor, an ibrutinib, IMBRUVICA^{®}, an acalabrutinib, CALQUENCE^{®}, a zanubrutinib, a PD-1 antibody, a PD-L1 antibody, a CTLA-4 antibody, a LAG3 antibody, an ICOS antibody, a TIGIT antibody, a TIM3 antibody, a CD40 antibody, a 4-1BB antibody, a CD47 antibody, a SIRP1α antibody or fusions protein, a CD70 antibody, and CLL1 antibody, a CD123 antibody, an antagonist of E-selectin, an antibody binding to a tumor antigen, an antibody binding to a T-cell surface marker, an antibody binding to a myeloid cell or NK cell surface marker, an alkylating agent, a nitrosourea agent, an antimetabolite, an antitumor antibiotic, an alkaloid derived from a plant, a hormone therapy medicine, a hormone antagonist, an aromatase inhibitor, and a P-glycoprotein inhibitor.

### V. Antibody Conjugates

Antibodies of the present disclosure may be linked to at least one agent to form an antibody conjugate. In order to increase the efficacy of antibody molecules as diagnostic or therapeutic agents, it is conventional to link or covalently bind or complex at least one desired molecule or moiety. Such a molecule or moiety may be, but is not limited to, at least one effector or reporter molecule. Effector molecules comprise molecules having a desired activity, *e.g*., cytotoxic activity. Non-limiting examples of effector molecules which have been attached to antibodies include toxins, anti-tumor agents, therapeutic enzymes, radionuclides, antiviral agents, chelating agents, cytokines, growth factors, and oligo- or polynucleotides. By contrast, a reporter molecule is defined as any moiety which may be detected using an assay. Non-limiting examples of reporter molecules which have been conjugated to antibodies include enzymes, radiolabels, haptens, fluorescent labels, phosphorescent molecules, chemiluminescent molecules, chromophores, photoaffinity molecules, colored particles or ligands, such as biotin.

Antibody-drug conjugates have emerged as a breakthrough approach to the development of cancer therapeutics. Antibody-drug conjugates (ADCs) comprise monoclonal antibodies (MAbs) that are covalently linked to cell-killing drugs. This approach combines the high specificity of MAbs against their antigen targets with highly potent cytotoxic drugs, resulting in "armed" MAbs that deliver the payload (drug) to tumor cells with enriched levels of the antigen. Targeted delivery of the drug also minimizes its exposure in normal tissues, resulting in decreased toxicity and improved therapeutic index. The approval of two ADC drugs, ADCETRIS^{®} (brentuximab vedotin) in 2011 and KADCYLA^{®} (trastuzumab emtansine or T-DM1) in 2013 by FDA validated the approach. There are currently more than 30 ADC drug candidates in various stages of clinical trials for cancer treatment (Leal *et al.,* 2014). As antibody engineering and linker-payload optimization are becoming more and more mature, the discovery and development of new ADCs are increasingly dependent on the identification and validation of new targets that are suitable to this approach and the generation of targeting MAbs. Two criteria for ADC targets are upregulated/high levels of expression in tumor cells and robust internalization.

Antibody conjugates are also preferred for use as diagnostic agents. Antibody diagnostics generally fall within two classes, those for use in *in vitro* diagnostics, such as in a variety of immunoassays, and those for use *in vivo* diagnostic protocols, generally known as "antibody-directed imaging." Many appropriate imaging agents are known in the art, as are methods for their attachment to antibodies (see, for *e.g.,* U.S. Patents 5,021,236, 4,938,948, and 4,472,509). The imaging moieties used can be paramagnetic ions, radioactive isotopes, fluorochromes, NMR-detectable substances, and X-ray imaging agents.

In the case of paramagnetic ions, one might mention by way of example ions such as chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) and/or erbium (III), with gadolinium being particularly preferred. Ions useful in other contexts, such as X-ray imaging, include but are not limited to lanthanum (III), gold (III), lead (II), and especially bismuth (III).

In the case of radioactive isotopes for therapeutic and/or diagnostic application, one might mention astatine²¹¹, ¹⁴carbon, ⁵¹chromium, ³⁶chlorine, ⁵⁷cobalt, ⁵⁸cobalt, copper⁶⁷, ¹⁵²Eu, gallium⁶⁷, ³hydrogen, iodine¹²³, iodine¹²⁵ , iodine¹³¹, indium¹¹¹, ⁵⁹iron, ³²phosphorus, rhenium¹⁸⁶, rhenium¹⁸⁸, ⁷⁵selenium, ³⁵sulphur, technicium^{99m} and/or yttrium⁹⁰. ¹²⁵I is often being preferred for use in certain embodiments, and technicium^{99m} and/or indium¹¹¹ are also often preferred due to their low energy and suitability for long range detection. Radioactively labeled monoclonal antibodies of the present disclosure may be produced according to well-known methods in the art. For instance, monoclonal antibodies can be iodinated by contact with sodium and/or potassium iodide and a chemical oxidizing agent such as sodium hypochlorite, or an enzymatic oxidizing agent, such as lactoperoxidase. Monoclonal antibodies according to the disclosure may be labeled with technetium^{99m} by ligand exchange process, for example, by reducing pertechnate with stannous solution, chelating the reduced technetium onto a Sephadex column and applying the antibody to this column. Alternatively, direct labeling techniques may be used, *e.g.,* by incubating pertechnate, a reducing agent such as SNCl₂, a buffer solution such as sodium-potassium phthalate solution, and the antibody. Intermediary functional groups which are often used to bind radioisotopes which exist as metallic ions to antibody are diethylenetriaminepentaacetic acid (DTPA) or ethylene diaminetetracetic acid (EDTA).

Among the fluorescent labels contemplated for use as conjugates include Alexa 350, Alexa 430, AMCA, BODIPY 630/650, BODIPY 650/665, BODIPY-FL, BODIPY-R6G, BODIPY-TMR, BODIPY-TRX, Cascade Blue, Cy3, Cy5,6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, TAMRA, TET, Tetramethylrhodamine, and/or Texas Red.

Another type of antibody conjugate contemplated in the present disclosure are those intended primarily for use *in vitro,* where the antibody is linked to a secondary binding ligand and/or to an enzyme (an enzyme tag) that will generate a colored product upon contact with a chromogenic substrate. Examples of suitable enzymes include urease, alkaline phosphatase, (horseradish) hydrogen peroxidase or glucose oxidase. Preferred secondary binding ligands are biotin and avidin and streptavidin compounds. The use of such labels is well known to those of skill in the art and are described, for example, in U.S. Patents 3,817,837, 3,850,752, 3,939,350, 3,996,345, 4,277,437, 4,275,149 and 4,366,241.

Yet another known method of site-specific attachment of molecules to antibodies comprises the reaction of antibodies with hapten-based affinity labels. Essentially, hapten-based affinity labels react with amino acids in the antigen binding site, thereby destroying this site and blocking specific antigen reaction. However, this may not be advantageous since it results in loss of antigen binding by the antibody conjugate.

Molecules containing azido groups may also be used to form covalent bonds to proteins through reactive nitrene intermediates that are generated by low intensity ultraviolet light (Potter and Haley, 1983). In particular, 2- and 8-azido analogues of purine nucleotides have been used as site-directed photoprobes to identify nucleotide binding proteins in crude cell extracts (Owens & Haley, 1987; Atherton *et al.,* 1985). The 2- and 8-azido nucleotides have also been used to map nucleotide binding domains of purified proteins (Khatoon *et al.,* 1989; King *et al.,* 1989; Dholakia *et al.,* 1989) and may be used as antibody binding agents.

Several methods are known in the art for the attachment or conjugation of an antibody to its conjugate moiety. Some attachment methods involve the use of a metal chelate complex employing, for example, an organic chelating agent such a diethylenetriaminepentaacetic acid anhydride (DTPA); ethylenetriaminetetraacetic acid; N-chloro-p-toluenesulfonamide; and/or tetrachloro-3α-6α-diphenylglycouril-3 attached to the antibody (U.S. Patents 4,472,509 and 4,938,948). Monoclonal antibodies may also be reacted with an enzyme in the presence of a coupling agent such as glutaraldehyde or periodate. Conjugates with fluorescein markers are prepared in the presence of these coupling agents or by reaction with an isothiocyanate. In U.S. Patent 4,938,948, imaging of breast tumors is achieved using monoclonal antibodies and the detectable imaging moieties are bound to the antibody using linkers such as methyl-p-hydroxybenzimidate or N-succinimidyl-3-(4-hydroxyphenyl)propionate.

In other embodiments, derivatization of immunoglobulins by selectively introducing sulfhydryl groups in the Fc region of an immunoglobulin, using reaction conditions that do not alter the antibody combining site are contemplated. Antibody conjugates produced according to this methodology are disclosed to exhibit improved longevity, specificity and sensitivity (U.S. Patent 5,196,066, incorporated herein by reference). Site-specific attachment of effector or reporter molecules, wherein the reporter or effector molecule is conjugated to a carbohydrate residue in the Fc region have also been disclosed in the literature (O' Shannessy *et al.,* 1987). This approach has been reported to produce diagnostically and therapeutically promising antibodies which are currently in clinical evaluation.

### VI. Immunodetection Methods

In still further embodiments, the present disclosure concerns immunodetection methods for binding, purifying, removing, quantifying and otherwise generally detecting LILRB-related cancers. While such methods can be applied in a traditional sense, another use will be in quality control and monitoring of vaccine and other virus stocks, where antibodies according to the present disclosure can be used to assess the amount or integrity (*i.e.,* long term stability) of H1 antigens in viruses. Alternatively, the methods may be used to screen various antibodies for appropriate/desired reactivity profiles.

Some immunodetection methods include enzyme linked immunosorbent assay (ELISA), radioimmunoassay (RIA), immunoradiometric assay, fluoroimmunoassay, chemiluminescent assay, bioluminescent assay, and Western blot to mention a few. In particular, a competitive assay for the detection and quantitation of LILRBs also is provided. The steps of various useful immunodetection methods have been described in the scientific literature, such as, *e.g.,* Doolittle and Ben-Zeev (1999), Gulbis and Galand (1993), De Jager *et al.* (1993), and Nakamura *et al.* (1987). In general, the immunobinding methods include obtaining a sample suspected of containing LILRB-related cancers and contacting the sample with a first antibody in accordance with the present disclosure, as the case may be, under conditions effective to allow the formation of immunocomplexes.

These methods include methods for detecting or purifying LILRBs or LILRB-related cancer cells from a sample. The antibody will preferably be linked to a solid support, such as in the form of a column matrix, and the sample suspected of containing the LILRB-related cancer cells will be applied to the immobilized antibody. The unwanted components will be washed from the column, leaving the LILRB-expressing cells immunocomplexed to the immobilized antibody, which is then collected by removing the organism or antigen from the column.

The immunobinding methods also include methods for detecting and quantifying the amount of LILRB-related cancer cells or related components in a sample and the detection and quantification of any immune complexes formed during the binding process. Here, one would obtain a sample suspected of containing LILRB-related cancer cells and contact the sample with an antibody that binds LILRBs or components thereof, followed by detecting and quantifying the amounts of immune complexes formed under the specific conditions. In terms of antigen detection, the biological sample analyzed may be any sample that is suspected of containing LILRB-related cancers, such as a tissue section or specimen, a homogenized tissue extract, a biological fluid, including blood and serum, or a secretion, such as feces or urine.

Contacting the chosen biological sample with the antibody under effective conditions and for a period of time sufficient to allow the formation of immune complexes (primary immune complexes) is generally a matter of simply adding the antibody composition to the sample and incubating the mixture for a period of time long enough for the antibodies to form immune complexes with, *i.e.,* to bind to LILRBs. After this time, the sample-antibody composition, such as a tissue section, ELISA plate, dot blot or Western blot, will generally be washed to remove any non-specifically bound antibody species, allowing only those antibodies specifically bound within the primary immune complexes to be detected.

In general, the detection of immunocomplex formation is well known in the art and may be achieved through the application of numerous approaches. These methods are generally based upon the detection of a label or marker, such as any of those radioactive, fluorescent, biological and enzymatic tags. Patents concerning the use of such labels include U.S. Patents 3,817,837, 3,850,752, 3,939,350, 3,996,345, 4,277,437, 4,275,149 and 4,366,241. Of course, one may find additional advantages through the use of a secondary binding ligand such as a second antibody and/or a biotin/avidin ligand binding arrangement, as is known in the art.

The antibody employed in the detection may itself be linked to a detectable label, wherein one would then simply detect this label, thereby allowing the amount of the primary immune complexes in the composition to be determined. Alternatively, the first antibody that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under effective conditions and for a period of time sufficient to allow the formation of secondary immune complexes. The secondary immune complexes are then generally washed to remove any non-specifically bound labeled secondary antibodies or ligands, and the remaining label in the secondary immune complexes is then detected.

Further methods include the detection of primary immune complexes by a two-step approach. A second binding ligand, such as an antibody that has binding affinity for the antibody, is used to form secondary immune complexes, as described above. After washing, the secondary immune complexes are contacted with a third binding ligand or antibody that has binding affinity for the second antibody, again under effective conditions and for a period of time sufficient to allow the formation of immune complexes (tertiary immune complexes). The third ligand or antibody is linked to a detectable label, allowing detection of the tertiary immune complexes thus formed. This system may provide for signal amplification if this is desired.

One method of immunodetection uses two different antibodies. A first biotinylated antibody is used to detect the target antigen, and a second antibody is then used to detect the biotin attached to the complexed biotin. In that method, the sample to be tested is first incubated in a solution containing the first step antibody. If the target antigen is present, some of the antibody binds to the antigen to form a biotinylated antibody/antigen complex. The antibody/antigen complex is then amplified by incubation in successive solutions of streptavidin (or avidin), biotinylated DNA, and/or complementary biotinylated DNA, with each step adding additional biotin sites to the antibody/antigen complex. The amplification steps are repeated until a suitable level of amplification is achieved, at which point the sample is incubated in a solution containing the second step antibody against biotin. This second step antibody is labeled, as for example with an enzyme that can be used to detect the presence of the antibody/antigen complex by histoenzymology using a chromogen substrate. With suitable amplification, a conjugate can be produced which is macroscopically visible.

Another known method of immunodetection takes advantage of the immuno-PCR (Polymerase Chain Reaction) methodology. The PCR method is similar to the Cantor method up to the incubation with biotinylated DNA, however, instead of using multiple rounds of streptavidin and biotinylated DNA incubation, the DNA/biotin/streptavidin/antibody complex is washed out with a low pH or high salt buffer that releases the antibody. The resulting wash solution is then used to carry out a PCR reaction with suitable primers with appropriate controls. At least in theory, the enormous amplification capability and specificity of PCR can be utilized to detect a single antigen molecule.

### 1. ELISAs

Immunoassays, in their most simple and direct sense, are binding assays. Certain preferred immunoassays are the various types of enzyme linked immunosorbent assays (ELISAs) and radioimmunoassays (RIA) known in the art. Immunohistochemical detection using tissue sections is also particularly useful. However, it will be readily appreciated that detection is not limited to such techniques, and western blotting, dot blotting, FACS analyses, and the like may also be used.

In one exemplary ELISA, the antibodies of the disclosure are immobilized onto a selected surface exhibiting protein affinity, such as a well in a polystyrene microtiter plate. Then, a test composition suspected of containing the LILRB-related cancer cells is added to the wells. After binding and washing to remove non-specifically bound immune complexes, the bound antigen may be detected. Detection may be achieved by the addition of another anti-LILRB antibody that is linked to a detectable label. This type of ELISA is a simple "sandwich ELISA." Detection may also be achieved by the addition of a second anti-LILRB3 antibody, followed by the addition of a third antibody that has binding affinity for the second antibody, with the third antibody being linked to a detectable label.

In another exemplary ELISA, the samples suspected of containing the LILRB3-related cancer cells are immobilized onto the well surface and then contacted with the anti- LILRB3 antibodies of the disclosure. After binding and washing to remove non-specifically bound immune complexes, the bound anti-LILRB3 antibodies are detected. Where the initial anti-LILRB3 antibodies are linked to a detectable label, the immune complexes may be detected directly. Again, the immune complexes may be detected using a second antibody that has binding affinity for the first anti-LILRB3 antibody, with the second antibody being linked to a detectable label.

Irrespective of the format employed, ELISAs have certain features in common, such as coating, incubating and binding, washing to remove non-specifically bound species, and detecting the bound immune complexes. These are described below.

In coating a plate with either antigen or antibody, one will generally incubate the wells of the plate with a solution of the antigen or antibody, either overnight or for a specified period of hours. The wells of the plate will then be washed to remove incompletely adsorbed material. Any remaining available surfaces of the wells are then "coated" with a nonspecific protein that is antigenically neutral with regard to the test antisera. These include bovine serum albumin (BSA), casein or solutions of milk powder. The coating allows for blocking of nonspecific adsorption sites on the immobilizing surface and thus reduces the background caused by nonspecific binding of antisera onto the surface.

In ELISAs, it is probably more customary to use a secondary or tertiary detection means rather than a direct procedure. Thus, after binding of a protein or antibody to the well, coating with a non-reactive material to reduce background, and washing to remove unbound material, the immobilizing surface is contacted with the biological sample to be tested under conditions effective to allow immune complex (antigen/antibody) formation. Detection of the immune complex then requires a labeled secondary binding ligand or antibody, and a secondary binding ligand or antibody in conjunction with a labeled tertiary antibody or a third binding ligand.

"Under conditions effective to allow immune complex (antigen/antibody) formation" means that the conditions preferably include diluting the antigens and/or antibodies with solutions such as BSA, bovine gamma globulin (BGG) or phosphate buffered saline (PBS)/Tween. These added agents also tend to assist in the reduction of nonspecific background.

The "suitable" conditions also mean that the incubation is at a temperature or for a period of time sufficient to allow effective binding. Incubation steps are typically from about 1 to 2 to 4 hours or so, at temperatures preferably on the order of 25°C to 27°C or may be overnight at about 4°C or so.

Following all incubation steps in an ELISA, the contacted surface is washed so as to remove non-complexed material. A preferred washing procedure includes washing with a solution such as PBS/Tween, or borate buffer. Following the formation of specific immune complexes between the test sample and the originally bound material, and subsequent washing, the occurrence of even minute amounts of immune complexes may be determined.

To provide a detecting means, the second or third antibody will have an associated label to allow detection. Preferably, this will be an enzyme that will generate color development upon incubating with an appropriate chromogenic substrate. Thus, for example, one will desire to contact or incubate the first and second immune complex with a urease, glucose oxidase, alkaline phosphatase or hydrogen peroxidase-conjugated antibody for a period of time and under conditions that favor the development of further immune complex formation (*e.g*., incubation for 2 hours at room temperature in a PBS-containing solution such as PBS-Tween).

After incubation with the labeled antibody, and subsequent to washing to remove unbound material, the amount of label is quantified, *e.g*., by incubation with a chromogenic substrate such as urea, or bromocresol purple, or 2,2'-azino-di-(3-ethyl-benzthiazoline-6-sulfonic acid (ABTS), or H₂O₂, in the case of peroxidase as the enzyme label. Quantification is then achieved by measuring the degree of color generated, *e.g*., using a visible spectra spectrophotometer.

### 2. Western Blot

The Western blot (alternatively, protein immunoblot) is an analytical technique used to detect specific proteins in a given sample of tissue homogenate or extract. It uses gel electrophoresis to separate native or denatured proteins by the length of the polypeptide (denaturing conditions) or by the 3-D structure of the protein (native/ non-denaturing conditions). The proteins are then transferred to a membrane (typically nitrocellulose or PVDF), where they are probed (detected) using antibodies specific to the target protein.

Samples may be taken from whole tissue or from cell culture. In most cases, solid tissues are first broken down mechanically using a blender (for larger sample volumes), using a homogenizer (smaller volumes), or by sonication. Cells may also be broken open by one of the above mechanical methods. However, it should be noted that bacteria, virus or environmental samples can be the source of protein and thus Western blotting is not restricted to cellular studies only. Assorted detergents, salts, and buffers may be employed to encourage lysis of cells and to solubilize proteins. Protease and phosphatase inhibitors are often added to prevent the digestion of the sample by its own enzymes. Tissue preparation is often done at cold temperatures to avoid protein denaturing.

The proteins of the sample are separated using gel electrophoresis. Separation of proteins may be by isoelectric point (pI), molecular weight, electric charge, or a combination of these factors. The nature of the separation depends on the treatment of the sample and the nature of the gel. This is a very useful way to determine a protein. It is also possible to use a two-dimensional (2-D) gel which spreads the proteins from a single sample out in two dimensions. Proteins are separated according to isoelectric point (pH at which they have neutral net charge) in the first dimension, and according to their molecular weight in the second dimension.

In order to make the proteins accessible to antibody detection, they are moved from within the gel onto a membrane made of nitrocellulose or polyvinylidene difluoride (PVDF). The membrane is placed on top of the gel, and a stack of filter papers placed on top of that. The entire stack is placed in a buffer solution which moves up the paper by capillary action, bringing the proteins with it. Another method for transferring the proteins is called electroblotting and uses an electric current to pull proteins from the gel into the PVDF or nitrocellulose membrane. The proteins move from within the gel onto the membrane while maintaining the organization they had within the gel. As a result of this blotting process, the proteins are exposed on a thin surface layer for detection (see below). Both varieties of membrane are chosen for their non-specific protein binding properties (*i.e.,* binds all proteins equally well). Protein binding is based upon hydrophobic interactions, as well as charged interactions between the membrane and protein. Nitrocellulose membranes are cheaper than PVDF but are far more fragile and do not stand up well to repeated probings. The uniformity and overall effectiveness of transfer of protein from the gel to the membrane can be checked by staining the membrane with Coomassie Brilliant Blue or Ponceau S dyes. Once transferred, proteins are detected using labeled primary antibodies, or unlabeled primary antibodies followed by indirect detection using labeled protein A or secondary labeled antibodies binding to the Fc region of the primary antibodies.

### 3. Immunohistochemistry

The antibodies of the present disclosure may also be used in conjunction with both fresh-frozen and/or formalin-fixed, paraffin-embedded tissue blocks prepared for study by immunohistochemistry (IHC). The method of preparing tissue blocks from these particulate specimens has been successfully used in previous IHC studies of various prognostic factors and is well known to those of skill in the art (Brown *et al.,* 1990; Abbondanzo *et al.,* 1990; Allred *et al.,* 1990).

Briefly, frozen-sections may be prepared by rehydrating 50 ng of frozen "pulverized" tissue at room temperature in phosphate buffered saline (PBS) in small plastic capsules; pelleting the particles by centrifugation; resuspending them in a viscous embedding medium (OCT); inverting the capsule and/or pelleting again by centrifugation; snap-freezing in -70°C isopentane; cutting the plastic capsule and/or removing the frozen cylinder of tissue; securing the tissue cylinder on a cryostat microtome chuck; and/or cutting 25-50 serial sections from the capsule. Alternatively, whole frozen tissue samples may be used for serial section cuttings.

Permanent-sections may be prepared by a similar method involving rehydration of the 50 mg sample in a plastic microfuge tube; pelleting; resuspending in 10% formalin for 4 hours fixation; washing/pelleting; resuspending in warm 2.5% agar; pelleting; cooling in ice water to harden the agar; removing the tissue/agar block from the tube; infiltrating and/or embedding the block in paraffin; and/or cutting up to 50 serial permanent sections. Again, whole tissue samples may be substituted.

### 4. Immunodetection Kits

In still further embodiments, the present disclosure concerns immunodetection kits for use with the immunodetection methods described above. As the antibodies may be used to detect LILRB-related cancer cells, the antibodies may be included in the kit. The immunodetection kits will thus comprise, in suitable container means, a first antibody that binds to an LILRB, and optionally an immunodetection reagent.

In certain embodiments, the antibody may be pre-bound to a solid support, such as a column matrix and/or well of a microtitre plate. The immunodetection reagents of the kit may take any one of a variety of forms, including those detectable labels that are associated with or linked to the given antibody. Detectable labels that are associated with or attached to a secondary binding ligand are also contemplated. Exemplary secondary ligands are those secondary antibodies that have binding affinity for the first antibody.

Further suitable immunodetection reagents for use in the present kits include the two-component reagent that comprises a secondary antibody that has binding affinity for the first antibody, along with a third antibody that has binding affinity for the second antibody, the third antibody being linked to a detectable label. As noted above, a number of exemplary labels are known in the art and all such labels may be employed in connection with the present disclosure.

The kits may further comprise a suitably aliquoted composition of LILRBs, whether labeled or unlabeled, as may be used to prepare a standard curve for a detection assay. The kits may contain antibody-label conjugates either in fully conjugated form, in the form of intermediates, or as separate moieties to be conjugated by the user of the kit. The components of the kits may be packaged either in aqueous media or in lyophilized form.

The container means of the kits will generally include at least one vial, test tube, flask, bottle, syringe or other container means, into which the antibody may be placed, or preferably, suitably aliquoted. The kits of the present disclosure will also typically include a means for containing the antibody, antigen, and any other reagent containers in close confinement for commercial sale. Such containers may include injection or blow-molded plastic containers into which the desired vials are retained.

### 5. Flow Cytometry and FACS

The antibodies of the present disclosure may also be used in flow cytometry or FACS. Flow cytometry is a laser- or impedance-based technology employed in many detection assays, including cell counting, cell sorting, biomarker detection and protein engineering. The technology suspends cells in a stream of fluid and passing them through an electronic detection apparatus, which allows simultaneous multiparametric analysis of the physical and chemical characteristics of up to thousands of particles per second. Flow cytometry is routinely used in the diagnosis disorders, especially blood cancers, but has many other applications in basic research, clinical practice and clinical trials.

Fluorescence-activated cell sorting (FACS) is a specialized type of cytometry. It provides a method for sorting a heterogenous mixture of biological cells into two or more containers, one cell at a time, based on the specific light scattering and fluorescent characteristics of each cell. In general, the technology involves a cell suspension entrained in the center of a narrow, rapidly flowing stream of liquid. The flow is arranged so that there is a large separation between cells relative to their diameter. A vibrating mechanism causes the stream of cells to break into individual droplets. Just before the stream breaks into droplets, the flow passes through a fluorescence measuring station where the fluorescence of each cell is measured. An electrical charging ring is placed just at the point where the stream breaks into droplets. A charge is placed on the ring based immediately prior to fluorescence intensity being measured, and the opposite charge is trapped on the droplet as it breaks form the stream. The charged droplets then fall through an electrostatic deflection system that diverts droplets into containers based upon their charge.

**In** certain embodiments, to be used in flow cytometry or FACS, the antibodies of the present disclosure are labeled with fluorophores and then allowed to bind to the cells of interest, which are analyzed in a flow cytometer or sorted by a FACS machine.

### VII. Examples

The following examples are included to demonstrate preferred embodiments of the disclosure. It should be appreciated by those of skill in the art that the techniques disclosed in the examples which follow represent techniques discovered by the inventor to function well in the practice of the disclosure, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the disclosure.

### Example 1 - Materials and Methods

**Mice.** C57BL/6J and NOD-SCID IL2Rγ-null (NSG) mice were purchased from and maintained at the animal core facility of the University of Texas Southwestern Medical Center. GFP-specific TCR mice (Jedi mice, JAX lab stock No: 028062) were purchased from the Jackson Lab. C57BL/6J CD45.1 mice were injected intraperitoneally with 1 x 10⁶ irradiated MLL-AF9 mouse AML cells (3000 cGy) and LPS or LPS alone as a control. The injection was repeated 10 days later. C57BL/6J CD45.2 recipient mice were lethally irradiated (1000 cGy) and injected with mouse MLL-AF9 AML cells and 0.5 x 10⁶ CD45.2 bone marrow cells. NSG mice at 5-8 weeks old were engrafted with AML cells or human T cells via tail injection. Mice in each experiment were 5-8 weeks old female mice. All work in this study was approved by the UT Southwestern Institutional Animal Care and Use Committee.

**LILRB3 transgenic mice.** LILRB3 cDNA was constructed into pR26 CAG AsiSI/MluI. Then the plasmid was purified with high concentration in the absence of endotoxin. Cas9 RNA, gRNA targeting mouse Rosa26 locus and the LILRB3 plasmid co-injected into mouse oocytes at the transgenic core facility of UTSW. The LILRB3 positive mice were identified by LILRB3 specific primers and crossbred with LysMcre mice (JAX, 004781). The LILRB3⁺LysMcre⁺ mice were analyzed with co-expression of LELRB3 and Mac-1 or Gr-1 in peripheral blood, spleen and bone marrow.

**Cell culture.** THP-1, MV4-11, Molm13, U937, C1498 and 293T cells were purchased from ATCC. AML cell lines were cultured in RPMI-1640 with 10% FBS. Human anti-LILRB3 with the N297A mutation was coated onto the plate to activate LILRB3 signaling, and plates coated with human IgG (N297A) were used as controls. Cells infected with virus were cultured for at least an additional 3 weeks before analysis of LILRB3 signaling. Dead cells were identified using PI staining. Primary human T cells were isolated by autoMACS from donor PBMCs, stimulated with anti-CD3 and anti-CD28, and cultured in RPMI-1640 in the presence of IL-2. For the cytotoxic T lymphocyte assay, human AML cells were stained with CFSE and mixed at different ratios with activated T cells. After 10 hours, the percentage of PI-positive CFSE-stained AML cells was determined by FACS.

**Flow cytometry.** Primary antibodies including anti-mouse CD8a-PE (BioLegend, 100707, 53-6.7, 1:100), anti-mouse CD45-PE (BD Pharmingen,561087, 30-F11, 1:100), anti-mouse CD45.1-FITC (BioLegend, 110705, A20, 1:100), anti-mouse CD45.2-PE (BioLegend, 109807,104, 1:100), anti-human CD45-PE (BD Pharmingen, 555483, HI30, 1:100), anti-human CD33-APC (Biolegend,366605, P67.6, 1:100), anti-human CD3-FITC (BioLegend, 300305, HIT3a, 1:100), anti-human CD8-PE (BD Pharmingen, 555367, RPA-T8, 1:100) antibodies were used. Cells were run on Calibur for analysis or FACSAria for sorting.

**Plasmids.** TRAF2 and cFLIP were cloned from human cDNA. LILRB3, TRAF2, cFLIP, and dominant-negative TRAF2 (245-501) were constructed in the pLVX-IRES-ZsGreen vector. LILRBs, LAIR1, LILRB3 fragments, and chimeric protein CAR-LILRB3 were fused with hFc at C-termini in the pFLAG-CMV5.1 vector. LILRB3-specific shRNAs were constructed in pLL3.7. Tet-on cFLIP and Cre were constructed by replacing Cas9 in the plasmid pCW-Cas9 with FL-cFLIP and Cre, respectively. For infection of mouse cells, full-length LILRB3 was inserted into the MSCV-IRES-GFP vector to create B3-FL; LILRB3 with the intracellular domain deleted was inserted into the MSCV-IRES-GFP vector to create B3del ICD.

**NF-κB reporter assay and LILRB3 chimeric receptor reporter** assay. THP-1-Lucia^{™} NF-κB cells were purchased from InvivoGen. Human anti-LILRB3 antibody with the N297A mutation was coated onto the plate to activate LILRB3 signaling, and plates coated with hIgG (N297A) were used as the control. The activation of NF-κB signaling was evaluated by monitoring luciferase signal. Infected THP-1 reporter cells were cultured for an additional month before stimulation with anti-LILRB3. For the NF-κB reporter assay conducted in 293T cells, an NF-κB-driven firefly luciferase reporter plasmid co-transfected with a plasmid encoding CMV-driven Renilla luciferase along with plasmids expressing LILRB3, TRAF2, or cFLIP were transfected into cells. The luciferase activity was detected using the Dual-Luciferase^{®} Reporter (DLR^{™}) Assay System (Promega). LILRB3 chimeric receptor reporter cells were constructed as the inventors described (Deng *et al.,* 2014; 2018; Kang *et al.,* 2015), with LILRB3-ECD fused with the transmembrane and intracellular domains of paired immunoglobulin-like receptor β, which signals through the adaptor DAP-12 to activate the NFAT promoter.

**Virus production and infection.** For lentivirus production, plasmid pLL3.7 shRNA and pLVX-IRES-ZsGreen cFLIP, dominant-negative TRAF2, or tet-on pCW-Cre were mixed with psPAX2 and pMD2.G at a ratio of 4:3:1 and transfected into 293T cells using polyjet (SignaGen). To produce retrovirus, plasmid MSCV-IRES-GFP with B3-FL or B3del ICD were mixed with pCL-ECO (2:1) and transfected into 293T cells. The supernatant containing virus was collected 48-72 hours after transfection. Human AML cell lines were infected with virus supernatant by centrifugation at 1800 rpm at 37 °C for 2 hours following three hours' incubation before changing to the regular culture medium. Fresh mouse MLL-AF9 AML cells were infected with virus supernatant mixed with StemSpan (StemCell) with mSCF, IL3, and IL6. After infection, the virus supernatant was replaced with StemSpan with mSCF, IL3, and IL6. Cells were cultured for an additional 2 days before isolating infected cells.

**Primary human leukemia.** The primary human AML samples was obtained from the University of Texas Southwestern Medical Center. The informed consent was obtained and approved by the Institutional Review Board of the University of Texas Southwestern Medical Center (IRB STU 122013-023). For primary transplantation, leukemia cells isolated from donor peripheral blood was injected into irradiated NSG mice (200 cGy), and antibody or IgG was introduced intravenously 8 days after injection. For secondary transplantation, human leukemia cells from frozen BM of NSG mice that were engrafted with patient AML cells were transplanted into sublethally-irradiated NSG recipients.

**Western blotting and co-immunoprecipitation.** Cells were lysed with RIPA buffer containing protease inhibitor (Roche Diagnostic). Samples were mixed with 2X SDS loading buffer and separated by SDS-PAGE. After transfer to nitrocellulose membranes, the protein was detected with specific primary antibodies and HRP-conjugated secondary antibodies. The primary antibodies were anti-cFLIP (R&D Systems, MAB8430, 1:500), anti-LILRB3 (R&D Systems, MAB1806, 1:500), anti-TRAF2 (Novus Biologicals, NB100-56715, 1:500), anti-HA (BioLegend 901513 1:2000), anti-FLAG (BioLegend, 637319, 1:2000), Anti-MLKL (phospho S358, abcam, ab187091, 1:500), Anti-MLKL (abcam, ab184718, 1:1000) and anti-actin (BioLegend, 664801, 1:10000). For immunoprecipitation, cells were lysed with Pierce IP Lysis Buffer (Thermo Fisher Scientific, 87787). A total of 1x10⁹ primary M5 AML leukemia cells were lysed for analyzing the interaction of LILRB3 and TRAF2, and the LILRB3 and TRAF2 complex was immunoprecipitated with human anti-LILRB3 N297A mutant. HA- or FLAG-tagged TRAF2 and cFLIP were co-expressed with hFc-tagged LILRB3 or LILRB3 fragments in 293T cells. Dynabeads protein A (Thermo Fisher Scientific, 10001D) was used in all co-immunoprecipitation. For evaluating the interaction of TRAF2 and LILRB3 in vitro, purified GST-TRAF2 (MyBioSource, MBS515700) was incubated with Dynabeads protein A binding with the intracellular domain of LILRB3 fused with hFc at C terminal overnight at 4 °C.

**Quantitative RT-PCR.** Total RNA was isolated from AML cells or primary CD14⁺ monocytes isolated from PBMC. First-strand cDNA was synthesized with a High-Capacity cDNA Reverse Transcription Kit (Applied Biosystems). The following primers were ordered from Sigma: 5'-GAAGAAACTCAACTGGTGTCG-3' (SEQ ID NO: 422) and 5'-CCAAGTCTGTGTCCTGAACG-3' (SEQ ID NO: 423) to detect TNFAIP3F, 5'-GAAGGCTACCAACTACAATGG-3' (SEQ ID NO: 424) and 5'-TTCAACAGGAGTGACACCAG-3' (SEQ ID NO: 425) to detect NFKB1AF, 5'-GAATCACCAGCAGCAAGTG-3' (SEQ ID NO: 426) and 5'-CTTCGGAGTTTGGGTTTG-3' (SEQ ID NO: 427) to detect CCL2, 5'-TTGTGCGTCTCCTCAGTAAA-3' (SEQ ID NO: 428) and 5'-CAAGTGAAACCTCCAACCC-3' (SEQ ID NO: 429) to detect CCL20, 5'-CATTGAGGAGGATTGCCAAA-3' (SEQ ID NO: 430) and 5'-ACAAACTGGATGTCGCTGG-3' (SEQ ID NO: 431) to detect Myd88, 5'-ACGCTCTTCTGCCTGCT-3' (SEQ ID NO: 432) and 5'-GCTTGAGGGTTTGCTACAA-3' (SEQ ID NO: 433) to detect TNFa, 5'-TGGCTTATTACAGTGGCAATG-3' (SEQ ID NO: 434) and 5'-TGGTGGTCGGAGATTCGT-3' (SEQ ID NO: 435) to detect IL1B, 5'-CTTTCTGCTGACATCGCC-3' (SEQ ID NO: 436) and 5'-GTCTGCCGTAGGTTGTTGTA-3' (SEQ ID NO: 437) to detect BCL3, 5'-ACGCAGACATCGTCATCC-3' (SEQ ID NO: 438) and 5'-CAAACCGAGTTGGAACCAC-3' (SEQ ID NO: 439) to detect MMP9, 5'-CATACTTACCCACTTCAAGGG-3' (SEQ ID NO: 440) and 5'-TTGTAGCCATAGTCAGCATTGT-3' (SEQ ID NO: 441) to detect PTGS2, and 5'-GATGGGGTCTTCATCTG-3' (SEQ ID NO: 442) and 5'-CGTAGGTGGATGCCTCC-3' (SEQ ID NO: 443) to detect TRAF2. The mRNA levels were normalized to the level of GAPDH present in the same sample.

TCGA analyses. The AML patient data were obtained from TCGA (version: August 16, 2016) and classified into AML subtypes (FAB classification). The mRNA levels were determined by RNA-seq, and LILRB3 expression of each subtype was averaged. The overall survival was analyzed based on LILRB3 expression and the corresponding patient survival data. Patients were grouped based on significant LILRB3 expression cutoff for survival analysis.

RNA-seq analysis. RNA was extracted from AML cells using the Qiagen RNeasy Mini kit according to the manufacturer's instructions, and then reverse-transcribed with SuperScript III Reverse Transcriptase (Invitrogen). RNA-seq was performed as previously reported (Deng *et al.,* 2018).

**GESA analysis.** Gene set enrichment analysis was performed using GSEA v4.0 software (Subramanian *et al.,* 2005) (world-wide-web at broadinstitute.org/gsea/index.jsp) with 1000 phenotype permutations.

**Generation of LILRB3 antibody.** Phage panning: A complete human scFv phage library was generated in house and used for LILRB3 antibody panning. Briefly, human LILRB3 extracellular domain protein was coated onto wells of a 96-well plate, a pre-blocked phage library was added to LILRB3-coated wells, and samples were incubated for 2 hours at room temperature. Wells were washed to remove unbound phage, and bound phage were eluted and used to infect TG1 bacterial for amplification. The panning process was repeated to enrich for high-affinity binders.

V_{H} and V_{L} sequence evaluation: Sequences of phage bound to LILRB3 were analyzed using GeneBank IgBLAST1.10.0 to identify germline V(D)J gene segments. Individual V_{H} and V_{L} genes were mapped to the germline of major IGL and IGH locus. Framework and CDR sequences were annotated according to IMGT (world-wide-web at imgt.org/) nomenclature.

Cloning of VH and VL encoding genes into full human IgG vector: The V_{H} and V_{L} encoding genes from the phage plasmids were cloned into a human IgG-expressing vector. Briefly, DNA fragments encoding V_{H} and V_{L} were amplified by PCR using family-leader region-specific primers. The PCR product of V_{H} and V_{L} genes, around 400 bp, were collected and purified for infusion PCR. Infusion PCR was carried out using the In-Fusion^{®} HD Cloning kit (Clontech).

Expression of antibodies by HEK293F cells: Human anti-LILRB3 antibodies were expressed in mammalian cells (HEK293F) and purified using affinity chromatography with Protein A resin. Briefly, equal molar amounts of heavy-chain and light-chain plasmids were co-transfected into HEK293F cells for transient expression of antibodies. Supernatants were harvested after 7 days in culture, and IgGs were purified with Protein A resin (GE Healthcare).

Affinity measurement and epitope binning: Affinity measurement and epitope binning were done as described previously (Gui *et al.,* 2019). Briefly, antibody affinity was analyzed with the Octet RED96 instrument. Antibody (30 mg/mL) was loaded onto the protein A biosensors then exposed to a series of concentrations of recombinant LILRB3 (0.1-200 nmol/L), and background subtraction was used to correct for sensor drift. ForteBio's data analysis software was used to extract association and dissociation rates assuming a 1:1 binding model. The K_{d} was calculated as the ratio k_{off}/kₒₙ. Epitope binning of anti-LILRB3 rabbit antibodies was performed with an Octet RED96 instrument using a classical sandwich epitope binning assay. In these epitope binning assays, primary antibodies (40 µg/ml) were loaded onto protein A biosensors, and remaining Fc-binding sites on the sensor were blocked with a human non-targeting IgG (200 µg/ml). The sensors were then exposed to the 1 µM LILRB3 diluted in 1 × kinetics buffer, followed by the secondary antibodies (40 µg/ml). Raw data were processed using ForteBio's Data Analysis Software 7.0. Antibody pairs were assessed for competitive binding. Additional binding by the secondary antibody indicates an unoccupied epitope (the antibodies of the pair are not competitors), and no binding indicates epitope blocking (the antibodies of the pair are competitors for the same epitope).

**Statistics and Reproducibility.** Statistical significance of differences was assessed using the two-tailed Student's t-test. Animal survival analysis was assessed with the log rank test. A p-value of 0.05 or less was considered significant. Values are reported as mean ± s.e.m. All replicates for in vitro data are derived from independent experiments. No statistical method was used to predetermine sample size. No data were excluded from the analyses. Experiments using cultured cells and mice were randomized. For detecting protein levels inside the cells and interactions of proteins, immune blotting was conducted and repeated twice for confirming the results.

### Example 2 - Results

**LILRB3 supports AML by enhancing leukemia cell survival.** This analysis indicated that expression of LILRB3 is negatively correlated with the overall survival of AML patients (FIG. 1a). Further, these results showed that LILRB3 is highly expressed on monocytic AML cells (FAB M4 and M5 AML subtypes; FIG. 1b). Analysis of 35 AML patient samples indicates that LILRB3 is co-expressed with LILRB4, a monocytic AML cell marker (Deng *et al.,* 2018), on AML cells (FIG. S1a). This suggests that LILRB3 is mainly expressed on monocytic AML cells. Several AML cell lines, including THP-1, Molm13, and MV4, had cell-surface expression of LILRB3 (FIG. 1c). LILRB3 signaling was activated in AML cells by treatment with immobilized anti-LILRB3 antibody that leads to receptor clustering. The percentage of cell death was significantly lower for these AML cells treated with immobilized anti-LILRB3 antibody than in AML cells treated with a control IgG either in the presence or absence of AML drugs (FIG. 1d, FIG. S1b).

AML cells treated with LILRB3-specific shRNAs (FIG. 1e) had normal proliferation after 3 additional weeks of culture (FIG. S1c). In contrast, AML progression of NSG mice implanted with LILRB3-silenced Molm-13 cells was significantly delayed (FIG. 1f). Next, the inventors implanted NSG mice with THP-1 AML cells and applied an shRNA delivery system that can be induced by tet-on CRE to silence LILRB3 in these cells (Tiscornia *et al.,* 2004) (FIG. S1d). When induced at 19 days after AML cell transplantation, the expression of the shRNA targeting LILRB3 slowed AML development (FIG. 1σ).

Leukemia patients usually have higher TNF-α levels than healthy individuals (Sanchez-Correa *et al.,* 2013). The inventors observed that LILRB3 activation significantly reduced cell death associated with increasing levels of TNF-α (FIG. S1e). TNF-α has dual roles in apoptosis and survival (McEwan, 2002). These results suggest that LILRB3 enhances TNF-α survival signaling and attenuates its cell death signaling. Nevertheless, activated LILRB3 enhanced cell survival with treatment of anti-TNFα neutralizing antibodies (FIG. S1f), suggesting the function of LILRB3 does not depend on TNFα.

The inventors implanted PirB-defective MLL-AF9 AML cells overexpressing full-length LILRB3 (B3-FL) or a mutant LILRB3 with deletion of the intracellular domain (B3del ICD) by retroviral infection (Syken *et al.,* 2006) (FIG. 1h) into syngeneic immuno-competent C57BL/6 mice. The lack of LILRB3 intracellular domain led to significantly reduced AML load, decreased sizes of spleens and livers, lower colony-forming unit (CFU) activity, and prolonged survival (FIGS. 1i-m). Experiments with mouse AML C1498 cells that ectopically expressed the full-length LILRB3 or a mutant LILRB3 with the intracellular domain deletion also indicated that LILRB3 supports AML development in immuno-competent mice (FIGS. S1g-h). Immobilized anti-LILRB3 had little effect on THP-1 cell growth in culture (FIG. S11). MLL-AF9-expressing mouse AML cells with full-length or intracellular domain truncated LILRB3 remained the similar colony forming ability after serial replating (FIG. S1j), suggesting that LILRB3 does not affect AML cell self-renew in vitro. Overexpression of LILRB3 in LILRB3-negative U937 AML cells increased cell survival (FIG. S1k), confirming the survival-promoting function of LILRB3.

**LILRB3⁺ AML cells inhibit T cell activity.** Monocytic AML cells suppress T cell function (Deng *et al.,* 2018). LILRB3⁺ THP-1 cells activated with immobilized anti-LILRB3 antibodies significantly reduced the level of AML cell death in the presence of CTLs (FIGS. 2a,b). To further evaluate whether LILRB3 expressed on AML cells has an effect on T cell function, T cells were injected 4 days after Molm13 AML cell transplantation into NSG mice. In the presence of T cells, LILRB3-silenced Molm13 AML cells developed significantly more slowly than did AML cells expressing the control shRNA (FIGS. 2c,d). These results suggest that LILRB3 in AML cells inhibits T cell function.

To further test the significance of T cells, the inventors depleted CD8 T cells in C57BL/6 recipient mice with anti-mCD8 antibodies (FIG. S2a) and evaluated the leukemia development initiated by PirB-defective MIL-AF9 AML cells with B3-FL or B3del ICD. With CD8 T cell depletion, AML cells with B3-FL still enabled faster leukemia development than those with B3del ICD, but the difference between these two groups was much smaller than their difference in control conditions (FIGS. 2e,f). The results suggest that T cell play a critical role in LILRB3 function in AML development. The inventors then developed tumor-specific mouse T cells by immunizing CD45.1 C57BL/6 mice with mouse AML cells that express MLL-AF9 (Hasegawa *et al.,* 2015) twice with 10 days apart, and isolated CD3⁺ cells from the spleens. These T cells were co-injected with MLL-AF9 AML cells into recipient CD45.2 C57BL/6 mice (FIG. 2g). T cells from mice immunized with LPS were used as a control. T cells specific for MLL-AF9 AML cells (T-AF9 cells) showed a greater ability to kill MLL-AF9 AML cells than did T cells from LPS-treated mice (T-LPS cells) *in vitro* (FIG. 2h), though the T-AF9 cells did not kill the normal BM cells (FIG. S2b). AML cells with B3-FL were more resistant to killing by T-AF9 cells than their counterparts with B3del ICD, suggesting that LILRB3 signaling in AML cells decreases T cell-mediated killing.

The inventors then transplanted C57BL/6 mice with PirB-defective AML cells expressing B3del ICD or B3-FL (with a double number of AML cells with B3del ICD transplanted into each mouse than the AML cells with B3 FL, which made easier to compare the leukemia development in the presence of tumor-specific T cells). The two groups of mice had similar AML development in the absence of injected CD45.1 T cells or with non-specific T cells (T-LPS) (FIG. 2i). In contrast, mice co-injected with tumor-specific T cells (T-AF9) had significantly slower AML development. Importantly, B3del ICD AML developed more slowly than did B3-FL AML in the presence of the specific T cells (FIGS. 2i,j). There were higher percentages of tumor-specific CD45.1⁺ T cells in mice with B3del ICD AML. In contrast, the percentages of non-specific CD45.1⁺ T cells were the same in the two groups of mice (FIG. 2k).

To further investigate the function of LILRB3 in AML development using antigen-specific T cells, the inventors injected MIL-AF9 AML C57BL/6 CD45.2 mice with GFP specific CD3⁺ cells isolated from the spleens of CD45.1 transgenic mice whose T cell express GFP-targeting TCRs. PirB-defective MLL-AF9 AML cells with B3del ICD progressed much slower than those with B3-FL in mice injected with GFP-specific T cells, whereas these two groups of AML had similar development in mice injected with WT naive T cells (FIGS. 2l,m). A greater number of CD8 GFP-specific T cells and higher expression of INFγ and TNFα as well as lower expression of PD1 in T cells were detected in mice with B3del ICD AML cells than in mice with B3 FL AML cells (FIG. 2n and FIG. S2c). Together, these results indicate that LILRB3 expressed on AML cells inhibits T cell activity and that the signaling domain of LILRB3 is important in this function.

**LILRB3 activates NF-κB signaling by recruiting TRAF2.** RNA-seq was conducted in THP-1 cells treated with immobilized anti-LILRB3 antibody or control IgG. GO enrichment analysis indicates that LILRB3 activation is correlated with TNF signaling, Toll-like receptor signaling, and NOD-like receptor signaling (FIG. 3a). These signaling pathways are all known to stimulate NF-κB signaling (MacEwan, 2002; Kawasaki & Kawai, 2014; Shaw *et al.,* 2010). GSEA analysis showed that immobilized anti-LILRB3 antibody activates NF-κB signaling (FIG. 3b). RNA-seq analysis of MIL-AF9 mouse AML cells ectopically expressed B3-FL or B3del ICD was conducted. Results of this analysis also suggest that LILRB3 enhanced NF-κB signaling (FIGS. S3a-b). These results were in agreement with the previous finding that NF-κB signaling in tumor cells supports cell survival and T cell inhibitory activity (Taniguchi & Karin, 2018).

In THP-1 cells that express a luciferase reporter regulated by NF-κB signaling, culture in the presence of immobilized anti-LILRB3 antibodies stimulated the luciferase activity (FIG. 3c) and increased levels of phosphorylated p65 protein (FIG. 3d). To investigate whether LILRB3 interacts with TNFa signaling proteins, the inventors transfected LILRB3 together with TRADD, FADD, or TRAF2, which mediate TNFa signaling, into 293T cells. Then, they examined whether expression of the NF-κB reporter was affected. Overexpression of TRADD, FADD, or TRAF2 in 293T cells activated NF-κB signaling (FIG. 3e). LILRB3 significantly enhanced the activity of TRAF2 but did not alter the activity of TRADD or FADD (FIG. 3e). Overexpression of dominant-negative TRAF2 (dnTRAF2) (Cannons *et al.,* 2002) in THP-1 cells abolished the stimulation of NF-κB reporter and the effect on survival of AML cells in the presence of immobilized anti-LILRB3 antibody (FIGS. 3f-h). Xenograft experiments were conducted to evaluate disease progress in NSG mice engrafted with AML cells that overexpress dnTRAF2. Results showed this progress was similar to that in mice engrafted with cells in which LILRB3 expression was silenced (FIG. 3i). The interaction between LILRB3 and TRAF2 was detected in the primary M5 AML patient sample by co-immunoprecipitation (FIG. 4a) and confirmed in vitro by co-immunoprecipitation of purified recombinant proteins (FIG. 4b). In addition to stimulation of NF-κB signaling, TRAF2 was reported to activate JNK signaling in the TNFa pathway (Nishitoh *et al.,* 1998). LILRB3 expression did not enhance JNK signaling (FIG. S8c). This result suggests that TRAF2 interaction with LILRB3 activates NF-κB signaling through a path separate from TNFa signaling. This is concordant with the previous finding that TRAF2-mediated signaling does not require association with the TNF receptor-complex (Reinhard *et al.,* 1997).

Co-immunoprecipitation experiments in 293T cells indicated that the fragment containing amino acids 500-520 of LILRB3 is essential for the interaction with TRAF2 (FIG. 4c-e). LILRB3 with amino acids 509-511 mutated from QEE to AAA or with deletion of amino acids 505-515 did not interact with TRAF2 (FIG. 4f), and neither of these mutants activated expression of the NF-κB reporter (FIG. 4g). The sequences in LILRB3 related to the binding to TRAF2 are conserved in other LILRBs (FIG. 4h). Nevertheless, of LILRBs tested, only LILRB3 bound strongly to TRAF2 (FIG. 4i). Further analysis of interactions between the intracellular domains of LILRBs and TRAF2 suggests the membrane-proximal segments of the intracellular domains of LILRBs suppress the interactions between LILRBs and TRAF2 (FIG. 4j). Analyses of TRAF2 fragments indicate that the TRAF-C domain (aa 351-501) mediates the interaction with LILRB3 (FIG. 4k). PirB-defective MLL-AF9 mouse AML cells expressing LILRB3 with amino acids 509-511 mutated from QEE to AAA (that does not bind TRAF2) had slower AML development than their counterparts with full-length LILRB3 in immuno-competent mice (FIGS. 4l,m). This indicates that the ability of interaction with TRAF2 is important to the function of LILRB3 in AML cells.

**LILRB3 activates NF-κB signaling via cFLIP.** cFLIP inhibits apoptosis. The N-terminal fragment p22-FLIP, a product of cFLIP digestion by caspase 8 (Golks *et al.,* 2006), activated NF-κB signaling in 293T cells (FIG. S4a) as previously reported (Kataoka & Tschopp, 2004). Caspase 8 inhibitors z-VAD-FMK and z-IETD-FMK (Kataoka & Tschopp, 2004), inhibited the NF-κB reporter activity activated by LILRB3 (FIG. 5a). This result suggests that caspase 8 is required for NF-κB signaling stimulated by LILRB3. Immobilized anti-LILRB3 treatment increased cFLIP and p22-FLIP protein levels in THP-1 cells (FIG. 5b). A low level of cFLIP stimulates whereas a high level of cFLIP inhibits caspase 8 activity (Hughes *et al.,* 2016; Chang *et al.,* 2002). When a Tet-on cFLIP construct was transfected into 293T cells, a low concentration of doxycycline (dox) stimulated NF-κB reporter activity (FIG. S4b). LILRB3 enhanced the ability of cFLIP to activate NF-κB at a low concentration of dox (FIG. 5c). These results suggest that NF-κB activation by LILRB3 depends on a low level of cFLIP. As in THP-1 cells, z-VAD-FMK also blocked LILRB3-induced NF-κB activity in 293T cells (FIG. 5d).

TRAF2 can bind to cFLIP (FIG. S9c) (Kataoka & Tschopp, 2004). Unlike TRAF2, only full-length LILRB3 could recruit cFLIP when co-expressed in 293T cells (FIG. 5e). The inability of the intracellular domain of LELRB3 alone to recruit cFLIP suggests that crosslinking of LILRB3 on the membrane is required for formation of the complex of LILRB3 with cFLIP. Co-expression of TRAF2 enhanced the interaction of LELRB3 and cFLIP (FIG. 5f), suggesting that LILRB3 simultaneously interacts with TRAF2 and cFLIP. Overexpression of dnTRAF2 blocked LILRB3-mediated enhancement of NF-κB signaling in the presence of a low level of cFLIP (FIG. 5g). z-VAD-FMK also blocked the ability of LILRB3 to stimulate NF-κB through TRAF2 (FIG. 5h). Together, these results suggest that the interaction of cFLIP and TRAF2 is critical for LILRB3 signaling. Endogenous TRAF2 does not stimulate NF-κB signaling without cFLIP in 293T cells (Kataoka *et al.,* 2004). In the presence and absence of exogenous TRAF2 in 293T cells, LILRB3 enhanced NF-κB signaling when a low level of cFLIP was present, but not at a high level of cFLIP (FIG. 5i), confirming the cooperation of TRAF2 and cFLIP in LILRB3 signaling.

When THP-1 cells overexpressing TRAF2 or cFLIP were co-cultured with activated T cells, the cytotoxicity of T cells was significantly decreased (FIGS. S4d-e). With overexpression of TRAF2 or FLIP in THP-1 cells, activated T cells kill the same percentages of THP-1 cells in plates with coated anti-LILRB3 and coated IgG (FIG. 5j). These results suggest that LILRB3 protects AML cells from T cell killing via TRAF2 and FLIP. Caspase 8 could induce apoptosis and inhibit necroptosis (Fritsch *et al.,* 2019), and apoptosis does not induce immune response and necroptosis results in immune response (Bertheloot *et al.,* 2021). THP-1 cells treated with immobilized anti-LILRB3 decreased phospho-MLKL (a mediator of necroptosis), suggesting that LILRB3 signaling inhibits necroptosis and decreases immune response (FIG. S4f).

Blocking NF-κB signaling with its inhibitor QNZ in THP-1 cells partially abolished the effect of LILRB3 on survival of THP-1 cells (FIG. S4g). This result strengthens the inventors' finding that LILRB3 enhances AML cell survival at least partially by stimulating NF-κB signaling.

**LILRB3 inhibits NF-κB signaling upon NF-κB hyperactivation.** Next the inventors aimed to identify the context in which LILRB3 acts as an inhibitory receptor. With a relatively high level of LPS (200 µg/L), the activation of LILRB3 signaling in THP-1 cells by the immobilized anti-LILRB3 led to inhibition of NF-κB reporter activity (FIG. 6a) and decreased levels of phosphorylated p65 (FIG. 6b).

SHP-1 and SHP-2 mediate the inhibitory effect of LILRBs (Kang *et al.,* 2016). LILRB3 co-immunoprecipitated with endogenous SHP-1 and SHP-2 in THP-1 cells stably expressing LILRB3 (FIG. 6c). To identify the LILRB3 ITIMs that mediate the interactions, the inventors prepared a vector for expression of the transmembrane and intracellular domains of LILRB3 fused at the N-terminus to the extracellular domain of tight junction protein CAR (Wu & Zhang, 2020) and at the C-terminus to hFc (CARECD-B3ICD-hFc). They expected that homophilic interactions of CAR extracellular domains of the chimeric proteins would enhance LILRB3 clustering to facilitate activation of the receptor. When this fusion protein was ectopically co-expressed with SHP-1 and Lyn (a Src-like kinase required for LILRB ITIM phosphorylation) (Pereira & Lowell, 2003) in 293T cells, the inventors found that LILRB3 interacted with SHP-1 mainly via the last two C terminal ITIMs (FIG. 6d).

Next, the inventors performed studies to characterize the effect of LILRB3 on NF-κB signaling with SHP-1 and SHP-2 association. CARECD-B3ICD and the control CARECDTM (CARECD-B3ICD with LILRB3 intracellular domain deletion) were co-transfected with SHP-1 or SHP-2, the NF-κB reporter, and tet-on cFLIP in the presence or absence of Lyn. With 0.4 µg/ml dox treatment, CARECD-B3ICD enhanced NF-κB reporter activity without Lyn, but inhibited NF-κB reporter activity when Lyn was expressed in the presence of SHP-1 or SHP-2 (FIG. 6e).

Vectors for expression of CARECD-B3ICD with different ITIM mutations or CARECDTM were co-transfected into 293T cells exogenously expressing TLR4, CD14, and MD2 (Medvedev & Vogel, 2003). This enables the cells to respond to LPS treatment. In the presence of SHP-1 and Lyn, wild-type CARECD-B3ICD significantly inhibited NF-κB stimulated by LPS, whereas Y596/626F with double ITIM mutations and Y4xF with four ITIM mutations restored the inhibitory effect (FIG. 6f).

The high level of full-length cFLIP observed when NF-κB signaling is highly active could inhibit caspase 8 activity (Hughes *et al.,* 2016; Chang *et al.,* 2002; Micheau *et al.,* 2001). Indeed, the inventors found that co-expression of the full-length cFLIP or p22-FLIP with CARECD-B3ICD in 293T cells blocked the enhanced NF-κB activation by LILRB3 (FIG. 6g). Overexpression of full-length cFLIP also inhibited NF-κB activation in THP-1 cells cultured in plates coated with anti-LILRB3 antibodies (FIG. 6h).

Moreover, high LPS stimulation significantly decreased the association of TRAF2 and LILRB3 in THP-1 cells (FIG. 6i). A20 (also known as TNFAIP3), a protein that acts as a negative feedback regulator of NF-κB signaling (Shembade & Harhaj, 2012), mediates TRAF2 degradation (Li *et al.,* 2009). The A20 N-terminus (amino acids 1-386), which is known to be associated with TRAF2 (Song *et al.,* 1996), was ectopically expressed with HA-TRAF2, and CARECD-B3ICDhFc, B3ICDhFc, or CARECDTMhFc. The N-terminal fragment of A20 disrupted the interaction between TRAF2 and CARECD-B3ICDhFc, which is clustered through interactions of the CAR extracellular domains. However, it did not drastically affect the association of TRAF2 with B3ICDhFc, which is not crosslinked (FIG. 6j). In addition, co-expression of the A20 N-terminal fragment with CARECD-B3ICD in 293T cells disrupted the positive effect of LILRB3 on NF-κB signaling (FIG. 6k).

In THP-1 cells, LILRB3 enhanced NF-κB signaling in the presence of a low concentration of LPS but inhibited NF-κB signaling at a high level of LPS (FIG. 6l). The caspase 8 inhibitor z-VAD-FMK blocked LILRB3-induced NF-κB when the concentration of LPS was low. However, at a high concentration of LPS in the presence of z-VAD-FMK, LILRB3 activation by immobilized anti-LILRB3 inhibited NF-κB signaling (FIG. 6l). Interestingly, SHP-1/2 and TRAF2 were able to bind simultaneously to LILRB3 when they were co-overexpressed in 293T cells (FIGS. 6m,n).

In normal human monocytes, NF-κB target gene expression was significantly elevated after 5 hours of treatment with immobilized anti-LILRB3 and significantly decreased at 24 hours after the treatment especially in the presence of high level of LPS (FIG. 6o). This result implies that a relatively long-term activation of LILRB3 in normal monocytes results in the inhibitory effect, possibly due to increased expression of cFLIP (Micheau *et al.,* 2001) and A20 (Shembade * Harhaj, 2012), which block LILRB3 positive signaling. However, THP-1 AML cells could sustain NF-κB signaling activated by anti-LILRB3 antibody for as long as 2 days (FIG. S5a). One possible explanation is that AML cells express higher levels of TRAF2 than do normal monocytes (FIG. S5b).

Concordantly, MLL-AF9-expressing mouse AML cells with full-length LILRB3 led to decreased leukemia development when the immuno-competent mice were treated with LPS, whereas the development of AML characterized with LILRB3 intracellular domain truncation was unchanged with LPS treatment (FIGS. S5c-d). Together, these results suggest that LILRB3/TRAF2/cFLIP loop maintains a balanced NF-κB signaling (FIG. S5e).

**LILRB3 blocking antibodies inhibit AML progression.** In order to develop fully human anti-LILRB3 blocking antibodies, the inventors used sequential panning rounds of a highly diverse naive scFv phage library with increased stringency to select LILRB3-ECD bound phages (FIG. S6a). Unique scFv sequences were converted to fully humanized IgG format (FIG. S6a). Fifty of the 62 unique IgGs bind to LILRB3 on cells (FIG. S6a). Thirty had high affinity for LILRB3 as confirmed by ELISA (FIGS. S6b-c). These IgGs were grouped into three LILRB3 epitope bins by an Octet-based epitope binning assay (FIG. 7a). Only IgGs in the third bin (#1 and #26) blocked colony formation by MLL-AF9 cells (FIG. 7b). The affinities of selected IgGs in each epitope bin were evaluated (FIG. S6d, FIG. 7c). Both antibodies #1 and #26 are specific to LILBR3 (FIGS. S6e-f). Because IgG #1 had a higher affinity than #26 (FIG. 7c), this antibody was produced. This antibody, IgG#1, was further developed with an Fc glycosylation N297A mutation or LALAPG mutation, which has defective Fc-mediated effector functions (Tao & Morrison, 1989; Hristodorov *et al.,* 2013; Lo *et al.,* 2017).

Anti-LILRB3 #1 N297A was injected into immuno-competent mice transplanted with MLL-AF9 AML cells expressing B3-FL or B3delICD. Compared to injection with control IgG, anti-LILRB3 #1 N297A significantly delayed development of LILRB3-expressing AML (FIGS. 7d,e). In contrast, there was no detectable difference in B3del ICD AML development in mice treated with anti-LILRB3 #1 N297A and control IgG (FIGS. 7d,e). In addition, anti-LILRB3 #1 N297A decreased CFU formation by B3-FL but not B3del ICD AML cells (FIG. 7f). Treatment with anti-LILRB3 #1 N297A did not decrease CFU formation of AML cells that express a mutant LILRB3 that cannot interact with TRAF2 (FIG. S6g). When the recipient immuno-competent mice were injected with anti-mCD8 to deplete mouse CD8 T cells, the anti-AML effect of anti-LILRB3 #1 (with LALAPG mutant) diminished on AML with B3-FL (FIGS. 7g,h). This suggests that blocking LILRB3 signaling enhances T cell killing of AML cells. The inventors also injected AML immuno-competent mice with anti-LILRB3 #6 antibody, which cannot inhibit LILRB3 signaling (FIG. 7b). Anti-LILRB3 #6 suppresses the progresses of both AML with B3-FL and AML with B3del ICD with similar efficacies (FIGS. S6h-i). This result suggests that the Fc-mediated functions including antibody-dependent cellular cytotoxicity contribute to the anti-AML effects of anti-LILRB3 without LILRB3 signaling involved.

Anti-LILRB3 #1 N297A treatment of NSG mice xeongrafted with THP-1 cells also significantly delayed leukemia development compared to controls (FIG. 7i,j). The LILRB3 signaling in THP-1 cells decreased in NSG mice treated with anti-LILRB3 #1 N297A (FIG. S6j).

The inventors then injected NSG mice with activated human T cells four days after Molm13 AML cell transplantation (FIG. 7k). Anti-LILRB3 #1 N297A and control IgG were injected at day 8. Leukemia progression was significantly slower following anti-LILRB3 treatment than following IgG treatment. The leukemia in one of the anti-LILRB3-treated mice was largely eliminated (FIGS. 7k,l). Under these conditions, however, the anti-LILRB3 showed little effect on mice without the injection of T cells (FIGS. 7k-m), suggesting anti-LILRB3 #1 N297A enhances the anti-AML activity of T cells. There were significantly more T cells in the anti-LILRB3-treated mice than in the IgG-treated mice (FIG. 7n).

The efficacy of anti-LILRB3 antibody treatment was further tested in an M5 AML patient-derived xenograft models. LILRB3 was expressed on AML cells in the bone marrow of engrafted NSG mice as shown by flow cytometry analysis (FIG. 8a). Mice treated with anti-LILRB3 #1 N297A had significantly lower AML burden in peripheral blood, bone marrow, spleen, and liver than did mice treated with IgG (FIG. 8b). The anti-LILRB3 treatment also increased the percentages of human autologous T cells in the NSG mice (FIG. 8c). The mice treated with anti-LILRB3 had a significant survival advantage compared to mice treated with control IgG (FIG. 8d). In order to further dissect cell-autonomous and immune effects of the anti-LILRB3, NSG mice were transplanted with monocytic AML cells (derived from BM of NSG mice engrafted with monocytic AML patient peripheral blood samples) followed by treatment of IgG or anti-LILRB3 #1 LALAPG (FIGS. 8e-i). The NSG mice were then injected with activated human T cells or PBS. The anti-LILRB3 #1 LALAPG significantly decreased AML development in this model, and transplantation of activated human T cells enhanced the anti-AML effect of anti-LILRB3 #1 LALAPG (FIGS. 8e-i). These results indicate that the anti-AML efficacy of the anti-LILRB3 resulted from the combined effects of enhanced activity of tumor-specific T cells and direct leukemia killing.

Finally, the inventors developed myeloid LysM-Cre driven LILRB3-transgenic C57BL/6 mice (FIGS. S7a-c). The transgenic LILRB3 is expressed on myeloid cells. The treatment of these mice with anti-LILRB3 #1N297A antibody did not affect normal hematopoiesis and leukocytosis (FIGS. S7a-c), suggesting low toxicity of the anti-LILRB3 #1N297A.

### Example 3 - Discussion

Here the inventors demonstrated that LILRB3, expressed on AML cells, stimulates NF-κB signaling by recruiting TRAF2 and cFLIP and that this upregulation of NF-κB signaling enhances survival of AML cells and inhibits the anti-leukemia activity of T cells. The inventors also developed a blocking antibody that binds to LILRB3 and inhibits AML progression. Moreover, they showed that hyperactivation of NF-κB signaling resulted in negative feedback and the predominance of LILRB3 inhibitory signaling.

ITIMs were the only known signaling motifs in LILRBs; recruitment of phosphatase SHP-1 or SHP-2 to the activated ITIMs leads to signaling inhibition (Kang *et al.,* 2016). Here the inventors demonstrate that LILRB3 can also act as an activating receptor by interacting with TRAF2 and cFLIP. The unliganded LILRB3 constitutively associates with TRAF2. Once LILRB3 is activated by ligand binding, cFLIP is recruited to LILRB3/TRAF2 complex leading to NF-κB activation. The activated LILRB3 also recruits SHP-1 or SHP-2 to inhibit downstream signaling including NF-κB pathway. The inventors showed that LILRB3, TRAF2, cFLIP, SHP-1, and SHP-2 are present in the same complex under certain conditions. However, a high level of NF-κB activation can result in multiple negative feedback signals, including upregulation of A20 that mediates TRAF2 degradation (Shembade & Harhaj, 2012). These, in turn destabilize the LILRB3/TRAF2 interaction; consequently, the inhibitory signaling initiated by SHP-1/2 becomes dominant.

It was suggested that the activity of the ITIM-containing inhibitory receptors requires ITAM-containing receptors (Dietrich *et al.,* 2001), and an ITIM-containing receptor cannot activate by itself but needs to interact with an activating receptor. When the ITAM-containing activating receptor is activated, its ITAM recruits the Src tyrosine kinase (Dietrich *et al.,* 2001), which phosphorylates and thus activates the ITIMs of the nearby inhibitory receptors. This model explains TCR, BCR, FcR coupled LILRB signaling in T and B cells. Nevertheless, in monocytic cells, LILRB4 clustering *per se* without crosslinking with an ITAM receptor can induce SHP-1 recruitment (Cella *et al.,* 1997). Here, the inventors' novel finding that LILRB3 can recruit TRAF2 and cFLIP to activate NF-κB further suggests that LILRB can mediate ITAM-independent signaling.

The balance of stimulatory and inhibitory effects of LILRB3 on NF-κB signaling may be different in different cell types. The inventors speculate that inhibitory signaling by LILRB3 is dominant in normal monocytes. In contrast, AML cells, which have abnormally high expression of TRAF2 (Sawanobori *et al.,* 2003), are biased toward more positive signaling. This supports tumor development by providing survival cues and by immune inhibition.

The LILRBs have been shown to be critical for leukemia progression ^{10,16,18,63}. The intracellular domains of different LILRBs differ (Li *et al.,* 2019), suggesting that these receptors mediate distinct downstream signaling events. In the current study, the inventors found that of LILRBs evaluated, only LILRB3 recruits TRAF2. TRAF2 can be specifically recruited by TNFR subfamily via the SKEE-like motif (Rodriguez *et al.,* 2011). Interestingly, they identified the motif in the intracellular domain of LILRB3 critical for binding to TRAF2 as VQEE. TRAF2 binds with relatively low affinity to TNFR family members in the absence of activation (Ye & Wu, 2000). LILRB3 constitutively binds to TRAF2, however.

Because LILRB3-mediated signaling in AML cells supports survival of these leukemia cells and inhibits the activity of cytotoxic T cells, it is desirable to develop anti-LILRB3 antagonizing antibodies that may block AML development. Here, the inventors used functional assays to screen phage libraries and identified anti-LILRB3 antagonizing antibodies that demonstrated anti-AML efficacy. Mice that lack PirB, the mouse orthologue of LILRB3, have overall normal hematopoiesis (Syken *et al.,* 2006; Takai *et al.,* 2011); therefore, targeting LILRB3 may effectively block AML development with a low toxicity. This study may lead to development of a new strategy that combines targeted therapy and immunotherapy for treatment of AML and other types of cancer.

**Table 1. LILRB3 antibodies-DNA sequences of heavy chain CDRs**

| **Heavy chain** | **CDR1** | **SEQ ID NO:** | **CDR2** | **SEQ ID NO:** | **CDR3** | **SEQ ID NO:** |
|---|---|---|---|---|---|---|
| B3-1_HC | ggattcaccttcagtagct atggc | 23 | atacggtatgatggaagtaataaa | 24 | gcgaaagccgggttcagtggctggtactactacg gtatggacgtc | 25 |
| B3-3_HC | ggggacagtgtctctagc aacagtgctgct | 26 | acatactacaggtccaagtggtataat | 27 | gccagagctttggctgggattgatggttttgatgta | 28 |
| B3-4_HC | ggatacaccttcaccagc tactat | 29 | atcaaccctagtggtggtagcaca | 30 | gcgagaggcgaaacagggaggttcggggagtt aatacggggtatggacgtc | 31 |
| B3-5_HC | ggattcacctttgatgatta tggc | 32 | attaattggaatggtggtagcaca | 33 | gcgagagagggccatgtgtctgcttttgatatc | 34 |
| B3-6_HC | ggggacagtgtctctagc aacagtgctgct | 35 | acatactacaggtccaagtggtataat | 36 | gccagagctttggctgggattgatggttttgatgta | 37 |
| B3-9_HC | ggggacagtgtctctagc aacagtgctgct | 38 | acatactacaggtccaagtggtataat | 39 | gcaagagtgtcgccggggcttgcttttgatatc | 40 |
| B3-10_HC | ggggacagtgtctctagc aacagtgctgct | 41 | acatactacaggtccaagtggtataat | 42 | gcaagacggggggaatacggaatctttgactac | 43 |
| B3-11_HC | ggggacagtgtctctagc aacagtgctgct | 44 | acatactacaggtccaagtggtataat | 45 | gcaagagattatacggttcggggagcgtctctcta ctactacggtatggacgtc | 46 |
| B3-13_HC | ggggacagtgtctctagc aacagtgctgct | 47 | acatactacaggtccaagtggtataat | 48 | gcaagagataggagtgtaggcagcatagagac aaggtactactactactacggtatggacgtc | 49 |
| B3-14_HC | ggggacagtgtctctagc aacagtgctgct | 50 | acatactacaggtccaagtggtataat | 51 | gcaagactccgctcctatggtgatgatgcttttgata tc | 52 |
| B3-16_HC | ggattcacctttgatgatta tggc | 53 | attaattggaatggtggtagcaca | 54 | gcgagagaaggtttcgttatgggatttgactac | 55 |
| B3-17_HC | ggattcacttttgaaacgt ctggc | 56 | attaattggaatggtcgtaataca | 57 | gcgagggtcaaaaaaggagagtacggcctgga cgtc | 58 |
| B3-19_HC | ggaggcaccttcagcag ctatgct | 59 | atcatccctatctttggtacagca | 60 | gcgagagcaccggtcgggtgtagtagta ccagctgctatacttggggctactactact acggtatggacgtc | 61 |
| B3-22_HC | ggggacagtgtctctagc aacagtgctgct | 62 | acatactacaggtccaagtggtataat | 63 | gcaagagcccgatacgcatcctttgactac | 64 |
| B3-26_HC | ggattcaccttcagtgacc actac | 65 | attgatctttctggtagtaccgta | 66 | gcgaggggtcactacggtttggacgtc | 67 |
| B3-28_HC | ggatacaccttcaccagc tactat | 68 | atcaaccctagtggtggtagcaca | 69 | gcgagatgggacagtggcgtccgggtctacggta tggacgtc | 70 |
| B3-31_HC | ggggacagtgtctccagc aacactgctgct | 71 | acatactacaggtccaagtggtatttt | 72 | gcaagactccgctcctatggtgatgatgcttttgata tc | 73 |
| B3-32_HC | ggaggcaccttcagcag ctatgct | 74 | atcatccctatctttggtacagca | 75 | gcgagaggctggaactggttcgacccc | 76 |
| B3-39_HC | ggatacaccttcaccagc tactat | 77 | atcaaccctagtggtggtagcaca | 78 | gcgagagattcgcgagtaaagcagtggcaggca catgatgcttttgatatc | 79 |
| B3-40_HC | ggggacagtgtctctagc aacagtgctgct | 80 | acatactacaggtccaagtggtataat | 81 | gcaagactccgctcctatggtgatgatgcttttgata tc | 82 |
| B3-41_HC | ggatacaccttcaccagc tactat | 83 | atcaaccctagtggtggtagcaca | 84 | gcgagagattgggggcgtttggggtac | 85 |
| B3-42_HC | ggggacagtgtctctagc aacagtgctgct | 86 | acatactacaggtccaagtggtataat | 87 | gcaagactccgctcctatggtgatgatgcttttgata tc | 88 |
| B3-45_HC | ggtgggtccttcagtggtt actac | 89 | ggtgggtccttcagtggttactac | 90 | gcgagaggcctcctgtatagcagtggttttgactac | 91 |
| B3-58_HC | ggggacaatctctctagg gacactgctgct | 92 | acatactacaggtccaagtggtataat | 93 | gcgagaagtataggaggttttgactat | 94 |
| B3-62_HC | ggattcactttcagtaacg cctgg | 95 | attaaaagcaaaactgatggtgggac aaca | 96 | accacgtatagtggttatttaggctac | 97 |
| B3-66_HC | ggggacagtgtctctagc aacagtgctgct | 98 | acatactacaggtccaagtggtataat | 99 | gcaaggtggctacgtgggggctttgactac | 100 |
| B3-67_HC | ggtgggtccttcagtggtt actac | 101 | atcaatcatagtggaagcacc | 102 | gcgagaggcttgctgtatagcagtggtctcgacaa c | 103 |
| B3-68_HC | ggggacagtgtctctagc aacagtgctgct | 104 | acatactacaggtccaagtggtataat | 105 | gcgcgtatagtgggaggtgccgttgactac | 106 |
| B3-76_HC | ggattcacctttgatgatta tggc | 107 | attaattggaatggtggtagcaca | 108 | gcgagagatcagttcttggcttttgatatc | 109 |

**Table 2. LILRB3 antibodies-nucleic acid sequences of light chain CDRs**

| **Light chain** | **CDR1** | **SEQ ID NO:** | **CDR2** | **CDR3** | **SEQ ID NO:** |
|---|---|---|---|---|---|
| B3-1 LC | cagagcattagcacttat | 110 | ggtgcatcc | cagcagagttacggtctccccctcact | 111 |
| B3-3 LC | agcagtgacgttggtggttataactat | 112 | gatgtcagt | agctcatatacaagcagcactttttgggtg | 113 |
| B3-4 LC | cagggcattagcaattat | 114 | gctgcatcc | caaaagtataacagtgcccctctcact | 115 |
| B3-5 LC | agcagtgacgttggtggttataactat | 116 | gaggtcagt | agctcatatacaagcagcagctcccatgtggta | 117 |
| B3-6 LC | cagagcattagcaaatat | 118 | gctgcatcc | caagagaaggacaat | 119 |
| B3-9 LC | cagagcattagcagctat | 120 | gctgcatcc | caacagagttacagtacccaagggacg | 121 |
| B3-10 LC | cagagtattagtagctgg | 122 | aaggcatct | caacagtataatagttattacact | 123 |
| B3-11 LC | agctccaccatcggaactaatcct | 124 | agtaataat | gcaacattggatgacagcctgaatggttatgtc | 125 |
| B3-13 LC | acctccaacatcagaggtaatact | 126 | aataataat | ggagcatgggatgacagcctgaatggtcctgtc | 127 |
| B3-14 LC | cagagcattagcagctat | 128 | gctgcatcc | caacagagttacagtacccacatcacc | 129 |
| B3-16 LC | agcagtgacgttggtggttataactat | 130 | gaggtcagt | agttcatatacaagcagcag | 131 |
| B3-17 LC | cagagcattagcagctat | 132 | gctgcatcc | tgtcaacagagtttacaggtacccctccgtgggacg | 133 |
| B3-19 LC | cagggtattagcagctgg | 134 | gctgcatcc | caacaggctaacagtttcccgctcact | 135 |
| B3-22 LC | cagggtattagcagctgg | 136 | gcatccagt | caggctaacagtttccctccgacg | 137 |
| B3-26 LC | cagagtattggtacgtgg | 138 | aaggcgtct | caacagtataatctttattctcggacg | 139 |
| B3-28 LC | cagagcctcctgcaacataacggat acaactat | 140 | ttgggctct | atgcaagctctacatagtccgacg | 141 |
| B3-31 LC | cagagcattagcaactat | 142 | gctgcatcc | caacagagttacagtacccccacc | 143 |
| B3-32 LC | agctccaacattgggaagaattat | 144 | gacgataat | ggagcttgggatagcagcctgagtgcttatgtc | 145 |
| B3-39 LC | agcagtgacattggtggttataagtct | 146 | gaggtcagt | agctcatatgcaggcagcaacaatataaattatgtc | 147 |
| B3-40 LC | cagagcattagcagctat | 148 | gctgcatcc | caacagagttacagtacccccact | 149 |
| B3-41 LC | agctccaacattgggaagaattat | 150 | gacgataat | ggagcttgggatagcagcctgagtgcttatgtc | 151 |
| B3-42 LC | cagagcattagcagctct | 152 | gctgcatcc | cagcagagtttcagtagccccacc | 153 |
| B3-45 LC | cagagcattagcagctat | 154 | gctgcatcc | caacagagttacagtaccccgctcact | 155 |
| B3-58 LC | cagagcgtcagcaacaat | 156 | gccgcatcc | caacagagttacagtatctcg | 157 |
| B3-62 LC | gagagttttagtacctgg | 158 | agggcgtct | caacaatataatggttaccctact | 159 |
| B3-66 LC | cagggcattagaaatgat | 160 | gctgcatcc | ctacagcataatagttaccctcccgacgtt | 161 |
| B3-67 LC | cagagcattagcagctat | 162 | gctgcatcc | caacagagttacagtaccccgctcact | 163 |
| B3-68 LC | cagggtattagcagatgg | 164 | gaagtatcc | caacaggctaacagtttcccgatcacc | 165 |
| B3-76 LC | agcagtgacgttggtggttataactat | 166 | gaggtcagt | agctcatatacaagcagcagccctcccttaatctct | 167 |

**Table 3. LILRB3 antibodies-amino acid sequences of heavy chain CDRs**

| **Heavy chain** | **CDR1** | **SEQ ID NO:** | **CDR2** | **SEQ ID NO:** | **CDR3** | **SEQ ID NO:** |
|---|---|---|---|---|---|---|
| B3-1_HC | GFTFSSYG | 168 | IRYDGSNK | 169 | AKAGFSGWYYYGMDV | 170 |
| B3-3_HC | GDSVSSNSAA | 171 | TYYRSKWYN | 172 | ARALAGIDGFDV | 173 |
| B3-4_HC | GYTFTSYY | 174 | INPSGGST | 175 | ARGETGRFGELIRGMDV | 176 |
| B3-5_HC | GFTFDDYG | 177 | INWNGGST | 178 | AREGHVSAFDI | 179 |
| B3-6_HC | GDSVSSNSAA | 180 | TYYRSKWYN | 181 | ARALAGIDGFDV | 182 |
| B3-9_HC | GDSVSSNSAA | 183 | TYYRSKWYN | 184 | ARVSPGLAFDI | 185 |
| B3-10_HC | GDSVSSNSAA | 186 | TYYRSKWYN | 187 | ARRGEYGIFDY | 188 |
| B3-11_HC | GDSVSSNSAA | 189 | TYYRSKWYN | 190 | ARDYTVRGASLYYYGMD V | 191 |
| B3-13_HC | GDSVSSNSAA | 192 | TYYRSKWYN | 193 | ARDRSVGSIETRYYYYY GMDV | 194 |
| B3-14_HC | GDSVSSNSAA | 195 | TYYRSKWYN | 196 | ARLRSYGDDAFDI | 197 |
| B3-16_HC | GFTFDDYG | 198 | INWNGGST | 199 | AREGFVMGFDY | 200 |
| B3-17_HC | GFTFETSG | 201 | INWNGRNT | 202 | ARVKKGEYGLDV | 203 |
| B3-19_HC | GGTFSSYA | 204 | IIPIFGTA | 205 | ARAPVGCSSTSCYTWGY YYYGMDV | 206 |
| B3-22_HC | GDSVSSNSAA | 207 | TYYRSKWYN | 208 | ARARYASFDY | 209 |
| B3-26_HC | GFTFSDHY | 210 | IDLSGSTV | 211 | ARGHYGLDV | 212 |
| B3-28_HC | GYTFTSYY | 213 | INPSGGST | 214 | ARWDSGVRVYGMDV | 215 |
| B3-31_HC | GDSVSSNTAA | 216 | TYYRSKWYF | 217 | ARLRSYGDDAFDI | 218 |
| B3-32_HC | GGTFSSYA | 219 | IIPIFGTA | 220 | ARGWNWFDP | 221 |
| B3-39_HC | GYTFTSYY | 222 | INPSGGST | 223 | ARDSRVKQWQAHDAFDI | 224 |
| B3-40_HC | GDSVSSNSAA | 225 | TYYRSKWYN | 226 | ARLRSYGDDAFDI | 227 |
| B3-41_HC | GYTFTSYY | 228 | INPSGGST | 229 | ARDWGRLGY | 230 |
| B3-42_HC | GDSVSSNSAA | 231 | TYYRSKWYN | 232 | ARLRSYGDDAFDI | 234 |
| B3-45_HC | GGSFSGYY | 235 | INHSGST | 236 | ARGLLYSSGFDY | 237 |
| B3-58_HC | GDNLSRDTAA | 238 | TYYRSKWYN | 239 | ARSIGGFDY | 240 |
| B3-62_HC | GFTFSNAW | 241 | IKSKTDGGTT | 242 | TTYSGYLGY | 243 |
| B3-66_HC | GDSVSSNSAA | 244 | TYYRSKWYN | 245 | ARWLRGGFDY | 246 |
| B3-67_HC | GGSFSGYY | 247 | INHSGST | 248 | ARGLLYSSGLDN | 249 |
| B3-68_HC | GDSVSSNSAA | 250 | TYYRSKWYN | 251 | ARIVGGAVDY | 252 |
| B3-76_HC | GFTFDDYG | 253 | INWNGGST | 254 | ARDQFLAFDI | 255 |

**Table 4. LILRB3 antibodies-amino acid sequences of light chain CDRs**

| **Light chain** | **CDR1** | **SEQ ID NO:** | **CDR2** | **CDR3** | **SEQ ID NO:** |
|---|---|---|---|---|---|
| B3-1_LC | QSISTY | 256 | GAS | QQSYGLPLT | 257 |
| B3-3_LC | SSDVGGYNY | 258 | DVS | SSYTSSTFWV | 259 |
| B3-4_LC | QGISNY | 260 | AAS | QKYNSAPLT | 261 |
| B3-5_LC | SSDVGGYNY | 262 | EVS | SSYTSSSSHVV | 263 |
| B3-6_LC | QSISKY | 264 | AAS | QEKDN | 265 |
| B3-9_LC | QSISSY | 266 | AAS | QQSYSTQGT | 267 |
| B3-10_LC | QSISSW | 268 | KAS | QQYNSYYT | 269 |
| B3-11_LC | SSTIGTNP | 270 | SNN | ATLDDSLNGYV | 271 |
| B3-13_LC | TSNIRGNT | 272 | NNN | GAWDDSLNGPV | 273 |
| B3-14_LC | QSISSY | 274 | AAS | QQSYSTHIT | 275 |
| B3-16_LC | SSDVGGYNY | 276 | EVS | SSYTSS | 277 |
| B3-17_LC | QSISSY | 278 | AAS | CQQSLQVPLRGT | 279 |
| B3-19_LC | QGISSW | 280 | AAS | QQANSFPLT | 281 |
| B3-22_LC | QGISSW | 282 | ASS | QANSFPPT | 283 |
| B3-26_LC | QSIGTW | 284 | KAS | QQYNLYSRT | 285 |
| B3-28_LC | QSLLQHNGYNY | 286 | LGS | MQALHSPT | 287 |
| B3-31_LC | QSISNY | 288 | AAS | QQSYSTPT | 289 |
| B3-32_LC | SSNIGKNY | 290 | DDN | GAWDSSLSAYV | 291 |
| B3-39_LC | SSDIGGYKS | 292 | EVS | SSYAGSNNINYV | 293 |
| B3-40_LC | QSISSY | 294 | AAS | QQSYSTPT | 295 |
| B3-41_LC | SSNIGKNY | 296 | DDN | GAWDSSLSAYV | 297 |
| B3-42_LC | QSISSS | 298 | AAS | QQSFSSPT | 299 |
| B3-45_LC | QSISSY | 300 | AAS | QQSYSTPLT | 301 |
| B3-58_LC | QSVSNN | 302 | AAS | QQSYSIS | 303 |
| B3-62_LC | ESFSTW | 304 | RAS | QQYNGYPT | 305 |
| B3-66_LC | QGIRND | 306 | AAS | LQHNSYPPDV | 307 |
| B3-67_LC | QSISSY | 308 | AAS | QQSYSTPLT | 309 |
| B3-68_LC | QGISRW | 310 | EVS | QQANSFPIT | 311 |
| B3-76_LC | SSDVGGYNY | 312 | EVS | SSYTSSSPPLIS | 313 |

**Table 5. LILRB3 antibodies-DNA sequences of heavy chains**

| |
|---|
| >B3-1_HC |
| Gaggtgcagctggtggagtctgggggaggcgtggtccagcctgggaggtccctgagactctcctgtgcagcgtctgg attcaccttcagtagctatggcatgcactgggtccgccaggctccaggcaaggggctggagtgggtggcatttatacg gtatgatggaagtaataaatactatgcagactccgtgaagggccgattcaccatctccagagacaattccaagaaca cgctgtatctgcaaatgaacagcctgagagctgaggacacggctgtgtattactgtgcgaaagccgggttcagtggct ggtactactacggtatggacgtctggggccaagggacaatggtcaccgtctcttca (SEQ ID NO: 314) |
| >B3-3_HC |
| Caggtacagctgcagcagtcaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataataattatgcagtgtctgtgaaaagtcgaataaccatcaacccagacacat ccaagaaccagttctccctgcagctgaactctgtgactcccgaggacacggctgtgtattactgtgccagagctttggct gggattgatggttttgatgtatggggccaagggaccacggtcagcgtctcttca (SEQ ID NO: 315) |
| >B3-4_HC |
| Gaagtgcagctggtgcagtctggggctgaggtgaagaagcctggggcctcagtgaaggtttcctgcaaggcatctg gatacaccttcaccagctactatatgcactgggtgcgacaggcccctggacaagggcttgagtggatgggaataatc aaccctagtggtggtagcacaagctacgcacagaagttccagggcagagtcaccatgaccagggacacgtccac gagcacagtctacatggagctgagcagcctgagatctgaggacacggccgtgtattactgtgcgagaggcgaaac agggaggttcggggagttaatacgggggtatggacgtctggggccaagggaccacggtcaccgtctcttca (SEQ ID NO: 316) |
| >B3-5_HC |
| Gaagtgcagctggtgcagtctgggggaggtgtggtacggcctggggggtccctgagactctcctgtgcagcctctgg attcacctttgatgattatggcatgagctgggtccgccaagctccagggaaggggctggagtgggtctctggtattaatt ggaatggtggtagcacaggttatgcagactctgtgaagggccgattcaccatctccagagacaacgccaagaactc cctgtatctgcaaatgaacagtctgagagccgaggacacggccttgtattactgtgcgagagagggccatgtgtctgc ttttgatatctggggccaagggacaatggtcaccgtctcctca (SEQ ID NO: 317) |
| >B3-6_HC |
| Caggtacagctgcagcagtcaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataataattatgcagtgtctgtgaaaagtcgaataaccatcaacccagacacat ccaagaaccagttctccctgcagctgaactctgtgactcccgaggacacggctgtgtattactgtgccagagctttggct gggattgatggttttgatgtatggggccaagggaccacggtcagcgtctcttca (SEQ ID NO: 318) |
| >B3-9_HC |
| Caggtacagctgcagcagtcaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataatgattatgcagtatctgtgaaaagtcgaataaccatcaacccagacacat ccaagaaccagttctccctgcagctgaactctgtgactcccgaggacacggctgtgtattactgtgcaagagtgtcgcc ggggcttgcttttgatatctggggccaagggacaatggtcaccgtctcttca (SEQ ID NO: 319) |
| >83-10_HC |
| Caggtacagctgcagcagtcaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataatgattatgcagtatctgtgaaaagtcgaataaccatcaacccagacacat |
| ccaagaaccagttctccctgcagctgaactctgtgactcccgaggacacggctgtgtattactgtgcaagacggggg gaatacggaatctttgactactggggccagggaaccctggtcaccgtctcttca (SEQ ID NO: 320) |
| >B3-11_HC |
| Caggtacagctgcagcagtcaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataatgattatgcagtatctgtgaaaagtcgaataaccatcaacccagacacat ccaagaaccagttctccctgcagctgaactctgtgactcccgaggacacggctgtgtattactgtgcaagagattatac ggttcggggagcgtctctctactactacggtatggacgtctggggccaagggaccacggtcaccgtctcttca (SEQ ID NO: 321) |
| >B3-13_HC |
| Caggtacagctgcagcagtcaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataatgattatgcagtatctgtgaaaagtcgaataaccatcaacccagacacat ccaagaaccagttctccctgcagctgaactctgtgactcccgaggacacggctgtgtattactgtgcaagagatagga gtgtaggcagcatagagacaaggtactactactactacggtatggacgtctggggccaagggaccacggtcagcgt ctcctca (SEQ ID NO: 322) |
| >B3-14_HC |
| Caggtacagctgcagcagttaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataatgattatgcagtatctgtgaaaagtcgaataaccatcaacccagacacat ccaagaaccagttctccctgcagctgaactctgtgactcccgaggacacggctgtgtattactgtgcaagactccgctc ctatggtgatgatgcttttgatatctggggccaagggacaatggtcaccgtctcctca (SEQ ID NO: 323) |
| >B3-19_HC |
| Caggtgcagctggtgcagtctggggctgaggtgaagaagcctgggtcctcggtgaaggtctcctgcaaggcttctgg aggcaccttcagcagctatgctatcagctgggtgcgacaggcccctggacaagggcttgagtggatgggagggatc atccctatctttggtacagcaaactacgcacagaagttccagggcagagtcacgattaccgcggacaaatccacga gcacagcctacatggagctgagcagcctgagatctgaggacacggccgtgtattactgtgcgagagcaccggtcgg gtgtagtagtaccagctgctatacttggggctactactactacggtatggacgtctggggccaagggaccacggtcac cgtctcttca (SEQ ID NO: 324) |
| >B3-22_HC |
| Caggtacagctgcagcagtcaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataatgattatgcagtatctgtgaaaagtcgaataaccatcaacccagacacat ccaagaaccagttctccctgcagctgaactctgtgactcccgaggacacggctgtgtattactgtgcaagagcccgat acgcatcctttgactactggggccagggaaccctggtcactgtctcctca (SEQ ID NO: 325) |
| >B3-26_HC |
| Caggtgcagatggtggagtctgggggaggcttggtcaagcctggagggtccctgagactctcctgtgcagcctctgg attcaccttcagtgaccactacctgagctggatccgccaggctccagggaaggggctggagtgggtttcatacattgat ctttctggtagtaccgtatactacggagactctgtgaagggtcgatttaccatctccagggacaacgccaggaactcag tatacttgcaaatgaacagcctgagagccgaggacacggccgtatattactgtgcgaggggtcactacggtttggac gtctggggccaagggacaatggtcaccgtctcttca (SEQ ID NO: 326) |
| >B3-28_HC |
| Caggtgcagctggtgcagtctggggctgaggtgaagaagcctggggcctcagtgaaggtttcctgcaaggcatctg gatacaccttcaccagctactatatgcactgggtgcgacaggcccctggacaagggcttgagtggatgggaataatc aaccctagtggtggtagcacaagctacgcacagaagttccagggcagagtcaccatgaccagggacacgtccac gagcacagtctacatggagctgagcagcctgagatctgaggacacggccgtgtattactgtgcgagatgggacagt ggcgtccgggtctacggtatggacgtctggggccaagggaccacggtcaccgtctcctca (SEQ ID NO: 327) |
| >B3-31_HC |
| Caggtacagctgcagcagtcaggtccaggactggtgaagccctcgcagaccctctcactcacctgtgccatctccgg ggacagtgtctccagcaacactgctgcttggaattggatcaggcagtccccatcgagaggccttgagtggctgggaa ggacatactacaggtccaagtggtattttgattatgcagtttctgtgaaaagtcgaatgaccatcaacccagacacatc caagaaccagttctccctgcaactgaactctctgactcccgaggacacggctgtgtattactgtgcaagactccgctcc tatggtgatgatgcttttgatatctggggccaagggaccacggtcaccgtctcctca (SEQ ID NO: 328) |
| >B3-32_HC |
| Caggtccagctggtgcagtctggggctgaggtgaagaagcctgggtcctcggtgaaggtctcctgcaaggcttctgg aggcaccttcagcagctatgctatcagctgggtgcgacaggcccctggacaagggcttgagtggatgggagggatc atccctatctttggtacagcaaactacgcacagaagttccagggcagagtcacgattaccgcggacgaatccacga gcacagcctacatggagctgagcagcctgagatctgaggacacggccgtgtattactgtgcgagaggctggaactg gttcgacccctggggccagggaacaatggtcatcgtctcctca (SEQ ID NO: 329) |
| >B3-39_HC |
| Caggtgcagctggtgcagtctggggctgaggtgaagaagcctggggcctcagtgaaggtttcctgcaaggcatctg gatacaccttcaccagctactatatgcactgggtgcgacaggcccctggacaagggcttgagtggatgggaataatc aaccctagtggtggtagcacaagctacgcacagaagttccagggcagagtcaccatgaccagggacacgtccac gagcacagtctacatggagctgagcagcctgagatctgaggacacggccgtgtattactgtgcgagagattcgcga gtaaagcagtggcaggcacatgatgcttttgatatctggggccaagggacaatggtcaccgtctcttca (SEQ ID NO: 330) |
| >B3-40_HC |
| Caggtacagctgcagcagtcaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataatgattatgcagtatctgtgaaaagtcgaataaccatcaacccagacacat ccaagaaccagttctccctgcagctgaactctgtgactcccgaggacacggctgtgtattactgtgcaagactccgctc ctatggtgatgatgcttttgatatctggggccaagggacaatggtcaccgtctcctca (SEQ ID NO: 331) |
| >B3-41_HC |
| Caggtgcttctggtgcagtctggggctgaggtgaagaagcctggggcctcagtgaaggtttcctgcaaggcatctgg atacaccttcaccagctactatatgcactgggtgcgacaggcccctggacaagggcttgagtggatgggaataatca accctagtggtggtagcacaagctacgcacagaagttccagggcagagtcaccatgaccagggacacgtccacg agcacagtctacatggagctgagcagcctgagatctgaggacacggccgtgtattactgtgcgagagattgggggc gtttggggtactggggccagggaaccacggtcaccgtctcttca (SEQ ID NO: 332) |
| >B3-42_HC |
| Caggtacagctgcagcagtcaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataatgattatgcagtatctgtgaaaagtcgaataaccatcaacccagacacat ccaagaaccagttctccctgcagctgaactctgtgactcccgaggacacggctgtgtattactgtgcaagactccgctc ctatggtgatgatgcttttgatatctggggccaagggaccacggtcaccgtctcttca (SEQ ID NO: 333) |
| >B3-45_HC |
| Caggtgcagctacagcagtggggcgcaggactgttgaagccttcggagaccctgtccctcacctgcgctgtctatggt gggtccttcagtggttactactggagctggatccgccagcccccagggaaggggctggagtggattggggaaatca atcatagtggaagcaccaactacaacccgtccctcaagagtcgagtcaccatatcagtagacacgtccaagaacca gttctccctgaagctgagctctgtgaccgccgcggacacggctgtgtattactgtgcgagaggcctcctgtatagcagt ggttttgactactggggccagggaaccctggtcaccgtctcctca (SEQ ID NO: 334) |
| >B3-58_HC |
| Caggtacagctgcagcagtcaggtccaggactggtgaagccctcgcagaccctctcactcacctgtgccatctccgg ggacaatctctctagggacactgctgcttggaattggatcaggcagtccccatcgagaggccttgagtggctgggaa ggacatactacaggtccaagtggtataatgattatgcagtatctgtgaaaagtcgaataaccatcaacccagacacat ccaagaaccaattctccctgcagctgaactctgtgactcccgaggacacggctgtgtattactgtgcgagaagtatag gaggttttgactattggggccagggaaccatggtcaccgtctcttca (SEQ ID NO: 335) |
| >B3-62_HC |
| Caggtgaagctggtggagtctgggggaggcttggtaaagcctggggggtcccttagactctcctgtgcagcctctgg attcactttcagtaacgcctggatgagctgggtccgccaggctccagggaaggggctggagtgggttggccgtattaa aagcaaaactgatggtgggacaacagactacgctgcacccgtgaaaggcagattcaccatctcaagagatgattc aaaaaacacgctgtatctgcaaatgaacagcctgaaaaccgaggacacagccgtgtattactgtaccacgtatagt ggttatttaggctactggggccagggaaccctggtcactgtctcctca (SEQ ID NO: 336) |
| >B3-66_HC |
| Caggtacagctgcagcagtcaggtccaggactggtgaagccctcgcagaccctctcactcacctgtgccatctccgg ggacagtgtctctagcaacagtgctgcttggatctggatcaggcagtccccatcgagaggccttgagtggctgggaa ggacatactacaggtccaagtggtataatgattatgcagtatctgtgaaaagtcgaataaccatcaacccagacacat ccaagaaccacttctccctgcagctgaactctgtgactcccgaggacacggctgtgtattactgtgcaaggtggctacg tggggggctttgactactggggccaagggacaatggtcaccgtctcttca (SEQ ID NO: 337) |
| >B3-67_HC |
| Caggtgcagctacaacagtggggcgcaggactgttgaagccttcggagaccctgtccctcacctgcgctgtctatggt gggtccttcagtggttactactggagctggatccgccagcccccagggaaggggctggagtggattggggaaatca atcatagtggaagcaccaactacaacccgtccctcaagagtcgagtcaccatatcagtagacacgtccaagaacca gttctccctgaagctgagctctgtgaccgccgcggacacggctgtgtattactgtgcgagaggcttgctgtatagcagtg gtctcgacaactggggccagggaaccctggtcaccgtctcttca (SEQ ID NO: 338) |
| >B3-68_HC |
| Caggtacagctgcagcagtcaggtccaggactggtgaagccctcgcagaccctctcactcacctgtgccatctccgg ggacagtgtctctagcaacagtgctgcttggaactggatcaggcagtccccatcgagaggccttgagtggctgggaa ggacatactacaggtccaagtggtataatgattatgcagtttcagtgagaagtcgaataaccatcaacccagacacat ccaagaaccagttctccctgcagctaaactctgtgactcccgaggacacggctgtgtattactgtgcgcgtatagtggg aggtgccgttgactactggggccagggaaccctggtcagcgtctcctca (SEQ ID NO: 339) |
| >B3-76_HC |
| Caggtgaagctggtggagtctgggggaggtgtggtacggcctggggggtccctgagactctcctgtgcagcctctgg attcacctttgatgattatggcatgagctgggtccgccaagctccagggaaggggctggagtgggtctctggtattaatt ggaatggtggtagcacaggttatgcagactctgtgaagggccgattcaccatctccagagacaacgccaagaactc cctgtatctgcaaatgaacagtctgagagccgaggacacggccttgtattactgtgcgagagatcagttcttggcttttg atatctggggccaagggaccacggtcatcgtctcctca (SEQ ID NO: 340) |

**Table 6. LILRB3 antibodies-amino acid sequences of heavy chains**

| |
|---|
| >B3-1_HC |
| EVQLVESGGGVVQPGRSLRLSCAASGFTFSSYGMHWVRQAPGKGLEWVAFIRYD GSNKYYADSVKGRFTISRDNSKNTLYLQMNSLRAEDTAVYYCAKAGFSGWYYYGM DVWGQGTMVTVSS (SEQ ID NO: 341) |
| >B3-3_HC |
| QVQLQQSGPGLVKPSQTLSLTCAISGDSVSSNSAAWNWIRQSPSRGLEWLGRTYY RSKWYNNYAVSVKSRITINPDTSKNQFSLQLNSVTPEDTAVYYCARALAGIDGFDV WGQGTTVSVSS (SEQ ID NO: 342) |
| >B3-4_HC |
| EVQLVQSGAEVKKPGASVKVSCKASGYTFTSYYMHWVRQAPGQGLEWMGIINPS GGSTSYAQKFQGRVTMTRDTSTSTVYMELSSLRSEDTAVYYCARGETGRFGELIR GMDVWGQGTTVTVSS (SEQ ID NO: 343) |
| >B3-5_HC |
| EVQLVQSGGGVVRPGGSLRLSCAASGFTFDDYGMSWVRQAPGKGLEWVSGINW NGGSTGYADSVKGRFTISRDNAKNSLYLQMNSLRAEDTALYYCAREGHVSAFDIW GQGTMVTVSS (SEQ ID NO: 344) |
| >B3-6_HC |
| QVQLQQSGPGLVKPSQTLSLTCAISGDSVSSNSAAWNWIRQSPSRGLEWLGRTYY RSKWYNNYAVSVKSRITINPDTSKNQFSLQLNSVTPEDTAVYYCARALAGIDGFDV WGQGTTVSVSS (SEQ ID NO: 345) |
| >B3-9_HC |
| QVQLQQSGPGLVKPSQTLSLTCAISGDSVSSNSAAWNWIRQSPSRGLEWLGRTYY RSKWYNDYAVSVKSRITINPDTSKNQFSLQLNSVTPEDTAVYYCARVSPGLAFDIW GQGTMVTVSS (SEQ ID NO: 346) |
| >B3-10HC |
| QVQLQQSGPGLVKPSQTLSLTCAISGDSVSSNSAAWNWIRQSPSRGLEWLGRTYY RSKWYNDYAVSVKSRITINPDTSKNQFSLQLNSVTPEDTAVYYCARRGEYGIFDYW GQGTLVTVSS (SEQ ID NO: 347) |
| >B3-11HC |
| QVQLQQSGPGLVKPSQTLSLTCAISGDSVSSNSAAWNWIRQSPSRGLEWLGRTYY RSKWYNDYAVSVKSRITINPDTSKNQFSLQLNSVTPEDTAVYYCARDYTVRGASLY YYGMDVWGQGTTVTVSS (SEQ ID NO: 348) |
| >B3-13_HC |
| QVQLQQSGPGLVKPSQTLSLTCAISGDSVSSNSAAWNWIRQSPSRGLEWLGRTYY RSKWYNDYAVSVKSRITINPDTSKNQFSLQLNSVTPEDTAVYYCARDRSVGSIETR YYYYYGMDVWGQGTTVSVSS (SEQ ID NO: 349) |
| >B3-14_HC |
| QVQLQQLGPGLVKPSQTLSLTCAISGDSVSSNSAAWNWIRQSPSRGLEWLGRTYY RSKWYNDYAVSVKSRITINPDTSKNQFSLQLNSVTPEDTAVYYCARLRSYGDDAFD IWGQGTMVTVSS (SEQ ID NO: 350) |
| >B3-19H |
| QVQLVQSGAEVKKPGSSVKVSCKASGGTFSSYAISWVRQAPGQGLEWMGGIIPIF GTANYAQKFQGRVTITADKSTSTAYMELSSLRSEDTAVYYCARAPVGCSSTSCYT WGYYYYGMDVWGQGTTVTVSS (SEQ ID NO: 351) |
| >B3-22_HC |
| QVQLQQSGPGLVKPSQTLSLTCAISGDSVSSNSAAWNWIRQSPSRGLEWLGRTYY RSKWYNDYAVSVKSRITINPDTSKNQFSLQLNSVTPEDTAVYYCARARYASFDYW GQGTLVTVSS (SEQ ID NO: 352) |
| >B3-26_HC |
| QVQMVESGGGLVKPGGSLRLSCAASGFTFSDHYLSWIRQAPGKGLEWVSYIDLSG STVYYGDSVKGRFTISRDNARNSVYLQMNSLRAEDTAVYYCARGHYGLDVWGQG TMVTVSS (SEQ ID NO: 353) |
| >B3-28_HC |
| QVQLVQSGAEVKKPGASVKVSCKASGYTFTSYYMHWVRQAPGQGLEWMGIINPS GGSTSYAQKFQGRVTMTRDTSTSTVYMELSSLRSEDTAVYYCARWDSGVRVYGM DVWGQGTTVTVSS (SEQ ID NO: 354) |
| >B3-31_HC |
| QVQLQQSGPGLVKPSQTLSLTCAISGDSVSSNTAAWNWIRQSPSRGLEWLGRTYY RSKWYFDYAVSVKSRMTINPDTSKNQFSLQLNSLTPEDTAVYYCARLRSYGDDAF DIWGQGTTVTVSS (SEQ ID NO: 355) |
| >B3-32_HC |
| QVQLVQSGAEVKKPGSSVKVSCKASGGTFSSYAISWVRQAPGQGLEWMGGIIPIF GTANYAQKFQGRVTITADESTSTAYMELSSLRSEDTAVYYCARGWNWFDPWGQG TMVIVSS (SEQ ID NO: 356) |
| >B3-39 HC |
| QVQLVQSGAEVKKPGASVKVSCKASGYTFTSYYMHWVRQAPGQGLEWMGIINPS GGSTSYAQKFQGRVTMTRDTSTSTVYMELSSLRSEDTAVYYCARDSRVKQWQAH DAFDIWGQGTMVTVSS (SEQ ID NO: 357) |
| >B3-40_HC |
| QVQLQQSGPGLVKPSQTLSLTCAISGDSVSSNSAAWNWIRQSPSRGLEWLGRTYY RSKWYNDYAVSVKSRITINPDTSKNQFSLQLNSVTPEDTAVYYCARLRSYGDDAFD IWGQGTMVTVSS (SEQ ID NO: 358) |
| >B3-41_HC |
| QVLLVQSGAEVKKPGASVKVSCKASGYTFTSYYMHWVRQAPGQGLEWMGIINPS GGSTSYAQKFQGRVTMTRDTSTSTVYMELSSLRSEDTAVYYCARDWGRLGYWG QGTTVTVSS (SEQ ID NO: 359) |
| >B3-42_HC |
| QVQLQQSGPGLVKPSQTLSLTCAISGDSVSSNSAAWNWIRQSPSRGLEWLGRTYY RSKWYNDYAVSVKSRITINPDTSKNQFSLQLNSVTPEDTAVYYCARLRSYGDDAFD IWGQGTTVTVSS (SEQ ID NO: 360) |
| >B3-45_HC |
| QVQLQQWGAGLLKPSETLSLTCAVYGGSFSGYYWSWIRQPPGKGLEWIGEINHSG STNYNPSLKSRVTISVDTSKNQFSLKLSSVTAADTAVYYCARGLLYSSGFDYWGQG TLVTVSS (SEQ ID NO: 361) |
| >B3-58_HC |
| QVQLQQSGPGLVKPSQTLSLTCAISGDNLSRDTAAWNWIRQSPSRGLEWLGRTYY RSKWYNDYAVSVKSRITINPDTSKNQFSLQLNSVTPEDTAVYYCARSIGGFDYWGQ GTMVTVSS (SEQ ID NO: 362) |
| >B3-62_HC |
| QVKLVESGGGLVKPGGSLRLSCAASGFTFSNAWMSWVRQAPGKGLEWVGRIKSK TDGGTTDYAAPVKGRFTISRDDSKNTLYLQMNSLKTEDTAVYYCTTYSGYLGYWG QGTLVTVSS (SEQ ID NO: 363) |
| >B3-66_HC |
| QVQLQQSGPGLVKPSQTLSLTCAISGDSVSSNSAAWIWIRQSPSRGLEWLGRTYY RSKWYNDYAVSVKSRITINPDTSKNHFSLQLNSVTPEDTAVYYCARWLRGGFDYW GQGTMVTVSS (SEQ ID NO: 364) |
| >B3-67_HC |
| QVQLQQWGAGLLKPSETLSLTCAVYGGSFSGYYWSWIRQPPGKGLEWIGEINHSG STNYNPSLKSRVTISVDTSKNQFSLKLSSVTAADTAVYYCARGLLYSSGLDNWGQG TLVTVSS (SEQ ID NO: 365) |
| >B3-68_HC |
| QVQLQQSGPGLVKPSQTLSLTCAISGDSVSSNSAAWNWIRQSPSRGLEWLGRTYY RSKWYNDYAVSVRSRITINPDTSKNQFSLQLNSVTPEDTAVYYCARIVGGAVDYWG QGTLVSVSS (SEQ ID NO: 366) |
| >B3-76_HC |
| QVKLVESGGGVVRPGGSLRLSCAASGFTFDDYGMSWVRQAPGKGLEWVSGINW NGGSTGYADSVKGRFTISRDNAKNSLYLQMNSLRAEDTALYYCARDQFLAFDIWG QGTTVIVSS (SEQ ID NO: 367) |

**Table 7. LILRB3 antibodies-DNA sequences of light chains**

| |
|---|
| >B3-1_LC |
| Gacatcgtgatgacccagtctccatcctccctgtctgcatctgtgggagacagactcaccatcacttgccgggcaagt cagagcattagcacttatttaaattggtatcaacaaaaaccagggaaagcccctaagctcctgatctatggtgcatcca gtttgcaaagtggggtcccatcacggttcagtggcagtggatctgggacagatttcactctcaccatcagcagtctgca acctgaagattttgcaacttacttctgtcagcagagttacggtctcccccctcactttcggcggagggaccaaagtggata tcaaa (SEQ ID NO: 368) |
| >B3-3_LC |
| Cagcctgtgctgactcagcctgcctccgtgtctgggtctcctggacagtcgatcaccatctcctgcactggaaccagca gtgacgttggtggttataactatgtctcctggtaccaacaacacccaggcaaagcccccaaactcatgatttatgatgtc agtaatcggccctcaggggtttctaatcgcttctctggctccaagtctggcaacacggcctccctgaccatctctgggct ccaggctgaggacgaggctgattattactgcagctcatatacaagcagcactttttgggtgttcggcggagggaccaa ggtcaccgtccta (SEQ ID NO: 369) |
| >B3-4_LC |
| Gacatcgtgatgacccagtctccatcctccctgtctgcatctgtaggagacagagtcaccatcacttgccgggcgagt cagggcattagcaattatttagcctggtatcagcagaaaccagggaaagttcctaagctcctgatctatgctgcatcca ctttgcaatcaggggtcccatctcggttcagtggcagtggatctgggacagatttcactctcaccatcagcagcctgca gcctgaagatgttgcaacttattactgtcaaaagtataacagtgcccctctcactttcggcggagggaccaaggtgga gatcaaa (SEQ ID NO: 370) |
| >B3-5_LC |
| Cagtctgccctgaatcagcctgcctccgtgtctgggtctcctggacagtcgatcaccatctcctgcactggaaccagca gtgacgttggtggttataactatgtctcctggtaccaacagcacccaggcaaagcccccaaactcatgatttatgaggt cagtaatcggccctcaggggtttctgatcgcttctctggctccaagtctggcaacacggcctccctgaccatctctgggc tccaggctgaggacgaggctgattattactgcagctcatatacaagcagcagctcccatgtggtattcggcggaggg accaaggtcaccgtccta (SEQ ID NO: 371) |
| >B3-6_LC |
| Gccatccagttgacccagtctccatcctccctgtctgcctctgtaggagacagaatcaccatcacttgccgggcaagtc agagcattagcaaatatttaaattggtatcaacaaaagccaggagaagcccctaaactcctgatctatgctgcatcca gtttgcagagtggggtcccatccaggttcagtggcagtggatctgggacagatttcactctcaccatcagcagtctgca acctgaagattttgcaacttattattgtcaagagaaggacaattttggccaggggaccaagctggagatcaaa (SEQ ID NO: 372) |
| >B3-9_LC |
| Aacatccagatgacccagtctccatcctccctgtctgcatctgtaggagacagagtcaccatcacttgccgggcaagt cagagcattagcagctatttaaattggtatcagcagaaaccagggaaagcccctaagctcctgatctatgctgcatcc agtttgcaaagtggggtcccatcaaggttcagtggcagtggatctgggacagatttcactctcaccatcagcagtctgc aacctgaagattttgcaacttactactgtcaacagagttacagtacccaagggacgttcggccaagggaccaaagtg gatatcaaa (SEQ ID NO: 373) |
| >B3-10_LC |
| Aacatccagatgacccagtctccttccaccctgtctgcatctgtaggagacagagtcaccatcacttgccgggccagt cagagtattagtagctggttggcctggtatcagcagaaaccagggaaagcccctaagctcctgatctataaggcatct agtttagaaagtggggtcccatcaaggttcagcggcagtggatctgggacagaattcactctcaccatcagcagcctg cagcctgatgattttgcaacttattactgccaacagtataatagttattacacttttggccaggggaccaagctggagatc aaa (SEQ ID NO: 374) |
| >B3-11_LC |
| Cagcctgtgctgactcagccaccctcagcgtctgggacccccgggcagagggtcaccatctcttgttctggaagcag ctccaccatcggaactaatcctgtaaactggtaccggcacctcccaggaacggcccccaaactcctcatctatagtaa taatcagcggccctcaggggtccctgaccgagtctctggctccaagtccggcacctcagcctccctggccatcagtgg gctccagtctgaggatgaggctgattattactgtgcaacattggatgacagcctgaatggttatgtcttcggaactggga cccagctgaccgtccta (SEQ ID NO: 375) |
| >B3-13_LC |
| Cagcctgtgctgactcagccaccctcagcgtctgggacccccgggcagagggtcaccatctcttgttctggaagcac ctccaacatcagaggtaatactgtaaactggtaccaacaactcccaggaacggcccccaaactcctcatctataata ataatcagcggccctcaggggtccctgaccgattctctggctccaagtctggcacctcagcctccctggccatcagtgg gctccagtctgaggatgaggctatttattactgtggagcatgggatgacagcctgaatggtcctgtcttcggaactggga ccaaggtcaccgtccta (SEQ ID NO: 376) |
| >B3-14_LC |
| Gacatccagatgacccagtctccatcctccctgtctgcatctgtaggagacagagtcaccatcacttgccgggcaagt cagagcattagcagctatttaaattggtatcagcagaaaccagggaaagcccctaagctcctgatctatgctgcatcc agtttgcaaagtggggtcccatcaaggttcagtggcagtggatctgggacagatttcactctcaccatcagcagtctgc aacctgaagattttgcaacttactactgtcaacagagttacagtacccacatcaccttcggccaagggacacgactgg acattaaa (SEQ ID NO: 377) |
| >B3-19_LC |
| Aacatccagatgacccagtctccatcttccgtgtctgcatctgtaggagacagagtcaccatcacttgtcgggcgagtc agggtattagcagctggttagcctggtatcagcagaaaccagggaaagcccctaagctcctgatctatgctgcatcca gtttgcaaagtggggtcccatcaaggttcagcggcagtggatctgggacagatttcactctcaccatcagcagcctgc agcctgaagattttgcaacttactattgtcaacaggctaacagtttcccgctcactttcggcggagggaccaaggtggat atcaaa (SEQ ID NO: 378) |
| >B3-22_LC |
| Aacatccagatgacccagtctccatcttccgtgtctgcatctgtaggagacagagtcaccatcacttgtcgggcgagtc agggtattagcagctggttagcctggtatcagcagaaaccagggaaagcccctaaactcctgatctatgcatccagttt gcaaagtggggtcccatcaaggttcagcggcagtggatctgggacagatttcactctcaccatcagcagcctgcagc ctgaagattttgcaacttactattgtcaacaggctaacagtttccctccgacgttcggccaagggaccaaggtggagat caaa (SEQ ID NO: 379) |
| >B3-26_LC |
| Aacatccagatgacccagtctccttccaccctgtctgcatctgtaggagacagagtcaccatctcttgccgggccagtc agagtattggtacgtggttggcctggtatcagcagaaaccagggaaagcccctcaactcctgatctataaggcgtcta gtctacatagtggggtcccatcaaggttcagcggcagtggatctgggacagaattcactctcaccatcagcagcctgc agcctgatgattttgcaacctattattgccaacagtataatctttattctcggacgttcggccaagggaccaaggtggaa atcaaa (SEQ ID NO: 380) |
| >B3-28_LC |
| Gatattgtgatgacccagtctccactctccctggccgtcacccctggagagccggcctccatctcttgcacgtctagtca gagcctcctgcaacataacggatacaactatttggattggtacctgcagaagccagggcagtctccacagctcctgat ctttttgggctctactcgggcctccggggtccctgacaggttcagtgccagtggatcaggcacggattttacactgaaga tcagcagagtggaggctgaggatgttggagtatattattgcatgcaagctctacatagtccgacgttcggccaaggga ccaaggtggagatcaaa (SEQ ID NO: 381) |
| >B3-31_LC |
| Gacatcgtgatgacccagtctccatcctccctgtctgcatctgtaggagacagagtcaccatcacttgccgggcaagt cagagcattagcaactatttaaattggtatcaacagaaaccagggacagcccctaagctcctgatctatgctgcatcc agtttgcagagtggggtcccatcaaggttcagtggcagtgggtctgggacagatttcactctcaccatcagcagtctgc aacctgaagattttgctacttactactgtcaacagagttacagtacccccaccttcggccaagggacacgactggaga ttaaa (SEQ ID NO: 382) |
| >B3-32_LC |
| Cagtctgtgttgacgcagccgccctcagtgtctgcggacccagggcagaaggtcaccatctcctgctctggaagcag ctccaacattgggaagaattatgtatcgtggtatcagcagctcccaggaacagcccccaaactcctcatttatgacgat aataggcgactctcagggactcctgaccgattctccggctccaagtctggcacgtcagccaccctgggcatcaccgg actccagactggggacgaggccgattattactgcggagcttgggatagcagcctgagtgcttatgtcttcggaactgg gaccacggtgaccgtcctg (SEQ ID NO: 383) |
| >B3-39_LC |
| Cagtctgtgctgactcagcctccctccgcgtccgggtctcctggacagtcagtcaccatctcctgcactggaattagca gtgacattggtggttataagtctgtctcctggtaccaacagcacccaggcaaagcccccaaactcatgatttatgaggt cagtaagcggccctcaggggtccctgatcgcttctctggctccaagtctggcaacacggcctccctgaccgtctctggc ctccaggctgaagatgaggctgattattactgcagctcatatgcaggcagcaacaatataaattatgtcttcggaactg ggacccagctgaccgtccta (SEQ ID NO: 384) |
| >B3-40_LC |
| Gacatccagttgacccagtctccatcctccctgtctgcatctgtaggagacagagtcaccatcacttgccgggcaagt cagagcattagcagctatttaaattggtatcagcagaaaccagggaaagcccctaagctcctgatctatgctgcatcc agtttgcaaagtggggtcccatcaaggttcagtggcagtggatctgggacagatttcactctcaccatcagcagtctgc aacctgaagattttgcaacttactactgtcaacagagttacagtacccccactttcggcggagggaccaagctggaga tcaaa (SEQ ID NO: 385) |
| >B3-41_LC |
| Cagtctgtgttgacgcagccgccctcagtgtctgcggacccagggcagaaggtcaccatctcctgctctggaagcag ctccaacattgggaagaattatgtatcgtggtatcagcagctcccaggaacagcccccaaactcctcatttatgacgat aataggcgactctcagggactcctgaccgattctccggctccaagtctggcacgtcagccaccctgggcatcaccgg actccagactggggacgaggccgattattactgcggagcttgggatagcagcctgagtgcttatgtcttcggaactgg gaccaaggtcaccgtccta (SEQ ID NO: 386) |
| >B3-42_LC |
| Aacatccagatgacccagtctccatcctccctgtctgcatctgtaggagacagagtcaccattacttgccgggcaagtc agagcattagcagctctttaaattggtatgcgcagaaaccagggaaagcccctaagctcctgatctatgctgcatcca ctttgcaaagtggggtcccatcaaggttcagtggcagtggatctgggacagatttcagtctcagcatcagcagtctgca acctgaagattttgcaacttattactgtcagcagagtttcagtagccccaccttcggccaagggacacgactggacatt aaa (SEQ ID NO: 387) |
| >B3-45_LC |
| Gacatccagttgacccagtctccatcctccctgtctgcatctgtaggagacagagtcaccatcacttgccgggcaagt cagagcattagcagctatttaaattggtatcagcagaaaccagggaaagcccctaagctcctgatctatgctgcatcc agtttgcaaagtggggtcccatcaaggttcagtggcagtggatctgggacagatttcactctcaccatcagcagtctgc aacctgaagattttgcaacttactactgtcaacagagttacagtaccccgctcactttcggcggagggaccaaggtgg agatcaaa (SEQ ID NO: 388) |
| >B3-58_LC |
| Gacatcgtgatgacccagtctccatcctccctgtcggcatctgtgggagacagagtcaccatcacttgccgggcaagt cagagcgtcagcaacaatttaaattggtatcagcagaaaacgggaaaagcccctaagctcctgatctctgccgcatc cagtttgcaaagtggggtcccattaaggttcagtggcagtgggtctgggacagacttcactctcaccatcagcagtctg caacctgacgattatgcaacttactactgtcaacagagttacagtatctcgttcggccaggggactacagtggatatca aa (SEQ ID NO: 389) |
| >B3-62_LC |
| Gccatccggatgacccagtctccttccaccctgtctgcttctgtaggagacacagtcaccatcacttgccgggccagtg agagttttagtacctggttggcctggtatcagcagaaaccagggaaagcccctaaactcctgatctatagggcgtctac tttacaaagtggggtcccgtcaagattcagcggccataaatctgggacagagttcactctcaccatcagcagcctgca gcctgatgattttgcgacttattattgtcaacaatataatggttaccctactttcggcggagggaccaaggtggaaatcaa a (SEQ ID NO: 390) |
| >B3-66_LC |
| Aacatccagatgacccagtctccatcctccctgtctgcatctgtaggagacagagtcaccatcacttgccgggcaagt cagggcattagaaatgatttaggctggtatcagcagaaaccagggacagcccctaagcgcctaatctatgctgcatc caatttgcaaagtggggtcccatcaaggttcagcggcagtggatctgggacagaattcactctcacaatcagcagcct gcagcctgaagattttgcgacttattactgtctacagcataatagttaccctcccgacgtttcggccaggggaccaaa gtggatatcaaa (SEQ ID NO: 391) |
| >B3-67_LC |
| Gccatccagatgacccagtctccatcctccctgtctgcatctgtaggagacagagtcaccatcacttgccgggcaagt cagagcattagcagctatttaaattggtatcagcagaaaccagggaaagcccctaagctcctgatctatgctgcatcc agtttgcaaagtggggtcccatcaaggttcagtggcagtggatctgggacagatttcactctcaccatcagcagtctgc aacctgaagattttgcaacttactactgtcaacagagttacagtaccccgctcactttcggcggagggaccaagctgg agatcaaa (SEQ ID NO: 392) |
| >B3-68_LC |
| Gacgtccagatgacccagtctccatcttccgtgtctgcatctgtaggagacagagtcaccatcacttgtcgggcgagtc agggtattagcagatggttagcctggtatcagcagaaaccagggaaagcccctaaactcctgatctacgaagtatcc agtttgcaaagtggggtcccatcaaggttcagcggcagtggatctggggctgatttcactctcaccatcagtagcctgc agcctgaagattatggaacttactattgtcaacaggctaacagtttcccgatcaccttcggccaagggacacgactgg agattaaa (SEQ ID NO: 393) |
| >B3-76_LC |
| Cagtctgccctgactcagcctgcctccgtgtctgggtctcctggacagtcgatcaccatctcctgcactggaaccagca gtgacgttggtggttataactatgtctcctggtaccaacagcacccaggtaaagcccccaaactcatgatttatgaggtc agtaatcggccctcaggggtttctaatcgcttctctggctccaagtctggcaacacggcctccctgaccatctctgggct ccaggctgaggacgaggctgattattactgcagctcatatacaagcagcagccctcccttaatctctatcggaactgg gaccaaggtcacggtccta (SEQ ID NO: 394) |

**Table 8. LILRB3 antibodies-amino acid sequences of light chains**

| |
|---|
| >B3-1_LC |
| DIVMTQSPSSLSASVGDRLTITCRASQSISTYLNWYQQKPGKAPKLLIYGASSLQSG VPSRFSGSGSGTDFTLTISSLQPEDFATYFCQQSYGLPLTFGGGTKVDIK (SEQ ID NO: 395) |
| >B3-3_LC |
| QPVLTQPASVSGSPGQSITISCTGTSSDVGGYNYVSWYQQHPGKAPKLMIYDVSN RPSGVSNRFSGSKSGNTASLTISGLQAEDEADYYCSSYTSSTFWVFGGGTKVTVL (SEQ ID NO: 396) |
| >B3-4_LC |
| DIVMTQSPSSLSASVGDRVTITCRASQGISNYLAWYQQKPGKVPKLLIYAASTLQSG VPSRFSGSGSGTDFTLTISSLQPEDVATYYCQKYNSAPLTFGGGTKVEIK (SEQ ID NO: 397) |
| >B3-5_LC |
| QSALNQPASVSGSPGQSITISCTGTSSDVGGYNYVSWYQQHPGKAPKLMIYEVSN RPSGVSDRFSGSKSGNTASLTISGLQAEDEADYYCSSYTSSSSHVVFGGGTKVTVL (SEQ ID NO: 398) |
| >B3-6_LC |
| AIQLTQSPSSLSASVGDRITITCRASQSISKYLNWYQQKPGEAPKLLIYAASSLQSGV PSRFSGSGSGTDFTLTISSLQPEDFATYYCQEKDNFGQGTKLEIK (SEQ ID NO: 399) |
| >B3-9_LC |
| NIQMTQSPSSLSASVGDRVTITCRASQSISSYLNWYQQKPGKAPKLLIYAASSLQSG VPSRFSGSGSGTDFTLTISSLQPEDFATYYCQQSYSTQGTFGQGTKVDIK (SEQ ID NO: 400) |
| >B3-10_LC |
| NIQMTQSPSTLSASVGDRVTITCRASQSISSWLAWYQQKPGKAPKLLIYKASSLESG VPSRFSGSGSGTEFTLTISSLQPDDFATYYCQQYNSYYTFGQGTKLEIK (SEQ ID NO: 401) |
| >B3-11LC |
| QPVLTQPPSASGTPGQRVTISCSGSSSTIGTNPVNWYRHLPGTAPKLLIYSNNQRP SGVPDRVSGSKSGTSASLAISGLQSEDEADYYCATLDDSLNGYVFGTGTQLTVL (SEQ ID NO: 402) _ |
| >B3-13_LC |
| QPVLTQPPSASGTPGQRVTISCSGSTSNIRGNTVNWYQQLPGTAPKLLIYNNNQRP SGVPDRFSGSKSGTSASLAISGLQSEDEAIYYCGAWDDSLNGPVFGTGTKVTVL (SEQ ID NO: 403) |
| >B3-14_LC |
| DIQMTQSPSSLSASVGDRVTITCRASQSISSYLNWYQQKPGKAPKLLIYAASSLQSG VPSRFSGSGSGTDFTLTISSLQPEDFATYYCQQSYSTHITFGQGTRLDIK (SEQ ID NO: 404) |
| >B3-19_LC |
| NIQMTQSPSSVSASVGDRVTITCRASQGISSWLAWYQQKPGKAPKLLIYAASSLQS GVPSRFSGSGSGTDFTLTISSLQPEDFATYYCQQANSFPLTFGGGTKVDIK (SEQ ID NO: 405) |
| >B3-22_LC |
| NIQMTQSPSSVSASVGDRVTITCRASQGISSWLAWYQQKPGKAPKLLIYASSLQSG VPSRFSGSGSGTDFTLTISSLQPEDFATYYCQQANSFPPTFGQGTKVEIK (SEQ ID NO: 406) |
| >B3-26_LC |
| NIQMTQSPSTLSASVGDRVTISCRASQSIGTWLAWYQQKPGKAPQLLIYKASSLHS GVPSRFSGSGSGTEFTLTISSLQPDDFATYYCQQYNLYSRTFGQGTKVEIK (SEQ ID NO: 407) |
| >B3-28_LC |
| DIVMTQSPLSLAVTPGEPASISCTSSQSLLQHNGYNYLDWYLQKPGQSPQLLIFLGS TRASGVPDRFSASGSGTDFTLKISRVEAEDVGVYYCMQALHSPTFGQGTKVEIK (SEQ ID NO: 408) |
| >B3-31_LC |
| DIVMTQSPSSLSASVGDRVTITCRASQSISNYLNWYQQKPGTAPKLLIYAASSLQSG VPSRFSGSGSGTDFTLTISSLQPEDFATYYCQQSYSTPTFGQGTRLEIK (SEQ ID NO: 409) |
| >B3-32_LC |
| QSVLTQPPSVSADPGQKVTISCSGSSSNIGKNYVSWYQQLPGTAPKLLIYDDNRRL SGTPDRFSGSKSGTSATLGITGLQTGDEADYYCGAWDSSLSAYVFGTGTTVTVL (SEQ ID NO: 410) |
| >B3-39_LC |
| QSVLTQPPSASGSPGQSVTISCTGISSDIGGYKSVSWYQQHPGKAPKLMIYEVSKR PSGVPDRFSGSKSGNTASLTVSGLQAEDEADYYCSSYAGSNNINYVFGTGTQLTV L (SEQ ID NO: 411) |
| >B3-40_LC |
| DIQLTQSPSSLSASVGDRVTITCRASQSISSYLNWYQQKPGKAPKLLIYAASSLQSG VPSRFSGSGSGTDFTLTISSLQPEDFATYYCQQSYSTPTFGGGTKLEIK (SEQ ID NO: 412) |
| >B3-41_LC |
| QSVLTQPPSVSADPGQKVTISCSGSSSNIGKNYVSWYQQLPGTAPKLLIYDDNRRL SGTPDRFSGSKSGTSATLGITGLQTGDEADYYCGAWDSSLSAYVFGTGTKVTVL (SEQ ID NO: 413) |
| >B3-42_LC |
| NIQMTQSPSSLSASVGDRVTITCRASQSISSSLNWYAQKPGKAPKLLIYAASTLQSG VPSRFSGSGSGTDFSLSISSLQPEDFATYYCQQSFSSPTFGQGTRLDIK (SEQ ID NO: 414) |
| >B3-45_LC |
| DIQLTQSPSSLSASVGDRVTITCRASQSISSYLNWYQQKPGKAPKLLIYAASSLQSG VPSRFSGSGSGTDFTLTISSLQPEDFATYYCQQSYSTPLTFGGGTKVEIK (SEQ ID NO: 415) |
| >B3-58_LC |
| DIVMTQSPSSLSASVGDRVTITCRASQSVSNNLNWYQQKTGKAPKLLISAASSLQS GVPLRFSGSGSGTDFTLTISSLQPDDYATYYCQQSYSISFGQGTTVDIK (SEQ ID NO: 416) |
| >B3-62_LC |
| AIRMTQSPSTLSASVGDTVTITCRASESFSTWLAWYQQKPGKAPKLLIYRASTLQS GVPSRFSGHKSGTEFTLTISSLQPDDFATYYCQQYNGYPTFGGGTKVEIK (SEQ ID NO: 417) |
| >B3-66_LC |
| NIQMTQSPSSLSASVGDRVTITCRASQGIRNDLGWYQQKPGTAPKRLIYAASNLQS GVPSRFSGSGSGTEFTLTISSLQPEDFATYYCLQHNSYPPDVSARGTKVDIK (SEQ ID NO: 418) |
| >B3-67_LC |
| AIQMTQSPSSLSASVGDRVTITCRASQSISSYLNWYQQKPGKAPKLLIYAASSLQSG VPSRFSGSGSGTDFTLTISSLQPEDFATYYCQQSYSTPLTFGGGTKLEIK (SEQ ID NO: 419) |
| >B3-68_LC |
| DVQMTQSPSSVSASVGDRVTITCRASQGISRWLAWYQQKPGKAPKLLIYEVSSLQ SGVPSRFSGSGSGADFTLTISSLQPEDYGTYYCQQANSFPITFGQGTRLEIK (SEQ ID NO: 420) |
| >B3-76_LC |
| QSALTQPASVSGSPGQSITISCTGTSSDVGGYNYVSWYQQHPGKAPKLMIYEVSN RPSGVSNRFSGSKSGNTASLTISGLQAEDEADYYCSSYTSSSPPLISIGTGTKVTVL (SEQ ID NO: 421) |

SEQUENCE LISTING

<110>  THE BOARD OF REGENTS OF THE UNIVERSITY OF TEXAS SYSTEM

<120>  METHODS FOR IDENTIFYING LILRB-BLOCKING ANTIBODIES

<130>  UTFH.P0372WO

<150>  US 63/121,057
<151>  2020-12-03

<150>  US 63/238,717
<151>  2021-08-30

<150>  US63/240,684
<151>  2021-09-03

<160>  448

<170>  PatentIn version 3.5

<210>  1
<211>  21
<212>  DNA
<213>  Artificial sequence

<220>
<223>  Synthetic primer

<400>  1
gaagaaactc aactggtgtc g                                                21


<210>  2
<211>  20
<212>  DNA
<213>  Artificial sequence

<220>
<223>  Synthetic primer

<400>  2
ccaagtctgt gtcctgaacg                                                  20


<210>  3
<211>  21
<212>  DNA
<213>  Artificial sequence

<220>
<223>  Synthetic primer

<400>  3
gaaggctacc aactacaatg g                                                21


<210>  4
<211>  20
<212>  DNA
<213>  Artificial sequence

<220>
<223>  Synthetic primer

<400>  4
ttcaacagga gtgacaccag                                                   20


<210>  5
<211>  19
<212>  DNA
<213>  Artificial sequence

<220>
<223>  Synthetic primer

<400>  5
gaatcaccag cagcaagtg                                                    19


<210>  6
<211>  18
<212>  DNA
<213>  Artificial sequence

<220>
<223>  Synthetic primer

<400>  6
cttcggagtt tgggtttg                                                     18


<210>  7
<211>  20
<212>  DNA
<213>  Artificial sequence

<220>
<223>  Synthetic primer

<400>  7
ttgtgcgtct cctcagtaaa                                                   20


<210>  8
<211>  19
<212>  DNA
<213>  Artificial sequence

<220>
<223>  Synthetic primer

<400>  8
caagtgaaac ctccaaccc                                                    19


<210>  9
<211>  20
<212>  DNA
<213>  Artificial sequence

<220>
<223>  Synthetic primer

<400>  9
cattgaggag gattgccaaa                                                   20


<210>  10
<211>  19
<212>  DNA
<213>  Artificial sequence

<220>
<223>  Synthetic primer

<400>  10
acaaactgga tgtcgctgg                                                    19


<210>  11
<211>  17
<212>  DNA
<213>  Artificial sequence

<220>
<223>  Synthetic primer

<400>  11
acgctcttct gcctgct                                                      17


<210>  12
<211>  19
<212>  DNA
<213>  Artificial sequence

<220>
<223>  Synthetic primer

<400>  12
gcttgagggt ttgctacaa                                                    19


<210>  13
<211>  21
<212>  DNA
<213>  Artificial sequence

<220>
<223>  Synthetic primer

<400>  13
tggcttatta cagtggcaat g                                                 21


<210>  14
<211>  18
<212>  DNA
<213>  Artificial sequence

<220>
<223>  Synthetic primer

<400>  14
tggtggtcgg agattcgt                                                     18


<210>  15
<211>  18
<212>  DNA
<213>  Artificial sequence

<220>
<223>  Synthetic primer

<400>  15
ctttctgctg acatcgcc                                                     18

<210>  16
<211>  20
<212>  DNA
<213>  Artificial sequence

<220>
<223>  Synthetic primer

<400>  16
gtctgccgta ggttgttgta                                                    20


<210>  17
<211>  18
<212>  DNA
<213>  Artificial sequence

<220>
<223>  Synthetic primer

<400>  17
acgcagacat cgtcatcc                                                      18


<210>  18
<211>  19
<212>  DNA
<213>  Artificial sequence

<220>
<223>  Synthetic primer

<400>  18
caaaccgagt tggaaccac                                                     19


<210>  19
<211>  21
<212>  DNA
<213>  Artificial sequence

<220>
<223>  Synthetic primer

<400>  19
catacttacc cacttcaagg g                                                  21


<210>  20
<211>  22
<212>  DNA
<213>  Artificial sequence

<220>
<223>  Synthetic primer

<400>  20
ttgtagccat agtcagcatt gt                                                 22


<210>  21
<211>  17
<212>  DNA
<213>  Artificial sequence

<220>
<223>  Synthetic primer

<400>  21
gatggggtct tcatctg                                                      17


<210>  22
<211>  17
<212>  DNA
<213>  Artificial sequence

<220>
<223>  Synthetic primer

<400>  22
cgtaggtgga tgcctcc                                                      17


<210>  23
<211>  24
<212>  DNA
<213>  Artificial sequence

<220>
<223>  Synthetic antibody

<400>  23
ggattcacct tcagtagcta tggc                                              24


<210>  24
<211>  24
<212>  DNA
<213>  Artificial sequence

<220>
<223>  Synthetic antibody

<400>  24
atacggtatg atggaagtaa taaa                                              24


<210>  25
<211>  45
<212>  DNA
<213>  Artificial sequence

<220>
<223>  Synthetic antibody

<400>  25
gcgaaagccg ggttcagtgg ctggtactac tacggtatgg acgtc                       45


<210>  26
<211>  30
<212>  DNA
<213>  Artificial sequence

<220>
<223>  Synthetic antibody

<400>  26
ggggacagtg tctctagcaa cagtgctgct                                        30


<210>  27
<211>  27
<212>  DNA
<213>  Artificial sequence

<220>
<223>  Synthetic antibody

<400>  27
acatactaca ggtccaagtg gtataat                                          27


<210>  28
<211>  36
<212>  DNA
<213>  Artificial sequence

<220>
<223>  Synthetic antibody

<400>  28
gccagagctt tggctgggat tgatggtttt gatgta                                36


<210>  29
<211>  24
<212>  DNA
<213>  Artificial sequence

<220>
<223>  Synthetic antibody

<400>  29
ggatacacct tcaccagcta ctat                                             24


<210>  30
<211>  24
<212>  DNA
<213>  Artificial sequence

<220>
<223>  Synthetic antibody

<400>  30
atcaacccta gtggtggtag caca                                             24


<210>  31
<211>  51
<212>  DNA
<213>  Artificial sequence

<220>
<223>  Synthetic antibody

<400>  31
gcgagaggcg aaacagggag gttcggggag ttaatacggg gtatggacgt c               51


<210>  32
<211>  24
<212>  DNA
<213>  Artificial sequence

<220>
<223>  Synthetic antibody

<400>  32
ggattcacct ttgatgatta tggc                                             24

<210>  33
<211>  24
<212>  DNA
<213>  Artificial sequence

<220>
<223>  Synthetic antibody

<400>  33
attaattgga atggtggtag caca                                              24


<210>  34
<211>  33
<212>  DNA
<213>  Artificial sequence

<220>
<223>  Synthetic antibody

<400>  34
gcgagagagg gccatgtgtc tgcttttgat atc                                    33


<210>  35
<211>  30
<212>  DNA
<213>  Artificial sequence

<220>
<223>  Synthetic antibody

<400>  35
ggggacagtg tctctagcaa cagtgctgct                                        30


<210>  36
<211>  27
<212>  DNA
<213>  Artificial sequence

<220>
<223>  Synthetic antibody

<400>  36
acatactaca ggtccaagtg gtataat                                           27


<210>  37
<211>  36
<212>  DNA
<213>  Artificial sequence

<220>
<223>  Synthetic antibody

<400>  37
gccagagctt tggctgggat tgatggtttt gatgta                                 36


<210>  38
<211>  30
<212>  DNA
<213>  Artificial sequence

<220>
<223>  Synthetic antibody

<400>  38
ggggacagtg tctctagcaa cagtgctgct                                        30


<210>  39
<211>  27
<212>  DNA
<213>  Artificial sequence

<220>
<223>  Synthetic antibody

<400>  39
acatactaca ggtccaagtg gtataat                                           27


<210>  40
<211>  33
<212>  DNA
<213>  Artificial sequence

<220>
<223>  Synthetic antibody

<400>  40
gcaagagtgt cgccggggct tgcttttgat atc                                    33


<210>  41
<211>  30
<212>  DNA
<213>  Artificial sequence

<220>
<223>  Synthetic antibody

<400>  41
ggggacagtg tctctagcaa cagtgctgct                                        30


<210>  42
<211>  27
<212>  DNA
<213>  Artificial sequence

<220>
<223>  Synthetic antibody

<400>  42
acatactaca ggtccaagtg gtataat                                           27


<210>  43
<211>  33
<212>  DNA
<213>  Artificial sequence

<220>
<223>  Synthetic antibody

<400>  43
gcaagacggg gggaatacgg aatctttgac tac                                    33


<210>  44
<211>  30
<212>  DNA
<213>  Artificial sequence

<220>
<223>  Synthetic antibody

<400>  44
ggggacagtg tctctagcaa cagtgctgct                                        30


<210>  45
<211>  27
<212>  DNA
<213>  Artificial sequence

<220>
<223>  Synthetic antibody

<400>  45
acatactaca ggtccaagtg gtataat                                           27


<210>  46
<211>  54
<212>  DNA
<213>  Artificial sequence

<220>
<223>  Synthetic antibody

<400>  46
gcaagagatt atacggttcg gggagcgtct ctctactact acggtatgga cgtc             54


<210>  47
<211>  30
<212>  DNA
<213>  Artificial sequence

<220>
<223>  Synthetic antibody

<400>  47
ggggacagtg tctctagcaa cagtgctgct                                        30


<210>  48
<211>  27
<212>  DNA
<213>  Artificial sequence

<220>
<223>  Synthetic antibody

<400>  48
acatactaca ggtccaagtg gtataat                                           27


<210>  49
<211>  63
<212>  DNA
<213>  Artificial sequence

<220>
<223>  Synthetic antibody

<400>  49
gcaagagata ggagtgtagg cagcatagag acaaggtact actactacta cggtatggac       60

gtc                                                                     63

<210>  50
<211>  30
<212>  DNA
<213>  Artificial sequence

<220>
<223>  Synthetic antibody

<400>  50
ggggacagtg tctctagcaa cagtgctgct                                        30


<210>  51
<211>  27
<212>  DNA
<213>  Artificial sequence

<220>
<223>  Synthetic antibody

<400>  51
acatactaca ggtccaagtg gtataat                                           27


<210>  52
<211>  39
<212>  DNA
<213>  Artificial sequence

<220>
<223>  Synthetic antibody

<400>  52
gcaagactcc gctcctatgg tgatgatgct tttgatatc                              39


<210>  53
<211>  24
<212>  DNA
<213>  Artificial sequence

<220>
<223>  Synthetic antibody

<400>  53
ggattcacct ttgatgatta tggc                                              24


<210>  54
<211>  24
<212>  DNA
<213>  Artificial sequence

<220>
<223>  Synthetic antibody

<400>  54
attaattgga atggtggtag caca                                              24


<210>  55
<211>  33
<212>  DNA
<213>  Artificial sequence

<220>

<223>  Synthetic antibody

<400>  55
gcgagagaag gtttcgttat gggatttgac tac                                    33


<210>  56
<211>  24
<212>  DNA
<213>  Artificial sequence

<220>
<223>  Synthetic antibody

<400>  56
ggattcactt ttgaaacgtc tggc                                              24


<210>  57
<211>  24
<212>  DNA
<213>  Artificial sequence

<220>
<223>  Synthetic antibody

<400>  57
attaattgga atggtcgtaa taca                                              24


<210>  58
<211>  36
<212>  DNA
<213>  Artificial sequence

<220>
<223>  Synthetic antibody

<400>  58
gcgagggtca aaaaaggaga gtacggcctg gacgtc                                 36


<210>  59
<211>  24
<212>  DNA
<213>  Artificial sequence

<220>
<223>  Synthetic antibody

<400>  59
ggaggcacct tcagcagcta tgct                                              24


<210>  60
<211>  24
<212>  DNA
<213>  Artificial sequence

<220>
<223>  Synthetic antibody

<400>  60
atcatcccta tctttggtac agca                                              24


<210>  61
<211>  72

<212>  DNA
<213>  Artificial sequence

<220>
<223>  Synthetic antibody

<400>  61
gcgagagcac cggtcgggtg tagtagtacc agctgctata cttggggcta ctactactac      60

ggtatggacg tc                                                          72


<210>  62
<211>  30
<212>  DNA
<213>  Artificial sequence

<220>
<223>  Synthetic antibody

<400>  62
ggggacagtg tctctagcaa cagtgctgct                                       30


<210>  63
<211>  27
<212>  DNA
<213>  Artificial sequence

<220>
<223>  Synthetic antibody

<400>  63
acatactaca ggtccaagtg gtataat                                          27


<210>  64
<211>  30
<212>  DNA
<213>  Artificial sequence

<220>
<223>  Synthetic antibody

<400>  64
gcaagagccc gatacgcatc ctttgactac                                       30


<210>  65
<211>  24
<212>  DNA
<213>  Artificial sequence

<220>
<223>  Synthetic antibody

<400>  65
ggattcacct tcagtgacca ctac                                             24


<210>  66
<211>  24
<212>  DNA
<213>  Artificial sequence

<220>
<223>  Synthetic antibody

<400>  66
attgatcttt ctggtagtac cgta                                              24


<210>  67
<211>  27
<212>  DNA
<213>  Artificial sequence

<220>
<223>  Synthetic antibody

<400>  67
gcgaggggtc actacggttt ggacgtc                                           27


<210>  68
<211>  24
<212>  DNA
<213>  Artificial sequence

<220>
<223>  Synthetic antibody

<400>  68
ggatacacct tcaccagcta ctat                                              24


<210>  69
<211>  24
<212>  DNA
<213>  Artificial sequence

<220>
<223>  Synthetic antibody

<400>  69
atcaacccta gtggtggtag caca                                              24


<210>  70
<211>  42
<212>  DNA
<213>  Artificial sequence

<220>
<223>  Synthetic antibody

<400>  70
gcgagatggg acagtggcgt ccgggtctac ggtatggacg tc                          42


<210>  71
<211>  30
<212>  DNA
<213>  Artificial sequence

<220>
<223>  Synthetic antibody

<400>  71
ggggacagtg tctccagcaa cactgctgct                                        30


<210>  72
<211>  27
<212>  DNA
<213>  Artificial sequence

<220>
<223>  Synthetic antibody

<400>  72
acatactaca ggtccaagtg gtatttt                                          27


<210>  73
<211>  39
<212>  DNA
<213>  Artificial sequence

<220>
<223>  Synthetic antibody

<400>  73
gcaagactcc gctcctatgg tgatgatgct tttgatatc                             39


<210>  74
<211>  24
<212>  DNA
<213>  Artificial sequence

<220>
<223>  Synthetic antibody

<400>  74
ggaggcacct tcagcagcta tgct                                             24


<210>  75
<211>  24
<212>  DNA
<213>  Artificial sequence

<220>
<223>  Synthetic antibody

<400>  75
atcatcccta tctttggtac agca                                             24


<210>  76
<211>  27
<212>  DNA
<213>  Artificial sequence

<220>
<223>  Synthetic antibody

<400>  76
gcgagaggct ggaactggtt cgacccc                                          27


<210>  77
<211>  24
<212>  DNA
<213>  Artificial sequence

<220>
<223>  Synthetic antibody

<400>  77
ggatacacct tcaccagcta ctat                                             24

<210>  78
<211>  24
<212>  DNA
<213>  Artificial sequence

<220>
<223>  Synthetic antibody

<400>  78
atcaacccta gtggtggtag caca                                              24


<210>  79
<211>  51
<212>  DNA
<213>  Artificial sequence

<220>
<223>  Synthetic antibody

<400>  79
gcgagagatt cgcgagtaaa gcagtggcag gcacatgatg cttttgatat c                51


<210>  80
<211>  30
<212>  DNA
<213>  Artificial sequence

<220>
<223>  Synthetic antibody

<400>  80
ggggacagtg tctctagcaa cagtgctgct                                        30


<210>  81
<211>  27
<212>  DNA
<213>  Artificial sequence

<220>
<223>  Synthetic antibody

<400>  81
acatactaca ggtccaagtg gtataat                                           27


<210>  82
<211>  39
<212>  DNA
<213>  Artificial sequence

<220>
<223>  Synthetic antibody

<400>  82
gcaagactcc gctcctatgg tgatgatgct tttgatatc                              39


<210>  83
<211>  24
<212>  DNA
<213>  Artificial sequence

<220>
<223>  Synthetic antibody

<400>  83
ggatacacct tcaccagcta ctat                                              24


<210>  84
<211>  24
<212>  DNA
<213>  Artificial sequence

<220>
<223>  Synthetic antibody

<400>  84
atcaacccta gtggtggtag caca                                              24


<210>  85
<211>  27
<212>  DNA
<213>  Artificial sequence

<220>
<223>  Synthetic antibody

<400>  85
gcgagagatt gggggcgttt ggggtac                                           27


<210>  86
<211>  30
<212>  DNA
<213>  Artificial sequence

<220>
<223>  Synthetic antibody

<400>  86
ggggacagtg tctctagcaa cagtgctgct                                        30


<210>  87
<211>  27
<212>  DNA
<213>  Artificial sequence

<220>
<223>  Synthetic antibody

<400>  87
acatactaca ggtccaagtg gtataat                                           27


<210>  88
<211>  39
<212>  DNA
<213>  Artificial sequence

<220>
<223>  Synthetic antibody

<400>  88
gcaagactcc gctcctatgg tgatgatgct tttgatatc                              39


<210>  89
<211>  24
<212>  DNA
<213>  Artificial sequence

<220>
<223>  Synthetic antibody

<400>  89
ggtgggtcct tcagtggtta ctac                                              24


<210>  90
<211>  24
<212>  DNA
<213>  Artificial sequence

<220>
<223>  Synthetic antibody

<400>  90
ggtgggtcct tcagtggtta ctac                                              24


<210>  91
<211>  36
<212>  DNA
<213>  Artificial sequence

<220>
<223>  Synthetic antibody

<400>  91
gcgagaggcc tcctgtatag cagtggtttt gactac                                 36


<210>  92
<211>  30
<212>  DNA
<213>  Artificial sequence

<220>
<223>  Synthetic antibody

<400>  92
ggggacaatc tctctaggga cactgctgct                                        30


<210>  93
<211>  27
<212>  DNA
<213>  Artificial sequence

<220>
<223>  Synthetic antibody

<400>  93
acatactaca ggtccaagtg gtataat                                           27


<210>  94
<211>  27
<212>  DNA
<213>  Artificial sequence

<220>
<223>  Synthetic antibody

<400>  94
gcgagaagta taggaggttt tgactat                                           27

<210>  95
<211>  24
<212>  DNA
<213>  Artificial sequence

<220>
<223>  Synthetic antibody

<400>  95
ggattcactt tcagtaacgc ctgg                                              24


<210>  96
<211>  30
<212>  DNA
<213>  Artificial sequence

<220>
<223>  Synthetic antibody

<400>  96
attaaaagca aaactgatgg tgggacaaca                                        30


<210>  97
<211>  27
<212>  DNA
<213>  Artificial sequence

<220>
<223>  Synthetic antibody

<400>  97
accacgtata gtggttattt aggctac                                           27


<210>  98
<211>  30
<212>  DNA
<213>  Artificial sequence

<220>
<223>  Synthetic antibody

<400>  98
ggggacagtg tctctagcaa cagtgctgct                                        30


<210>  99
<211>  27
<212>  DNA
<213>  Artificial sequence

<220>
<223>  Synthetic antibody

<400>  99
acatactaca ggtccaagtg gtataat                                           27


<210>  100
<211>  30
<212>  DNA
<213>  Artificial sequence

<220>
<223>  Synthetic antibody

<400>  100
gcaaggtggc tacgtggggg ctttgactac                                        30


<210>  101
<211>  24
<212>  DNA
<213>  Artificial sequence

<220>
<223>  Synthetic antibody

<400>  101
ggtgggtcct tcagtggtta ctac                                              24


<210>  102
<211>  21
<212>  DNA
<213>  Artificial sequence

<220>
<223>  Synthetic antibody

<400>  102
atcaatcata gtggaagcac c                                                 21


<210>  103
<211>  36
<212>  DNA
<213>  Artificial sequence

<220>
<223>  Synthetic antibody

<400>  103
gcgagaggct tgctgtatag cagtggtctc gacaac                                 36


<210>  104
<211>  30
<212>  DNA
<213>  Artificial sequence

<220>
<223>  Synthetic antibody

<400>  104
ggggacagtg tctctagcaa cagtgctgct                                        30


<210>  105
<211>  27
<212>  DNA
<213>  Artificial sequence

<220>
<223>  Synthetic antibody

<400>  105
acatactaca ggtccaagtg gtataat                                           27


<210>  106
<211>  30
<212>  DNA
<213>  Artificial sequence

<220>
<223>  Synthetic antibody

<400>  106
gcgcgtatag tgggaggtgc cgttgactac                                        30


<210>  107
<211>  24
<212>  DNA
<213>  Artificial sequence

<220>
<223>  Synthetic antibody

<400>  107
ggattcacct ttgatgatta tggc                                              24


<210>  108
<211>  24
<212>  DNA
<213>  Artificial sequence

<220>
<223>  Synthetic antibody

<400>  108
attaattgga atggtggtag caca                                              24


<210>  109
<211>  30
<212>  DNA
<213>  Artificial sequence

<220>
<223>  Synthetic antibody

<400>  109
gcgagagatc agttcttggc ttttgatatc                                        30


<210>  110
<211>  18
<212>  DNA
<213>  Artificial sequence

<220>
<223>  Synthetic antibody

<400>  110
cagagcatta gcacttat                                                     18


<210>  111
<211>  27
<212>  DNA
<213>  Artificial sequence

<220>
<223>  Synthetic antibody

<400>  111
cagcagagtt acggtctccc cctcact                                           27

<210>  112
<211>  27
<212>  DNA
<213>  Artificial sequence

<220>
<223>  Synthetic antibody

<400>  112
agcagtgacg ttggtggtta taactat                                          27


<210>  113
<211>  30
<212>  DNA
<213>  Artificial sequence

<220>
<223>  Synthetic antibody

<400>  113
agctcatata caagcagcac tttttgggtg                                       30


<210>  114
<211>  18
<212>  DNA
<213>  Artificial sequence

<220>
<223>  Synthetic antibody

<400>  114
cagggcatta gcaattat                                                    18


<210>  115
<211>  27
<212>  DNA
<213>  Artificial sequence

<220>
<223>  Synthetic antibody

<400>  115
caaaagtata acagtgcccc tctcact                                          27


<210>  116
<211>  27
<212>  DNA
<213>  Artificial sequence

<220>
<223>  Synthetic antibody

<400>  116
agcagtgacg ttggtggtta taactat                                          27


<210>  117
<211>  33
<212>  DNA
<213>  Artificial sequence

<220>
<223>  Synthetic antibody

<400>  117
agctcatata caagcagcag ctcccatgtg gta                                    33


<210>  118
<211>  18
<212>  DNA
<213>  Artificial sequence

<220>
<223>  Synthetic antibody

<400>  118
cagagcatta gcaaatat                                                     18


<210>  119
<211>  15
<212>  DNA
<213>  Artificial sequence

<220>
<223>  Synthetic antibody

<400>  119
caagagaagg acaat                                                        15


<210>  120
<211>  18
<212>  DNA
<213>  Artificial sequence

<220>
<223>  Synthetic antibody

<400>  120
cagagcatta gcagctat                                                     18


<210>  121
<211>  27
<212>  DNA
<213>  Artificial sequence

<220>
<223>  Synthetic antibody

<400>  121
caacagagtt acagtaccca agggacg                                           27


<210>  122
<211>  18
<212>  DNA
<213>  Artificial sequence

<220>
<223>  Synthetic antibody

<400>  122
cagagtatta gtagctgg                                                     18


<210>  123
<211>  24
<212>  DNA
<213>  Artificial sequence

<220>
<223>  Synthetic antibody

<400>  123
caacagtata atagttatta cact                                              24


<210>  124
<211>  24
<212>  DNA
<213>  Artificial sequence

<220>
<223>  Synthetic antibody

<400>  124
agctccacca tcggaactaa tcct                                              24


<210>  125
<211>  33
<212>  DNA
<213>  Artificial sequence

<220>
<223>  Synthetic antibody

<400>  125
gcaacattgg atgacagcct gaatggttat gtc                                    33


<210>  126
<211>  24
<212>  DNA
<213>  Artificial sequence

<220>
<223>  Synthetic antibody

<400>  126
acctccaaca tcagaggtaa tact                                              24


<210>  127
<211>  33
<212>  DNA
<213>  Artificial sequence

<220>
<223>  Synthetic antibody

<400>  127
ggagcatggg atgacagcct gaatggtcct gtc                                    33


<210>  128
<211>  18
<212>  DNA
<213>  Artificial sequence

<220>
<223>  Synthetic antibody

<400>  128
cagagcatta gcagctat                                                     18

<210>  129
<211>  27
<212>  DNA
<213>  Artificial sequence

<220>
<223>  Synthetic antibody

<400>  129
caacagagtt acagtaccca catcacc                                          27


<210>  130
<211>  27
<212>  DNA
<213>  Artificial sequence

<220>
<223>  Synthetic antibody

<400>  130
agcagtgacg ttggtggtta taactat                                          27


<210>  131
<211>  20
<212>  DNA
<213>  Artificial sequence

<220>
<223>  Synthetic antibody

<400>  131
agttcatata caagcagcag                                                  20


<210>  132
<211>  18
<212>  DNA
<213>  Artificial sequence

<220>
<223>  Synthetic antibody

<400>  132
cagagcatta gcagctat                                                    18


<210>  133
<211>  36
<212>  DNA
<213>  Artificial sequence

<220>
<223>  Synthetic antibody

<400>  133
tgtcaacaga gtttacaggt acccctccgt gggacg                                36


<210>  134
<211>  18
<212>  DNA
<213>  Artificial sequence

<220>
<223>  Synthetic antibody

<400>  134
cagggtatta gcagctgg                                                    18


<210>  135
<211>  27
<212>  DNA
<213>  Artificial sequence

<220>
<223>  Synthetic antibody

<400>  135
caacaggcta acagtttccc gctcact                                          27


<210>  136
<211>  18
<212>  DNA
<213>  Artificial sequence

<220>
<223>  Synthetic antibody

<400>  136
cagggtatta gcagctgg                                                    18


<210>  137
<211>  24
<212>  DNA
<213>  Artificial sequence

<220>
<223>  Synthetic antibody

<400>  137
caggctaaca gtttccctcc gacg                                             24


<210>  138
<211>  18
<212>  DNA
<213>  Artificial sequence

<220>
<223>  Synthetic antibody

<400>  138
cagagtattg gtacgtgg                                                    18


<210>  139
<211>  27
<212>  DNA
<213>  Artificial sequence

<220>
<223>  Synthetic antibody

<400>  139
caacagtata atctttattc tcggacg                                          27


<210>  140
<211>  33
<212>  DNA
<213>  Artificial sequence

<220>
<223>  Synthetic antibody

<400>  140
cagagcctcc tgcaacataa cggatacaac tat                                    33


<210>  141
<211>  24
<212>  DNA
<213>  Artificial sequence

<220>
<223>  Synthetic antibody

<400>  141
atgcaagctc tacatagtcc gacg                                              24


<210>  142
<211>  18
<212>  DNA
<213>  Artificial sequence

<220>
<223>  Synthetic antibody

<400>  142
cagagcatta gcaactat                                                     18


<210>  143
<211>  24
<212>  DNA
<213>  Artificial sequence

<220>
<223>  Synthetic antibody

<400>  143
caacagagtt acagtacccc cacc                                              24


<210>  144
<211>  24
<212>  DNA
<213>  Artificial sequence

<220>
<223>  Synthetic antibody

<400>  144
agctccaaca ttgggaagaa ttat                                              24


<210>  145
<211>  33
<212>  DNA
<213>  Artificial sequence

<220>
<223>  Synthetic antibody

<400>  145
ggagcttggg atagcagcct gagtgcttat gtc                                    33

<210>  146
<211>  27
<212>  DNA
<213>  Artificial sequence

<220>
<223>  Synthetic antibody

<400>  146
agcagtgaca ttggtggtta taagtct                                          27


<210>  147
<211>  36
<212>  DNA
<213>  Artificial sequence

<220>
<223>  Synthetic antibody

<400>  147
agctcatatg caggcagcaa caatataaat tatgtc                                36


<210>  148
<211>  18
<212>  DNA
<213>  Artificial sequence

<220>
<223>  Synthetic antibody

<400>  148
cagagcatta gcagctat                                                    18


<210>  149
<211>  24
<212>  DNA
<213>  Artificial sequence

<220>
<223>  Synthetic antibody

<400>  149
caacagagtt acagtacccc cact                                             24


<210>  150
<211>  24
<212>  DNA
<213>  Artificial sequence

<220>
<223>  Synthetic antibody

<400>  150
agctccaaca ttgggaagaa ttat                                             24


<210>  151
<211>  33
<212>  DNA
<213>  Artificial sequence

<220>
<223>  Synthetic antibody

<400>  151
ggagcttggg atagcagcct gagtgcttat gtc                                    33


<210>  152
<211>  18
<212>  DNA
<213>  Artificial sequence

<220>
<223>  Synthetic antibody

<400>  152
cagagcatta gcagctct                                                     18


<210>  153
<211>  24
<212>  DNA
<213>  Artificial sequence

<220>
<223>  Synthetic antibody

<400>  153
cagcagagtt tcagtagccc cacc                                              24


<210>  154
<211>  18
<212>  DNA
<213>  Artificial sequence

<220>
<223>  Synthetic antibody

<400>  154
cagagcatta gcagctat                                                     18


<210>  155
<211>  27
<212>  DNA
<213>  Artificial sequence

<220>
<223>  Synthetic antibody

<400>  155
caacagagtt acagtacccc gctcact                                           27


<210>  156
<211>  18
<212>  DNA
<213>  Artificial sequence

<220>
<223>  Synthetic antibody

<400>  156
cagagcgtca gcaacaat                                                     18


<210>  157
<211>  21
<212>  DNA
<213>  Artificial sequence

<220>
<223>  Synthetic antibody

<400>  157
caacagagtt acagtatctc g                                                21


<210>  158
<211>  18
<212>  DNA
<213>  Artificial sequence

<220>
<223>  Synthetic antibody

<400>  158
gagagtttta gtacctgg                                                    18


<210>  159
<211>  24
<212>  DNA
<213>  Artificial sequence

<220>
<223>  Synthetic antibody

<400>  159
caacaatata atggttaccc tact                                             24


<210>  160
<211>  18
<212>  DNA
<213>  Artificial sequence

<220>
<223>  Synthetic antibody

<400>  160
cagggcatta gaaatgat                                                    18


<210>  161
<211>  30
<212>  DNA
<213>  Artificial sequence

<220>
<223>  Synthetic antibody

<400>  161
ctacagcata atagttaccc tcccgacgtt                                       30


<210>  162
<211>  18
<212>  DNA
<213>  Artificial sequence

<220>
<223>  Synthetic antibody

<400>  162
cagagcatta gcagctat                                                    18

<210>  163
<211>  27
<212>  DNA
<213>  Artificial sequence

<220>
<223>  Synthetic antibody

<400>  163
caacagagtt acagtacccc gctcact                                          27


<210>  164
<211>  18
<212>  DNA
<213>  Artificial sequence

<220>
<223>  Synthetic antibody

<400>  164
cagggtatta gcagatgg                                                    18


<210>  165
<211>  27
<212>  DNA
<213>  Artificial sequence

<220>
<223>  Synthetic antibody

<400>  165
caacaggcta acagtttccc gatcacc                                          27


<210>  166
<211>  27
<212>  DNA
<213>  Artificial sequence

<220>
<223>  Synthetic antibody

<400>  166
agcagtgacg ttggtggtta taactat                                          27


<210>  167
<211>  36
<212>  DNA
<213>  Artificial sequence

<220>
<223>  Synthetic antibody

<400>  167
agctcatata caagcagcag ccctccctta atctct                                36


<210>  168
<211>  8
<212>  PRT
<213>  Artificial sequence

<220>
<223>  Synthetic antibody

<400>  168

Gly Phe Thr Phe Ser Ser Tyr Gly
1               5


<210>  169
<211>  8
<212>  PRT
<213>  Artificial sequence

<220>
<223>  Synthetic antibody

<400>  169

Ile Arg Tyr Asp Gly Ser Asn Lys
1               5


<210>  170
<211>  15
<212>  PRT
<213>  Artificial sequence

<220>
<223>  Synthetic antibody

<400>  170

Ala Lys Ala Gly Phe Ser Gly Trp Tyr Tyr Tyr Gly Met Asp Val
1               5                   10                  15


<210>  171
<211>  10
<212>  PRT
<213>  Artificial sequence

<220>
<223>  Synthetic antibody

<400>  171

Gly Asp Ser Val Ser Ser Asn Ser Ala Ala
1               5                   10


<210>  172
<211>  9
<212>  PRT
<213>  Artificial sequence

<220>
<223>  Synthetic antibody

<400>  172

Thr Tyr Tyr Arg Ser Lys Trp Tyr Asn
1               5


<210>  173
<211>  12
<212>  PRT
<213>  Artificial sequence

<220>

<223>  Synthetic antibody

<400>  173

Ala Arg Ala Leu Ala Gly Ile Asp Gly Phe Asp Val
1               5                   10


<210>  174
<211>  8
<212>  PRT
<213>  Artificial sequence

<220>
<223>  Synthetic antibody

<400>  174

Gly Tyr Thr Phe Thr Ser Tyr Tyr
1               5


<210>  175
<211>  8
<212>  PRT
<213>  Artificial sequence

<220>
<223>  Synthetic antibody

<400>  175

Ile Asn Pro Ser Gly Gly Ser Thr
1               5


<210>  176
<211>  17
<212>  PRT
<213>  Artificial sequence

<220>
<223>  Synthetic antibody

<400>  176

Ala Arg Gly Glu Thr Gly Arg Phe Gly Glu Leu Ile Arg Gly Met Asp
1               5                   10                  15


Val




<210>  177
<211>  8
<212>  PRT
<213>  Artificial sequence

<220>
<223>  Synthetic antibody

<400>  177

Gly Phe Thr Phe Asp Asp Tyr Gly
1               5

<210>  178
<211>  8
<212>  PRT
<213>  Artificial sequence

<220>
<223>  Synthetic antibody

<400>  178

Ile Asn Trp Asn Gly Gly Ser Thr
1               5


<210>  179
<211>  11
<212>  PRT
<213>  Artificial sequence

<220>
<223>  Synthetic antibody

<400>  179

Ala Arg Glu Gly His Val Ser Ala Phe Asp Ile
1               5                   10


<210>  180
<211>  10
<212>  PRT
<213>  Artificial sequence

<220>
<223>  Synthetic antibody

<400>  180

Gly Asp Ser Val Ser Ser Asn Ser Ala Ala
1               5                   10


<210>  181
<211>  9
<212>  PRT
<213>  Artificial sequence

<220>
<223>  Synthetic antibody

<400>  181

Thr Tyr Tyr Arg Ser Lys Trp Tyr Asn
1               5


<210>  182
<211>  12
<212>  PRT
<213>  Artificial sequence

<220>
<223>  Synthetic antibody

<400>  182

Ala Arg Ala Leu Ala Gly Ile Asp Gly Phe Asp Val
1               5                   10

<210>  183
<211>  10
<212>  PRT
<213>  Artificial sequence

<220>
<223>  Synthetic antibody

<400>  183

Gly Asp Ser Val Ser Ser Asn Ser Ala Ala
1               5                   10


<210>  184
<211>  9
<212>  PRT
<213>  Artificial sequence

<220>
<223>  Synthetic antibody

<400>  184

Thr Tyr Tyr Arg Ser Lys Trp Tyr Asn
1               5


<210>  185
<211>  11
<212>  PRT
<213>  Artificial sequence

<220>
<223>  Synthetic antibody

<400>  185

Ala Arg Val Ser Pro Gly Leu Ala Phe Asp Ile
1               5                   10


<210>  186
<211>  10
<212>  PRT
<213>  Artificial sequence

<220>
<223>  Synthetic antibody

<400>  186

Gly Asp Ser Val Ser Ser Asn Ser Ala Ala
1               5                   10


<210>  187
<211>  9
<212>  PRT
<213>  Artificial sequence

<220>
<223>  Synthetic antibody

<400>  187

Thr Tyr Tyr Arg Ser Lys Trp Tyr Asn
1               5

<210>  188
<211>  11
<212>  PRT
<213>  Artificial sequence

<220>
<223>  Synthetic antibody

<400>  188

Ala Arg Arg Gly Glu Tyr Gly Ile Phe Asp Tyr
1               5                   10

<210>  189
<211>  10
<212>  PRT
<213>  Artificial sequence

<220>
<223>  Synthetic antibody

<400>  189

Gly Asp Ser Val Ser Ser Asn Ser Ala Ala
1               5                   10

<210>  190
<211>  9
<212>  PRT
<213>  Artificial sequence

<220>
<223>  Synthetic antibody

<400>  190

Thr Tyr Tyr Arg Ser Lys Trp Tyr Asn
1               5

<210>  191
<211>  18
<212>  PRT
<213>  Artificial sequence

<220>
<223>  Synthetic antibody

<400>  191

Ala Arg Asp Tyr Thr Val Arg Gly Ala Ser Leu Tyr Tyr Tyr Gly Met
1               5                   10                  15

Asp Val

<210>  192
<211>  10
<212>  PRT
<213>  Artificial sequence

<220>
<223>  Synthetic antibody

<400>  192

Gly Asp Ser Val Ser Ser Asn Ser Ala Ala
1               5                   10


<210>  193
<211>  9
<212>  PRT
<213>  Artificial sequence

<220>
<223>  Synthetic antibody

<400>  193

Thr Tyr Tyr Arg Ser Lys Trp Tyr Asn
1               5


<210>  194
<211>  21
<212>  PRT
<213>  Artificial sequence

<220>
<223>  Synthetic antibody

<400>  194

Ala Arg Asp Arg Ser Val Gly Ser Ile Glu Thr Arg Tyr Tyr Tyr Tyr
1               5                   10                  15


Tyr Gly Met Asp Val
            20


<210>  195
<211>  10
<212>  PRT
<213>  Artificial sequence

<220>
<223>  Synthetic antibody

<400>  195

Gly Asp Ser Val Ser Ser Asn Ser Ala Ala
1               5                   10


<210>  196
<211>  9
<212>  PRT
<213>  Artificial sequence

<220>
<223>  Synthetic antibody

<400>  196

Thr Tyr Tyr Arg Ser Lys Trp Tyr Asn
1               5

<210>  197
<211>  13
<212>  PRT
<213>  Artificial sequence

<220>
<223>  Synthetic antibody

<400>  197

Ala Arg Leu Arg Ser Tyr Gly Asp Asp Ala Phe Asp Ile
1               5                   10


<210>  198
<211>  8
<212>  PRT
<213>  Artificial sequence

<220>
<223>  Synthetic antibody

<400>  198

Gly Phe Thr Phe Asp Asp Tyr Gly
1               5


<210>  199
<211>  8
<212>  PRT
<213>  Artificial sequence

<220>
<223>  Synthetic antibody

<400>  199

Ile Asn Trp Asn Gly Gly Ser Thr
1               5


<210>  200
<211>  11
<212>  PRT
<213>  Artificial sequence

<220>
<223>  Synthetic antibody

<400>  200

Ala Arg Glu Gly Phe Val Met Gly Phe Asp Tyr
1               5                   10


<210>  201
<211>  8
<212>  PRT
<213>  Artificial sequence

<220>
<223>  Synthetic antibody

<400>  201

Gly Phe Thr Phe Glu Thr Ser Gly
1               5

<210>  202
<211>  8
<212>  PRT
<213>  Artificial sequence

<220>
<223>  Synthetic antibody

<400>  202

Ile Asn Trp Asn Gly Arg Asn Thr
1               5

<210>  203
<211>  12
<212>  PRT
<213>  Artificial sequence

<220>
<223>  Synthetic antibody

<400>  203

Ala Arg Val Lys Lys Gly Glu Tyr Gly Leu Asp Val
1               5                   10

<210>  204
<211>  8
<212>  PRT
<213>  Artificial sequence

<220>
<223>  Synthetic antibody

<400>  204

Gly Gly Thr Phe Ser Ser Tyr Ala
1               5

<210>  205
<211>  8
<212>  PRT
<213>  Artificial sequence

<220>
<223>  Synthetic antibody

<400>  205

Ile Ile Pro Ile Phe Gly Thr Ala
1               5

<210>  206
<211>  24
<212>  PRT
<213>  Artificial sequence

<220>
<223>  Synthetic antibody

<400>  206

Ala Arg Ala Pro Val Gly Cys Ser Ser Thr Ser Cys Tyr Thr Trp Gly
1               5                   10                  15

Tyr Tyr Tyr Tyr Gly Met Asp Val
            20

<210>  207
<211>  10
<212>  PRT
<213>  Artificial sequence

<220>
<223>  Synthetic antibody

<400>  207

Gly Asp Ser Val Ser Ser Asn Ser Ala Ala
1               5                   10

<210>  208
<211>  9
<212>  PRT
<213>  Artificial sequence

<220>
<223>  Synthetic antibody

<400>  208

Thr Tyr Tyr Arg Ser Lys Trp Tyr Asn
1               5

<210>  209
<211>  10
<212>  PRT
<213>  Artificial sequence

<220>
<223>  Synthetic antibody

<400>  209

Ala Arg Ala Arg Tyr Ala Ser Phe Asp Tyr
1               5                   10

<210>  210
<211>  8
<212>  PRT
<213>  Artificial sequence

<220>
<223>  Synthetic antibody

<400>  210

Gly Phe Thr Phe Ser Asp His Tyr
1               5

<210>  211
<211>  8

<212>  PRT
<213>  Artificial sequence

<220>
<223>  Synthetic antibody

<400>  211

Ile Asp Leu Ser Gly Ser Thr Val
1               5


<210>  212
<211>  9
<212>  PRT
<213>  Artificial sequence

<220>
<223>  Synthetic antibody

<400>  212

Ala Arg Gly His Tyr Gly Leu Asp Val
1               5


<210>  213
<211>  8
<212>  PRT
<213>  Artificial sequence

<220>
<223>  Synthetic antibody

<400>  213

Gly Tyr Thr Phe Thr Ser Tyr Tyr
1               5


<210>  214
<211>  8
<212>  PRT
<213>  Artificial sequence

<220>
<223>  Synthetic antibody

<400>  214

Ile Asn Pro Ser Gly Gly Ser Thr
1               5


<210>  215
<211>  14
<212>  PRT
<213>  Artificial sequence

<220>
<223>  Synthetic antibody

<400>  215

Ala Arg Trp Asp Ser Gly Val Arg Val Tyr Gly Met Asp Val
1               5                   10

<210>  216
<211>  10
<212>  PRT
<213>  Artificial sequence

<220>
<223>  Synthetic antibody

<400>  216

Gly Asp Ser Val Ser Ser Asn Thr Ala Ala
1               5                   10


<210>  217
<211>  9
<212>  PRT
<213>  Artificial sequence

<220>
<223>  Synthetic antibody

<400>  217

Thr Tyr Tyr Arg Ser Lys Trp Tyr Phe
1               5


<210>  218
<211>  13
<212>  PRT
<213>  Artificial sequence

<220>
<223>  Synthetic antibody

<400>  218

Ala Arg Leu Arg Ser Tyr Gly Asp Asp Ala Phe Asp Ile
1               5                   10


<210>  219
<211>  8
<212>  PRT
<213>  Artificial sequence

<220>
<223>  Synthetic antibody

<400>  219

Gly Gly Thr Phe Ser Ser Tyr Ala
1               5


<210>  220
<211>  8
<212>  PRT
<213>  Artificial sequence

<220>
<223>  Synthetic antibody

<400>  220

Ile Ile Pro Ile Phe Gly Thr Ala
1               5

<210>  221
<211>  9
<212>  PRT
<213>  Artificial sequence

<220>
<223>  Synthetic antibody

<400>  221

Ala Arg Gly Trp Asn Trp Phe Asp Pro
1               5


<210>  222
<211>  8
<212>  PRT
<213>  Artificial sequence

<220>
<223>  Synthetic antibody

<400>  222

Gly Tyr Thr Phe Thr Ser Tyr Tyr
1               5


<210>  223
<211>  8
<212>  PRT
<213>  Artificial sequence

<220>
<223>  Synthetic antibody

<400>  223

Ile Asn Pro Ser Gly Gly Ser Thr
1               5


<210>  224
<211>  17
<212>  PRT
<213>  Artificial sequence

<220>
<223>  Synthetic antibody

<400>  224

Ala Arg Asp Ser Arg Val Lys Gln Trp Gln Ala His Asp Ala Phe Asp
1               5                   10                  15


Ile


<210>  225
<211>  10
<212>  PRT
<213>  Artificial sequence

<220>

<223>  Synthetic antibody

<400>  225

Gly Asp Ser Val Ser Ser Asn Ser Ala Ala
1               5                   10


<210>  226
<211>  9
<212>  PRT
<213>  Artificial sequence

<220>
<223>  Synthetic antibody

<400>  226

Thr Tyr Tyr Arg Ser Lys Trp Tyr Asn
1               5


<210>  227
<211>  13
<212>  PRT
<213>  Artificial sequence

<220>
<223>  Synthetic antibody

<400>  227

Ala Arg Leu Arg Ser Tyr Gly Asp Asp Ala Phe Asp Ile
1               5                   10


<210>  228
<211>  8
<212>  PRT
<213>  Artificial sequence

<220>
<223>  Synthetic antibody

<400>  228

Gly Tyr Thr Phe Thr Ser Tyr Tyr
1               5


<210>  229
<211>  8
<212>  PRT
<213>  Artificial sequence

<220>
<223>  Synthetic antibody

<400>  229

Ile Asn Pro Ser Gly Gly Ser Thr
1               5


<210>  230
<211>  9
<212>  PRT
<213>  Artificial sequence

<220>
<223>  Synthetic antibody

<400>  230

Ala Arg Asp Trp Gly Arg Leu Gly Tyr
1               5


<210>  231
<211>  10
<212>  PRT
<213>  Artificial sequence

<220>
<223>  Synthetic antibody

<400>  231

Gly Asp Ser Val Ser Ser Asn Ser Ala Ala
1               5                   10


<210>  232
<211>  9
<212>  PRT
<213>  Artificial sequence

<220>
<223>  Synthetic antibody

<400>  232

Thr Tyr Tyr Arg Ser Lys Trp Tyr Asn
1               5


<210>  233

<400>  233
000

<210>  234
<211>  13
<212>  PRT
<213>  Artificial sequence

<220>
<223>  Synthetic antibody

<400>  234

Ala Arg Leu Arg Ser Tyr Gly Asp Asp Ala Phe Asp Ile
1               5                   10


<210>  235
<211>  8
<212>  PRT
<213>  Artificial sequence

<220>
<223>  Synthetic antibody

<400>  235

Gly Gly Ser Phe Ser Gly Tyr Tyr

1               5


<210>  236
<211>  7
<212>  PRT
<213>  Artificial sequence

<220>
<223>  Synthetic antibody

<400>  236

Ile Asn His Ser Gly Ser Thr
1               5


<210>  237
<211>  12
<212>  PRT
<213>  Artificial sequence

<220>
<223>  Synthetic antibody

<400>  237

Ala Arg Gly Leu Leu Tyr Ser Ser Gly Phe Asp Tyr
1               5                   10


<210>  238
<211>  10
<212>  PRT
<213>  Artificial sequence

<220>
<223>  Synthetic antibody

<400>  238

Gly Asp Asn Leu Ser Arg Asp Thr Ala Ala
1               5                   10


<210>  239
<211>  9
<212>  PRT
<213>  Artificial sequence

<220>
<223>  Synthetic antibody

<400>  239

Thr Tyr Tyr Arg Ser Lys Trp Tyr Asn
1               5


<210>  240
<211>  9
<212>  PRT
<213>  Artificial sequence

<220>
<223>  Synthetic antibody

<400>  240

Ala Arg Ser Ile Gly Gly Phe Asp Tyr
1               5

<210>  241
<211>  8
<212>  PRT
<213>  Artificial sequence

<220>
<223>  Synthetic antibody

<400>  241

Gly Phe Thr Phe Ser Asn Ala Trp
1               5

<210>  242
<211>  10
<212>  PRT
<213>  Artificial sequence

<220>
<223>  Synthetic antibody

<400>  242

Ile Lys Ser Lys Thr Asp Gly Gly Thr Thr
1               5                   10

<210>  243
<211>  9
<212>  PRT
<213>  Artificial sequence

<220>
<223>  Synthetic antibody

<400>  243

Thr Thr Tyr Ser Gly Tyr Leu Gly Tyr
1               5

<210>  244
<211>  10
<212>  PRT
<213>  Artificial sequence

<220>
<223>  Synthetic antibody

<400>  244

Gly Asp Ser Val Ser Ser Asn Ser Ala Ala
1               5                   10

<210>  245
<211>  9
<212>  PRT
<213>  Artificial sequence

<220>
<223>  Synthetic antibody

<400>  245

Thr Tyr Tyr Arg Ser Lys Trp Tyr Asn
1               5


<210>  246
<211>  10
<212>  PRT
<213>  Artificial sequence

<220>
<223>  Synthetic antibody

<400>  246

Ala Arg Trp Leu Arg Gly Gly Phe Asp Tyr
1               5                   10


<210>  247
<211>  8
<212>  PRT
<213>  Artificial sequence

<220>
<223>  Synthetic antibody

<400>  247

Gly Gly Ser Phe Ser Gly Tyr Tyr
1               5


<210>  248
<211>  7
<212>  PRT
<213>  Artificial sequence

<220>
<223>  Synthetic antibody

<400>  248

Ile Asn His Ser Gly Ser Thr
1               5


<210>  249
<211>  12
<212>  PRT
<213>  Artificial sequence

<220>
<223>  Synthetic antibody

<400>  249

Ala Arg Gly Leu Leu Tyr Ser Ser Gly Leu Asp Asn
1               5                   10


<210>  250
<211>  10
<212>  PRT
<213>  Artificial sequence

<220>
<223>  Synthetic antibody

<400>  250

Gly Asp Ser Val Ser Ser Asn Ser Ala Ala
1               5                   10


<210>  251
<211>  9
<212>  PRT
<213>  Artificial sequence

<220>
<223>  Synthetic antibody

<400>  251

Thr Tyr Tyr Arg Ser Lys Trp Tyr Asn
1               5


<210>  252
<211>  10
<212>  PRT
<213>  Artificial sequence

<220>
<223>  Synthetic antibody

<400>  252

Ala Arg Ile Val Gly Gly Ala Val Asp Tyr
1               5                   10


<210>  253
<211>  8
<212>  PRT
<213>  Artificial sequence

<220>
<223>  Synthetic antibody

<400>  253

Gly Phe Thr Phe Asp Asp Tyr Gly
1               5


<210>  254
<211>  8
<212>  PRT
<213>  Artificial sequence

<220>
<223>  Synthetic antibody

<400>  254

Ile Asn Trp Asn Gly Gly Ser Thr
1               5


<210>  255
<211>  10
<212>  PRT

<213>  Artificial sequence

<220>
<223>  Synthetic antibody

<400>  255

Ala Arg Asp Gln Phe Leu Ala Phe Asp Ile
1               5                   10


<210>  256
<211>  6
<212>  PRT
<213>  Artificial sequence

<220>
<223>  Synthetic antibody

<400>  256

Gln Ser Ile Ser Thr Tyr
1               5


<210>  257
<211>  9
<212>  PRT
<213>  Artificial sequence

<220>
<223>  Synthetic antibody

<400>  257

Gln Gln Ser Tyr Gly Leu Pro Leu Thr
1               5


<210>  258
<211>  9
<212>  PRT
<213>  Artificial sequence

<220>
<223>  Synthetic antibody

<400>  258

Ser Ser Asp Val Gly Gly Tyr Asn Tyr
1               5


<210>  259
<211>  10
<212>  PRT
<213>  Artificial sequence

<220>
<223>  Synthetic antibody

<400>  259

Ser Ser Tyr Thr Ser Ser Thr Phe Trp Val
1               5                   10


<210>  260

<211>  6
<212>  PRT
<213>  Artificial sequence

<220>
<223>  Synthetic antibody

<400>  260

Gln Gly Ile Ser Asn Tyr
1               5


<210>  261
<211>  9
<212>  PRT
<213>  Artificial sequence

<220>
<223>  Synthetic antibody

<400>  261

Gln Lys Tyr Asn Ser Ala Pro Leu Thr
1               5


<210>  262
<211>  9
<212>  PRT
<213>  Artificial sequence

<220>
<223>  Synthetic antibody

<400>  262

Ser Ser Asp Val Gly Gly Tyr Asn Tyr
1               5


<210>  263
<211>  11
<212>  PRT
<213>  Artificial sequence

<220>
<223>  Synthetic antibody

<400>  263

Ser Ser Tyr Thr Ser Ser Ser Ser His Val Val
1               5                   10


<210>  264
<211>  6
<212>  PRT
<213>  Artificial sequence

<220>
<223>  Synthetic antibody

<400>  264

Gln Ser Ile Ser Lys Tyr
1               5

<210>  265
<211>  5
<212>  PRT
<213>  Artificial sequence

<220>
<223>  Synthetic antibody

<400>  265

Gln Glu Lys Asp Asn
1               5


<210>  266
<211>  6
<212>  PRT
<213>  Artificial sequence

<220>
<223>  Synthetic antibody

<400>  266

Gln Ser Ile Ser Ser Tyr
1               5


<210>  267
<211>  9
<212>  PRT
<213>  Artificial sequence

<220>
<223>  Synthetic antibody

<400>  267

Gln Gln Ser Tyr Ser Thr Gln Gly Thr
1               5


<210>  268
<211>  6
<212>  PRT
<213>  Artificial sequence

<220>
<223>  Synthetic antibody

<400>  268

Gln Ser Ile Ser Ser Trp
1               5


<210>  269
<211>  8
<212>  PRT
<213>  Artificial sequence

<220>
<223>  Synthetic antibody

<400>  269

Gln Gln Tyr Asn Ser Tyr Tyr Thr

1               5


<210>  270
<211>  8
<212>  PRT
<213>  Artificial sequence

<220>
<223>  Synthetic antibody

<400>  270

Ser Ser Thr Ile Gly Thr Asn Pro
1               5


<210>  271
<211>  11
<212>  PRT
<213>  Artificial sequence

<220>
<223>  Synthetic antibody

<400>  271

Ala Thr Leu Asp Asp Ser Leu Asn Gly Tyr Val
1               5                   10


<210>  272
<211>  8
<212>  PRT
<213>  Artificial sequence

<220>
<223>  Synthetic antibody

<400>  272

Thr Ser Asn Ile Arg Gly Asn Thr
1               5


<210>  273
<211>  11
<212>  PRT
<213>  Artificial sequence

<220>
<223>  Synthetic antibody

<400>  273

Gly Ala Trp Asp Asp Ser Leu Asn Gly Pro Val
1               5                   10


<210>  274
<211>  6
<212>  PRT
<213>  Artificial sequence

<220>
<223>  Synthetic antibody

<400>  274

Gln Ser Ile Ser Ser Tyr
1               5


<210>  275
<211>  9
<212>  PRT
<213>  Artificial sequence

<220>
<223>  Synthetic antibody

<400>  275

Gln Gln Ser Tyr Ser Thr His Ile Thr
1               5


<210>  276
<211>  9
<212>  PRT
<213>  Artificial sequence

<220>
<223>  Synthetic antibody

<400>  276

Ser Ser Asp Val Gly Gly Tyr Asn Tyr
1               5


<210>  277
<211>  6
<212>  PRT
<213>  Artificial sequence

<220>
<223>  Synthetic antibody

<400>  277

Ser Ser Tyr Thr Ser Ser
1               5


<210>  278
<211>  6
<212>  PRT
<213>  Artificial sequence

<220>
<223>  Synthetic antibody

<400>  278

Gln Ser Ile Ser Ser Tyr
1               5


<210>  279
<211>  12
<212>  PRT
<213>  Artificial sequence

<220>
<223>  Synthetic antibody

<400>  279

Cys Gln Gln Ser Leu Gln Val Pro Leu Arg Gly Thr
1               5                   10


<210>  280
<211>  6
<212>  PRT
<213>  Artificial sequence

<220>
<223>  Synthetic antibody

<400>  280

Gln Gly Ile Ser Ser Trp
1               5


<210>  281
<211>  9
<212>  PRT
<213>  Artificial sequence

<220>
<223>  Synthetic antibody

<400>  281

Gln Gln Ala Asn Ser Phe Pro Leu Thr
1               5


<210>  282
<211>  6
<212>  PRT
<213>  Artificial sequence

<220>
<223>  Synthetic antibody

<400>  282

Gln Gly Ile Ser Ser Trp
1               5


<210>  283
<211>  8
<212>  PRT
<213>  Artificial sequence

<220>
<223>  Synthetic antibody

<400>  283

Gln Ala Asn Ser Phe Pro Pro Thr
1               5


<210>  284
<211>  6
<212>  PRT
<213>  Artificial sequence

<220>
<223>  Synthetic antibody

<400>  284

Gln Ser Ile Gly Thr Trp
1               5


<210>  285
<211>  9
<212>  PRT
<213>  Artificial sequence

<220>
<223>  Synthetic antibody

<400>  285

Gln Gln Tyr Asn Leu Tyr Ser Arg Thr
1               5


<210>  286
<211>  11
<212>  PRT
<213>  Artificial sequence

<220>
<223>  Synthetic antibody

<400>  286

Gln Ser Leu Leu Gln His Asn Gly Tyr Asn Tyr
1               5                   10


<210>  287
<211>  8
<212>  PRT
<213>  Artificial sequence

<220>
<223>  Synthetic antibody

<400>  287

Met Gln Ala Leu His Ser Pro Thr
1               5


<210>  288
<211>  6
<212>  PRT
<213>  Artificial sequence

<220>
<223>  Synthetic antibody

<400>  288

Gln Ser Ile Ser Asn Tyr
1               5


<210>  289
<211>  8
<212>  PRT

<213>  Artificial sequence

<220>
<223>  Synthetic antibody

<400>  289

Gln Gln Ser Tyr Ser Thr Pro Thr
1               5


<210>  290
<211>  8
<212>  PRT
<213>  Artificial sequence

<220>
<223>  Synthetic antibody

<400>  290

Ser Ser Asn Ile Gly Lys Asn Tyr
1               5


<210>  291
<211>  11
<212>  PRT
<213>  Artificial sequence

<220>
<223>  Synthetic antibody

<400>  291

Gly Ala Trp Asp Ser Ser Leu Ser Ala Tyr Val
1               5                   10


<210>  292
<211>  9
<212>  PRT
<213>  Artificial sequence

<220>
<223>  Synthetic antibody

<400>  292

Ser Ser Asp Ile Gly Gly Tyr Lys Ser
1               5


<210>  293
<211>  12
<212>  PRT
<213>  Artificial sequence

<220>
<223>  Synthetic antibody

<400>  293

Ser Ser Tyr Ala Gly Ser Asn Asn Ile Asn Tyr Val
1               5                   10


<210>  294

<211>  6
<212>  PRT
<213>  Artificial sequence

<220>
<223>  Synthetic antibody

<400>  294

Gln Ser Ile Ser Ser Tyr
1               5


<210>  295
<211>  8
<212>  PRT
<213>  Artificial sequence

<220>
<223>  Synthetic antibody

<400>  295

Gln Gln Ser Tyr Ser Thr Pro Thr
1               5


<210>  296
<211>  8
<212>  PRT
<213>  Artificial sequence

<220>
<223>  Synthetic antibody

<400>  296

Ser Ser Asn Ile Gly Lys Asn Tyr
1               5


<210>  297
<211>  11
<212>  PRT
<213>  Artificial sequence

<220>
<223>  Synthetic antibody

<400>  297

Gly Ala Trp Asp Ser Ser Leu Ser Ala Tyr Val
1               5                   10


<210>  298
<211>  6
<212>  PRT
<213>  Artificial sequence

<220>
<223>  Synthetic antibody

<400>  298

Gln Ser Ile Ser Ser Ser
1               5

<210>  299
<211>  8
<212>  PRT
<213>  Artificial sequence

<220>
<223>  Synthetic antibody

<400>  299

Gln Gln Ser Phe Ser Ser Pro Thr
1               5


<210>  300
<211>  6
<212>  PRT
<213>  Artificial sequence

<220>
<223>  Synthetic antibody

<400>  300

Gln Ser Ile Ser Ser Tyr
1               5


<210>  301
<211>  9
<212>  PRT
<213>  Artificial sequence

<220>
<223>  Synthetic antibody

<400>  301

Gln Gln Ser Tyr Ser Thr Pro Leu Thr
1               5


<210>  302
<211>  6
<212>  PRT
<213>  Artificial sequence

<220>
<223>  Synthetic antibody

<400>  302

Gln Ser Val Ser Asn Asn
1               5


<210>  303
<211>  7
<212>  PRT
<213>  Artificial sequence

<220>
<223>  Synthetic antibody

<400>  303

Gln Gln Ser Tyr Ser Ile Ser

1               5


<210>  304
<211>  6
<212>  PRT
<213>  Artificial sequence

<220>
<223>  Synthetic antibody

<400>  304

Glu Ser Phe Ser Thr Trp
1               5


<210>  305
<211>  8
<212>  PRT
<213>  Artificial sequence

<220>
<223>  Synthetic antibody

<400>  305

Gln Gln Tyr Asn Gly Tyr Pro Thr
1               5


<210>  306
<211>  6
<212>  PRT
<213>  Artificial sequence

<220>
<223>  Synthetic antibody

<400>  306

Gln Gly Ile Arg Asn Asp
1               5


<210>  307
<211>  10
<212>  PRT
<213>  Artificial sequence

<220>
<223>  Synthetic antibody

<400>  307

Leu Gln His Asn Ser Tyr Pro Pro Asp Val
1               5                   10


<210>  308
<211>  6
<212>  PRT
<213>  Artificial sequence

<220>
<223>  Synthetic antibody

<400>  308

Gln Ser Ile Ser Ser Tyr
1               5


<210>  309
<211>  9
<212>  PRT
<213>  Artificial sequence

<220>
<223>  Synthetic antibody

<400>  309

Gln Gln Ser Tyr Ser Thr Pro Leu Thr
1               5


<210>  310
<211>  6
<212>  PRT
<213>  Artificial sequence

<220>
<223>  Synthetic antibody

<400>  310

Gln Gly Ile Ser Arg Trp
1               5


<210>  311
<211>  9
<212>  PRT
<213>  Artificial sequence

<220>
<223>  Synthetic antibody

<400>  311

Gln Gln Ala Asn Ser Phe Pro Ile Thr
1               5


<210>  312
<211>  9
<212>  PRT
<213>  Artificial sequence

<220>
<223>  Synthetic antibody

<400>  312

Ser Ser Asp Val Gly Gly Tyr Asn Tyr
1               5


<210>  313
<211>  12
<212>  PRT
<213>  Artificial sequence

<220>
<223>  Synthetic antibody

<400>  313

Ser Ser Tyr Thr Ser Ser Ser Pro Pro Leu Ile Ser
1               5                   10


<210>  314
<211>  366
<212>  DNA
<213>  Artificial sequence

<220>
<223>  Synthetic antibody

<400>  314
gaggtgcagc tggtggagtc tgggggaggc gtggtccagc ctgggaggtc cctgagactc        60

tcctgtgcag cgtctggatt caccttcagt agctatggca tgcactgggt ccgccaggct       120

ccaggcaagg ggctggagtg ggtggcattt atacggtatg atggaagtaa taaatactat       180

gcagactccg tgaagggccg attcaccatc tccagagaca attccaagaa cacgctgtat       240

ctgcaaatga acagcctgag agctgaggac acggctgtgt attactgtgc gaaagccggg       300

ttcagtggct ggtactacta cggtatggac gtctggggcc aagggacaat ggtcaccgtc       360

tcttca                                                                  366


<210>  315
<211>  366
<212>  DNA
<213>  Artificial sequence

<220>
<223>  Synthetic antibody

<400>  315
caggtacagc tgcagcagtc aggtccagga ctggtgaagc cctcgcagac cctctcactc        60

acctgtgcca tctccgggga cagtgtctct agcaacagtg ctgcttggaa ctggatcagg       120

cagtccccat cgagaggcct tgagtggctg ggaaggacat actacaggtc caagtggtat       180

aataattatg cagtgtctgt gaaaagtcga ataaccatca acccagacac atccaagaac       240

cagttctccc tgcagctgaa ctctgtgact cccgaggaca cggctgtgta ttactgtgcc       300

agagctttgg ctgggattga tggttttgat gtatggggcc aagggaccac ggtcagcgtc       360

tcttca                                                                  366


<210>  316
<211>  372
<212>  DNA
<213>  Artificial sequence

<220>
<223>  Synthetic antibody

<400>  316
gaagtgcagc tggtgcagtc tggggctgag gtgaagaagc ctggggcctc agtgaaggtt        60

tcctgcaagg catctggata caccttcacc agctactata tgcactgggt gcgacaggcc       120

cctggacaag ggcttgagtg gatgggaata atcaacccta gtggtggtag cacaagctac 180

gcacagaagt tccagggcag agtcaccatg accagggaca cgtccacgag cacagtctac 240

atggagctga gcagcctgag atctgaggac acggccgtgt attactgtgc gagaggcgaa 300

acagggaggt tcggggagtt aatacggggt atggacgtct ggggccaagg gaccacggtc 360

accgtctctt ca 372

<210> 317
<211> 354
<212> DNA
<213> Artificial sequence

<220>
<223> Synthetic antibody

<400> 317
gaagtgcagc tggtgcagtc tgggggaggt gtggtacggc ctggggggtc cctgagactc 60

tcctgtgcag cctctggatt cacctttgat gattatggca tgagctgggt ccgccaagct 120

ccagggaagg ggctggagtg ggtctctggt attaattgga atggtggtag cacaggttat 180

gcagactctg tgaagggccg attcaccatc tccagagaca acgccaagaa ctccctgtat 240

ctgcaaatga acagtctgag agccgaggac acggccttgt attactgtgc gagagagggc 300

catgtgtctg cttttgatat ctggggccaa gggacaatgg tcaccgtctc ctca 354

<210> 318
<211> 366
<212> DNA
<213> Artificial sequence

<220>
<223> Synthetic antibody

<400> 318
caggtacagc tgcagcagtc aggtccagga ctggtgaagc cctcgcagac cctctcactc 60

acctgtgcca tctccgggga cagtgtctct agcaacagtg ctgcttggaa ctggatcagg 120

cagtccccat cgagaggcct tgagtggctg ggaaggacat actacaggtc caagtggtat 180

aataattatg cagtgtctgt gaaaagtcga ataaccatca acccagacac atccaagaac 240

cagttctccc tgcagctgaa ctctgtgact cccgaggaca cggctgtgta ttactgtgcc 300

agagctttgg ctgggattga tggttttgat gtatggggcc aagggaccac ggtcagcgtc 360

tcttca 366

<210> 319
<211> 363
<212> DNA
<213> Artificial sequence

<220>
<223> Synthetic antibody

<400> 319
caggtacagc tgcagcagtc aggtccagga ctggtgaagc cctcgcagac cctctcactc 60

acctgtgcca tctccgggga cagtgtctct agcaacagtg ctgcttggaa ctggatcagg 120

cagtccccat cgagaggcct tgagtggctg ggaaggacat actacaggtc caagtggtat 180

aatgattatg cagtatctgt gaaaagtcga ataaccatca acccagacac atccaagaac 240

cagttctccc tgcagctgaa ctctgtgact cccgaggaca cggctgtgta ttactgtgca 300

agagtgtcgc cggggcttgc ttttgatatc tggggccaag ggacaatggt caccgtctct 360

tca 363

<210> 320
<211> 363
<212> DNA
<213> Artificial sequence

<220>
<223> Synthetic antibody

<400> 320
caggtacagc tgcagcagtc aggtccagga ctggtgaagc cctcgcagac cctctcactc 60

acctgtgcca tctccgggga cagtgtctct agcaacagtg ctgcttggaa ctggatcagg 120

cagtccccat cgagaggcct tgagtggctg ggaaggacat actacaggtc caagtggtat 180

aatgattatg cagtatctgt gaaaagtcga ataaccatca acccagacac atccaagaac 240

cagttctccc tgcagctgaa ctctgtgact cccgaggaca cggctgtgta ttactgtgca 300

agacgggggg aatacggaat ctttgactac tggggccagg gaaccctggt caccgtctct 360

tca 363

<210> 321
<211> 384
<212> DNA
<213> Artificial sequence

<220>
<223> Synthetic antibody

<400> 321
caggtacagc tgcagcagtc aggtccagga ctggtgaagc cctcgcagac cctctcactc 60

acctgtgcca tctccgggga cagtgtctct agcaacagtg ctgcttggaa ctggatcagg 120

cagtccccat cgagaggcct tgagtggctg ggaaggacat actacaggtc caagtggtat 180

aatgattatg cagtatctgt gaaaagtcga ataaccatca acccagacac atccaagaac 240

cagttctccc tgcagctgaa ctctgtgact cccgaggaca cggctgtgta ttactgtgca 300

agagattata cggttcgggg agcgtctctc tactactacg gtatggacgt ctggggccaa 360

gggaccacgg tcaccgtctc ttca 384

<210> 322
<211> 393
<212> DNA
<213> Artificial sequence

<220>
<223> Synthetic antibody

<400>  322
caggtacagc tgcagcagtc aggtccagga ctggtgaagc cctcgcagac cctctcactc      60

acctgtgcca tctccgggga cagtgtctct agcaacagtg ctgcttggaa ctggatcagg     120

cagtccccat cgagaggcct tgagtggctg ggaaggacat actacaggtc caagtggtat     180

aatgattatg cagtatctgt gaaaagtcga ataaccatca acccagacac atccaagaac     240

cagttctccc tgcagctgaa ctctgtgact cccgaggaca cggctgtgta ttactgtgca     300

agagatagga gtgtaggcag catagagaca aggtactact actactacgg tatggacgtc     360

tggggccaag ggaccacggt cagcgtctcc tca                                  393


<210>  323
<211>  369
<212>  DNA
<213>  Artificial sequence

<220>
<223>  Synthetic antibody

<400>  323
caggtacagc tgcagcagtt aggtccagga ctggtgaagc cctcgcagac cctctcactc      60

acctgtgcca tctccgggga cagtgtctct agcaacagtg ctgcttggaa ctggatcagg     120

cagtccccat cgagaggcct tgagtggctg ggaaggacat actacaggtc caagtggtat     180

aatgattatg cagtatctgt gaaaagtcga ataaccatca acccagacac atccaagaac     240

cagttctccc tgcagctgaa ctctgtgact cccgaggaca cggctgtgta ttactgtgca     300

agactccgct cctatggtga tgatgctttt gatatctggg gccaagggac aatggtcacc     360

gtctcctca                                                             369


<210>  324
<211>  393
<212>  DNA
<213>  Artificial sequence

<220>
<223>  Synthetic antibody

<400>  324
caggtgcagc tggtgcagtc tggggctgag gtgaagaagc ctgggtcctc ggtgaaggtc      60

tcctgcaagg cttctggagg caccttcagc agctatgcta tcagctgggt gcgacaggcc     120

cctggacaag ggcttgagtg gatgggaggg atcatcccta tctttggtac agcaaactac     180

gcacagaagt tccagggcag agtcacgatt accgcggaca aatccacgag cacagcctac     240

atggagctga gcagcctgag atctgaggac acggccgtgt attactgtgc gagagcaccg     300

gtcgggtgta gtagtaccag ctgctatact tggggctact actactacgg tatggacgtc     360

tggggccaag ggaccacggt caccgtctct tca                                  393


<210>  325
<211>  360
<212>  DNA

<213>  Artificial sequence

<220>
<223>  Synthetic antibody

<400>  325
caggtacagc tgcagcagtc aggtccagga ctggtgaagc cctcgcagac cctctcactc      60

acctgtgcca tctccgggga cagtgtctct agcaacagtg ctgcttggaa ctggatcagg     120

cagtccccat cgagaggcct tgagtggctg ggaaggacat actacaggtc caagtggtat     180

aatgattatg cagtatctgt gaaaagtcga ataaccatca acccagacac atccaagaac     240

cagttctccc tgcagctgaa ctctgtgact cccgaggaca cggctgtgta ttactgtgca     300

agagcccgat acgcatcctt tgactactgg ggccagggaa ccctggtcac tgtctcctca     360


<210>  326
<211>  348
<212>  DNA
<213>  Artificial sequence

<220>
<223>  Synthetic antibody

<400>  326
caggtgcaga tggtggagtc tgggggaggc ttggtcaagc ctggagggtc cctgagactc      60

tcctgtgcag cctctggatt caccttcagt gaccactacc tgagctggat ccgccaggct     120

ccagggaagg ggctggagtg ggtttcatac attgatcttt ctggtagtac cgtatactac     180

ggagactctg tgaagggtcg atttaccatc tccagggaca acgccaggaa ctcagtatac     240

ttgcaaatga acagcctgag agccgaggac acggccgtat attactgtgc gaggggtcac     300

tacggtttgg acgtctgggg ccaagggaca atggtcaccg tctcttca                  348


<210>  327
<211>  363
<212>  DNA
<213>  Artificial sequence

<220>
<223>  Synthetic antibody

<400>  327
caggtgcagc tggtgcagtc tggggctgag gtgaagaagc ctggggcctc agtgaaggtt      60

tcctgcaagg catctggata caccttcacc agctactata tgcactgggt gcgacaggcc     120

cctggacaag ggcttgagtg gatgggaata atcaacccta gtggtggtag cacaagctac     180

gcacagaagt tccagggcag agtcaccatg accagggaca cgtccacgag cacagtctac     240

atggagctga gcagcctgag atctgaggac acggccgtgt attactgtgc gagatgggac     300

agtggcgtcc gggtctacgg tatggacgtc tggggccaag ggaccacggt caccgtctcc     360

tca                                                                   363


<210>  328
<211>  363
<212>  DNA

<213>  Artificial sequence

<220>
<223>  Synthetic antibody

<400>  328
caggtgcagc tggtgcagtc tggggctgag gtgaagaagc ctggggcctc agtgaaggtt      60

tcctgcaagg catctggata caccttcacc agctactata tgcactgggt gcgacaggcc     120

cctggacaag ggcttgagtg gatgggaata atcaacccta gtggtggtag cacaagctac     180

gcacagaagt tccagggcag agtcaccatg accagggaca cgtccacgag cacagtctac     240

atggagctga gcagcctgag atctgaggac acggccgtgt attactgtgc gagatgggac     300

agtggcgtcc gggtctacgg tatggacgtc tggggccaag ggaccacggt caccgtctcc     360

tca                                                                   363


<210>  329
<211>  348
<212>  DNA
<213>  Artificial sequence

<220>
<223>  Synthetic antibody

<400>  329
caggtccagc tggtgcagtc tggggctgag gtgaagaagc ctgggtcctc ggtgaaggtc      60

tcctgcaagg cttctggagg caccttcagc agctatgcta tcagctgggt gcgacaggcc     120

cctggacaag ggcttgagtg gatgggaggg atcatcccta tctttggtac agcaaactac     180

gcacagaagt tccagggcag agtcacgatt accgcggacg aatccacgag cacagcctac     240

atggagctga gcagcctgag atctgaggac acggccgtgt attactgtgc gagaggctgg     300

aactggttcg acccctgggg ccagggaaca atggtcatcg tctcctca                 348


<210>  330
<211>  372
<212>  DNA
<213>  Artificial sequence

<220>
<223>  Synthetic antibody

<400>  330
caggtgcagc tggtgcagtc tggggctgag gtgaagaagc ctggggcctc agtgaaggtt      60

tcctgcaagg catctggata caccttcacc agctactata tgcactgggt gcgacaggcc     120

cctggacaag ggcttgagtg gatgggaata atcaacccta gtggtggtag cacaagctac     180

gcacagaagt tccagggcag agtcaccatg accagggaca cgtccacgag cacagtctac     240

atggagctga gcagcctgag atctgaggac acggccgtgt attactgtgc gagagattcg     300

cgagtaaagc agtggcaggc acatgatgct tttgatatct ggggccaagg gacaatggtc     360

accgtctctt ca                                                         372


<210>  331

<211>  369
<212>  DNA
<213>  Artificial sequence

<220>
<223>  Synthetic antibody

<400>  331
caggtacagc tgcagcagtc aggtccagga ctggtgaagc cctcgcagac cctctcactc      60

acctgtgcca tctccgggga cagtgtctct agcaacagtg ctgcttggaa ctggatcagg     120

cagtccccat cgagaggcct tgagtggctg ggaaggacat actacaggtc caagtggtat     180

aatgattatg cagtatctgt gaaaagtcga ataaccatca acccagacac atccaagaac     240

cagttctccc tgcagctgaa ctctgtgact cccgaggaca cggctgtgta ttactgtgca     300

agactccgct cctatggtga tgatgctttt gatatctggg gccaagggac aatggtcacc     360

gtctcctca                                                             369


<210>  332
<211>  348
<212>  DNA
<213>  Artificial sequence

<220>
<223>  Synthetic antibody

<400>  332
caggtgcttc tggtgcagtc tggggctgag gtgaagaagc ctggggcctc agtgaaggtt      60

tcctgcaagg catctggata caccttcacc agctactata tgcactgggt gcgacaggcc     120

cctggacaag ggcttgagtg gatgggaata atcaacccta gtggtggtag cacaagctac     180

gcacagaagt tccagggcag agtcaccatg accagggaca cgtccacgag cacagtctac     240

atggagctga gcagcctgag atctgaggac acggccgtgt attactgtgc gagagattgg     300

gggcgtttgg ggtactgggg ccagggaacc acggtcaccg tctcttca                  348


<210>  333
<211>  369
<212>  DNA
<213>  Artificial sequence

<220>
<223>  Synthetic antibody

<400>  333
caggtacagc tgcagcagtc aggtccagga ctggtgaagc cctcgcagac cctctcactc      60

acctgtgcca tctccgggga cagtgtctct agcaacagtg ctgcttggaa ctggatcagg     120

cagtccccat cgagaggcct tgagtggctg ggaaggacat actacaggtc caagtggtat     180

aatgattatg cagtatctgt gaaaagtcga ataaccatca acccagacac atccaagaac     240

cagttctccc tgcagctgaa ctctgtgact cccgaggaca cggctgtgta ttactgtgca     300

agactccgct cctatggtga tgatgctttt gatatctggg gccaagggac cacggtcacc     360

gtctcttca                                                             369

<210>  334
<211>  354
<212>  DNA
<213>  Artificial sequence

<220>
<223>  Synthetic antibody

<400>  334
caggtgcagc tacagcagtg gggcgcagga ctgttgaagc cttcggagac cctgtccctc      60

acctgcgctg tctatggtgg gtccttcagt ggttactact ggagctggat ccgccagccc     120

ccagggaagg ggctggagtg gattggggaa atcaatcata gtggaagcac caactacaac     180

ccgtccctca agagtcgagt caccatatca gtagacacgt ccaagaacca gttctccctg     240

aagctgagct ctgtgaccgc cgcggacacg gctgtgtatt actgtgcgag aggcctcctg     300

tatagcagtg gttttgacta ctggggccag ggaaccctgg tcaccgtctc ctca           354


<210>  335
<211>  357
<212>  DNA
<213>  Artificial sequence

<220>
<223>  Synthetic antibody

<400>  335
caggtacagc tgcagcagtc aggtccagga ctggtgaagc cctcgcagac cctctcactc      60

acctgtgcca tctccgggga caatctctct agggacactg ctgcttggaa ttggatcagg     120

cagtccccat cgagaggcct tgagtggctg ggaaggacat actacaggtc caagtggtat     180

aatgattatg cagtatctgt gaaaagtcga ataaccatca acccagacac atccaagaac     240

caattctccc tgcagctgaa ctctgtgact cccgaggaca cggctgtgta ttactgtgcg     300

agaagtatag gaggttttga ctattggggc cagggaacca tggtcaccgt ctcttca        357


<210>  336
<211>  354
<212>  DNA
<213>  Artificial sequence

<220>
<223>  Synthetic antibody

<400>  336
caggtgaagc tggtggagtc tgggggaggc ttggtaaagc ctggggggtc ccttagactc      60

tcctgtgcag cctctggatt cactttcagt aacgcctgga tgagctgggt ccgccaggct     120

ccagggaagg ggctggagtg ggttggccgt attaaaagca aaactgatgg tgggacaaca     180

gactacgctg cacccgtgaa aggcagattc accatctcaa gagatgattc aaaaaacacg     240

ctgtatctgc aaatgaacag cctgaaaacc gaggacacag ccgtgtatta ctgtaccacg     300

tatagtggtt atttaggcta ctggggccag ggaaccctgg tcactgtctc ctca           354


<210>  337

<211>  360
<212>  DNA
<213>  Artificial sequence

<220>
<223>  Synthetic antibody

<400>  337
caggtacagc tgcagcagtc aggtccagga ctggtgaagc cctcgcagac cctctcactc      60

acctgtgcca tctccgggga cagtgtctct agcaacagtg ctgcttggat ctggatcagg     120

cagtccccat cgagaggcct tgagtggctg ggaaggacat actacaggtc caagtggtat     180

aatgattatg cagtatctgt gaaaagtcga ataaccatca acccagacac atccaagaac     240

cacttctccc tgcagctgaa ctctgtgact cccgaggaca cggctgtgta ttactgtgca     300

aggtggctac gtgggggctt tgactactgg ggccaaggga caatggtcac cgtctcttca     360


<210>  338
<211>  354
<212>  DNA
<213>  Artificial sequence

<220>
<223>  Synthetic antibody

<400>  338
caggtgcagc tacaacagtg gggcgcagga ctgttgaagc cttcggagac cctgtccctc      60

acctgcgctg tctatggtgg gtccttcagt ggttactact ggagctggat ccgccagccc     120

ccagggaagg ggctggagtg gattggggaa atcaatcata gtggaagcac caactacaac     180

ccgtccctca agagtcgagt caccatatca gtagacacgt ccaagaacca gttctccctg     240

aagctgagct ctgtgaccgc cgcggacacg gctgtgtatt actgtgcgag aggcttgctg     300

tatagcagtg gtctcgacaa ctggggccag ggaaccctgg tcaccgtctc ttca           354


<210>  339
<211>  360
<212>  DNA
<213>  Artificial sequence

<220>
<223>  Synthetic antibody

<400>  339
caggtacagc tgcagcagtc aggtccagga ctggtgaagc cctcgcagac cctctcactc      60

acctgtgcca tctccgggga cagtgtctct agcaacagtg ctgcttggaa ctggatcagg     120

cagtccccat cgagaggcct tgagtggctg ggaaggacat actacaggtc caagtggtat     180

aatgattatg cagtttcagt gagaagtcga ataaccatca acccagacac atccaagaac     240

cagttctccc tgcagctaaa ctctgtgact cccgaggaca cggctgtgta ttactgtgcg     300

cgtatagtgg gaggtgccgt tgactactgg ggccagggaa ccctggtcag cgtctcctca     360


<210>  340
<211>  351
<212>  DNA

<213>  Artificial sequence

<220>
<223>  Synthetic antibody

<400>  340
caggtgaagc tggtggagtc tgggggaggt gtggtacggc ctggggggtc cctgagactc        60

tcctgtgcag cctctggatt cacctttgat gattatggca tgagctgggt ccgccaagct      120

ccagggaagg ggctggagtg ggtctctggt attaattgga atggtggtag cacaggttat      180

gcagactctg tgaagggccg attcaccatc tccagagaca acgccaagaa ctccctgtat      240

ctgcaaatga acagtctgag agccgaggac acggccttgt attactgtgc gagagatcag      300

ttcttggctt ttgatatctg gggccaaggg accacggtca tcgtctcctc a               351


<210>  341
<211>  122
<212>  PRT
<213>  Artificial sequence

<220>
<223>  Synthetic antibody

<400>  341

Glu Val Gln Leu Val Glu Ser Gly Gly Gly Val Val Gln Pro Gly Arg
1               5                   10                  15


Ser Leu Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
        35                  40                  45


Ala Phe Ile Arg Tyr Asp Gly Ser Asn Lys Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Ala Gly Phe Ser Gly Trp Tyr Tyr Tyr Gly Met Asp Val Trp
            100                 105                 110


Gly Gln Gly Thr Met Val Thr Val Ser Ser
        115                 120


<210>  342
<211>  122
<212>  PRT
<213>  Artificial sequence

<220>
<223>  Synthetic antibody

<400>  342

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asn Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Ala Leu Ala Gly Ile Asp Gly Phe Asp Val Trp
            100                 105                 110

Gly Gln Gly Thr Thr Val Ser Val Ser Ser
        115                 120


<210>  343
<211>  124
<212>  PRT
<213>  Artificial sequence

<220>
<223>  Synthetic antibody

<400>  343

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Glu Thr Gly Arg Phe Gly Glu Leu Ile Arg Gly Met Asp
            100                 105                 110

Val Trp Gly Gln Gly Thr Thr Val Thr Val Ser Ser
        115                 120

<210>  344
<211>  118
<212>  PRT
<213>  Artificial sequence

<220>
<223>  Synthetic antibody

<400>  344

Glu Val Gln Leu Val Gln Ser Gly Gly Gly Val Val Arg Pro Gly Gly
1               5                   10                  15

Ser Leu Arg Leu Ser Cys Ala Ala Ser Gly Phe Thr Phe Asp Asp Tyr
            20                  25                  30

Gly Met Ser Trp Val Arg Gln Ala Pro Gly Lys Gly Leu Glu Trp Val
        35                  40                  45

Ser Gly Ile Asn Trp Asn Gly Gly Ser Thr Gly Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Leu Tyr Tyr Cys
                85                  90                  95

Ala Arg Glu Gly His Val Ser Ala Phe Asp Ile Trp Gly Gln Gly Thr
            100                 105                 110

Met Val Thr Val Ser Ser
        115

<210>  345
<211>  122
<212>  PRT
<213>  Artificial sequence

<220>
<223>  Synthetic antibody

<400>  345

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asn Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Ala Leu Ala Gly Ile Asp Gly Phe Asp Val Trp
            100                 105                 110

Gly Gln Gly Thr Thr Val Ser Val Ser Ser
        115                 120

<210> 346
<211> 121
<212> PRT
<213> Artificial sequence

<220>
<223> Synthetic antibody

<400> 346

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Val Ser Pro Gly Leu Ala Phe Asp Ile Trp Gly
            100                 105                 110

Gln Gly Thr Met Val Thr Val Ser Ser
        115                 120

<210> 347

<211>  121
<212>  PRT
<213>  Artificial sequence

<220>
<223>  Synthetic antibody

<400>  347

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Arg Gly Glu Tyr Gly Ile Phe Asp Tyr Trp Gly
            100                 105                 110

Gln Gly Thr Leu Val Thr Val Ser Ser
        115                 120


<210>  348
<211>  128
<212>  PRT
<213>  Artificial sequence

<220>
<223>  Synthetic antibody

<400>  348

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn

65                  70                  75                  80

Gln Phe Ser Leu Gln Leu Asn Ser Val Thr Pro Glu Asp Thr Ala Val
                85                  90                  95

Tyr Tyr Cys Ala Arg Asp Tyr Thr Val Arg Gly Ala Ser Leu Tyr Tyr
            100                 105                 110

Tyr Gly Met Asp Val Trp Gly Gln Gly Thr Thr Val Thr Val Ser Ser
        115                 120                 125

<210>  349
<211>  131
<212>  PRT
<213>  Artificial sequence

<220>
<223>  Synthetic antibody

<400>  349

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Asp Arg Ser Val Gly Ser Ile Glu Thr Arg Tyr
            100                 105                 110

Tyr Tyr Tyr Tyr Gly Met Asp Val Trp Gly Gln Gly Thr Thr Val Ser
        115                 120                 125

Val Ser Ser
    130

<210>  350
<211>  123
<212>  PRT
<213>  Artificial sequence

<220>
<223>  Synthetic antibody

<400>  350

Gln Val Gln Leu Gln Gln Leu Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Leu Arg Ser Tyr Gly Asp Asp Ala Phe Asp Ile
            100                 105                 110

Trp Gly Gln Gly Thr Met Val Thr Val Ser Ser
        115                 120


<210>  351
<211>  131
<212>  PRT
<213>  Artificial sequence

<220>
<223>  Synthetic antibody

<400>  351

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ala Pro Val Gly Cys Ser Ser Thr Ser Cys Tyr Thr Trp Gly
            100                 105                 110

Tyr Tyr Tyr Tyr Gly Met Asp Val Trp Gly Gln Gly Thr Thr Val Thr
        115                 120                 125

Val Ser Ser
    130

<210>  352
<211>  120
<212>  PRT
<213>  Artificial sequence

<220>
<223>  Synthetic antibody

<400>  352

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Ala Arg Tyr Ala Ser Phe Asp Tyr Trp Gly Gln
            100                 105                 110

Gly Thr Leu Val Thr Val Ser Ser
        115                 120

<210>  353
<211>  116
<212>  PRT
<213>  Artificial sequence

<220>
<223>  Synthetic antibody

<400>  353

Gln Val Gln Met Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp His
            20                  25                  30

Tyr Leu Ser Trp Ile Arg Gln Ala Pro Gly Lys Gly Leu Glu Trp Val
        35                  40                  45

Ser Tyr Ile Asp Leu Ser Gly Ser Thr Val Tyr Tyr Gly Asp Ser Val
    50                  55                  60

Lys Gly Arg Phe Thr Ile Ser Arg Asp Asn Ala Arg Asn Ser Val Tyr
65                  70                  75                  80

Leu Gln Met Asn Ser Leu Arg Ala Glu Asp Thr Ala Val Tyr Tyr Cys
                85                  90                  95

Ala Arg Gly His Tyr Gly Leu Asp Val Trp Gly Gln Gly Thr Met Val
            100                 105                 110

Thr Val Ser Ser
        115

<210> 354
<211> 121
<212> PRT
<213> Artificial sequence

<220>
<223> Synthetic antibody

<400> 354

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Trp Asp Ser Gly Val Arg Val Tyr Gly Met Asp Val Trp Gly
            100                 105                 110

Gln Gly Thr Thr Val Thr Val Ser Ser

115                 120

<210>  355
<211>  123
<212>  PRT
<213>  Artificial sequence

<220>
<223>  Synthetic antibody

<400>  355

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Thr Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Phe Asp Tyr Ala
    50                  55                  60

Val Ser Val Lys Ser Arg Met Thr Ile Asn Pro Asp Thr Ser Lys Asn
65                  70                  75                  80

Gln Phe Ser Leu Gln Leu Asn Ser Leu Thr Pro Glu Asp Thr Ala Val
                85                  90                  95

Tyr Tyr Cys Ala Arg Leu Arg Ser Tyr Gly Asp Asp Ala Phe Asp Ile
            100                 105                 110

Trp Gly Gln Gly Thr Thr Val Thr Val Ser Ser
        115                 120

<210>  356
<211>  116
<212>  PRT
<213>  Artificial sequence

<220>
<223>  Synthetic antibody

<400>  356

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe

50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Trp Asn Trp Phe Asp Pro Trp Gly Gln Gly Thr Met Val
            100                 105                 110

Ile Val Ser Ser
        115

<210>  357
<211>  124
<212>  PRT
<213>  Artificial sequence

<220>
<223>  Synthetic antibody

<400>  357

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Asp Ser Arg Val Lys Gln Trp Gln Ala His Asp Ala Phe Asp
            100                 105                 110

Ile Trp Gly Gln Gly Thr Met Val Thr Val Ser Ser
        115                 120

<210>  358
<211>  123
<212>  PRT
<213>  Artificial sequence

<220>
<223>  Synthetic antibody

<400>  358

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Leu Arg Ser Tyr Gly Asp Asp Ala Phe Asp Ile
            100                 105                 110

Trp Gly Gln Gly Thr Met Val Thr Val Ser Ser
        115                 120


<210>  359
<211>  116
<212>  PRT
<213>  Artificial sequence

<220>
<223>  Synthetic antibody

<400>  359

Gln Val Leu Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Asp Trp Gly Arg Leu Gly Tyr Trp Gly Gln Gly Thr Thr Val
            100                 105                 110

Thr Val Ser Ser
        115

<210>  360
<211>  123
<212>  PRT
<213>  Artificial sequence

<220>
<223>  Synthetic antibody

<400>  360

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Leu Arg Ser Tyr Gly Asp Asp Ala Phe Asp Ile
            100                 105                 110

Trp Gly Gln Gly Thr Thr Val Thr Val Ser Ser
        115                 120

<210>  361
<211>  118
<212>  PRT
<213>  Artificial sequence

<220>
<223>  Synthetic antibody

<400>  361

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Gly Leu Leu Tyr Ser Ser Gly Phe Asp Tyr Trp Gly Gln Gly Thr
            100                 105                 110

Leu Val Thr Val Ser Ser
        115

<210> 362
<211> 119
<212> PRT
<213> Artificial sequence

<220>
<223> Synthetic antibody

<400> 362

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Asn Leu Ser Arg Asp
            20                  25                  30

Thr Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Ser Ile Gly Gly Phe Asp Tyr Trp Gly Gln Gly
            100                 105                 110

Thr Met Val Thr Val Ser Ser
        115

<210> 363

<211>  118
<212>  PRT
<213>  Artificial sequence

<220>
<223>  Synthetic antibody

<400>  363

Gln Val Lys Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Gly Arg Ile Lys Ser Lys Thr Asp Gly Gly Thr Thr Asp Tyr Ala Ala
    50                  55                  60

Pro Val Lys Gly Arg Phe Thr Ile Ser Arg Asp Asp Ser Lys Asn Thr
65                  70                  75                  80

Leu Tyr Leu Gln Met Asn Ser Leu Lys Thr Glu Asp Thr Ala Val Tyr
                85                  90                  95

Tyr Cys Thr Thr Tyr Ser Gly Tyr Leu Gly Tyr Trp Gly Gln Gly Thr
            100                 105                 110

Leu Val Thr Val Ser Ser
        115


<210>  364
<211>  120
<212>  PRT
<213>  Artificial sequence

<220>
<223>  Synthetic antibody

<400>  364

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Ile Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn

65                  70                  75                  80

His Phe Ser Leu Gln Leu Asn Ser Val Thr Pro Glu Asp Thr Ala Val
                85                  90                  95

Tyr Tyr Cys Ala Arg Trp Leu Arg Gly Gly Phe Asp Tyr Trp Gly Gln
            100                 105                 110

Gly Thr Met Val Thr Val Ser Ser
        115                 120

<210>  365
<211>  118
<212>  PRT
<213>  Artificial sequence

<220>
<223>  Synthetic antibody

<400>  365

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Gly Leu Leu Tyr Ser Ser Gly Leu Asp Asn Trp Gly Gln Gly Thr
            100                 105                 110

Leu Val Thr Val Ser Ser
        115

<210>  366
<211>  120
<212>  PRT
<213>  Artificial sequence

<220>
<223>  Synthetic antibody

<400>  366

Gln Val Gln Leu Gln Gln Ser Gly Pro Gly Leu Val Lys Pro Ser Gln

1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Arg Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Ile Val Gly Gly Ala Val Asp Tyr Trp Gly Gln
            100                 105                 110

Gly Thr Leu Val Ser Val Ser Ser
        115                 120

<210>  367
<211>  117
<212>  PRT
<213>  Artificial sequence

<220>
<223>  Synthetic antibody

<400>  367

Gln Val Lys Leu Val Glu Ser Gly Gly Gly Val Val Arg Pro Gly Gly
1               5                   10                  15

Ser Leu Arg Leu Ser Cys Ala Ala Ser Gly Phe Thr Phe Asp Asp Tyr
            20                  25                  30

Gly Met Ser Trp Val Arg Gln Ala Pro Gly Lys Gly Leu Glu Trp Val
        35                  40                  45

Ser Gly Ile Asn Trp Asn Gly Gly Ser Thr Gly Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Leu Tyr Tyr Cys
                85                  90                  95

Ala Arg Asp Gln Phe Leu Ala Phe Asp Ile Trp Gly Gln Gly Thr Thr
            100                 105                 110

Val Ile Val Ser Ser
        115


<210>  368
<211>  321
<212>  DNA
<213>  Artificial sequence

<220>
<223>  Synthetic antibody

<400>  368
gacatcgtga tgacccagtc tccatcctcc ctgtctgcat ctgtgggaga cagactcacc        60

atcacttgcc gggcaagtca gagcattagc acttatttaa attggtatca acaaaaacca       120

gggaaagccc ctaagctcct gatctatggt gcatccagtt tgcaaagtgg ggtcccatca       180

cggttcagtg gcagtggatc tgggacagat ttcactctca ccatcagcag tctgcaacct       240

gaagattttg caacttactt ctgtcagcag agttacggtc tccccctcac tttcggcgga        300

gggaccaaag tggatatcaa a                                                 321


<210>  369
<211>  330
<212>  DNA
<213>  Artificial sequence

<220>
<223>  Synthetic antibody

<400>  369
cagcctgtgc tgactcagcc tgcctccgtg tctgggtctc ctggacagtc gatcaccatc        60

tcctgcactg gaaccagcag tgacgttggt ggttataact atgtctcctg gtaccaacaa       120

cacccaggca aagcccccaa actcatgatt tatgatgtca gtaatcggcc ctcaggggtt       180

tctaatcgct tctctggctc caagtctggc aacacggcct ccctgaccat ctctgggctc       240

caggctgagg acgaggctga ttattactgc agctcatata caagcagcac tttttgggtg       300

ttcggcggag ggaccaaggt caccgtccta                                        330


<210>  370
<211>  321
<212>  DNA
<213>  Artificial sequence

<220>
<223>  Synthetic antibody

<400>  370
gacatcgtga tgacccagtc tccatcctcc ctgtctgcat ctgtaggaga cagagtcacc        60

atcacttgcc gggcgagtca gggcattagc aattatttag cctggtatca gcagaaacca       120

gggaaagttc ctaagctcct gatctatgct gcatccactt tgcaatcagg ggtcccatct       180

cggttcagtg gcagtggatc tgggacagat ttcactctca ccatcagcag cctgcagcct       240

gaagatgttg caacttatta ctgtcaaaag tataacagtg cccctctcac tttcggcgga       300

gggaccaagg tggagatcaa a                                              321

<210>  371
<211>  333
<212>  DNA
<213>  Artificial sequence

<220>
<223>  Synthetic antibody

<400>  371
cagtctgccc tgaatcagcc tgcctccgtg tctgggtctc ctggacagtc gatcaccatc      60

tcctgcactg gaaccagcag tgacgttggt ggttataact atgtctcctg gtaccaacag     120

cacccaggca aagcccccaa actcatgatt tatgaggtca gtaatcggcc ctcaggggtt     180

tctgatcgct tctctggctc caagtctggc aacacggcct ccctgaccat ctctgggctc     240

caggctgagg acgaggctga ttattactgc agctcatata caagcagcag ctcccatgtg     300

gtattcggcg gagggaccaa ggtcaccgtc cta                                  333

<210>  372
<211>  309
<212>  DNA
<213>  Artificial sequence

<220>
<223>  Synthetic antibody

<400>  372
gccatccagt tgacccagtc tccatcctcc ctgtctgcct ctgtaggaga cagaatcacc      60

atcacttgcc gggcaagtca gagcattagc aaatatttaa attggtatca acaaaagcca     120

ggagaagccc ctaaactcct gatctatgct gcatccagtt tgcagagtgg ggtcccatcc     180

aggttcagtg gcagtggatc tgggacagat ttcactctca ccatcagcag tctgcaacct     240

gaagattttg caacttatta ttgtcaagag aaggacaatt ttggccaggg gaccaagctg     300

gagatcaaa                                                             309

<210>  373
<211>  321
<212>  DNA
<213>  Artificial sequence

<220>
<223>  Synthetic antibody

<400>  373
aacatccaga tgacccagtc tccatcctcc ctgtctgcat ctgtaggaga cagagtcacc      60

atcacttgcc gggcaagtca gagcattagc agctatttaa attggtatca gcagaaacca     120

gggaaagccc ctaagctcct gatctatgct gcatccagtt tgcaaagtgg ggtcccatca     180

aggttcagtg gcagtggatc tgggacagat ttcactctca ccatcagcag tctgcaacct     240

gaagattttg caacttacta ctgtcaacag agttacagta cccaagggac gttcggccaa     300

gggaccaaag tggatatcaa a                                               321

<210>  374
<211>  318
<212>  DNA
<213>  Artificial sequence

<220>
<223>  Synthetic antibody

<400>  374
aacatccaga tgacccagtc tccttccacc ctgtctgcat ctgtaggaga cagagtcacc      60

atcacttgcc gggccagtca gagtattagt agctggttgg cctggtatca gcagaaacca     120

gggaaagccc ctaagctcct gatctataag gcatctagtt tagaaagtgg ggtcccatca     180

aggttcagcg gcagtggatc tgggacagaa ttcactctca ccatcagcag cctgcagcct     240

gatgattttg caacttatta ctgccaacag tataatagtt attacacttt tggccagggg     300

accaagctgg agatcaaa                                                   318


<210>  375
<211>  330
<212>  DNA
<213>  Artificial sequence

<220>
<223>  Synthetic antibody

<400>  375
cagcctgtgc tgactcagcc accctcagcg tctgggaccc ccgggcagag ggtcaccatc      60

tcttgttctg gaagcagctc caccatcgga actaatcctg taaactggta ccggcacctc     120

ccaggaacgg cccccaaact cctcatctat agtaataatc agcggccctc aggggtccct     180

gaccgagtct ctggctccaa gtccggcacc tcagcctccc tggccatcag tgggctccag     240

tctgaggatg aggctgatta ttactgtgca acattggatg acagcctgaa tggttatgtc     300

ttcggaactg ggacccagct gaccgtccta                                      330


<210>  376
<211>  330
<212>  DNA
<213>  Artificial sequence

<220>
<223>  Synthetic antibody

<400>  376
cagcctgtgc tgactcagcc accctcagcg tctgggaccc ccgggcagag ggtcaccatc      60

tcttgttctg gaagcacctc caacatcaga ggtaatactg taaactggta ccaacaactc     120

ccaggaacgg cccccaaact cctcatctat aataataatc agcggccctc aggggtccct     180

gaccgattct ctggctccaa gtctggcacc tcagcctccc tggccatcag tgggctccag     240

tctgaggatg aggctattta ttactgtgga gcatgggatg acagcctgaa tggtcctgtc     300

ttcggaactg ggaccaaggt caccgtccta                                      330


<210>  377

<211>  321
<212>  DNA
<213>  Artificial sequence

<220>
<223>  Synthetic antibody

<400>  377
gacatccaga tgacccagtc tccatcctcc ctgtctgcat ctgtaggaga cagagtcacc      60

atcacttgcc gggcaagtca gagcattagc agctatttaa attggtatca gcagaaacca     120

gggaaagccc ctaagctcct gatctatgct gcatccagtt tgcaaagtgg ggtcccatca     180

aggttcagtg gcagtggatc tgggacagat ttcactctca ccatcagcag tctgcaacct     240

gaagattttg caacttacta ctgtcaacag agttacagta cccacatcac cttcggccaa     300

gggacacgac tggacattaa a                                               321


<210>  378
<211>  321
<212>  DNA
<213>  Artificial sequence

<220>
<223>  Synthetic antibody

<400>  378
aacatccaga tgacccagtc tccatcttcc gtgtctgcat ctgtaggaga cagagtcacc      60

atcacttgtc gggcgagtca gggtattagc agctggttag cctggtatca gcagaaacca     120

gggaaagccc ctaagctcct gatctatgct gcatccagtt tgcaaagtgg ggtcccatca     180

aggttcagcg gcagtggatc tgggacagat ttcactctca ccatcagcag cctgcagcct     240

gaagattttg caacttacta ttgtcaacag gctaacagtt tcccgctcac tttcggcgga     300

gggaccaagg tggatatcaa a                                               321


<210>  379
<211>  318
<212>  DNA
<213>  Artificial sequence

<220>
<223>  Synthetic antibody

<400>  379
aacatccaga tgacccagtc tccatcttcc gtgtctgcat ctgtaggaga cagagtcacc      60

atcacttgtc gggcgagtca gggtattagc agctggttag cctggtatca gcagaaacca     120

gggaaagccc ctaaactcct gatctatgca tccagtttgc aaagtggggt cccatcaagg     180

ttcagcggca gtggatctgg gacagatttc actctcacca tcagcagcct gcagcctgaa     240

gattttgcaa cttactattg tcaacaggct aacagtttcc ctccgacgtt cggccaaggg     300

accaaggtgg agatcaaa                                                   318


<210>  380
<211>  321
<212>  DNA

<213>  Artificial sequence

<220>
<223>  Synthetic antibody

<400>  380
aacatccaga tgacccagtc tccttccacc ctgtctgcat ctgtaggaga cagagtcacc      60

atctcttgcc gggccagtca gagtattggt acgtggttgg cctggtatca gcagaaacca     120

gggaaagccc ctcaactcct gatctataag gcgtctagtc tacatagtgg ggtcccatca     180

aggttcagcg gcagtggatc tgggacagaa ttcactctca ccatcagcag cctgcagcct     240

gatgattttg caacctatta ttgccaacag tataatcttt attctcggac gttcggccaa     300

gggaccaagg tggaaatcaa a                                               321


<210>  381
<211>  333
<212>  DNA
<213>  Artificial sequence

<220>
<223>  Synthetic antibody

<400>  381
gatattgtga tgacccagtc tccactctcc ctggccgtca cccctggaga gccggcctcc      60

atctcttgca cgtctagtca gagcctcctg caacataacg gatacaacta tttggattgg     120

tacctgcaga agccagggca gtctccacag ctcctgatct ttttgggctc tactcgggcc      180

tccggggtcc ctgacaggtt cagtgccagt ggatcaggca cggattttac actgaagatc     240

agcagagtgg aggctgagga tgttggagta tattattgca tgcaagctct acatagtccg     300

acgttcggcc aagggaccaa ggtggagatc aaa                                  333


<210>  382
<211>  318
<212>  DNA
<213>  Artificial sequence

<220>
<223>  Synthetic antibody

<400>  382
gacatcgtga tgacccagtc tccatcctcc ctgtctgcat ctgtaggaga cagagtcacc      60

atcacttgcc gggcaagtca gagcattagc aactatttaa attggtatca acagaaacca     120

gggacagccc ctaagctcct gatctatgct gcatccagtt tgcagagtgg ggtcccatca     180

aggttcagtg gcagtgggtc tgggacagat ttcactctca ccatcagcag tctgcaacct     240

gaagattttg ctacttacta ctgtcaacag agttacagta cccccacctt cggccaaggg     300

acacgactgg agattaaa                                                   318


<210>  383
<211>  330
<212>  DNA
<213>  Artificial sequence

<220>
<223>  Synthetic antibody

<400>  383
cagtctgtgt tgacgcagcc gccctcagtg tctgcggacc cagggcagaa ggtcaccatc      60

tcctgctctg gaagcagctc caacattggg aagaattatg tatcgtggta tcagcagctc     120

ccaggaacag cccccaaact cctcatttat gacgataata ggcgactctc agggactcct     180

gaccgattct ccggctccaa gtctggcacg tcagccaccc tgggcatcac cggactccag     240

actggggacg aggccgatta ttactgcgga gcttgggata gcagcctgag tgcttatgtc     300

ttcggaactg ggaccacggt gaccgtcctg                                      330


<210>  384
<211>  336
<212>  DNA
<213>  Artificial sequence

<220>
<223>  Synthetic antibody

<400>  384
cagtctgtgc tgactcagcc tccctccgcg tccgggtctc ctggacagtc agtcaccatc      60

tcctgcactg gaattagcag tgacattggt ggttataagt ctgtctcctg gtaccaacag     120

cacccaggca aagcccccaa actcatgatt tatgaggtca gtaagcggcc ctcaggggtc     180

cctgatcgct tctctggctc caagtctggc aacacggcct ccctgaccgt ctctggcctc     240

caggctgaag atgaggctga ttattactgc agctcatatg caggcagcaa caatataaat     300

tatgtcttcg gaactgggac ccagctgacc gtccta                               336


<210>  385
<211>  318
<212>  DNA
<213>  Artificial sequence

<220>
<223>  Synthetic antibody

<400>  385
gacatccagt tgacccagtc tccatcctcc ctgtctgcat ctgtaggaga cagagtcacc      60

atcacttgcc gggcaagtca gagcattagc agctatttaa attggtatca gcagaaacca     120

gggaaagccc ctaagctcct gatctatgct gcatccagtt tgcaaagtgg ggtcccatca     180

aggttcagtg gcagtggatc tgggacagat ttcactctca ccatcagcag tctgcaacct     240

gaagattttg caacttacta ctgtcaacag agttacagta cccccacttt cggcggaggg     300

accaagctgg agatcaaa                                                   318


<210>  386
<211>  330
<212>  DNA
<213>  Artificial sequence

<220>
<223>  Synthetic antibody

<400>  386
cagtctgtgt tgacgcagcc gccctcagtg tctgcggacc cagggcagaa ggtcaccatc      60

tcctgctctg gaagcagctc caacattggg aagaattatg tatcgtggta tcagcagctc     120

ccaggaacag cccccaaact cctcatttat gacgataata ggcgactctc agggactcct     180

gaccgattct ccggctccaa gtctggcacg tcagccaccc tgggcatcac cggactccag     240

actggggacg aggccgatta ttactgcgga gcttgggata gcagcctgag tgcttatgtc     300

ttcggaactg ggaccaaggt caccgtccta                                      330


<210>  387
<211>  318
<212>  DNA
<213>  Artificial sequence

<220>
<223>  Synthetic antibody

<400>  387
aacatccaga tgacccagtc tccatcctcc ctgtctgcat ctgtaggaga cagagtcacc      60

attacttgcc gggcaagtca gagcattagc agctctttaa attggtatgc gcagaaacca     120

gggaaagccc ctaagctcct gatctatgct gcatccactt tgcaaagtgg ggtcccatca     180

aggttcagtg gcagtggatc tgggacagat ttcagtctca gcatcagcag tctgcaacct     240

gaagattttg caacttatta ctgtcagcag agtttcagta gccccacctt cggccaaggg     300

acacgactgg acattaaa                                                   318


<210>  388
<211>  321
<212>  DNA
<213>  Artificial sequence

<220>
<223>  Synthetic antibody

<400>  388
gacatccagt tgacccagtc tccatcctcc ctgtctgcat ctgtaggaga cagagtcacc      60

atcacttgcc gggcaagtca gagcattagc agctatttaa attggtatca gcagaaacca     120

gggaaagccc ctaagctcct gatctatgct gcatccagtt tgcaaagtgg ggtcccatca     180

aggttcagtg gcagtggatc tgggacagat ttcactctca ccatcagcag tctgcaacct     240

gaagattttg caacttacta ctgtcaacag agttacagta ccccgctcac tttcggcgga     300

gggaccaagg tggagatcaa a                                               321


<210>  389
<211>  315
<212>  DNA
<213>  Artificial sequence

<220>
<223>  Synthetic antibody

<400>  389

gacatcgtga tgacccagtc tccatcctcc ctgtcggcat ctgtgggaga cagagtcacc 60

atcacttgcc gggcaagtca gagcgtcagc aacaatttaa attggtatca gcagaaaacg 120

ggaaaagccc ctaagctcct gatctctgcc gcatccagtt tgcaaagtgg ggtcccatta 180

aggttcagtg gcagtgggtc tgggacagac ttcactctca ccatcagcag tctgcaacct 240

gacgattatg caacttacta ctgtcaacag agttacagta tctcgttcgg ccaggggact 300

acagtggata tcaaa 315

<210> 390
<211> 318
<212> DNA
<213> Artificial sequence

<220>
<223> Synthetic antibody

<400> 390
gccatccgga tgacccagtc tccttccacc ctgtctgctt ctgtaggaga cacagtcacc 60

atcacttgcc gggccagtga gagttttagt acctggttgg cctggtatca gcagaaacca 120

gggaaagccc ctaaactcct gatctatagg gcgtctactt tacaaagtgg ggtcccgtca 180

agattcagcg gccataaatc tgggacagag ttcactctca ccatcagcag cctgcagcct 240

gatgattttg cgacttatta ttgtcaacaa tataatggtt accctacttt cggcggaggg 300

accaaggtgg aaatcaaa 318

<210> 391
<211> 324
<212> DNA
<213> Artificial sequence

<220>
<223> Synthetic antibody

<400> 391
aacatccaga tgacccagtc tccatcctcc ctgtctgcat ctgtaggaga cagagtcacc 60

atcacttgcc gggcaagtca gggcattaga aatgatttag gctggtatca gcagaaacca 120

gggacagccc ctaagcgcct aatctatgct gcatccaatt tgcaaagtgg ggtcccatca 180

aggttcagcg gcagtggatc tgggacagaa ttcactctca caatcagcag cctgcagcct 240

gaagattttg cgacttatta ctgtctacag cataatagtt accctcccga cgtttcggcc 300

agggggacca aagtggatat caaa 324

<210> 392
<211> 321
<212> DNA
<213> Artificial sequence

<220>
<223> Synthetic antibody

<400> 392
gccatccaga tgacccagtc tccatcctcc ctgtctgcat ctgtaggaga cagagtcacc 60

atcacttgcc gggcaagtca gagcattagc agctatttaa attggtatca gcagaaacca 120

gggaaagccc ctaagctcct gatctatgct gcatccagtt tgcaaagtgg ggtcccatca 180

aggttcagtg gcagtggatc tgggacagat ttcactctca ccatcagcag tctgcaacct 240

gaagattttg caacttacta ctgtcaacag agttacagta ccccgctcac tttcggcgga 300

gggaccaagc tggagatcaa a 321

<210> 393
<211> 321
<212> DNA
<213> Artificial sequence

<220>
<223> Synthetic antibody

<400> 393
gacgtccaga tgacccagtc tccatcttcc gtgtctgcat ctgtaggaga cagagtcacc 60

atcacttgtc gggcgagtca gggtattagc agatggttag cctggtatca gcagaaacca 120

gggaaagccc ctaaactcct gatctacgaa gtatccagtt tgcaaagtgg ggtcccatca 180

aggttcagcg gcagtggatc tggggctgat ttcactctca ccatcagtag cctgcagcct 240

gaagattatg gaacttacta ttgtcaacag gctaacagtt tcccgatcac cttcggccaa 300

gggacacgac tggagattaa a 321

<210> 394
<211> 336
<212> DNA
<213> Artificial sequence

<220>
<223> Synthetic antibody

<400> 394
cagtctgccc tgactcagcc tgcctccgtg tctgggtctc ctggacagtc gatcaccatc 60

tcctgcactg gaaccagcag tgacgttggt ggttataact atgtctcctg gtaccaacag 120

cacccaggta aagcccccaa actcatgatt tatgaggtca gtaatcggcc ctcaggggtt 180

tctaatcgct tctctggctc caagtctggc aacacggcct ccctgaccat ctctgggctc 240

caggctgagg acgaggctga ttattactgc agctcatata caagcagcag ccctccctta 300

atctctatcg gaactgggac caaggtcacg gtccta 336

<210> 395
<211> 107
<212> PRT
<213> Artificial sequence

<220>
<223> Synthetic antibody

<400> 395

Asp Ile Val Met Thr Gln Ser Pro Ser Ser Leu Ser Ala Ser Val Gly
1               5                   10                  15

Asp Arg Leu Thr Ile Thr Cys Arg Ala Ser Gln Ser Ile Ser Thr Tyr
            20                  25                  30

Leu Asn Trp Tyr Gln Gln Lys Pro Gly Lys Ala Pro Lys Leu Leu Ile
        35                  40                  45

Tyr Gly Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Gly Leu Pro Leu
                85                  90                  95

Thr Phe Gly Gly Gly Thr Lys Val Asp Ile Lys
            100                 105

<210>  396
<211>  110
<212>  PRT
<213>  Artificial sequence

<220>
<223>  Synthetic antibody

<400>  396

Gln Pro Val Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Thr Phe Trp Val Phe Gly Gly Gly Thr Lys Val Thr Val Leu
            100                 105                 110

<210>  397
<211>  107
<212>  PRT
<213>  Artificial sequence

<220>
<223>  Synthetic antibody

<400>  397

Asp Ile Val Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Asn Tyr
            20                  25                  30

Leu Ala Trp Tyr Gln Gln Lys Pro Gly Lys Val Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Lys Tyr Asn Ser Ala Pro Leu
                85                  90                  95

Thr Phe Gly Gly Gly Thr Lys Val Glu Ile Lys
            100                 105


<210>  398
<211>  111
<212>  PRT
<213>  Artificial sequence

<220>
<223>  Synthetic antibody

<400>  398

Gln Ser Ala Leu Asn Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Glu Val Ser Asn Arg Pro Ser Gly Val Ser Asp Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Ser His Val Val Phe Gly Gly Gly Thr Lys Val Thr Val Leu

            100                 105                 110

<210> 399
<211> 103
<212> PRT
<213> Artificial sequence

<220>
<223> Synthetic antibody

<400> 399

Ala Ile Gln Leu Thr Gln Ser Pro Ser Ser Leu Ser Ala Ser Val Gly
1               5                   10                  15

Asp Arg Ile Thr Ile Thr Cys Arg Ala Ser Gln Ser Ile Ser Lys Tyr
            20                  25                  30

Leu Asn Trp Tyr Gln Gln Lys Pro Gly Glu Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Glu Lys Asp Asn Phe Gly Gln
                85                  90                  95

Gly Thr Lys Leu Glu Ile Lys
            100

<210> 400
<211> 107
<212> PRT
<213> Artificial sequence

<220>
<223> Synthetic antibody

<400> 400

Asn Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro

65                  70                  75                  80

Glu Asp Phe Ala Thr Tyr Tyr Cys Gln Gln Ser Tyr Ser Thr Gln Gly
                85                  90                  95

Thr Phe Gly Gln Gly Thr Lys Val Asp Ile Lys
            100                 105

<210>  401
<211>  106
<212>  PRT
<213>  Artificial sequence

<220>
<223>  Synthetic antibody

<400>  401

Asn Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ser Tyr Tyr Thr
                85                  90                  95

Phe Gly Gln Gly Thr Lys Leu Glu Ile Lys
            100                 105

<210>  402
<211>  110
<212>  PRT
<213>  Artificial sequence

<220>
<223>  Synthetic antibody

<400>  402

Gln Pro Val Leu Thr Gln Pro Pro Ser Ala Ser Gly Thr Pro Gly Gln
1               5                   10                  15

Arg Val Thr Ile Ser Cys Ser Gly Ser Ser Ser Thr Ile Gly Thr Asn
            20                  25                  30

Pro Val Asn Trp Tyr Arg His Leu Pro Gly Thr Ala Pro Lys Leu Leu

35                  40                  45

Ile Tyr Ser Asn Asn Gln Arg Pro Ser Gly Val Pro Asp Arg Val Ser
    50                  55                  60

Gly Ser Lys Ser Gly Thr Ser Ala Ser Leu Ala Ile Ser Gly Leu Gln
65                  70                  75                  80

Ser Glu Asp Glu Ala Asp Tyr Tyr Cys Ala Thr Leu Asp Asp Ser Leu
                85                  90                  95

Asn Gly Tyr Val Phe Gly Thr Gly Thr Gln Leu Thr Val Leu
            100                 105                 110

<210>  403
<211>  110
<212>  PRT
<213>  Artificial sequence

<220>
<223>  Synthetic antibody

<400>  403

Gln Pro Val Leu Thr Gln Pro Pro Ser Ala Ser Gly Thr Pro Gly Gln
1               5                   10                  15

Arg Val Thr Ile Ser Cys Ser Gly Ser Thr Ser Asn Ile Arg Gly Asn
            20                  25                  30

Thr Val Asn Trp Tyr Gln Gln Leu Pro Gly Thr Ala Pro Lys Leu Leu
        35                  40                  45

Ile Tyr Asn Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Gln
65                  70                  75                  80

Ser Glu Asp Glu Ala Ile Tyr Tyr Cys Gly Ala Trp Asp Asp Ser Leu
                85                  90                  95

Asn Gly Pro Val Phe Gly Thr Gly Thr Lys Val Thr Val Leu
            100                 105                 110

<210>  404
<211>  107
<212>  PRT
<213>  Artificial sequence

<220>
<223>  Synthetic antibody

<400>  404

Asp Ile Gln Met Thr Gln Ser Pro Ser Ser Leu Ser Ala Ser Val Gly

1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr His Ile
                85                  90                  95

Thr Phe Gly Gln Gly Thr Arg Leu Asp Ile Lys
            100                 105

<210>  405
<211>  107
<212>  PRT
<213>  Artificial sequence

<220>
<223>  Synthetic antibody

<400>  405

Asn Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ala Asn Ser Phe Pro Leu
                85                  90                  95

Thr Phe Gly Gly Gly Thr Lys Val Asp Ile Lys
            100                 105

<210>  406
<211>  106
<212>  PRT

<213>  Artificial sequence

<220>
<223>  Synthetic antibody

<400>  406

Asn Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Gly Lys Ala Pro Lys Leu Leu Ile
        35                  40                  45

Tyr Ala Ser Ser Leu Gln Ser Gly Val Pro Ser Arg Phe Ser Gly Ser
    50                  55                  60

Gly Ser Gly Thr Asp Phe Thr Leu Thr Ile Ser Ser Leu Gln Pro Glu
65                  70                  75                  80

Asp Phe Ala Thr Tyr Tyr Cys Gln Gln Ala Asn Ser Phe Pro Pro Thr
                85                  90                  95

Phe Gly Gln Gly Thr Lys Val Glu Ile Lys
            100                 105


<210>  407
<211>  107
<212>  PRT
<213>  Artificial sequence

<220>
<223>  Synthetic antibody

<400>  407

Asn Ile Gln Met Thr Gln Ser Pro Ser Thr Leu Ser Ala Ser Val Gly
1               5                   10                  15

Asp Arg Val Thr Ile Ser Cys Arg Ala Ser Gln Ser Ile Gly Thr Trp
            20                  25                  30

Leu Ala Trp Tyr Gln Gln Lys Pro Gly Lys Ala Pro Gln Leu Leu Ile
        35                  40                  45

Tyr Lys Ala Ser Ser Leu His Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Leu Tyr Ser Arg
                85                  90                  95

Thr Phe Gly Gln Gly Thr Lys Val Glu Ile Lys
            100                 105

<210>  408
<211>  111
<212>  PRT
<213>  Artificial sequence

<220>
<223>  Synthetic antibody

<400>  408

Asp Ile Val Met Thr Gln Ser Pro Leu Ser Leu Ala Val Thr Pro Gly
1               5                   10                  15

Glu Pro Ala Ser Ile Ser Cys Thr Ser Ser Gln Ser Leu Leu Gln His
            20                  25                  30

Asn Gly Tyr Asn Tyr Leu Asp Trp Tyr Leu Gln Lys Pro Gly Gln Ser
        35                  40                  45

Pro Gln Leu Leu Ile Phe Leu Gly Ser Thr Arg Ala Ser Gly Val Pro
    50                  55                  60

Asp Arg Phe Ser Ala Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ala
                85                  90                  95

Leu His Ser Pro Thr Phe Gly Gln Gly Thr Lys Val Glu Ile Lys
            100                 105                 110

<210>  409
<211>  106
<212>  PRT
<213>  Artificial sequence

<220>
<223>  Synthetic antibody

<400>  409

Asp Ile Val Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Asn Tyr
            20                  25                  30

Leu Asn Trp Tyr Gln Gln Lys Pro Gly Thr Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Thr
                85                  90                  95

Phe Gly Gln Gly Thr Arg Leu Glu Ile Lys
            100                 105

<210>  410
<211>  110
<212>  PRT
<213>  Artificial sequence

<220>
<223>  Synthetic antibody

<400>  410

Gln Ser Val Leu Thr Gln Pro Pro Ser Val Ser Ala Asp Pro Gly Gln
1               5                   10                  15

Lys Val Thr Ile Ser Cys Ser Gly Ser Ser Ser Asn Ile Gly Lys Asn
            20                  25                  30

Tyr Val Ser Trp Tyr Gln Gln Leu Pro Gly Thr Ala Pro Lys Leu Leu
        35                  40                  45

Ile Tyr Asp Asp Asn Arg Arg Leu Ser Gly Thr Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Tyr Tyr Cys Gly Ala Trp Asp Ser Ser Leu
                85                  90                  95

Ser Ala Tyr Val Phe Gly Thr Gly Thr Thr Val Thr Val Leu
            100                 105                 110

<210>  411
<211>  112
<212>  PRT
<213>  Artificial sequence

<220>
<223>  Synthetic antibody

<400>  411

Gln Ser Val Leu Thr Gln Pro Pro Ser Ala Ser Gly Ser Pro Gly Gln
1               5                   10                  15

Ser Val Thr Ile Ser Cys Thr Gly Ile Ser Ser Asp Ile Gly Gly Tyr
            20                  25                  30

Lys Ser Val Ser Trp Tyr Gln Gln His Pro Gly Lys Ala Pro Lys Leu
35                  40                  45

Met Ile Tyr Glu Val Ser Lys Arg Pro Ser Gly Val Pro Asp Arg Phe
    50                  55                  60

Ser Gly Ser Lys Ser Gly Asn Thr Ala Ser Leu Thr Val Ser Gly Leu
65                  70                  75                  80

Gln Ala Glu Asp Glu Ala Asp Tyr Tyr Cys Ser Ser Tyr Ala Gly Ser
                85                  90                  95

Asn Asn Ile Asn Tyr Val Phe Gly Thr Gly Thr Gln Leu Thr Val Leu
            100                 105                 110

<210>  412
<211>  106
<212>  PRT
<213>  Artificial sequence

<220>
<223>  Synthetic antibody

<400>  412

Asp Ile Gln Leu Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Thr
                85                  90                  95

Phe Gly Gly Gly Thr Lys Leu Glu Ile Lys
            100                 105

<210>  413
<211>  110
<212>  PRT
<213>  Artificial sequence

<220>
<223>  Synthetic antibody

<400>  413

Gln Ser Val Leu Thr Gln Pro Pro Ser Val Ser Ala Asp Pro Gly Gln
1               5                   10                  15

Lys Val Thr Ile Ser Cys Ser Gly Ser Ser Ser Asn Ile Gly Lys Asn
            20                  25                  30

Tyr Val Ser Trp Tyr Gln Gln Leu Pro Gly Thr Ala Pro Lys Leu Leu
        35                  40                  45

Ile Tyr Asp Asp Asn Arg Arg Leu Ser Gly Thr Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Tyr Tyr Cys Gly Ala Trp Asp Ser Ser Leu
                85                  90                  95

Ser Ala Tyr Val Phe Gly Thr Gly Thr Lys Val Thr Val Leu
            100                 105                 110

<210>  414
<211>  106
<212>  PRT
<213>  Artificial sequence

<220>
<223>  Synthetic antibody

<400>  414

Asn Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Ser
            20                  25                  30

Leu Asn Trp Tyr Ala Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Ser Leu Ser Ile Ser Ser Leu Gln Pro
65                  70                  75                  80

Glu Asp Phe Ala Thr Tyr Tyr Cys Gln Gln Ser Phe Ser Ser Pro Thr
                85                  90                  95

Phe Gly Gln Gly Thr Arg Leu Asp Ile Lys
            100                 105

<210>  415

<211>  107
<212>  PRT
<213>  Artificial sequence

<220>
<223>  Synthetic antibody

<400>  415

Asp Ile Gln Leu Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Leu
                85                  90                  95

Thr Phe Gly Gly Gly Thr Lys Val Glu Ile Lys
            100                 105


<210>  416
<211>  105
<212>  PRT
<213>  Artificial sequence

<220>
<223>  Synthetic antibody

<400>  416

Asp Ile Val Met Thr Gln Ser Pro Ser Ser Leu Ser Ala Ser Val Gly
1               5                   10                  15

Asp Arg Val Thr Ile Thr Cys Arg Ala Ser Gln Ser Val Ser Asn Asn
            20                  25                  30

Leu Asn Trp Tyr Gln Gln Lys Thr Gly Lys Ala Pro Lys Leu Leu Ile
        35                  40                  45

Ser Ala Ala Ser Ser Leu Gln Ser Gly Val Pro Leu Arg Phe Ser Gly
    50                  55                  60

Ser Gly Ser Gly Thr Asp Phe Thr Leu Thr Ile Ser Ser Leu Gln Pro
65                  70                  75                  80

Asp Asp Tyr Ala Thr Tyr Tyr Cys Gln Gln Ser Tyr Ser Ile Ser Phe

85                  90                  95

Gly Gln Gly Thr Thr Val Asp Ile Lys
            100                 105

<210>  417
<211>  106
<212>  PRT
<213>  Artificial sequence

<220>
<223>  Synthetic antibody

<400>  417

Ala Ile Arg Met Thr Gln Ser Pro Ser Thr Leu Ser Ala Ser Val Gly
1               5                   10                  15

Asp Thr Val Thr Ile Thr Cys Arg Ala Ser Glu Ser Phe Ser Thr Trp
            20                  25                  30

Leu Ala Trp Tyr Gln Gln Lys Pro Gly Lys Ala Pro Lys Leu Leu Ile
        35                  40                  45

Tyr Arg Ala Ser Thr Leu Gln Ser Gly Val Pro Ser Arg Phe Ser Gly
    50                  55                  60

His Lys Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Gly Tyr Pro Thr
                85                  90                  95

Phe Gly Gly Gly Thr Lys Val Glu Ile Lys
            100                 105

<210>  418
<211>  108
<212>  PRT
<213>  Artificial sequence

<220>
<223>  Synthetic antibody

<400>  418

Asn Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Arg Asn Asp
            20                  25                  30

Leu Gly Trp Tyr Gln Gln Lys Pro Gly Thr Ala Pro Lys Arg Leu Ile
        35                  40                  45

Tyr Ala Ala Ser Asn Leu Gln Ser Gly Val Pro Ser Arg Phe Ser Gly

50                  55                  60

Ser Gly Ser Gly Thr Glu Phe Thr Leu Thr Ile Ser Ser Leu Gln Pro
65                  70                  75                  80

Glu Asp Phe Ala Thr Tyr Tyr Cys Leu Gln His Asn Ser Tyr Pro Pro
                85                  90                  95

Asp Val Ser Ala Arg Gly Thr Lys Val Asp Ile Lys
            100                 105

<210>  419
<211>  107
<212>  PRT
<213>  Artificial sequence

<220>
<223>  Synthetic antibody

<400>  419

Ala Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Leu
                85                  90                  95

Thr Phe Gly Gly Gly Thr Lys Leu Glu Ile Lys
            100                 105

<210>  420
<211>  107
<212>  PRT
<213>  Artificial sequence

<220>
<223>  Synthetic antibody

<400>  420

Asp Val Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Arg Trp

20                  25                  30

Leu Ala Trp Tyr Gln Gln Lys Pro Gly Lys Ala Pro Lys Leu Leu Ile
        35                  40                  45

Tyr Glu Val Ser Ser Leu Gln Ser Gly Val Pro Ser Arg Phe Ser Gly
    50                  55                  60

Ser Gly Ser Gly Ala Asp Phe Thr Leu Thr Ile Ser Ser Leu Gln Pro
65                  70                  75                  80

Glu Asp Tyr Gly Thr Tyr Tyr Cys Gln Gln Ala Asn Ser Phe Pro Ile
                85                  90                  95

Thr Phe Gly Gln Gly Thr Arg Leu Glu Ile Lys
            100                 105

<210>  421
<211>  112
<212>  PRT
<213>  Artificial sequence

<220>
<223>  Synthetic antibody

<400>  421

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Glu Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Pro Pro Leu Ile Ser Ile Gly Thr Gly Thr Lys Val Thr Val Leu
            100                 105                 110

<210>  422
<211>  21
<212>  DNA
<213>  Artificial sequence

<220>
<223>  Synthetic primer

<400>  422
gaagaaactc aactggtgtc g                                                21


<210>  423
<211>  20
<212>  DNA
<213>  Artificial sequence

<220>
<223>  Synthetic primer

<400>  423
ccaagtctgt gtcctgaacg                                                  20


<210>  424
<211>  21
<212>  DNA
<213>  Artificial sequence

<220>
<223>  Synthetic primer

<400>  424
gaaggctacc aactacaatg g                                                21


<210>  425
<211>  20
<212>  DNA
<213>  Artificial sequence

<220>
<223>  Synthetic primer

<400>  425
ttcaacagga gtgacaccag                                                  20


<210>  426
<211>  19
<212>  DNA
<213>  Artificial sequence

<220>
<223>  Synthetic primer

<400>  426
gaatcaccag cagcaagtg                                                   19


<210>  427
<211>  18
<212>  DNA
<213>  Artificial sequence

<220>
<223>  Synthetic primer

<400>  427
cttcggagtt tgggtttg                                                    18


<210>  428
<211>  20
<212>  DNA

<213>  Artificial sequence

<220>
<223>  Synthetic primer

<400>  428
ttgtgcgtct cctcagtaaa                                                   20


<210>  429
<211>  19
<212>  DNA
<213>  Artificial sequence

<220>
<223>  Synthetic primer

<400>  429
caagtgaaac ctccaaccc                                                    19


<210>  430
<211>  20
<212>  DNA
<213>  Artificial sequence

<220>
<223>  Synthetic primer

<400>  430
cattgaggag gattgccaaa                                                   20


<210>  431
<211>  19
<212>  DNA
<213>  Artificial sequence

<220>
<223>  Synthetic primer

<400>  431
acaaactgga tgtcgctgg                                                    19


<210>  432
<211>  17
<212>  DNA
<213>  Artificial sequence

<220>
<223>  Synthetic primer

<400>  432
acgctcttct gcctgct                                                      17


<210>  433
<211>  19
<212>  DNA
<213>  Artificial sequence

<220>
<223>  Synthetic primer

<400>  433
gcttgagggt ttgctacaa                                                    19

<210>  434
<211>  21
<212>  DNA
<213>  Artificial sequence

<220>
<223>  Synthetic primer

<400>  434
tggcttatta cagtggcaat g                                                 21


<210>  435
<211>  18
<212>  DNA
<213>  Artificial sequence

<220>
<223>  Synthetic primer

<400>  435
tggtggtcgg agattcgt                                                     18


<210>  436
<211>  18
<212>  DNA
<213>  Artificial sequence

<220>
<223>  Synthetic primer

<400>  436
ctttctgctg acatcgcc                                                     18


<210>  437
<211>  20
<212>  DNA
<213>  Artificial sequence

<220>
<223>  Synthetic primer

<400>  437
gtctgccgta ggttgttgta                                                   20


<210>  438
<211>  18
<212>  DNA
<213>  Artificial sequence

<220>
<223>  Synthetic primer

<400>  438
acgcagacat cgtcatcc                                                     18


<210>  439
<211>  19
<212>  DNA
<213>  Artificial sequence

<220>
<223>  Synthetic primer

<400>  439
caaaccgagt tggaaccac                                                    19


<210>  440
<211>  21
<212>  DNA
<213>  Artificial sequence

<220>
<223>  Synthetic primer

<400>  440
catacttacc cacttcaagg g                                                 21


<210>  441
<211>  22
<212>  DNA
<213>  Artificial sequence

<220>
<223>  Synthetic primer

<400>  441
ttgtagccat agtcagcatt gt                                                22


<210>  442
<211>  17
<212>  DNA
<213>  Artificial sequence

<220>
<223>  Synthetic primer

<400>  442
gatggggtct tcatctg                                                      17


<210>  443
<211>  17
<212>  DNA
<213>  Artificial sequence

<220>
<223>  Synthetic primer

<400>  443
cgtaggtgga tgcctcc                                                      17


<210>  444
<211>  21
<212>  PRT
<213>  Artificial sequence

<220>
<223>  Synthetic peptide

<400>  444

Arg Arg Ser Ser Pro Ala Ala Asp Val Gln Glu Glu Asn Leu Tyr Ala
1               5                   10                  15


Ala Val Lys Asp Thr

            20

<210>  445
<211>  21
<212>  PRT
<213>  Artificial sequence

<220>
<223>  Synthetic peptide

<400>  445

Trp Arg Ser Ser Pro Ala Ala Asp Ala Gln Glu Glu Asn Leu Tyr Ala
1               5                   10                  15

Ala Val Lys His Thr
            20

<210>  446
<211>  21
<212>  PRT
<213>  Artificial sequence

<220>
<223>  Synthetic peptide

<400>  446

Trp Arg Ser Ser Pro Ala Ala Asp Ala Gln Glu Glu Asn Leu Tyr Ala
1               5                   10                  15

Ala Val Lys Asp Thr
            20

<210>  447
<211>  21
<212>  PRT
<213>  Artificial sequence

<220>
<223>  Synthetic peptide

<400>  447

Arg Arg Ser Ser Pro Ala Ala Asp Val Gln Gly Glu Asn Phe Cys Ala
1               5                   10                  15

Ala Val Lys Asn Thr
            20

<210>  448
<211>  21
<212>  PRT
<213>  Artificial sequence

<220>
<223>  Synthetic peptide

<400>  448

Lys Arg Ala Ser Pro Val Ala Asp Ile Gln Glu Glu Ile Leu Asn Ala

1                   5                        10                       15

Ala Val Lys Asp Thr
                20

The present application provides the subject matter as defined by the following numbered paragraphs:
1. A method of identifying a modulator of LELRB3 activation comprising:
   (a) contacting a reporter cell with galectin-4 and a candidate substance, wherein said reporter cell expresses a chimeric receptor having an external domain of LILRB3; and
   (b) detecting a level of receptor activation in the reporter cell, wherein a change in the level of receptor activation as compared to a reference level indicates that the candidate substance is a modulator of LILRB3 activation.
2. The method of paragraph 1, wherein the cell is a mouse T-cell hybridoma cell.
3. The method of paragraphs 1-2, wherein the receptor comprises an intracellular domain of paired immunoglobulin-like receptor β (PILRβ).
4. The method of paragraphs 1-3, wherein the receptor is expressed in the cell through a viral expression vector.
5. The method of paragraph 4, wherein the viral expression vector is a retroviral expression vector.
6. The method of paragraphs 1-5, wherein the reporter cell expresses a reporter gene that encodes a detectable label and is operably linked to a promoter regulated by activation of the receptor.
7. The method of paragraph 6, wherein the promoter is a nuclear factor of activated T cells (NFAT) promoter.
8. The method of paragraph 6, wherein the promoter is an inducible promoter, a tissue specific promoter or a constitutive promoter.
9. The method of paragraph 6, wherein the detectable label is a colorometric label, fluorescent label, bioluminescent label, or chemiluminescent label.
10. The method of paragraph 6, wherein the detectable label is GFP, YFP, RFP, or D-luciferin.
11. The method of paragraph 6, wherein detecting step comprises flow cytometry analysis or quantification of luminescence.
12. The method of paragraphs 1-11, wherein the candidate substance is an antibody.
13. The method of paragraph 12, wherein the antibody is a monoclonal antibody, a chimeric antibody, a CDR-grafted antibody, a humanized antibody, a Fab, a Fab', a F(ab')2, a Fv, or a scFv.
14. The method of paragraph 12, wherein the antibody is a monoclonal antibody.
15. The method of paragraphs 1-14, wherein the reference level is obtained in the reporter cell when it is contacted with galectin-4.
16. The method of paragraphs 1-15, wherein an increase in the level of receptor activation as compared to the reference level indicates that the modulator is an agonist.
17. The method of paragraphs 1-15, wherein a decrease in the level of receptor activation as compared to the reference level indicates that the modulator is an antagonist.
18. The method of paragraphs 1-17, wherein the candidate substance is linked to a substrate.
19. The method of paragraphs 1-18, wherein the candidate substance is linked to a cell expressing FcR.
20. A composition comprising:
   a candidate LILRB3 modulator;
   galectin-4; and
   a reporter cell that expresses a chimeric receptor having an extracellular domain of LILRB3, wherein the reporter cell has a phenotype indicating receptor activation.
21. An isolated monoclonal antibody or an antigen-binding fragment thereof comprising a heavy chain (HC) variable region (VH) and a light chain (LC) variable region (VL) comprising clone-paired CDR sequences as set forth in Tables 3 and 4; and variants thereof wherein one or more of the HC-CDRs and/or LC-CDRs has one, two, or three amino acid substitutions, additions, deletions, or combinations thereof.
22. The isolated monoclonal antibody or an antigen binding fragment thereof of paragraph 21, wherein the isolated monoclonal antibody is a murine, a rodent, a rabbit, a chimeric, humanized, or human antibody.
23. The isolated monoclonal antibody or an antigen-binding fragment thereof of paragraph 21, wherein the antigen-binding fragment is a recombinant ScFv (single chain fragment variable) antibody, Fab fragment, F(ab')2 fragment, or Fv fragment.
24. The isolated monoclonal antibody or an antigen binding fragment thereof of paragraph 21, wherein the isolated monoclonal antibody is a human antibody.
25. The isolated monoclonal antibody or an antigen-binding fragment thereof of paragraph 21, wherein the VH and VL chains have amino acid sequences at least 90% or 95% identical to clone-paired sequences of Appendices II and IV, respectively.
26. The isolated monoclonal antibody or an antigen-binding fragment thereof of paragraph 21, wherein the VH and VL chains are encoded by nucleic acid sequences at least 80% or 90% identical to clone-paired sequences of Appendices I and III, respectively.
27. The isolated monoclonal antibody or an antigen-binding fragment thereof of paragraph 21, wherein the VH and VL chains have amino acid sequences identical to clone-paired sequences of Appendices II and IV, respectively.
28. The isolated monoclonal antibody or an antigen binding fragment thereof of paragraph 21 wherein the VH and VL chains are encoded by nucleic acid sequences identical to clone-paired sequences of Appendices I and III**,** respectively.
29. The isolated monoclonal antibody or an antigen binding fragment thereof of paragraphs 21-28, wherein the isolated monoclonal antibody is a humanized antibody.
30. The isolated monoclonal antibody or an antigen binding fragment thereof of paragraph 21-28, wherein the antibody is a chimeric antibody.
31. The isolated monoclonal antibody or an antigen binding fragment thereof of paragraph 21-30, which induces the activation of LILRB3.
32. The isolated monoclonal antibody or an antigen binding fragment thereof of paragraph 21-30, which suppresses the activation of LILRB3.
33. An isolated monoclonal antibody or an antigen binding fragment thereof, which competes for the same epitope with the isolated monoclonal antibody or an antigen-binding fragment thereof according to any of paragraphs 21-32.
34. A pharmaceutical composition comprising the isolated monoclonal antibody or an antigen-binding fragment thereof according to any one of paragraphs 21-33, and a pharmaceutically acceptable carrier.
35. An isolated nucleic acid that encodes the isolated monoclonal antibody according to any one of paragraphs 21-33.
36. A vector comprising the isolated nucleic acid of paragraph 35.
37. A host cell comprising the vector of paragraph 36.
38. The host cell of paragraph 37, wherein the host cell is a mammalian cell.
39. The host cell of paragraph 37, wherein the host cell is a CHO cell.
40. A hybridoma or engineered cell encoding and/or producing the isolated monoclonal antibody according to any one of paragraphs 21-33.
41. A process of producing an antibody, comprising culturing the host cell of paragraph 37 under conditions suitable for expressing the antibody, and recovering the antibody.
42. A chimeric antigen receptor (CAR) protein comprising an antigen-binding fragment according to any one of paragraph 21-33.
43. An isolated nucleic acid that encodes a CAR protein of paragraph 42.
44. A vector comprising the isolated nucleic acid of paragraph 43.
45. An engineered cell comprising the isolated nucleic acid of paragraph 43.
46. The engineered cell of paragraph 45, wherein the cell is a T cell, NK cell, or macrophage.
47. A method of treating or ameliorating the effect of a cancer in a subject, the method comprising administering to the subject a therapeutically effective amount of the antibody or an antigen-binding fragment thereof according to any one of paragraphs 21-33 or the engineered cell of paragraphs 45 or 46.
48. The method of paragraph 47, wherein the method reduces or eradicates the tumor burden in the subject.
49. The method of paragraph 47, wherein the method reduces the number of tumor cells.
50. The method of paragraph 47, wherein the method reduces tumor size.
51. The method of paragraph 47, wherein the method reduces or prevents tumor metastasis.
52. The method of paragraph 47, wherein the method eradicates the tumor in the subject.
53. The method of paragraph 47, wherein the cancer is a solid cancer.
54. The method of paragraph 53, wherein the solid cancer is selected from the group consisting of adrenal cancer, bile duct carcinoma, bone cancer, brain cancer, breast cancer, cervical cancer, choriocarcinoma, colon cancer, colorectal cancer, esophageal cancer, eye cancer, gastric cancer, glioblastoma, head and neck cancer, kidney cancer, liver cancer, lung cancer, mesothelioma, melanoma, merkel cell cancer, nasopharyngeal carcinoma, neuroblastoma, oral cancer, ovarian cancer, pancreatic cancer, penile cancer, pinealoma, prostate cancer, renal cell cancer, retinoblastoma, sarcoma, skin cancer, testicular cancer, thymic carcinoma, thyroid cancer, uterine cancer, and vaginal cancer.
55. The method of paragraph 47, wherein the monocytes, macrophages, dendritic cells, neutrophils and other myeloid cells, myeloid-derived suppressor cells, tumor-associated macrophages, and other immunosuppressive myeloid cells are targeted.
56. The method of paragraph 47, wherein the cancer is a hematologic malignancy.
57. The method of paragraph 56, wherein the hematologic malignancy is selected from the group consisting of acute lymphocytic leukemia (ALL), acute myeloid leukemia (AML), B-cell leukemia, chronic lymphoblastic leukemia (CLL), blastic plasmacytoid dendritic cell neoplasm (BPDCN), chronic myelomonocytic leukemia (CMML), chronic myelocytic leukemia (CML), pre-B acute lymphocytic leukemia (Pre-B ALL), diffuse large B-cell lymphoma (DLBCL), extranodal NK/T-cell lymphoma, hairy cell leukemia, heavy chain disease, HHV8-associated primary effusion lymphoma, plasmablastic lymphoma, primary CNS lymphoma, primary mediastinal large B-cell lymphoma, T-cell/histiocyte-rich B-cell lymphoma, Hodgkin's lymphoma, non-Hodgkin's lymphoma, Waldenstrom's macroglobulinemia, multiple myeloma (MM), myelodysplastic syndromes (MDS), myeloproliferative neoplasms, and polycythemia vera.
58. The method of paragraph 47, wherein the antibody or an antigen-binding fragment thereof is administered intravenously, intra-arterially, intra-tumorally, or subcutaneously.
59. The method of paragraph 47, further comprising administering to the subject one or more drugs selected from the group consisting of a topoisomerase inhibitor, an anthracycline topoisomerase inhibitor, an anthracycline, a daunorubicin, a nucleoside metabolic inhibitor, a cytarabine, a hypomethylating agent, a low dose cytarabine (LDAC), a combination of daunorubicin and cytarabine, a daunorubicin and cytarabine liposome for injection, Vyxeos^{®}, an azacytidine, Vidaza^{®}, a decitabine, an all-trans-retinoic acid (ATRA), an arsenic, an arsenic trioxide, a histamine dihydrochloride, Ceplene^{®}, an interleukin-2, an aldesleukin, Proleukin^{®}, a gemtuzumab ozogamicin, Mylotarg^{®}, an FLT-3 inhibitor, a midostaurin, Rydapt^{®}, a clofarabine, a farnesyl transferase inhibitor, a decitabine, an IDH1 inhibitor, an ivosidenib, Tibsovo^{®}, an IDH2 inhibitor, an enasidenib, Idhifa^{®}, a smoothened (SMO) inhibitor, a glasdegib, an arginase inhibitor, an IDO inhibitor, an epacadostat, a BCL-2 inhibitor, a venetoclax, Venclexta^{®}, a platinum complex derivative, oxaliplatin, a kinase inhibitor, a tyrosine kinase inhibitor, a PI3 kinase inhibitor, a BTK inhibitor, an ibrutinib, IMBRUVICA^{®}, an acalabrutinib, CALQUENCE^{®}, a zanubrutinib, a PD-1 antibody, a PD-L1 antibody, a CTLA-4 antibody, a LAG3 antibody, an ICOS antibody, a TIGIT antibody, a TIM3 antibody, a CD40 antibody, a 4-1BB antibody, a CD47 antibody, a SIRP1α antibody or fusions protein, a CD70 antibody, and CLL1 antibody, a CD123 antibody, an antagonist of E-selectin, an antibody binding to a tumor antigen, an antibody binding to a T-cell surface marker, an antibody binding to a myeloid cell or NK cell surface marker, an alkylating agent, a nitrosourea agent, an antimetabolite, an antitumor antibiotic, an alkaloid derived from a plant, a hormone therapy medicine, a hormone antagonist, an aromatase inhibitor, and a P-glycoprotein inhibitor.
60. The method according to any of paragraphs 47-59, wherein said isolated monoclonal antibody or an antigen binding fragment thereof further comprises an antitumor drug linked thereto.
61. The method of paragraph 60, wherein said antitumor drug is linked to said antibody through a photolabile linker.
62. The method of paragraph 60, wherein said antitumor drug is linked to said antibody through an enzymatically cleaved linker.
63. The method of paragraph 60, wherein said antitumor drug is a toxin, a radioisotope, a cytokine, or an enzyme.
64. A method of detecting a cancer cell or cancer stem cell in a sample or subject comprising:
   (a) contacting a subject or a sample from the subject with the antibody or an antigen-binding fragment thereof according to any one of claims 21-33; and
   (b) detecting binding of said antibody to a cancer cell or cancer stem cell in said subject or sample.
65. The method of paragraph 64, wherein the sample is a body fluid or biopsy.
66. The method of paragraph 64, wherein the sample is blood, bone marrow, sputum, tears, saliva, mucous, serum, urine or feces.
67. The method of paragraph 64, wherein detection comprises immunohistochemistry, flow cytometry, an immunoassay (including ELISA, RIA etc.) or Western blot.
68. The method of paragraph 64, further comprising performing steps (a) and (b) a second time and determining a change in detection levels as compared to the first time.
69. The method of paragraph 64, wherein said isolated monoclonal antibody or an antigen binding fragment thereof further comprises a label.
70. The method of paragraph 69, wherein said label is a peptide tag, an enzyme, a magnetic particle, a chromophore, a fluorescent molecule, a chemo-luminescent molecule, or a dye.
71. The method according to any of paragraphs 47-70, wherein said isolated monoclonal antibody or an antigen binding fragment thereof is conjugated to a liposome or nanoparticle.
72. A method of treating or ameliorating the effect of an autoimmune disease in a subject, the method comprising administering to the subject a therapeutically effective amount of the antibody or an antigen-binding fragment thereof according to any one of paragraphs 21-33 or the engineered cell of paragraphs 45 or 46.
73. The method of paragraph 72, wherein the monocytes, macrophages, dendritic cells, and neutrophils and other myeloid cells are targeted.
74. The method of paragraph 72, wherein the antibody or an antigen-binding fragment thereof is administered intravenously, intra-arterially, intra-tumorally, or subcutaneously.
75. The method of paragraph 72, further comprising administering to the subject one or more drugs selected from the group consisting of a steroid or an NSAID.
76. The method of paragraph 72, wherein the autoimmune disease is Guillain-Barre syndrome, Chronic inflammatory demyelinating polyneuropathy, ankylosing spondylitis, psoriatic arthritis, enteropathic arthritis, reactive arthritis, undifferentiated spondyloarthropathy, juvenile spondyloarthropathy, Behcet's disease, enthesitis, ulcerative colitis, Crohn's disease, irritable bowel syndrome, inflammatory bowel disease, fibromyalgia, chronic fatigue syndrome, pain conditions associated with systemic inflammatory disease, systemic lupus erythematosus, Sjogren's syndrome, rheumatoid arthritis, juvenile rheumatoid arthritis, juvenile onset diabetes mellitus (also known as Type I diabetes mellitus), Wegener's granulomatosis, polymyositis, dermatomyositis, inclusion body myositis, multiple endocrine failure, Schmidt's syndrome, autoimmune uveitis, Addison's disease, Grave's Disease, Hashimoto's thyroiditis, autoimmune thyroid disease, pernicious anemia, gastric atrophy, chronic hepatitis, lupoid hepatitis, atherosclerosis, multiple sclerosis, amyotrophic lateral sclerosis, hypoparathyroidism, Dressler's syndrome, myasthenia gravis, Eaton-Lambert syndrome, autoimmune thrombocytopenia, idiopathic thrombocytopenic purpura, hemolytic anemia, pemphigus vulgaris, pemphigus, dermatitis herpetiformis, alopecia, scleroderma, progressive systemic sclerosis, CREST syndrome (calcinosis, Raynaud's phenomenon, esophageal dysmotility, sclerodactyly, and telangtasia), adult onset diabetes mellitus (also known as Type II diabetes mellitus), mixed connective tissue disease, polyarteritis nodosa, systemic necrotizing vasculitis, glomerulonephritis, atopic dermatitis, atopic rhinitis, Goodpasture's syndrome, Chagas' disease, sarcoidosis, rheumatic fever, asthma, anti-phospholipidsyndrome, erythema multiforme, Cushing's syndrome, autoimmune chronic active hepatitis, allergic disease, allergic encephalomyelitis, transfusion reaction, leprosy, malaria, leshmaniasis, trypanosomiasis, Takayasu's arteritis, polymyalgia rheumatica, temporal arteritis, shistosomiasis, giant cell arteritis, eczema, lymphomatoid granulomatosis, Kawasaki's disease, endophthalmitis, psoriasis, erythroblastosis fetalis, eosinophilic faciitis, Shulman's syndrome, Felty's syndrome, Fuch's cyclitis, IgA nephropathy, Henoch-Schonlein purpura, graft versus host disease, transplantation rejection, tularemia, periodic fever syndromes, pyogenic arthritis, Familial Mediterranean Fever, TNF-receptor associated periodic syndrome (TRAPS), Muckle-Wells syndrome, or hyper-IgD syndrome.
77. A non-human cell comprising a coding region for human LILRB3, such as a mouse cell.
78. The cell of paragraph 77, wherein said LILBR3 coding region is under the control of a promoter, such as a heterologous promoter, including an inducible promoter or a constitutive promoter.
79. The cell of paragraphs 77-78, wherein expression of LILRB3 negatively regulated by transcription or translation inhibitor element, such as a stop codon flanked by Lox sites.
80. A transgenic non-human animal, such as a transgenic mouse, comprising the cell of paragraphs 77-79.

All of the methods disclosed and claimed herein can be made and executed without undue experimentation in light of the present disclosure. While the compositions and methods of this disclosure have been described in terms of preferred embodiments, it will be apparent to those of skill in the art that variations may be applied to the methods and in the steps or in the sequence of steps of the method described herein without departing from the concept, spirit and scope of the disclosure. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the disclosure as defined by the appended claims.

### REFERENCES

The following references, to the extent that they provide exemplary procedural or other details supplementary to those set forth herein, are specifically incorporated herein by reference.
Atwal, J.K., Pinkston-Gosse, J., Syken, J., Stawicki, S., Wu, Y., Shatz, C., and Tessier-Lavigne, M. (2008). PirB is a functional receptor for myelin inhibitors of axonal regeneration. Science 322, 967-970.
Barkal, A.A., Weiskopf, K., Kao, K.S., Gordon, S.R., Rosental, B., Yiu, Y.Y., George, B.M., Markovic, M., Ring, N.G., Tsai, J.M., et al. (2018). Engagement of MHC class I by the inhibitory receptor LILRB1 suppresses macrophages and is a target of cancer immunotherapy. Nat Immunol 19, 76-84.
Barrow, A.D., and Trowsdale, J. (2008). The extended human leukocyte receptor complex: diverse ways of modulating immune responses. Immunological reviews 224, 98-123.
Borghi, A., Verstrepen, L., and Beyaert, R. (2016). TRAF2 multitasking in TNF receptor-induced signaling to NF-κB, MAP kinases and cell death. Biochemical pharmacology 116, 1-10.
Cannons, J.L., Bertram, E.M., and Watts, T.H. (2002). Cutting edge: profound defect in T cell responses in TNF receptor-associated factor 2 dominant negative mice. The Journal of Immunology 169, 2828-2831.
Carosella, E.D., Rouas-Freiss, N., Roux, D.T., Moreau, P., and LeMaoult, J. (2015). HLA-G: An Immune Checkpoint Molecule. Adv Immunol 127, 33-144.
Chang, D.W., Xing, Z., Pan, Y., Algeciras-Schimnich, A., Barnhart, B.C., Yaish-Ohad, S., Peter, M.E., and Yang, X. (2002). c-FLIPL is a dual function regulator for caspase-8 activation and CD95-mediated apoptosis. The EMBO journal 21, 3704-3714.
Chen, H.M., van der Touw, W., Wang, Y.S., Kang, K., Mai, S., Zhang, J., Alsina-Beauchamp, D., Duty, J.A., Mungamuri, S.K., Zhang, B., et al. (2018). Blocking immunoinhibitory receptor LILRB2 reprograms tumor-associated myeloid cells and promotes antitumor immunity. J Clin Invest 128, 5647-5662.
Click, Z.R., Seddon, A.N., Bae, Y.R., Fisher, J.D., and Ogunniyi, A. (2018). New Food and Drug Administration-approved and emerging novel treatment options for acute myeloid leukemia. Pharmacotherapy 38, 1143-1154.
Coxon, C.H., Geer, M.J., and Senis, Y.A. (2017). ITIM receptors: more than just inhibitors of platelet activation. Blood 129, 3407-3418.
Curran, E.K., Godfrey, J., and Kline, J. (2017). Mechanisms of immune tolerance in leukemia and lymphoma. Trends in immunology 38, 513-525.
Daeron, M., Jaeger, S., Du Pasquier, L., and Vivier, E. (2008). Immunoreceptor tyrosine-based inhibition motifs: a quest in the past and future. Immunol Rev 224, 11-43.
Deng, M., Gui, X., Kim, J., Xie, L., Chen, W., Li, Z., He, L., Chen, Y., Chen, H., Luo, W., et al. (2018). LILRB4 signalling in leukaemia cells mediates T cell suppression and tumour infiltration. Nature 562, 605.
Deng, M., Lu, Z., Zheng, J., Wan, X., Chen, X., Hirayasu, K., Sun, H., Lam, Y., Chen, L., Wang, Q., et al. (2014). A motif in LILRB2 critical for Angptl2 binding and activation. Blood 124, 924-935.
Golks, A., Brenner, D., Krammer, P.H., and Lavrik, I.N. (2006). The c-FLIP-NH2 terminus (p22-FLIP) induces NF-κB activation. Journal of Experimental Medicine 203, 1295-1305.
Gui, X., Deng, M., Song, H., Chen, Y., Xie, J., Li, Z., He, L., Huang, F., Xu, Y., Anami, Y., et al. (2019). Disrupting LILRB4/APOE interaction by an efficacious humanized antibody reverses T-cell suppression and blocks AML development. Cancer immunology research 7, 1244-1257.
Hasegawa, K., Tanaka, S., Fujiki, F., Morimoto, S., Nakajima, H., Tatsumi, N., Nakata, J., Takashima, S., Nishida, S., Tsuboi, A., et al. (2015). An immunocompetent mouse model for MLL/AF9 leukemia reveals the potential of spontaneous cytotoxic T-cell response to an antigen expressed in leukemia cells. PloS one 10, e0144594.
Hirayasu, K., and Arase, H. (2015). Functional and genetic diversity of leukocyte immunoglobulin-like receptor and implication for disease associations. Journal of human genetics 60, 703-708.
Hristodorov, D., Fischer, R., and Linden, L. (2013). With or without sugar?(A) glycosylation of therapeutic antibodies. Molecular biotechnology 54, 1056-1068.
Hughes, M.A., Powley, I.R., Jukes-Jones, R., Horn, S., Feoktistova, M., Fairall, L., Schwabe, J.W., Leverkus, M., Cain, K., and MacFarlane, M. (2016). Co-operative and hierarchical binding of c-FLIP and caspase-8: a unified model defines how c-FLIP isoforms differentially control cell fate. Molecular cell 61, 834-849.
John, S., Chen, H., Deng, M., Gui, X., Wu, G., Chen, W., Li, Z., Zhang, N., An, Z., and Zhang, C.C. (2018). A Novel Anti-LILRB4 CAR-T Cell for the Treatment of Monocytic AML. Mol Ther 26, 2487-2495.
Kagoya, Y., Yoshimi, A., Kataoka, K., Nakagawa, M., Kumano, K., Arai, S., Kobayashi, H., Saito, T., Iwakura, Y., and Kurokawa, M. (2014). Positive feedback between NF-κB and TNF-α promotes leukemia-initiating cell capacity. J Clin Invest 124, 528-542.
Kang, X., Kim, J., Deng, M., John, S., Chen, H., Wu, G., Phan, H., and Zhang, C. (2016). Inhibitory leukocyte immunoglobulin-like receptors: immune checkpoint proteins and tumor sustaining factors. Cell cycle 15, 25-40.
Kang, X., Lu, Z., Cui, C., Deng, M., Fan, Y., Dong, B., Han, X., Xie, F., Tyner, J.W., Coligan, J.E., et al. (2015). The ITIM-containing receptor LAIR1 is essential for acute myeloid leukaemia development. Nature cell biology 17, 665-677.
Kataoka, T., and Tschopp, J. (2004). N-terminal fragment of c-FLIP (L) processed by caspase 8 specifically interacts with TRAF2 and induces activation of the NF-κB signaling pathway. Molecular cellular biology 24, 2627-2636.
Katz, H.R. (2006). Inhibition of inflammatory responses by leukocyte Ig-like receptors. Adv Immunol 91, 251-272.
Kawasaki, T., and Kawai, T. (2014). Toll-like receptor signaling pathways. Frontiers in immunology 5, 461.
Kim, T., Vidal, G.S., Djurisic, M., William, C.M., Birnbaum, M.E., Garcia, K.C., Hyman, B.T., and Shatz, C.J. (2013). Human LilrB2 is a β-amyloid receptor and its murine homolog PirB regulates synaptic plasticity in an Alzheimer's model. Science 341, 1399-1404.
Li, L., Soetandyo, N., Wang, Q., Ye, Y., and Research, B.e.B.A.-M.C. (2009). The zinc finger protein A20 targets TRAF2 to the lysosomes for degradation. 1793, 346-353.
Li, Z., Deng, M., Huang, F., Jin, C., Sun, S., Chen, H., Liu, X., He, L., Sadek, A.H., and Zhang, C.C. (2019). LILRB4 ITIMs mediate the T cell suppression and infiltration of acute myeloid leukemia cells. Cellular molecular immunology, 1-11.
Liu, X., Yu, X., Xie, J., Zhan, M., Yu, Z., Xie, L., Zeng, H., Zhang, F., Chen, G., Yi, X., et al. (2015). ANGPTL2/LILRB2 signaling promotes the propagation of lung cancer cells. Oncotarget 6, 21004.
Ma, G., Pan, P.-Y., Eisenstein, S., Divino, C.M., Lowell, C.A., Takai, T., and Chen, S.-H. (2011). Paired immunoglobin-like receptor-B regulates the suppressive function and fate of myeloid-derived suppressor cells. Immunity 34, 385-395.
MacEwan, D.J. (2002). TNF receptor subtype signalling: differences and cellular consequences. Cellular signalling 14, 477-492.
Medvedev, A.E., Vogel, S.N., and research, J.o.e. (2003). Overexpression of CD14, TLR4, and MD-2 in HEK 293T cells does not prevent induction of in vitro endotoxin tolerance. 9, 60-64.
Micheau, O., Lens, S., Gaide, O., Alevizopoulos, K., and Tschopp, J. (2001). NF-κB signals induce the expression of c-FLIP. Molecular cellular biology 21, 5299-5305.
Mori, Y., Tsuji, S., Inui, M., Sakamoto, Y., Endo, S., Ito, Y., Fujimura, S., Koga, T., Nakamura, A., and Takayanagi, H. (2008). Inhibitory immunoglobulin-like receptors LILRB and PIR-B negatively regulate osteoclast development. The Journal of Immunology 181, 4742-4751.
Nishitoh, H., Saitoh, M., Mochida, Y., Takeda, K., Nakano, H., Rothe, M., Miyazono, K., and Ichijo, H. (1998). ASK1 is essential for JNK/SAPK activation by TRAF2. Molecular cell 2, 389-395.
Pereira, S., and Lowell, C. (2003). The Lyn tyrosine kinase negatively regulates neutrophil integrin signaling. The Journal of Immunology 171, 1319-1327.
Pfistershammer, K., Lawitschka, A., Klauser, C., Leitner, J., Weigl, R., Heemskerk, M.H., Pickl, W.F., Majdic, O., Böhmig, G.A., and Fischer, G.F. (2009). Allogeneic disparities in immunoglobulin-like transcript 5 induce potent antibody responses in hematopoietic stem cell transplant recipients. Blood 114, 2323-2332.
Postow, M.A, Callahan, M.K., and Wolchok, J.D. (2015). Immune checkpoint blockade in cancer therapy. Journal of clinical oncology 33, 1974.
Reinhard, C., Shamoon, B., Shyamala, V., and Williams, L.T. (1997). Tumor necrosis factor alpha-induced activation of c-jun N-terminal kinase is mediated by TRAF2. EMBO J 16, 1080-1092.
Renauer, P., Saruhan-Direskeneli, G., Coit, P., Adler, A., Aksu, K., Keser, G., Alibaz-Oner, F., Aydin, S.Z., Kamali, S., Inanc, M., et al. (2015). Genome-wide association study identifies susceptibility loci in IL6, RPS9/LILRB3, and an intergenic locus on chromosome 21q22 in Takayasu's arteritis. Arthritis rheumatology 67, 1361.
Robert, C., Long, G.V., Brady, B., Dutriaux, C., Maio, M., Mortier, L., Hassel, J.C., Rutkowski, P., McNeil, C., and Kalinka-Warzocha, E. (2015a). Nivolumab in previously untreated melanoma without BRAF mutation. New England journal of medicine 372, 320-330.
Robert, C., Schachter, J., Long, G.V., Arance, A., Grob, J.J., Mortier, L., Daud, A., Carlino, M.S., McNeil, C., and Lotem, M. (2015b). Pembrolizumab versus ipilimumab in advanced melanoma. New England Journal of Medicine 372, 2521-2532.
Rodriguez, M., Cabal-Hierro, L., Carcedo, M.T., Iglesias, J.M., Artime, N., Darnay, B.G., and Lazo, P.S. (2011). NF-κB signal triggering and termination by tumor necrosis factor receptor 2. Journal of Biological Chemistry 286, 22814-22824.
Sanchez-Correa, B., Bergua, J.M., Campos, C., Gayoso, I., Arcos, M.J., Bañas, H., Morgado, S., Casado, J.G., Solana, R., and Tarazona, R. (2013). Cytokine profiles in acute myeloid leukemia patients at diagnosis: survival is inversely correlated with IL-6 and directly correlated with IL-10 levels. Cytokine 61, 885-891.
Sawanobori, M., Yamaguchi, S., Hasegawa, M., Inoue, M., Suzuki, K., Kamiyama, R., Hirokawa, K., and Kitagawa, M. (2003). Expression of TNF receptors and related signaling molecules in the bone marrow from patients with myelodysplastic syndromes. Leukemia research 27, 583-591.
Shaw, P.J., Lamkanfi, M., and Kanneganti, T.D. (2010). NOD-like receptor (NLR) signaling beyond the inflammasome. European journal of immunology 40, 624-627.
Shembade, N., Harhaj, E.W., and immunology, C.m. (2012). Regulation of NF-κB signaling by the A20 deubiquitinase. 9, 123-130.
Sloane, D.E., Tedla, N., Awoniyi, M., Macglashan, D.W., Jr., Borges, L., Austen, K.F., and Arm, J.P. (2004). Leukocyte immunoglobulin-like receptors: novel innate receptors for human basophil activation and inhibition. Blood 104, 2832-2839.
Song, H.Y., Rothe, M., Goeddel, D.V., and Sciences, P.o.t.N.A.o. (1996). The tumor necrosis factor-inducible zinc finger protein A20 interacts with TRAF1/TRAF2 and inhibits NF-kappaB activation. 93, 6721-6725.
Subramanian, A., Tamayo, P., Mootha, V.K., Mukherjee, S., Ebert, B.L., Gillette, M.A., Paulovich, A., Pomeroy, S.L., Golub, T.R., and Lander, E.S. (2005). Gene set enrichment analysis: a knowledge-based approach for interpreting genome-wide expression profiles. Proceedings of the National Academy of Sciences 102, 15545-15550.
Syken, J., GrandPre, T., Kanold, P.O., and Shatz, C.J. (2006). PirB restricts ocular-dominance plasticity in visual cortex. science 313, 1795-1800.
Takai, T., Nakamura, A., and Endo, S. (2011a). Role of PIR-B in autoimmune glomerulonephritis. J Biomed Biotechnol 2011, 275302.
Takai, T., Nakamura, A., and Endo, S. (2011b). Role of PIR-B in Autoimmune Glomerulonephritis. Journal of Biomedicine Biotechnology 2011*.*
Taniguchi, K., and Karin, M. (2018). NF-κB, inflammation, immunity and cancer: coming of age. Nature Reviews Immunology 18, 309-324.
Tao, M.-H., and Morrison, S.L. (1989). Studies of aglycosylated chimeric mouse-human IgG. Role of carbohydrate in the structure and effector functions mediated by the human IgG constant region. The Journal of Immunology 143, 2595-2601.
Tiscornia, G., Tergaonkar, V., Galimi, F., and Verma, I.M. (2004). CRE recombinase-inducible RNA interference mediated by lentiviral vectors. Proceedings of the National Academy of Sciences 101, 7347-7351.
Trowsdale, J., Jones, D.C., Barrow, A.D., and Traherne, J.A. (2015). Surveillance of cell and tissue perturbation by receptors in the LRC. Immunol Rev 267, 117-136.
Volk, A., Li, J., Xin, J., You, D., Zhang, J., Liu, X., Xiao, Y., Breslin, P., Li, Z., Wei, W., et al. (2014). Co-inhibition of NF-κB and JNK is synergistic in TNF-expressing human AML. J Exp Med 211, 1093-1108.
Vredevoogd, D.W., Kuilman, T., Ligtenberg, M.A., Boshuizen, J., Stecker, K.E., de Bruijn, B., Krijgsman, O., Huang, X., Kenski, J.C., and Lacroix, R. (2019). Augmenting immunotherapy impact by lowering tumor TNF cytotoxicity threshold. Cell 178, 585-599. e515.
Wu, G., and Zhang, C.C. (2020). Membrane protein CAR promotes hematopoietic regeneration upon stress. Haematologica doi: 10.3324/haematol.2019.243998. Online ahead of print*.*
Ye, H., and Wu, H. (2000). Thermodynamic characterization of the interaction between TRAF2 and tumor necrosis factor receptor peptides by isothermal titration calorimetry. Proceedings of the National Academy of Sciences 97, 8961-8966.
Zheng, J., Umikawa, M., Cui, C., Li, J., Chen, X., Zhang, C., Huynh, H., Kang, X., Silvany, R., Wan, X., et al. (2012). Inhibitory receptors bind ANGPTLs and support blood stem cells and leukaemia development. Nature 485, 656.
Cao ZQ, Guo XL. The role of galectin-4 in physiology and diseases. Protein Cell 2016; 7:314-24.
Rao US, Rao PS. Surface-bound galectin-4 regulates gene transcription and secretion of chemokines in human colorectal cancer cell lines. Tumour Biol 2017; 39:1010428317691687.
Huflejt ME, Jordan ET, Gitt MA, Barondes SH, Leffler H. Strikingly different localization of galectin-3 and galectin-4 in human colon adenocarcinoma T84 cells. Galectin-4 is localized at sites of cell adhesion. J Biol Chem 1997; 272:14294-303.
Deng M, Gui X, Kim J, Xie L, Chen W, Li Z, He L, Chen Y, Chen H, Luo W, et al. LILRB4 signalling in leukaemia cells mediates T cell suppression and tumour infiltration. Nature 2018; 562:605-9.
Gui X, Deng M, Song H, Chen Y, Xie J, Li Z, He L, Huang F, Xu Y, Anami Y, et al. Disrupting LILRB4/APOE Interaction by an Efficacious Humanized Antibody Reverses T-cell Suppression and Blocks AML Development. Cancer Immunol Res 2019; 7:1244-57.
Kang X, Lu Z, Cui C, Deng M, Fan Y, Dong B, Han X, Xie F, Tyner JW, Coligan JE, et al. The ITIM-containing receptor LAIR1 is essential for acute myeloid leukaemia development. Nat Cell Biol 2015; 17:665-77.
Chen H, Chen Y, Deng M, John S, Gui X, Kansagra A, Chen W, Kim J, Lewis C, Wu G, et al. An antagonistic anti-LILRB1 monoclonal antibody regulates anti-tumor functions of natural killer cells. Journal for ImmunoTherapy of Cancer 2020; In press.

1 Robert, C. et al. Nivolumab in previously untreated melanoma without BRAF mutation. New England journal of medicine 372, 320-330 (2015).
2 Robert, C. et al. Pembrolizumab versus ipilimumab in advanced melanoma. New England Journal of Medicine 372, 2521-2532 (2015).
3 Postow, M. A., Callahan, M. K. & Wolchok, J. D. Immune checkpoint blockade in cancer therapy. Journal of clinical oncology 33, 1974 (2015).
4 Curran, E. K., Godfrey, J. & Kline, J. Mechanisms of immune tolerance in leukemia and lymphoma. Trends in immunology 38, 513-525 (2017).
5 Click, Z. R., Seddon, A. N., Bae, Y. R., Fisher, J. D. & Ogunniyi, A. New Food and Drug Administration-approved and emerging novel treatment options for acute myeloid leukemia. Pharmacotherapy 38, 1143-1154 (2018).
6 Trowsdale, J., Jones, D. C., Barrow, A. D. & Traherne, J. A. Surveillance of cell and tissue perturbation by receptors in the LRC. Immunol Rev 267, 117-136, doi:10.1111/imr.12314 (2015).
7 Daeron, M., Jaeger, S., Du Pasquier, L. & Vivier, E. Immunoreceptor tyrosine-based inhibition motifs: a quest in the past and future. Immunol Rev 224, 11-43, doi:10.1111/j.1600-065X.2008.00666.x (2008).
8 Takai, T., Nakamura, A. & Endo, S. Role of PIR-B in autoimmune glomerulonephritis. J Biomed Biotechnol 2011, 275302, doi:10.1155/2011/275302 (2011).
9 Katz, H. R. Inhibition of inflammatory responses by leukocyte Ig-like receptors. Adv Immunol 91, 251-272, doi:10.1016/s0065-2776(06)91007-4 (2006).
10 Kang, X. et al. Inhibitory leukocyte immunoglobulin-like receptors: immune checkpoint proteins and tumor sustaining factors. Cell cycle 15, 25-40 (2016).
11 Hirayasu, K. & Arase, H. Functional and genetic diversity of leukocyte immunoglobulin-like receptor and implication for disease associations. Journal of human genetics 60, 703-708 (2015).
12 Deng, M. et al. Leukocyte immunoglobulin-like receptor subfamily B (LILRB): therapeutic targets in cancer Antibody Therapeutics 4, 16-33 (2021).
13 van der Touw, W., Chen, H. M., Pan, P. Y. & Chen, S. H. LILRB receptor-mediated regulation of myeloid cell maturation and function. Cancer Immunol Immunother 66, 1079-1087, doi:10.1007/s00262-017-2023-x (2017).
14 Carosella, E. D., Rouas-Freiss, N., Roux, D. T., Moreau, P. & LeMaoult, J. HLA-G: An Immune Checkpoint Molecule. Adv Immunol 127, 33-144, doi:10.1016/bs.ai.2015.04.001 (2015).
15 John, S. et al. A Novel Anti-LILRB4 CAR-T Cell for the Treatment of Monocytic AML. Mol Ther 26, 2487-2495, doi:10.1016/j.ymthe.2018.08.001 (2018).
16 Zheng, J. et al. Inhibitory receptors bind ANGPTLs and support blood stem cells and leukaemia development. Nature 485, 656 (2012).
17 Kang, X. et al. The ITIM-containing receptor LAIR1 is essential for acute myeloid leukaemia development. Nature cell biology 17, 665-677 (2015).
18 Deng, M. et al. LILRB4 signalling in leukaemia cells mediates T cell suppression and tumour infiltration. Nature 562, 605 (2018).
19 Gui, X. et al. Disrupting LILRB4/APOE interaction by an efficacious humanized antibody reverses T-cell suppression and blocks AML development. Cancer immunology research 7, 1244-1257 (2019).
20 Anami, Y. et al. LILRB4-Targeting Antibody-Drug Conjugates for the Treatment of Acute Myeloid Leukemia. Mol Cancer Ther, doi:10.1158/1535-7163.mct-20-0407 (2020).
21 Li, Z. et al. LILRB4 ITIMs mediate the T cell suppression and infiltration of acute myeloid leukemia cells. Cell Mol Immunol, doi:10.1038/s41423-019-0321-2 (2019).
22 Churchill, H. R. O. et al. Leukocyte immunoglobulin-like receptor B1 and B4 (LILRB1 and LILRB4): Highly sensitive and specific markers of acute myeloid leukemia with monocytic differentiation. Cytometry B Clin Cytom, doi:10.1002/cyto.b.21952 (2020).
23 Bergstrom, C. P. et al. The association of leukocyte immunoglobulin-like receptor subfamily B-4 expression in acute myeloid leukemia and central nervous system involvement. Leuk Res 100, 106480, doi:10.1016/j.leukres.2020.106480 (2021).
24 Barkal, A. A. et al. Engagement of MHC class I by the inhibitory receptor LILRB1 suppresses macrophages and is a target of cancer immunotherapy. Nat Immunol 19, 76-84, doi:10.1038/s41590-017-0004-z (2018).
25 Chen, H. M. et al. Blocking immunoinhibitory receptor LILRB2 reprograms tumor-associated myeloid cells and promotes antitumor immunity. J Clin Invest 128, 5647-5662, doi:10.1172/jci97570 (2018).
26 Tedla, N. et al. Activation of human eosinophils through leukocyte immunoglobulin-like receptor 7. Proc Natl Acad Sci U S A 100, 1174-1179, doi:10.1073/pnas.0337567100 (2003).
27 Coxon, C. H., Geer, M. J. & Senis, Y. A. ITIM receptors: more than just inhibitors of platelet activation. Blood 129, 3407-3418 (2017).
28 Sloane, D. E. et al. Leukocyte immunoglobulin-like receptors: novel innate receptors for human basophil activation and inhibition. Blood 104, 2832-2839, doi:10.1182/blood-2004-01-0268 (2004).
29 Yeboah, M. et al. LILRB3 (ILT5) is a myeloid cell checkpoint that elicits profound immunomodulation. JCI Insight 5, doi:10.1172/jci.insight.141593 (2020).
30 Renauer, P. et al. Genome-wide association study identifies susceptibility loci in IL6, RPS9/LILRB3, and an intergenic locus on chromosome 21q22 in Takayasu's arteritis. Arthritis rheumatology 67, 1361 (2015).
31 Jones, D. C. et al. Allele-specific recognition by LILRB3 and LILRA6 of a cytokeratin 8-associated ligand on necrotic glandular epithelial cells. Oncotarget 7, 15618-15631, doi:10.18632/oncotarget.6905 (2016).
32 Pfistershammer, K. et al. Allogeneic disparities in immunoglobulin-like transcript 5 induce potent antibody responses in hematopoietic stem cell transplant recipients. Blood 114, 2323-2332 (2009).
33 Tiscornia, G., Tergaonkar, V., Galimi, F. & Verma, I. M. CRE recombinase-inducible RNA interference mediated by lentiviral vectors. Proceedings of the National Academy of Sciences 101, 7347-7351 (2004).
34 Sanchez-Correa, B. et al. Cytokine profiles in acute myeloid leukemia patients at diagnosis: survival is inversely correlated with IL-6 and directly correlated with IL-10 levels. Cytokine 61, 885-891 (2013).
35 MacEwan, D. J. TNF receptor subtype signalling: differences and cellular consequences. Cellular signalling 14, 477-492 (2002).
36 Syken, J., GrandPre, T., Kanold, P. O. & Shatz, C. J. PirB restricts ocular-dominance plasticity in visual cortex. science 313, 1795-1800 (2006).
37 Hasegawa, K. et al. An immunocompetent mouse model for MLL/AF9 leukemia reveals the potential of spontaneous cytotoxic T-cell response to an antigen expressed in leukemia cells. PloS one 10, e0144594 (2015).
38 Kawasaki, T. & Kawai, T. Toll-like receptor signaling pathways. Frontiers in immunology 5, 461 (2014).
39 Shaw, P. J., Lamkanfi, M. & Kanneganti, T. D. NOD - like receptor (NLR) signaling beyond the inflammasome. European journal of immunology 40, 624-627 (2010).
40 Taniguchi, K. & Karin, M. NF-κB, inflammation, immunity and cancer: coming of age. Nature Reviews Immunology 18, 309-324 (2018).
41 Cannons, J. L., Bertram, E. M. & Watts, T. H. Cutting edge: profound defect in T cell responses in TNF receptor-associated factor 2 dominant negative mice. The Journal of Immunology 169, 2828-2831 (2002).
42 Nishitoh, H. et al. ASK1 is essential for JNK/SAPK activation by TRAF2. Molecular cell 2, 389-395 (1998).
43 Reinhard, C., Shamoon, B., Shyamala, V. & Williams, L. T. Tumor necrosis factor alpha-induced activation of c-jun N-terminal kinase is mediated by TRAF2. EMBO J 16, 1080-1092, doi:10.1093/emboj/16.5.1080 (1997).
44 Golks, A., Brenner, D., Krammer, P. H. & Lavrik, I. N. The c-FLIP-NH2 terminus (p22-FLIP) induces NF-κB activation. Journal of Experimental Medicine 203, 1295-1305 (2006).
45 Kataoka, T. & Tschopp, J. N-terminal fragment of c-FLIP (L) processed by caspase 8 specifically interacts with TRAF2 and induces activation of the NF-κB signaling pathway. Molecular cellular biology 24, 2627-2636 (2004).
46 Hughes, M. A. et al. Co-operative and hierarchical binding of c-FLIP and caspase-8: a unified model defines how c-FLIP isoforms differentially control cell fate. Molecular cell 61, 834-849 (2016).
47 Chang, D. W. et al. c - FLIPL is a dual function regulator for caspase - 8 activation and CD95 - mediated apoptosis. The EMBO journal 21, 3704-3714 (2002).
48 Fritsch, M. et al. Caspase-8 is the molecular switch for apoptosis, necroptosis and pyroptosis. Nature 575, 683-687 (2019).
49 Bertheloot, D., Latz, E. & Franklin, B. S. Necroptosis, pyroptosis and apoptosis: an intricate game of cell death. Cellular Molecular Immunology 18, 1106-1121 (2021).
50 Wu, G. & Zhang, C. C. Membrane protein CAR promotes hematopoietic regeneration upon stress. Haematologica doi: 10.3324/haematol.2019.243998. Online ahead of print. (2020).
51 Pereira, S. & Lowell, C. The Lyn tyrosine kinase negatively regulates neutrophil integrin signaling. The Journal of Immunology 171, 1319-1327 (2003).
52 Medvedev, A. E. & Vogel, S. N. Overexpression of CD14, TLR4, and MD-2 in HEK 293T cells does not prevent induction of in vitro endotoxin tolerance. Journal of endotoxin research 9, 60-64 (2003).
53 Micheau, O., Lens, S., Gaide, O., Alevizopoulos, K. & Tschopp, J. NF-κB signals induce the expression of c-FLIP. Molecular cellular biology 21, 5299-5305 (2001).
54 Shembade, N. & Harhaj, E. W. Regulation of NF-κB signaling by the A20 deubiquitinase. Cellular molecular immunology 9, 123-130 (2012).
55 Li, L., Soetandyo, N., Wang, Q. & Ye, Y. The zinc finger protein A20 targets TRAF2 to the lysosomes for degradation. Biochimica et Biophysica Acta -Molecular Cell Research 1793, 346-353 (2009).
56 Song, H. Y., Rothe, M. & Goeddel, D. V. The tumor necrosis factor-inducible zinc finger protein A20 interacts with TRAF1/TRAF2 and inhibits NF-kappaB activation. Proceedings of the National Academy of Sciences 93, 6721-6725 (1996).
57 Tao, M.-H. & Morrison, S. L. Studies of aglycosylated chimeric mouse-human IgG. Role of carbohydrate in the structure and effector functions mediated by the human IgG constant region. The Journal of Immunology 143, 2595-2601 (1989).
58 Hristodorov, D., Fischer, R. & Linden, L. With or without sugar?(A) glycosylation of therapeutic antibodies. Molecular biotechnology 54, 1056-1068 (2013).
59 Lo, M. et al. Effector-attenuating substitutions that maintain antibody stability and reduce toxicity in mice. Journal of Biological Chemistry 292, 3900-3908 (2017).
60 Dietrich, J., Cella, M. & Colonna, M. Ig-like transcript 2 (ILT2)/leukocyte Ig-like receptor 1 (LIR1) inhibits TCR signaling and actin cytoskeleton reorganization. J Immunol 166, 2514-2521 (2001).
61 Cella, M. et al. A novel inhibitory receptor (ILT3) expressed on monocytes, macrophages, and dendritic cells involved in antigen processing. J Exp Med 185, 1743-1751 (1997).
62 Sawanobori, M. et al. Expression of TNF receptors and related signaling molecules in the bone marrow from patients with myelodysplastic syndromes. Leukemia research 27, 583-591 (2003).
63 Barrow, A. D. & Trowsdale, J. The extended human leukocyte receptor complex: diverse ways of modulating immune responses. Immunological reviews 224, 98-123 (2008).
64 Li, Z. et al. LILRB4 ITIMs mediate the T cell suppression and infiltration of acute myeloid leukemia cells. Cellular molecular immunology 17, 272-282 (2019).
65 Rodriguez, M. et al. NF-κB signal triggering and termination by tumor necrosis factor receptor 2. Journal of Biological Chemistry 286, 22814-22824 (2011).
66 Ye, H. & Wu, H. Thermodynamic characterization of the interaction between TRAF2 and tumor necrosis factor receptor peptides by isothermal titration calorimetry. Proceedings of the National Academy of Sciences 97, 8961-8966 (2000).
67 Takai, T., Nakamura, A. & Endo, S. Role of PIR-B in Autoimmune Glomerulonephritis. Journal of Biomedicine Biotechnology 2011 (2011).
68 Deng, M. et al. A motif in LILRB2 critical for Angptl2 binding and activation. Blood 124, 924-935, doi:10.1182/blood-2014-01-549162 (2014).
69 Deng, M. et al. LILRB4 signalling in leukaemia cells mediates T cell suppression and tumour infiltration. Nature 562, 605-609 (2018).
70 Kang, X. et al. The ITIM-containing receptor LAIR1 is essential for acute myeloid leukaemia development. Nat Cell Biol 17, 665-677, doi:10.1038/ncb3158 (2015).
71 Subramanian, A. et al. Gene set enrichment analysis: a knowledge-based approach for interpreting genome-wide expression profiles. Proceedings of the National Academy of Sciences 102, 15545-15550 (2005).

## Claims

1. An isolated monoclonal antibody or an antigen-binding fragment thereof comprising a heavy chain (HC) variable region (VH) and a light chain (LC) variable region (VL) comprising clone-paired CDR sequences as set forth in Tables 3 and 4; and variants thereof wherein one or more of the HC-CDRs and/or LC-CDRs has one, two, or three amino acid substitutions, additions, deletions, or combinations thereof.

2. The isolated monoclonal antibody or an antigen binding fragment thereof of claim 1, wherein the isolated monoclonal antibody is a murine, a rodent, a rabbit, a chimeric, humanized, or human antibody.

3. The isolated monoclonal antibody or an antigen-binding fragment thereof of claim 1, wherein the antigen-binding fragment is a recombinant ScFv (single chain fragment variable) antibody, Fab fragment, F(ab')2 fragment, or Fv fragment.

4. The isolated monoclonal antibody or an antigen binding fragment thereof of claim 1, wherein the isolated monoclonal antibody is a human antibody.

5. The isolated monoclonal antibody or an antigen-binding fragment thereof of claim 1, wherein the VH and VL chains have amino acid sequences at least 90% or 95% identical to clone-paired sequences of Tables 6 and 8, respectively.

6. The isolated monoclonal antibody or an antigen-binding fragment thereof of claim 1, wherein the VH and VL chains have amino acid sequences identical to clone-paired sequences of Tables 6 and 8, respectively.

7. The isolated monoclonal antibody or an antigen binding fragment thereof of claim 1, wherein the isolated monoclonal antibody is a humanized antibody or a chimeric antibody.

8. A pharmaceutical composition comprising the isolated monoclonal antibody or an antigen-binding fragment thereof according to claim 1, and a pharmaceutically acceptable carrier.

9. A chimeric antigen receptor (CAR) protein comprising an antigen-binding fragment according to claim 1.

10. An isolated nucleic acid that encodes the isolated monoclonal antibody according to claim 1 or the CAR protein of claim 9.

11. A vector comprising the isolated nucleic acid of claim 10.

12. An engineered cell comprising the isolated nucleic acid of claim 10, optionally wherein the cell is a T cell, a NK cell, or a macrophage.

13. An antibody or an antigen-binding fragment thereof according to claim 1, or the engineered cell of claim 12, for use in a method of treating or ameliorating the effect of a cancer in a subject, the method comprising administering to the subject a therapeutically effective amount of the antibody or antigen-binding fragment thereof, or the engineered cell.

14. The antibody, antigen-binding fragment thereof, or engineered cell for use according to claim 13, wherein:
(a) the method reduces or eradicates the tumor burden in the subject;
(b) the method reduces the number of tumor cells;
(c) the method reduces tumor size;
(d) the method reduces or prevents tumor metastasis;
(e) the method eradicates the tumor in the subject;
(f) the cancer is a solid cancer, optionally wherein the solid cancer is selected from the group consisting of adrenal cancer, bile duct carcinoma, bone cancer, brain cancer, breast cancer, cervical cancer, choriocarcinoma, colon cancer, colorectal cancer, esophageal cancer, eye cancer, gastric cancer, glioblastoma, head and neck cancer, kidney cancer, liver cancer, lung cancer, mesothelioma, melanoma, merkel cell cancer, nasopharyngeal carcinoma, neuroblastoma, oral cancer, ovarian cancer, pancreatic cancer, penile cancer, pinealoma, prostate cancer, renal cell cancer, retinoblastoma, sarcoma, skin cancer, testicular cancer, thymic carcinoma, thyroid cancer, uterine cancer, and vaginal cancer;
(g) the monocytes, macrophages, dendritic cells, neutrophils and other myeloid cells, myeloid-derived suppressor cells, tumor-associated macrophages, and other immunosuppressive myeloid cells are targeted;
(h) the cancer is a hematologic malignancy, optionally wherein the hematologic malignancy is selected from the group consisting of acute lymphocytic leukemia (ALL), acute myeloid leukemia (AML), B-cell leukemia, chronic lymphoblastic leukemia (CLL), blastic plasmacytoid dendritic cell neoplasm (BPDCN), chronic myelomonocytic leukemia (CMML), chronic myelocytic leukemia (CML), pre-B acute lymphocytic leukemia (Pre-B ALL), diffuse large B-cell lymphoma (DLBCL), extranodal NK/T-cell lymphoma, hairy cell leukemia, heavy chain disease, HHV8-associated primary effusion lymphoma, plasmablastic lymphoma, primary CNS lymphoma, primary mediastinal large B-cell lymphoma, T-cell/histiocyte-rich B-cell lymphoma, Hodgkin's lymphoma, non-Hodgkin's lymphoma, Waldenstrom's macroglobulinemia, multiple myeloma (MM), myelodysplastic syndromes (MDS), myeloproliferative neoplasms, and polycythemia vera;
(i) the antibody or an antigen-binding fragment thereof is administered intravenously, intra-arterially, intra-tumorally, or subcutaneously; or
(j) the method further comprises administering to the subject one or more drugs selected from the group consisting of a topoisomerase inhibitor, an anthracycline topoisomerase inhibitor, an anthracycline, a daunorubicin, a nucleoside metabolic inhibitor, a cytarabine, a hypomethylating agent, a low dose cytarabine (LDAC), a combination of daunorubicin and cytarabine, a daunorubicin and cytarabine liposome for injection, Vyxeos^{®}, an azacytidine, Vidaza^{®}, a decitabine, an all-trans-retinoic acid (ATRA), an arsenic, an arsenic trioxide, a histamine dihydrochloride, Ceplene^{®}, an interleukin-2, an aldesleukin, Proleukin^{®}, a gemtuzumab ozogamicin, Mylotarg^{®}, an FLT-3 inhibitor, a midostaurin, Rydapt^{®}, a clofarabine, a farnesyl transferase inhibitor, a decitabine, an IDH1 inhibitor, an ivosidenib, Tibsovo^{®}, an IDH2 inhibitor, an enasidenib, Idhifa^{®}, a smoothened (SMO) inhibitor, a glasdegib, an arginase inhibitor, an IDO inhibitor, an epacadostat, a BCL-2 inhibitor, a venetoclax, Venclexta^{®}, a platinum complex derivative, oxaliplatin, a kinase inhibitor, a tyrosine kinase inhibitor, a PI3 kinase inhibitor, a BTK inhibitor, an ibrutinib, IMBRUVICA^{®}, an acalabrutinib, CALQUENCE^{®}, a zanubrutinib, a PD-1 antibody, a PD-L1 antibody, a CTLA-4 antibody, a LAG3 antibody, an ICOS antibody, a TIGIT antibody, a TIM3 antibody, a CD40 antibody, a 4-1BB antibody, a CD47 antibody, a SIRP1α antibody or fusions protein, a CD70 antibody, and CLL1 antibody, a CD123 antibody, an antagonist of E-selectin, an antibody binding to a tumor antigen, an antibody binding to a T-cell surface marker, an antibody binding to a myeloid cell or NK cell surface marker, an alkylating agent, a nitrosourea agent, an antimetabolite, an antitumor antibiotic, an alkaloid derived from a plant, a hormone therapy medicine, a hormone antagonist, an aromatase inhibitor, and a P-glycoprotein inhibitor.

15. The antibody, antigen-binding fragment thereof, or engineered cell for use according to claim 13 or 14, wherein said isolated monoclonal antibody or an antigen binding fragment thereof:
(a) further comprises an antitumor drug linked thereto, optionally wherein said antitumor drug is:
(i) linked to said antibody through a photolabile linker;
(ii) linked to said antibody through an enzymatically cleaved linker; or
(iii) a toxin, a radioisotope, a cytokine, or an enzyme;
and/or
(b) is conjugated to a liposome or nanoparticle.
